# EUROPÄISCHE PATENTSCHRIFT

(11) **EP 3 230 267 B1**
(45) Veröffentlichungstag und Bekanntmachung des Hinweises auf die Patenterteilung: **26.09.2018**
(21) Anmeldenummer: 15805217.5
(22) Anmeldetag: 08.12.2015
(51) Int. Cl.: C07D 233/56

(54) **FÜNFGLIEDRIGE C-N-VERKNÜPFTE ARYLSULFID- UND ARYLSULFOXID-DERIVATE ALS SCHÄDLINGSBEKÄMPFUNGSMITTEL**
FIVE-MEMBERED C-N LINKED ARYLSULFIDE AND ARYLSULFOXIDE DERIVATIVES AS PESTICIDES
DÉRIVÉS DE SULFURES D'ARYLES ET D'ARYLSULFOXYDE À CINQ ÉLÉMENTS LIÉS C-N COMME MOYENS DE LUTTE CONTRE LES PARASITES

(30) Priorität: 11.12.2014 EP 14197404
(43) Veröffentlichungstag der Anmeldung: 18.10.2017
(73) Patentinhaber: Bayer CropScience Aktiengesellschaft, 40789 Monheim am Rhein (DE)
(72) Erfinder: CEREZO-GALVEZ, Silvia, 40764 Langenfeld (DE); ALIG, Bernd, 53639 Königswinter (DE); FISCHER, Reiner, 40789 Monheim (DE); HAHN, Julia Johanna, 40589 Düsseldorf (DE); HARSCHNECK, Tobias, 40225 Düsseldorf (DE); WILCKE, David, 40219 Düsseldorf (DE); ILG, Kerstin, 50670 Köln (DE); MALSAM, Olga, 51503 Rösrath (DE); LÖSEL, Peter, 51371 Leverkusen (DE); KÖHLER, Adeline, 40764 Langenfeld (DE); PORTZ, Daniela, 52391 Vettweiß (DE); GÖRGENS, Ulrich, 40882 Ratingen (DE); EILMUS, Sascha, 42799 Leichlingen (DE)
(74) Vertreter: BIP Patents
(86) Internationale Anmeldenummer: PCT/EP2015/078934
(87) Internationale Veröffentlichungsnummer: WO 2016/091857

(56) Entgegenhaltungen:
- EP-A2- 0 364 797
- WO-A1-2013/092350
- WO-A1-2014/095979
- DE-A1- 19 536 842

## Beschreibung

Die vorliegende Anmeldung betrifft neue heterocyclische Verbindungen, Verfahren zu Ihrer Herstellung und ihre Verwendung zur Bekämpfung von tierischen Schädlingen, zu denen Arthropoden und insbesondere Insekten zählen.

Arylsulfid- und Arylsulfoxid- Derivate sowie deren insektizide und akarizide Wirkung sind beispielsweise bereits aus WO 1999/055668 A1 bekannt.

Imidazolidin-2,4-dione (auch Hydantoine genannt) und deren 2-Thio- oder 4-Thioanaloga sind als Pflanzenschutzmittel oder Pharmaka bekannt. 1-(Alkoxycarbonyl)- und 1-Carbamoyl-3-arylhydantoine und deren Analoga 2-Thiohydantoine sind beispielsweise bereits als Fungizide aus DE2144923 und FR2148868, sowie als Pflanzenwachstumsregulatoren aus DE2423273 bekannt. Von Iprodion (3-(3,5-Dichlorphenyl)-2,4-dioxo-*N*-isopropylimidazolidin-1-carboxamid) ist seine Anwendung für die Nematodenkontrolle aus WO2006/063848 bekannt. 1-Alkyl-3-arylhydantoine sind beispielsweise als Cannabinoid-Inhibitoren aus FR2845385 und Kinase-Inhibitoren aus WO2008/046216 bekannt. Herbizide Anwendungen von 1-Alkyl-3-arylhydantoine sind beispielsweise aus DE2212558 und EP300882 bekannt.

Pflanzenschutzmittel, zu denen auch Schädlingsbekämpfungsmittel gehören, müssen vielen Anforderungen genügen, beispielsweise in Bezug auf Höhe, Dauer und Breite ihrer Wirkung und möglichen Verwendung. Es spielen Fragen der Toxizität, der Kombinierbarkeit mit anderen Wirkstoffen oder Formulierhilfsmitteln eine Rolle sowie die Frage des Aufwands, der für die Synthese eines Wirkstoffs betrieben werden muss. Ferner können Resistenzen auftreten. Aus all diesen Gründen kann die Suche nach neuen Pflanzenschutzmitteln nicht als abgeschlossen betrachtet werden und es besteht ständig Bedarf an neuen Verbindungen mit gegenüber den bekannten Verbindungen zumindest in Bezug auf einzelne Aspekte verbesserten Eigenschaften.

Aufgabe der vorliegenden Erfindung war es, Verbindungen bereitzustellen, durch die das Spektrum der Schädlingsbekämpfungsmittel unter verschiedenen Aspekten verbreitert oder verbessert wird.

Gelöst wird die Aufgabe, sowie weitere nicht explizit genannte Aufgaben, die aus den hierin diskutierten Zusammenhängen ableitbar oder erschließbar sind, durch neue Verbindungen der Formel (I) 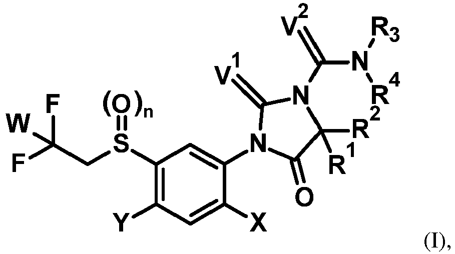 worin (Ausgestaltung 1-1)
- W: für Wasserstoff oder Halogen steht;
- n: für die Zahl 0, 1 oder 2 steht;
- Y: für Wasserstoff, Halogen, (C₁-C₆)Alkyl, Halogen(C₁-C₆)alkyl, (C₁-C₆)Alkoxy, Halogen(C₁-C₆)alkoxy oder Amino steht; oder
für NR"'R"" steht,
wobei R'" und R"" unabhängig voneinander für Wasserstoff, (C₁-C₆)Alkyl, oder Halogen(C₂-C₆)alkyl stehen;
- X: für Wasserstoff, Halogen, Cyano, (C₁-C₆)Alkyl, Halogen(C₁-C₆)alkyl oder (C₁-C₆)Alkoxy steht;
- V¹ und V²: unabhängig voneinander für Sauerstoff oder Schwefel stehen;
- R¹ und R²: unabhängig voneinander
für Wasserstoff, Halogen, Hydroxy, Cyano oder Nitro stehen; oder
für Alkyl, Alkenyl, Alkinyl, Alkoxy, Alkylsulfanyl, Alkylsulfinyl, Alkylsulfonyl, Halogenalkylcarbonyl, Alkylcarbonyl oder Alkoxycarbonyl stehen, wobei die vorgenannten Reste gegebenenfalls durch Halogen, Alkyl, Cycloalkyl, Cyano, Nitro, Alkoxy, Halogenalkyl oder Halogenalkoxy, insbesondere Fluor, Chlor, (C₁-C₃)Alkyl, (C₃-C₆)Cycloalkyl, Cyclopropyl, Cyano, (C₁-C₃)Alkoxy, (C₁-C₃)Halogenalkyl oder (C₁-C₃)Halogenalkoxy, substituiert sein können;
- oder R¹ und R²: bilden einen gegebenenfalls durch Halogen, Alkyl, Cycloalkyl, Cyano, Nitro, Alkoxy, Halogenalkyl oder Halogenalkoxy, insbesondere Fluor, Chlor, (C₁-C₃)Alkyl, (C₃-C₆)Cycloalkyl, Cyclopropyl, Cyano, (C₁-C₃)Alkoxy, (C₁-C₃)Halogenalkyl oder (C₁-C₃)Halogenalkoxy, substituierten und gegebenenfalls durch ein oder mehrere Heteroatome, welche unabhängig ausgewählt werden aus der Gruppe bestehend aus O, S und N mit der Maßgabe, dass zwei Sauerstoffatome nicht unmittelbar benachbart zueinander stehen, unterbrochenen gesättigten oder ungesättigten drei- bis sechsgliedrigen Ring;
- R³: für Alkyl, Halogenalkyl, Alkoxyalkyl, Halogenalkoxyalkyl, Alkenyl, Halogenalkenyl, Alkinyl, Halogenalkinyl, Alkyl-S(O)ₘ-alkyl, Halogenalkyl-S(O)ₘ-alkyl, *N-*Alkylaminocarbonylalkyl oder *N,N*-Dialkylaminocarbonylalkyl steht, oder
für gegebenenfalls einfach oder zweifach, gleich oder verschieden durch Halogen, Cyano, Nitro, Hydroxy, Amino, (C₁-C₄)Alkyl, Halogen(C₁-C₃)Alkyl, (C₁-C₃)Alkoxy, Halogen(C₁-C₃)Alkoxy oder Cyclopropyl substituiertes Cycloalkyl, Cycloalkylalkyl, Cycloalkenyl oder Cycloalkenylalkyl steht, oder
für gegebenenfalls einfach oder zweifach, gleich oder verschieden durch Halogen, Cyano, Nitro, Hydroxy, Amino, (C₁-C₄)Alkyl, Halogen(C₁-C₃)Alkyl, (C₁-C₃)Alkoxy, Halogen(C₁-C₃)Alkoxy oder Cyclopropyl substituiertes Heterocyclyl, Heterocyclylalkyl, Aryl, Arylalkyl, Hetaryl oder Hetarylalkyl steht;
- R⁴: für Wasserstoff, Alkyl, Halogenalkyl, Alkoxyalkyl, Halogenalkoxyalkyl, Alkenyl, Halogenalkenyl, Alkinyl, Halogenalkinyl, Alkyl-S(O)ₘ-alkyl, Halogenalkyl-S(O)ₘ-alkyl, *N*-Alkylaminocarbonylalkyl oder *N,N*-Dialkylaminocarbonylalkyl steht, oder
für gegebenenfalls einfach oder zweifach, gleich oder verschieden durch Halogen, Cyano, Nitro, Hydroxy, Amino, (C₁-C₄)Alkyl, Halogen(C₁-C₃)Alkyl, (C₁-C₃)Alkoxy, Halogen(C₁-C₃)Alkoxy oder Cyclopropyl substituiertes Cycloalkyl, Cycloalkylalkyl, Cycloalkenyl oder Cycloalkenylalkyl steht, oder
für gegebenenfalls einfach oder zweifach, gleich oder verschieden durch Halogen, Cyano, Nitro, Hydroxy, Amino, (C₁-C₄)Alkyl, Halogen(C₁-C₃)Alkyl, (C₁-C₃)Alkoxy, Halogen(C₁-C₃)Alkoxy oder Cyclopropyl substituiertes Heterocyclyl, Heterocyclylalkyl, Aryl, Arylalkyl, Hetaryl oder Hetarylalkyl steht;
- oder R³ und R⁴: bilden gemeinsam mit dem Stickstoffatom, an das sie gebunden sind, einen gegebenenfalls durch Halogen, Cyano, (C₁-C₄)Alkyl, Halogen(C₁-C₄)alkyl, (C₁-C₄)Alkoxy, Halogen(C₁-C₄)alkoxy oder (C₃-C₆)Cycloalkyl substituierten gesättigten bis dreifach ungesättigten 3- bis 6-gliedrigen Ring;
- m: für die Zahl 0, 1 oder 2 steht.

Weiterhin wurde gefunden, dass die neuen Verbindungen der Formel (I) eine gute Wirksamkeit als Schädlingsbekämpfungsmittel, beispielsweise gegen Arthropoden und insbesondere Insekten, Nematoden und Akariden, darüber hinaus in der Regel insbesondere gegenüber Kulturpflanzen sehr gut pflanzenverträglich sind und/oder über günstige toxikologische und/oder umweltrelevante Eigenschaften verfügen.

Bevorzugte Substituenten bzw. Bereiche der in den Verbindungen der Formel (I) aufgeführten Reste werden im Folgenden erläutert (Ausgestaltung 2-1):
- W: steht für Wasserstoff oder Halogen;
- n: steht für die Zahl 0 oder 1;
- Y: steht für Wasserstoff, Halogen, (C₁-C₃)Alkyl, (C₁-C₃)Halogenalkyl, (C₁-C₃)Alkoxy, (C₁-C₃)Halogenalkoxy oder Amino; oder
für NR'"R"",
wobei R'" und R"" unabhängig voneinander für Wasserstoff, (C₁-C₄)Alkyl, oder (C₂-C₄)Halogenalkyl stehen;
- X: steht für Wasserstoff, Halogen, Cyano, (C₁-C₃)Alkyl, (C₁-C₃)Halogenalkyl oder (C₁-C₃)Alkoxy;
- V¹ und V²: stehen unabhängig voneinander für Sauerstoff oder Schwefel;
- R¹ und R²: stehen unabhängig voneinander für Wasserstoff oder (C₁-C₃)Alkyl;
- oder R¹ und R²: bilden gemeinsam mit dem Kohlenstoffatom, an das sie gebunden sind, einen (C₃-C₆)Cycloalkyl-Ring;
- R³: steht für (C₁-C₆)Alkyl, Halogen(C₁-C₃)alkyl, (C₁-C₃)Alkoxy(C₁-C₃)alkyl, Halogen(C₁-C₃)alkoxy(C₁-C₃)alkyl, (C₁-C₆)Alkenyl, Halogen(C₁-C₃)alkenyl, (C₁-C₆)Alkinyl, Halogen(C₁-C₃)alkinyl, (C₁-C₃)Alkyl-S(O)ₘ-(C₁-C₃)alkyl, Halogen(C₁-C₃)Alkyl-S(O)ₘ-(C₁-C₃)alkyl, *N*-(C₁-C₃)Alkylaminocarbonyl(C₁-C₃)alkyl oder *N,N*-Di(C₁-C₃)Alkylaminocarbonyl(C₁-C₃)alkyl, oder
für gegebenenfalls einfach oder zweifach, gleich oder verschieden durch Halogen, Cyano, Nitro, Hydroxy, Amino, (C₁-C₄)Alkyl, Halogen(C₁-C₃)Alkyl, (C₁-C₃)Alkoxy, Halogen(C₁-C₃)Alkoxy oder Cyclopropyl substituiertes (C₃-C₆)Cycloalkyl, (C₃-C₆)Cycloalkyl(C₁-C₃)alkyl, (C₃-C₆)Cycloalkenyl oder (C₃-C₆)Cycloalkenyl(C₁-C₃)alkyl, oder
für gegebenenfalls einfach oder zweifach, gleich oder verschieden durch Halogen, Cyano, Nitro, Hydroxy, Amino, (C₁-C₄)Alkyl, Halogen(C₁-C₃)Alkyl, (C₁-C₃)Alkoxy, Halogen(C₁-C₃)Alkoxy oder Cyclopropyl substituiertes Heterocyclyl, Heterocyclyl(C₁-C₃)alkyl, Aryl, Aryl(C₁-C₃)alkyl, Hetaryl oder Hetaryl(C₁-C₃)alkyl;
- R⁴: steht für Wasserstoff, (C₁-C₆)Alkyl, Halogen(C₁-C₃)alkyl oder (C₁-C₃)Alkoxy(C₁-C₃)alkyl, oder
für gegebenenfalls einfach oder zweifach, gleich oder verschieden durch Halogen, Cyano, Nitro, Hydroxy, Amino, (C₁-C₄)Alkyl, Halogen(C₁-C₃)Alkyl, (C₁-C₃)Alkoxy, Halogen(C₁-C₃)Alkoxy oder Cyclopropyl substituiertes (C₃-C₆)Cycloalkyl, (C₃-C₆)Cycloalkyl(C₁-C₃)alkyl, Phenyl, Phenyl(C₁-C₃)alkyl, Pyridyl oder Pyridyl(C₁-C₃)alkyl;
- oder R³ und R⁴: bilden gemeinsam mit dem Stickstoffatom, an das sie gebunden sind, einen gegebenenfalls durch Halogen, Cyano, (C₁-C₄)Alkyl, Halogen(C₁-C₄)alkyl, (C₁-C₄)Alkoxy, Halogen(C₁-C₄)alkoxy, (C₃-C₆)Cycloalkyl substituierten gesättigten bis dreifach ungesättigten 3- bis 6-gliedrigen Ring, der ausgewählt ist aus Aziridinyl, Azirenyl, Diaziridinyl, Diazirenyl, Azetidinyl, Dihydroazetyl, Diazetidinyl, Dihydrodiazetyl, Oxazetidinyl, Oxazetyl, Thiazetidinyl, Thiazetyl, Pyrrolidinyl, Dihydropyrrolyl, Pyrazolidinyl, Dihydropyrazolyl, Imidazolidinyl, Dihydroimidazolyl, Oxazolidinyl, Dihydrooxazolyl, Thiazolidinyl, Dihydrothyazolyl, Piperidinyl, Piperazinyl, Hexahydropyridazinyl, Hexahydropyrimidinyl, Morpholin, Dioxazinanyl, Thiomorpholin, Dithiazinan, Pyrrolyl, Pyrazolyl, Imidazolyl, Triazolyl und Tetrazolyl;
- m: steht für die Zahl 0, 1 oder 2.

In einer weiteren Ausgestaltung sind die bevorzugten Substituenten bzw. Bereiche der in den Verbindungen der Formel (I) aufgeführten Reste wie folgt (Ausgestaltung 2-2):
- W: steht für Wasserstoff oder Halogen;
- n: steht für die Zahl 0 oder 1;
- Y: steht für Wasserstoff, Halogen, (C₁-C₃)Alkyl, (C₁-C₃)Halogenalkyl, (C₁-C₃)Alkoxy, (C₁-C₃)Halogenalkoxy oder Amino; oder
für NR'"R"",
wobei R'" und R"" unabhängig voneinander für Wasserstoff, (C₁-C₄)Alkyl, oder (C₂-C₄)Halogenalkyl stehen;
- X: steht für Wasserstoff, Halogen, Cyano, (C₁-C₃)Alkyl, (C₁-C₃)Halogenalkyl oder (C₁-C₃)Alkoxy;
- V¹ und V²: stehen unabhängig voneinander für Sauerstoff oder Schwefel;
- R¹ und R²: stehen unabhängig voneinander für Wasserstoff oder (C₁-C₃)Alkyl;
- oder R¹ und R²: bilden gemeinsam mit dem Kohlenstoffatom, an das sie gebunden sind, einen (C₃-C₆)Cycloalkyl-Ring;
- R³: steht für (C₁-C₆)Alkyl, Halogen(C₁-C₃)alkyl, (C₁-C₃)Alkoxy(C₁-C₃)alkyl, Halogen(C₁-C₃)alkoxy(C₁-C₃)alkyl, (C₁-C₆)Alkenyl, Halogen(C₁-C₃)alkenyl, (C₁-C₆)Alkinyl, Halogen(C₁-C₃)alkinyl, (C₁-C₃)Alkyl-S(O)ₘ-(C₁-C₃)alkyl, Halogen(C₁-C₃)Alkyl-S(O)ₘ-(C₁-C₃)alkyl, *N*-(C₁-C₃)Alkylaminocarbonyl(C₁-C₃)alkyl oder *N,N*-Di(C₁-C₃)Alkylaminocarbonyl(C₁-C₃)alkyl, oder
für gegebenenfalls einfach oder zweifach, gleich oder verschieden durch Halogen, Cyano, Nitro, Hydroxy, Amino, (C₁-C₄)Alkyl, Halogen(C₁-C₃)Alkyl, (C₁-C₃)Alkoxy, Halogen(C₁-C₃)Alkoxy oder Cyclopropyl substituiertes (C₃-C₆)Cycloalkyl, (C₃-C₆)Cycloalkyl(C₁-C₃)alkyl, (C₃-C₆)Cycloalkenyl oder (C₃-C₆)Cycloalkenyl(C₁-C₃)alkyl, oder
für gegebenenfalls einfach oder zweifach, gleich oder verschieden durch Halogen, Cyano, Nitro, Hydroxy, Amino, (C₁-C₄)Alkyl, Halogen(C₁-C₃)Alkyl, (C₁-C₃)Alkoxy, Halogen(C₁-C₃)Alkoxy oder Cyclopropyl substituiertes Heterocyclyl, Heterocyclyl(C₁-C₃)alkyl, Aryl, Aryl(C₁-C₃)alkyl, Hetaryl oder Hetaryl(C₁-C₃)alkyl;
- R⁴: steht für Wasserstoff, (C₁-C₆)Alkyl, Halogen(C₁-C₃)alkyl oder (C₁-C₃)Alkoxy(C₁-C₃)alkyl, oder
für gegebenenfalls einfach oder zweifach, gleich oder verschieden durch Halogen, Cyano, Nitro, Hydroxy, Amino, (C₁-C₄)Alkyl, Halogen(C₁-C₃)Alkyl, (C₁-C₃)Alkoxy, Halogen(C₁-C₃)Alkoxy oder Cyclopropyl substituiertes (C₃-C₆)Cycloalkyl, (C₃-C₆)Cycloalkyl(C₁-C₃)alkyl, Phenyl, Phenyl(C₁-C₃)alkyl, Pyridyl oder Pyridyl(C₁-C₃)alkyl;
- oder R³ und R⁴: bilden gemeinsam mit dem Stickstoffatom, an das sie gebunden sind, einen gegebenenfalls durch Halogen, Cyano, (C₁-C₄)Alkyl, Halogen(C₁-C₄)alkyl, (C₁-C₄)Alkoxy, Halogen(C₁-C₄)alkoxy, (C₃-C₆)Cycloalkyl substituierten gesättigten bis dreifach ungesättigten 3- bis 6-gliedrigen Ring, der ausgewählt ist aus Aziridinyl, Azirenyl, Diaziridinyl, Diazirenyl, Azetidinyl, Dihydroazetyl, Diazetidinyl, Dihydrodiazetyl, Oxazetidinyl, Oxazetyl, Thiazetidinyl, Thiazetyl, Pyrrolidinyl, Dihydropyrrolyl, Pyrazolidinyl, Dihydropyrazolyl, Imidazolidinyl, Dihydroimidazolyl, Oxazolidinyl, Dihydrooxazolyl, Thiazolidinyl, Dihydrothyazolyl, Piperidinyl, Piperazinyl, Hexahydropyridazinyl, Hexahydropyrimidinyl, Morpholin, Dioxazinanyl, Thiomorpholin, Dithiazinan, Dioxothiazinan, Pyrrolyl, Pyrazolyl, Imidazolyl, Triazolyl und Tetrazolyl.
- m: steht für die Zahl 0, 1 oder 2.

Besonders bevorzugten Substituenten bzw. Bereiche der in den Verbindungen der Formel (I) aufgeführten Reste werden im Folgenden erläutert (Ausgestaltung 3-1):
- W: steht für Wasserstoff oder Fluor;
- n: steht für die Zahl 0 oder 1;
- Y: steht für Fluor, Chlor, Brom, Methyl, Trifluoromethyl oder Methoxy;
- X: steht für Wasserstoff, Chlor, Fluor oder Methyl;
insbesondere wobei X und Y für folgende Kombinationen (Y,X) stehen: (Me, F), (Me,H), (Me,Cl), (Me,Me), (Cl,Cl), (Cl,F), (MeO,F), (MeO,H), (Cl,H), (Br,H), (Br,F), (F,F), (CF₃,H), (CF₃,F), besonders bevorzugt für (Me, F), (Me,Cl), (Me,Me), (Cl,Cl);
- V¹ und V²: stehen unabhängig voneinander für Sauerstoff oder Schwefel;
- R¹ und R²: stehen unabhängig voneinander für Wasserstoff, Methyl oder Ethyl;
- oder R¹ und R²: bilden einen Cyclopropyl- oder Cyclobutyl-Ring;
- R³: steht für Methyl, Ethyl, n-Propyl, Isopropyl, n-Butyl, Isobutyl, sec-Butyl, tert-Butyl, 1,1-Dimethylpropyl, 2,2,2-Trifluorethyl, 2,2-Difluorethyl, 2,2-Difluor-n-propyl, 2-Methoxyethyl, 2-Ethoxyethyl, 3-Methoxypropyl, 3-Ethoxypropyl, Dimethylaminocarbonylmethyl, Diethylaminocarbonylmethyl, *N*-Ethyl-*N-*methylaminocarbonylmethyl, *N*-Isopropyl-*N*-methylaminocarbonylmethyl, Dimethylaminocarbonylethyl, Diethylaminocarbonylethyl, *N*-Ethyl-*N-*methylaminocarbonylethyl, *N*-Isopropyl-*N*-methylaminocarbonylethyl, *N*-Cyclopropyl-*N*-methylaminocarbonylmethyl oder *N*-Cyclopropyl-*N*-methylaminocarbonylethyl, oder
für Cyclopropyl, Cyclobutyl, Cyclopentyl, Cyclohexyl, 2-Tetrahydrofurylmethyl, 3-Tetrahydrofurylmethyl, 2-Tetrahydrofurylethyl oder 3-Tetrahydrofurylethyl, oder
für gegebenenfalls einfach oder zweifach, gleich oder verschieden durch Fluor, Chlor, Brom, Cyano, Nitro, Hydroxy, Amino, Methyl, Ethyl, Isopropyl, tert-Butyl, Cyclopropyl, Trifluormethyl, Difluormethyl, Methoxy, Trifluormethoxy oder Difluormethoxy substituiertes Phenyl, oder
für gegebenenfalls einfach oder zweifach, gleich oder verschieden durch Fluor, Chlor, Brom, Cyano, Nitro, Hydroxy, Amino, Methyl, Ethyl, Isopropyl, tert-Butyl, Cyclopropyl, Trifluormethyl, Difluormethyl, Methoxy, Trifluormethoxy oder Difluormethoxy substituiertes Pyridyl, oder
für gegebenenfalls einfach oder zweifach, gleich oder verschieden durch Fluor, Chlor, Brom, Cyano, Nitro, Hydroxy, Amino, Methyl, Ethyl, Isopropyl, tert-Butyl, Trifluormethyl, Difluormethyl, Methoxy, Trifluormethoxy oder Difluormethoxy substituiertes Benzyl oder Pyridylmethyl;
- R⁴: steht für Wasserstoff, Methyl, Ethyl, n-Propyl, Isopropyl, n-Butyl, Isobutyl, tert-Butyl, sec-Butyl, 2,2,2-Trifluorethyl, 2,2-Difluorethyl, 2-Methoxyethyl, 2-Ethoxyethyl, Cyclopropyl, Cyclobutyl, Cyclopropylmethyl, Phenyl oder Benzyl;
- oder R³ und R⁴: bilden bilden gemeinsam mit dem Stickstoffatom, an das sie gebunden sind, einen Azetidin-, Oxetan-, Thiethan-, Morpholin-, Thiomorpholin- oder einen *N*-Methyl substituierten Piperazinring.

In einer weiteren Ausgestaltung sind die besonders bevorzugten Substituenten bzw. Bereiche der in den Verbindungen der Formel (I) aufgeführten Reste wie folgt (Ausgestaltung 3-2):
- W: steht für Wasserstoff oder Fluor;
- n: steht für die Zahl 0 oder 1;
- Y: steht für Fluor, Chlor, Brom, Methyl, Trifluoromethyl oder Methoxy;
- X: steht für Wasserstoff, Chlor, Fluor oder Methyl;
insbesondere wobei X und Y für folgende Kombinationen (Y,X) stehen: (Me, F), (Me,H), (Me,Cl), (Me,Me), (Cl,Cl), (Cl,F), (MeO,F), (MeO,H), (Cl,H), (Br,H), (Br,F), (F,F), (CF₃,H), (CF₃,F), besonders bevorzugt für (Me, F), (Me,Cl), (Me,Me), (Cl,Cl);
- V¹ und V²: stehen unabhängig voneinander für Sauerstoff oder Schwefel;
- R¹ und R²: stehen unabhängig voneinander für Wasserstoff, Methyl oder Ethyl;
- oder R¹ und R²: bilden einen Cyclopropyl- oder Cyclobutyl-Ring;
- R³: steht für Methyl, Ethyl, n-Propyl, Isopropyl, n-Butyl, Isobutyl, sec-Butyl, tert-Butyl, 1,1-Dimethylpropyl, 2,2,2-Trifluorethyl, 2,2-Difluorethyl, 2,2-Difluor-n-propyl, 2-Methoxyethyl, 2-Ethoxyethyl, 3-Methoxypropyl, 3-Ethoxypropyl, Dimethylaminocarbonylmethyl, Diethylaminocarbonylmethyl, *N*-Ethyl-*N-*methylaminocarbonylmethyl, *N*-Isopropyl-*N*-methylaminocarbonylmethyl, Dimethylaminocarbonylethyl, Diethylaminocarbonylethyl, *N*-Ethyl-*N-*methylaminocarbonylethyl, *N*-Isopropyl-*N*-methylaminocarbonylethyl, *N*-Cyclopropyl-*N*-methylaminocarbonylmethyl oder *N*-Cyclopropyl-*N*-methylaminocarbonylethyl, oder
für Cyclopropyl, Cyclobutyl, Cyclopentyl, Cyclohexyl, 2-Tetrahydrofurylmethyl, 3-Tetrahydrofurylmethyl, 2-Tetrahydrofurylethyl oder 3-Tetrahydrofurylethyl, oder
für gegebenenfalls einfach oder zweifach, gleich oder verschieden durch Fluor, Chlor, Brom, Cyano, Nitro, Hydroxy, Amino, Methyl, Ethyl, Isopropyl, tert-Butyl, Cyclopropyl, Trifluormethyl, Difluormethyl, Methoxy, Trifluormethoxy oder Difluormethoxy substituiertes Phenyl, oder
für gegebenenfalls einfach oder zweifach, gleich oder verschieden durch Fluor, Chlor, Brom, Cyano, Nitro, Hydroxy, Amino, Methyl, Ethyl, Isopropyl, tert-Butyl, Cyclopropyl, Trifluormethyl, Difluormethyl, Methoxy, Trifluormethoxy oder Difluormethoxy substituiertes Pyridyl, oder
für gegebenenfalls einfach oder zweifach, gleich oder verschieden durch Fluor, Chlor, Brom, Cyano, Nitro, Hydroxy, Amino, Methyl, Ethyl, Isopropyl, tert-Butyl, Trifluormethyl, Difluormethyl, Methoxy, Trifluormethoxy oder Difluormethoxy substituiertes Benzyl oder Pyridylmethyl;
- R⁴: steht für Wasserstoff, Methyl, Ethyl, n-Propyl, Isopropyl, n-Butyl, Isobutyl, tert-Butyl, sec-Butyl, 2,2,2-Trifluorethyl, 2,2-Difluorethyl, 2-Methoxyethyl, 2-Ethoxyethyl, Cyclopropyl, Cyclobutyl, Cyclopropylmethyl, Phenyl oder Benzyl;
- oder R³ und R⁴: bilden bilden gemeinsam mit dem Stickstoffatom, an das sie gebunden sind, einen Azetidin-, Oxetan-, Thiethan-, Morpholin-, Thiomorpholin-, Dioxothiazinan-, Piperidin- oder einen *N*-Methyl substituierten Piperazinring.

Ganz besonders bevorzugten Substituenten bzw. Bereiche der in den Verbindungen der Formel (I) aufgeführten Reste werden im Folgenden erläutert (Ausgestaltung 4-1):
- W: steht für Fluor;
- n: steht für die Zahl 0 oder 1;
- Y: steht für Chlor oder Methyl;
- X: steht für Wasserstoff, Fluor, Chlor oder Methyl;
insbesondere wobei X und Y für folgende Kombinationen (Y,X) stehen: (Me,Cl), (Me, F), (Me,Me), (Cl,Cl);
- V¹ und V²: stehen unabhängig voneinander für Sauerstoff oder Schwefel;
- R¹ und R²: stehen unabhängig voneinander für Wasserstoff oder Methyl;
- R³: steht für Ethyl, Isopropyl, Isobutyl, sec-Butyl, tert-Butyl, 1,1-Dimethylpropyl, 2,2,2-Trifluorethyl, 2-Methoxyethyl, oder
für Cyclopropyl oder 2-Tetrahydrofurylmethyl, oder
für Phenyl, 3-Fluorphenyl, 4-Fluorphenyl, 3-Chlorphenyl, 3-Trifluormethylphenyl, 2-Pyridyl, 3-Pyridyl oder Benzyl;
- R⁴: steht für Wasserstoff.

In einer weiteren Ausgestaltung sind die ganz besonders bevorzugten Substituenten bzw. Bereiche der in den Verbindungen der Formel (I) aufgeführten Reste wie folgt (Ausgestaltung 4-2):
- W: steht für Fluor;
- n: steht für die Zahl 0 oder 1;
- Y: steht für Chlor oder Methyl;
- X: steht für Wasserstoff, Fluor, Chlor oder Methyl;
insbesondere wobei X und Y für folgende Kombinationen (Y,X) stehen: (Me,Cl), (Me, F), (Me,Me), (Cl,Cl);
- V¹ und V²: stehen unabhängig voneinander für Sauerstoff oder Schwefel;
- R¹ und R²: stehen unabhängig voneinander für Wasserstoff oder Methyl;
- R³: steht für Methyl, Ethyl, Isopropyl, Isobutyl, sec-Butyl, tert-Butyl, 1,1-Dimethylpropyl, 2,2,2-Trifluorethyl, 2-Methoxyethyl, oder
für Cyclopropyl oder 2-Tetrahydrofurylmethyl, oder
für Phenyl, 3-Fluorphenyl, 4-Fluorphenyl, 3-Chlorphenyl, 3-Trifluormethylphenyl, 2-Pyridyl, 3-Pyridyl oder Benzyl;
- R⁴: steht für Wasserstoff oder Methyl;
- oder R³ und R⁴: bilden bilden gemeinsam einen der folgenden Ringe: 1-Morpholin, 1-(4-Methylpiperazin), 1-(1,1-Dioxo-1,4-thiazinan) oder 1-(4,4-Difluorpiperidin).

Wenn in obigen Definitionen in Ringen Schwefel und/oder Stickstoff vorkommen, wie beispielsweise in Ausdrücken wie "in welchen die Ringe mindestens ein Heteroatom aus der Reihe Schwefel, Sauerstoff (wobei Sauerstoffatome nicht unmittelbar benachbart sein dürfen) und Stickstoff enthalten können" oder "in welchen ein oder zwei Ringglieder jeweils durch ein Heteroatom aus der Reihe Schwefel, Sauerstoff (wobei Sauerstoffatome nicht unmittelbar benachbart sein dürfen) und Stickstoff ersetzt sein können", dann kann, sofern nichts anderes angegeben ist, der Schwefel auch als SO oder SO₂ vorliegen, der Stickstoff, sofern er nicht als -N= vorliegt, neben NH auch als N-Alkyl (insbesondere N-C₁-C₆-Alkyl) vorliegen.

In den Definitionen ist, sofern nichts anderes angegeben ist,
Halogen ausgewählt aus der Reihe Fluor, Chlor, Brom und Iod, bevorzugt wiederum aus der Reihe Fluor, Chlor und Brom,
Aryl (auch als Teil einer größeren Einheit, wie beispielsweise Arylalkyl) ausgewählt aus der Reihe Phenyl, Naphthyl, Anthryl und Phenanthrenyl, vorzugsweise Phenyl,
Hetaryl (gleichbedeutend mit Heteroaryl, auch als Teil einer größeren Einheit, wie beispielsweise Hetarylalkyl) ausgewählt aus der Reihe Furyl, Thienyl, Pyrrolyl, Pyrazolyl, Imidazolyl, 1,2,3-Triazolyl, 1,2,4-Triazolyl, Oxazolyl, Isoxazolyl, Thiazolyl, Isothiazolyl, 1,2,3-Oxadiazolyl, 1,2,4-Oxadiazolyl, 1,3,4-Oxadiazolyl, 1,2,5-Oxadiazolyl, 1,2,3-Thiadiazolyl, 1,2,4-Thiadiazolyl, 1,3,4-Thiadiazolyl, 1,2,5-Thiadiazolyl, Pyridyl, Pyrimidinyl, Pyridazinyl, Pyrazinyl, 1,2,3-Triazinyl, 1,2,4-Triazinyl, 1,3,5-Triazinyl, Benzofuryl, Benzisofuryl, Benzothienyl, Benzisothienyl, Indolyl, Isoindolyl, Indazolyl, Benzothiazolyl, Benzisothiazolyl, Benzoxazolyl, Benzisoxazolyl, Benzimidazolyl, 2,1,3-Benzoxadiazole, Chinolinyl, Isochinolinyl, Cinnolinyl, Phthalazinyl, Chinazolinyl, Chinoxalinyl, Naphthyridinyl, Benzotriazinyl, Purinyl, Pteridinyl und Indolizinyl, vorzugsweise wiederum aus der Reihe Pyrazolyl, Imidazolyl, 1,2,3-Triazolyl, 1,2,4-Triazolyl, Oxazolyl, Isoxazolyl, Thiazolyl, Isothiazolyl, Pyridyl, Pyrimidinyl, Pyridazinyl, Pyrazinyl, 1,2,3-Triazinyl, 1,2,4-Triazinyl und 1,3,5-Triazinyl, besonders bevorzugt Pyridyl,
Heterocyclyl ein gesättigter 4-, 5- oder 6-Ring, der 1 oder 2 Stickstoffatome und/oder ein Sauerstoffatom und/oder ein Schwefelatom enthält, vorzugsweise Azetidinyl, Azolidinyl, Azinanyl, Oxetanyl, Oxolanyl, Oxanyl, Dioxanyl, Thiethanyl, Thiolanyl, Thianyl, Tetrahydrofuryl, Piperazinyl oder Morpholinyl.

Durch Halogen substituierte Reste, z.B. Haloalkyl (= Halogenalkyl), sind einfach oder mehrfach bis zur maximal möglichen Substituentenzahl halogeniert. Bei mehrfacher Halogenierung können die Halogenatome gleich oder verschieden sein. Halogen steht hierbei wiederum für Fluor, Chlor, Brom und Iod, insbesondere für Fluor, Chlor und Brom.

Gesättigte oder ungesättigte Kohlenwasserstoffreste wie Alkyl oder Alkenyl können, auch in Verbindung mit Heteroatomen, wie z.B. in Alkoxy, soweit möglich, jeweils geradkettig oder verzweigt sein.

Gegebenenfalls substituierte Reste können einfach oder mehrfach substituiert sein, wobei bei Mehrfachsubstitutionen die Substituenten gleich oder verschieden sein können. Sofern Substituenten vorgesehen oder gegebenenfalls vorgesehen sind, handelt es sich bei den Substituenten, sofern nichts anderes angegeben ist, um Halogen, Alkyl, Cycloalkyl, Cyano, Nitro, Alkoxy, Halogenalkyl oder Halogenalkoxy, insbesondere um Fluor, Chlor, (C₁-C₃)Alkyl, (C₃-C₆)Cycloalkyl (insbesondere Cyclopropyl), Cyano, (C₁-C₃)Alkoxy, (C₁-C₃)Halogenalkyl oder (C₁-C₃)Halogenalkoxy.

Die oben aufgeführten allgemeinen oder in Vorzugsbereichen aufgeführten Restedefinitionen bzw. Erläuterungen gelten für die Endprodukte (auch für die Verbindungen der Formel (I) mit der später aufgeführten Substruktur (I-A) und für die Ausgangsprodukte und Zwischenprodukte entsprechend. Diese Restedefinitionen können untereinander, also auch zwischen den jeweiligen Vorzugsbereichen, beliebig kombiniert werden.

Erfindungsgemäß bevorzugt werden Verbindungen der Formel (I), in welchen eine Kombination der vorstehend als bevorzugt aufgeführten Bedeutungen vorliegt, wobei jede vorstehend als bevorzugt beschriebene Ausgestaltung eine eigenständige Kombination darstellt, insbesondere eine Kombination wie beschrieben in Ausgestaltung 2-1 oder in Ausgestaltung 2-2.

Erfindungsgemäß besonders bevorzugt werden Verbindungen der Formel (I), in welchen eine Kombination der vorstehend als besonders bevorzugt aufgeführten Bedeutungen vorliegt, wobei jede vorstehend als besonders bevorzugt beschriebene Ausgestaltung eine eigenständige Kombination darstellt, insbesondere eine Kombination wie beschrieben in Ausgestaltung 3-1 oder in Ausgestaltung 3-2.

Erfindungsgemäß ganz besonders bevorzugt werden Verbindungen der Formel (I), in welchen eine Kombination der vorstehend als ganz besonders bevorzugt aufgeführten Bedeutungen vorliegt, wobei jede vorstehend als ganz besonders bevorzugt beschriebene Ausgestaltung eine eigenständige Kombination darstellt, insbesondere eine Kombination wie beschrieben in Ausgestaltung 4-1 oder in Ausgestaltung 4-2.

In weiteren bevorzugten Ausführungsformen betrifft die Erfindung Verbindungen der Formel (I), in denen V¹ und V² für O stehen. Hieraus ergeben sich Verbindungen der Formel (I-A)

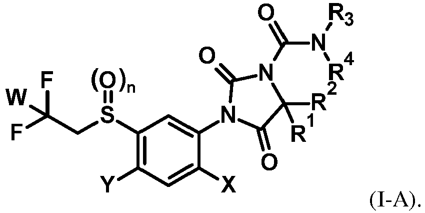

In der durch die Struktur (I-A) definierten Verbindungen der Formel (I) haben die Reste bzw. Strukturelemente W, n, m, Y, X, R¹, R², R³ und R⁴ die weiter oben genannten Bedeutungen, insbesondere wie beschrieben in Ausgestaltung 1-1 (Ausgestaltung I-A-1-1).

Bevorzugt innerhalb der durch die Struktur (I-A) definierten Verbindungen der Formel (I) werden diejenigen Verbindungen, in welchen eine Kombination der vorstehend als bevorzugt aufgeführten Bedeutungen vorliegt, wobei jede vorstehend als bevorzugt beschriebene Ausgestaltung eine eigenständige Kombination darstellt, insbesondere eine Kombination wie beschrieben in Ausgestaltung 2-1 (Ausgestaltung I-A-2-1) oder in Ausgestaltung 2-2 (Ausgestaltung I-A-2-2).

Besonders bevorzugt innerhalb der durch die Struktur (I-A) definierten Verbindungen der Formel (I) werden diejenigen Verbindungen, in welchen eine Kombination der vorstehend als besonders bevorzugt aufgeführten Bedeutungen vorliegt, wobei jede vorstehend als besonders bevorzugt beschriebene Ausgestaltung eine eigenständige Kombination darstellt, insbesondere eine Kombination wie beschrieben in Ausgestaltung 3-1 (Ausgestaltung I-A-3-1) oder in Ausgestaltung 3-2 (Ausgestaltung I-A-3-2).

Ganz besonders bevorzugt innerhalb der durch die Struktur (I-A) definierten Verbindungen der Formel (I) werden diejenigen Verbindungen, in welchen eine Kombination der vorstehend als ganz besonders bevorzugt aufgeführten Bedeutungen vorliegt, wobei jede vorstehend als ganz besonders bevorzugt beschriebene Ausgestaltung eine eigenständige Kombination darstellt, insbesondere eine Kombination wie beschrieben in Ausgestaltung 4-1 (Ausgestaltung I-A-4-1) oder in Ausgestaltung 4-2 (Ausgestaltung I-A-4-2).

In weiteren bevorzugten Ausführungsformen der Verbindungen der Formel (I-A), insbesondere in den Ausgestaltungen I-A-1-1, I-A-2-1,I-A-2-2, I-A-3-1, I-A-3-2, I-A-4-1 und I-A-4-2, stehen X und Y für folgende Kombinationen (Y,X): (Me, F), (Me,H), (Me,Cl), (Me,Me), (Cl,Cl), (Cl,F), (MeO,F), (MeO,H), (Cl,H), (Br,H), (Br,F), (F,F), (CF₃,H), (CF₃,F), wobei die folgenden Kombinationen (Y,X) besonders bevorzugt sind: (Me, F), (Me,Cl), (Me,Me), (Cl,Cl).

In weiteren bevorzugten Ausführungsformen betrifft die Erfindung Verbindungen der Formel (I), in denen n gleich 0 ist, bezeichnet als Verbindungen der Formel (Ia). In solchen Verbindungen der Formel (Ia) haben die Reste bzw. Strukturelemente W, m, Y, X, R¹, R², R³, R⁴, V¹ und V² die weiter oben genannten Bedeutungen, insbesondere wie beschrieben in Ausgestaltung 1-1 (Ausgestaltung Ia-1-1).

Bevorzugte Verbindungen der Formel (Ia) sind solche, in welchen eine Kombination der vorstehend als bevorzugt aufgeführten Bedeutungen vorliegt, wobei jede vorstehend als bevorzugt beschriebene Ausgestaltung eine eigenständige Kombination darstellt, insbesondere eine Kombination wie beschrieben in Ausgestaltung 2-1 (Ausgestaltung Ia-2-1) oder in Ausgestaltung 2-2 (Ausgestaltung Ia-2-2).

Besonders bevorzugte Verbindungen der Formel (Ia) sind solche, in welchen eine Kombination der vorstehend als besonders bevorzugt aufgeführten Bedeutungen vorliegt, wobei jede vorstehend als besonders bevorzugt beschriebene Ausgestaltung eine eigenständige Kombination darstellt, insbesondere eine Kombination wie beschrieben in Ausgestaltung 3-1 (Ausgestaltung Ia-3-1) oder in Ausgestaltung 3-2 (Ausgestaltung Ia-3-2).

Ganz besonders bevorzugte Verbindungen der Formel (Ia) sind solche, in welchen eine Kombination der vorstehend als ganz besonders bevorzugt aufgeführten Bedeutungen vorliegt, wobei jede vorstehend als ganz besonders bevorzugt beschriebene Ausgestaltung eine eigenständige Kombination darstellt, insbesondere eine Kombination wie beschrieben in Ausgestaltung 4-1 (Ausgestaltung Ia-4-1) oder in Ausgestaltung 4-2 (Ausgestaltung Ia-4-2).

In weiteren bevorzugten Ausführungsformen der Verbindungen der Formel (Ia), insbesondere in den Ausgestaltungen Ia-1-1, Ia-2-1, Ia-2-2, Ia-3-1, Ia-3-2, Ia-4-1 und Ia-4-2, stehen X und Y für folgende Kombinationen (Y,X): (Me, F), (Me,H), (Me,Cl), (Me,Me), (Cl,Cl), (Cl,F), (MeO,F), (MeO,H), (Cl,H), (Br,H), (Br,F), (F,F), (CF₃,H), (CF₃,F), wobei die folgenden Kombinationen (Y,X) besonders bevorzugt sind: (Me, F), (Me,Cl), (Me,Me), (Cl,Cl).

In weiteren bevorzugten Ausführungsformen betrifft die Erfindung Verbindungen der Formel (I), in denen n gleich 1 ist, bezeichnet als Verbindungen der Formel (Ib). In solchen Verbindungen der Formel (Ib) haben die Reste bzw. Strukturelemente W, m, Y, X, R¹, R², R³, R⁴, V¹ und V² die weiter oben genannten Bedeutungen, insbesondere wie beschrieben in Ausgestaltung 1-1 (Ausgestaltung Ib-1-1).

Bevorzugte Verbindungen der Formel (Ib) sind solche, in welchen eine Kombination der vorstehend als bevorzugt aufgeführten Bedeutungen vorliegt, wobei jede vorstehend als bevorzugt beschriebene Ausgestaltung eine eigenständige Kombination darstellt, insbesondere eine Kombination wie beschrieben in Ausgestaltung 2-1 (Ausgestaltung Ib-2-1) oder in Ausgestaltung 2-2 (Ausgestaltung Ib-2-2).

Besonders bevorzugte Verbindungen der Formel (Ib) sind solche, in welchen eine Kombination der vorstehend als besonders bevorzugt aufgeführten Bedeutungen vorliegt, wobei jede vorstehend als besonders bevorzugt beschriebene Ausgestaltung eine eigenständige Kombination darstellt, insbesondere eine Kombination wie beschrieben in Ausgestaltung 3-1 (Ausgestaltung Ib-3-1) oder in Ausgestaltung 3-2 (Ausgestaltung Ib-3-2).

Ganz besonders bevorzugte Verbindungen der Formel (Ib) sind solche, in welchen eine Kombination der vorstehend als ganz besonders bevorzugt aufgeführten Bedeutungen vorliegt, wobei jede vorstehend als ganz besonders bevorzugt beschriebene Ausgestaltung eine eigenständige Kombination darstellt, insbesondere eine Kombination wie beschrieben in Ausgestaltung 4-1 (Ausgestaltung Ib-4-1) oder in Ausgestaltung 4-2 (Ausgestaltung Ib-4-2).

In weiteren bevorzugten Ausführungsformen der Verbindungen der Formel (Ib), insbesondere in den Ausgestaltungen Ib-1-1, Ib-2-1, Ib-2-2, Ib-3-1, Ib-3-2, Ib-4-1 und Ib-4-2, stehen X und Y für folgende Kombinationen (Y,X): (Me, F), (Me,H), (Me,Cl), (Me,Me), (Cl,Cl), (Cl,F), (MeO,F), (MeO,H), (Cl,H), (Br,H), (Br,F), (F,F), (CF₃,H), (CF₃,F), wobei die folgenden Kombinationen (Y,X) besonders bevorzugt sind: (Me, F), (Me,Cl), (Me,Me), (Cl,Cl).

In weiteren Ausführungsformen betrifft die Erfindung Verbindungen der Formel (I), in denen n gleich 2 ist, bezeichnet als Verbindungen der Formel (Ic). In solchen Verbindungen der Formel (Ic) haben die Reste bzw. Strukturelemente W, m, Y, X, R¹, R², R³, R⁴, V¹ und V² die weiter oben genannten Bedeutungen, insbesondere wie beschrieben in Ausgestaltung 1-1 (Ausgestaltung Ic-1-1).

Bevorzugte Verbindungen der Formel (Ic) sind solche, in welchen eine Kombination der vorstehend als bevorzugt aufgeführten Bedeutungen vorliegt, wobei jede vorstehend als bevorzugt beschriebene Ausgestaltung eine eigenständige Kombination darstellt, insbesondere eine Kombination wie beschrieben in Ausgestaltung 2-1 (Ausgestaltung Ic-2-1) oder in Ausgestaltung 2-2 (Ausgestaltung Ic-2-2).

Besonders bevorzugte Verbindungen der Formel (Ic) sind solche, in welchen eine Kombination der vorstehend als besonders bevorzugt aufgeführten Bedeutungen vorliegt, wobei jede vorstehend als besonders bevorzugt beschriebene Ausgestaltung eine eigenständige Kombination darstellt, insbesondere eine Kombination wie beschrieben in Ausgestaltung 3-1 (Ausgestaltung Ic-3-1) oder in Ausgestaltung 3-2 (Ausgestaltung Ic-3-2).

Ganz besonders bevorzugte Verbindungen der Formel (Ic) sind solche, in welchen eine Kombination der vorstehend als ganz besonders bevorzugt aufgeführten Bedeutungen vorliegt, wobei jede vorstehend als ganz besonders bevorzugt beschriebene Ausgestaltung eine eigenständige Kombination darstellt, insbesondere eine Kombination wie beschrieben in Ausgestaltung 4-1 (Ausgestaltung Ic-4-1) oder in Ausgestaltung 4-2 (Ausgestaltung Ic-4-2).

In weiteren bevorzugten Ausführungsformen der Verbindungen der Formel (Ic), insbesondere in den Ausgestaltungen Ic-1-1, Ic-2-1, Ic-2-2, Ic-3-1, Ic-3-2, Ic-4-1 und Ic-4-2, stehen X und Y für folgende Kombinationen (Y,X): (Me, F), (Me,H), (Me,Cl), (Me,Me), (Cl,Cl), (Cl,F), (MeO,F), (MeO,H), (Cl,H), (Br,H), (Br,F), (F,F), (CF₃,H), (CF₃,F), wobei die folgenden Kombinationen (Y,X) besonders bevorzugt sind: (Me, F), (Me,Cl), (Me,Me), (Cl,Cl).

Gegenüber den Verbindungen der Formel (Ic) sind die Verbindungen der Formel (Ia) und die Verbindungen der Formel (Ib) bevorzugt.

Weitere bevorzugte Ausführungsformen der Erfindung sind Verbindungen der Formel (I), in denen eine Kombination der Bedeutungen der Strukturelemente der Verbindungen der Formel (I-A) und der Formel (Ia) vorliegt, beispielsweise eine Kombination der Ausgestaltungen I-A-1-1 und Ia-1-1, I-A-2-1 und Ia-2-1, I-A-3-1 und Ia-3-1, I-A-4-1 und Ia-4-1, I-A-2-2 und Ia-2-2, I-A-3-2 und Ia-3-2 oder I-A-4-2 und Ia-4-2.

Weitere bevorzugte Ausführungsformen der Erfindung sind Verbindungen der Formel (I), in denen eine Kombination der Bedeutungen der Strukturelemente der Verbindungen der Formel (I-A) und der Formel (Ib) vorliegt, beispielsweise eine Kombination der Ausgestaltungen I-A-1-1 und Ib-1-1, I-A-2-1 und Ib-2-1, I-A-3-1 und Ib-3-1, I-A-4-1 und Ib-4-1, I-A-2-2 und Ib-2-2, I-A-3-2 und Ib-3-2 oder I-A-4-2 und Ib-4-2.

Weitere Ausführungsformen der Erfindung sind Verbindungen der Formel (I), in denen eine Kombination der Strukturelemente der Verbindungen der Formel (I-A) und der Formel (Ic) vorliegt, beispielsweise eine Kombination der Ausgestaltungen I-A-1-1 und Ic-1-1, I-A-2-1 und Ic-2-1, I-A-3-1 und Ic-3-1, I-A-4-1 und Ic-4-1, I-A-2-2 und Ic-2-2, I-A-3-2 und Ic-3-2 oder I-A-4-2 und Ic-4-2.

Die Verbindungen der Formel (I) können auch als Salze, insbesondere Säureadditionssalze und Metallsalzkomplexe, vorliegen. Die Verbindungen der Formel (I) und deren Säureadditionssalze und Metallsalzkomplexe besitzen gute Wirksamkeit, insbesondere zur Bekämpfung von tierischen Schädlingen, zu denen Arthropoden und insbesondere Insekten und Akariden zählen.

Als geeignete Salze der Verbindungen der allgemeinen Formel (I) können übliche nicht toxische Salze, d. h. Salze mit entsprechenden Basen und Salze mit zugesetzten Säuren genannt werden. Vorzugsweise sind Salze mit anorganischen Basen, wie Alkalimetallsalze, beispielsweise Natrium-, Kalium- oder Cäsiumsalze, Erdalkalimetallsalze, beispielsweise Calzium- oder Magnesiumsalze, Ammoniumsalze, Salze mit organischen Basen sowie mit anorganischen Aminen, beispielsweise Triethylammonium-, Dicyclohexylammonium-, N,N'-Dibenzylethylendiammonium-, Pyridinium-, Picolinium- oder Ethanolammoniumsalze, Salze mit anorganischen Säuren, beispielsweise Hydrochloride, Hydrobromide, Dihydrosulfate, Trihydrosulfate, oder Phosphate, Salze mit organischen Carbonsäuren oder organischen Sulfosäure, beispielsweise Formiate, Acetate, Trifluoracetate, Maleate, Tartrate, Methansulfonate, Benzolsulfonate oder para-Toluolsulfonate, Salze mit basischen Aminosäuren, beispielsweise Arginate, Aspartate oder Glutamate und Ähnliches zu nennen.

Die Verbindungen der Formel (I) können gegebenenfalls auch in Abhängigkeit von der Art der Substituenten als Stereoisomere, d.h. als geometrische und/oder als optische Isomere oder Isomerengemische vorliegen in unterschiedlichen Zusammensetzungen vorliegen. Sowohl die reinen Stereoisomeren als auch beliebige Gemische dieser Isomeren sind Gegenstand dieser Erfindung, auch wenn hier im Allgemeinen nur von Verbindungen der Formel (I) die Rede ist.

Vorzugsweise werden jedoch die optisch aktiven, stereoisomeren Formen der Verbindungen der Formel (I) und deren Salze erfindungsgemäß verwendet.

Die Erfindung betrifft daher sowohl die reinen Enantiomeren und Diastereomeren, als auch deren Gemische zur Bekämpfung von tierischen Schädlingen, zu denen Arthropoden und insbesondere Insekten und Akariden zählen. Eine individuelle Ausgestaltung der Erfindung ist daher auf das Vorliegen des R-Enantiomers gerichtet bzw. auf ein Gemisch, welches mehrheitlich das R-Enantiomer umfasst, vorzugsweise wobei das Verhältnis von R- zu S-Enantiomer mindestens 60:40 und zunehmend bevorzugt mindestens 70:30, 75:25, 80:20, 85:15 und 90:10 beträgt. Eine weitere individuelle Ausgestaltung der Erfindung ist daher auf das Vorliegen des S-Enantiomers gerichtet bzw. auf ein Gemisch, welches mehrheitlich das S-Enantiomer umfasst, vorzugsweise wobei das Verhältnis von S-zu R-Enantiomer mindestens 60:40 und zunehmend bevorzugt mindestens 70:30, 75:25, 80:20, 85:15 und 90:10 beträgt.

Die Verbindungen der Formel (I) können in verschiedenen polymorphen Formen oder als Mischung verschiedener polymorpher Formen vorliegen. Sowohl die reinen Polymorphe als auch die Polymorphgemische sind Gegenstand der Erfindung und können erfindungsgemäß verwendet werden.

Die erfindungsgemäßen Verbindungen sind durch die Formel (I) allgemein definiert und die beinhaltet auch alle möglichen Rotamere und Gemische davon.

Die erfindungsgemässen Verbindungen der Formel (I) können nach üblichen, dem Fachmann bekannten Methoden hergestellt werden. Verschiedene Herstellungsverfahren, die gleichfalls Gegenstand der Erfindung sind, werden nachfolgend beschrieben.

### Herstellverfahren

Die Verbindungen der allgemeinen Formel (I) lassen sich in Verbindungen mit n=0 (Ia), n=1 (Ib) und n=2 (Ic) unterteilen und können beispielsweise nach Verfahren A, B oder C hergestellt werden.

### Verfahren A

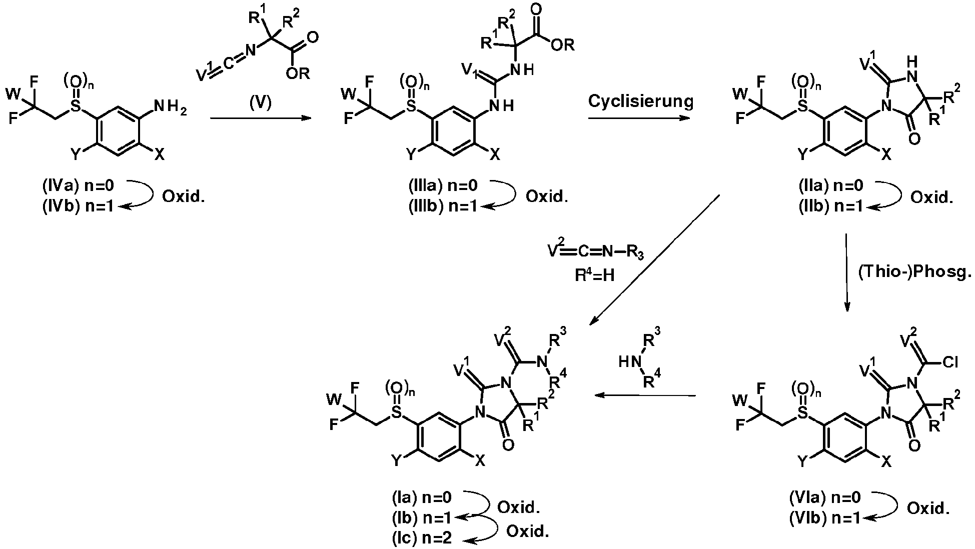

Aniline der Formel (IV) können durch Einwirkung eines Isocyanats (für V¹=O) oder Isothiocyanats (für V¹=S) der Formel (V), wobei R für Wasserstoff oder eine kleine Alkylgruppe (vorzugsweise Methyl, Ethyl) steht, zu den Harnstoffen (V¹=O) bzw. Thioharnstoffen (V¹=S) der Formel (III) nach literaturbekannten Methoden umgesetzt werden, gegebenenfalls in Gegenwart eines Lösungs- und Verdünnungsmittels, gegebenfalls in Anwesenheit einer anorganischen oder organischen Base (wie z.B. eines tertiären Amines, vorzugsweise Triethylamin, Trimethylamin, Diisopropylethylamin) in katalytischen oder stöchiometrischen Mengen oder in Überschuss oder anstelle des Lösungs- oder Verdünnungsmittels. Beispiele dieser Reaktionsbedingungen finden sich in DE2658220 und JP53015373.

Harnstoffe (V¹=O) bzw. Thioharnstoffe (V¹=S) der Formel (III) können zu 3-Arylhydantoinen der Formel (II) überführt werden, beispielsweise durch Cyclisierung in wässrigem Medium oder organischem Medium in Gegenwart eines Dehydratisierungsmittels.

Dieses zweistufige Verfahren ist in J. Med. Chem. 2006, 49, 417-425, beschrieben für die Umsetzung von Aminen mit Ethyl Isocyanatocarbonsäureestern (V¹=O, R=Ethyl) und Isothiocyanatocarbonsäureestern (V¹=S, R=Ethyl) der Formel (V). Der erste Schritt erfolgt in refluxierendem Chloroform; die daraus isolierten 3-substituierten Ureidoessigsäureethylester werden dann in einem Gemisch aus Ethanol und Salzsäure zu den 3-substituierten Imidazolidin-2,4-dionen bzw. -2-thio-4-onen umgesetzt werden.

Isocyanate (V¹=O) oder Isothiocyanate (V¹=S) der Formel (V) sind kommerziell erhältlich oder können nach literaturbekannten Methoden aus den entsprechenden Aminen hergestellt werden. Isocyanatoessigsäureethylester (V¹=O, R¹=R²=H), Isothiocyanatoessigsäureethylester (V¹=S, R¹=R²=H), Ethyl-2-isocyanatopropionat (V¹=O, R¹=Methyl, R²=H) und Ethyl-2-isocyanatopropionat (V'=O, R¹=Methyl, R²=Methyl) sind kommerziell erhältlich.

Die 3-Arylhydantoinen der Formel (II) lassen sich durch Phosgenierung (für V²=O) bzw. Thiophosgenierung (für V²=S) nach literaturbekannten Methoden zu den Carbamoylhydantoinen der Formel (VI) umgesetzt werden und diese können zu den Verbindungen der Formel (I) überführt werden durch Versetzung mit Aminen, gegebenenfalls in Gegenwart eines Lösungs- und Verdünnungsmittels, gegebenenfalls in Gegenwart einer Base (vorzugsweise Triethylamin, Trimethylamin, Diisopropylethylamin), wie in EP41465 beschrieben.

Die Herstellung von 3-Arylhydantoinen der Formel (II) kann auch aus den teilweise substituierten Hydantoinen der Formel (II-H) mit R² gleich Wasserstoff und diese wiederum aus unsubtituierten Hydantoinen der Formel (II-H) mit R¹ und R² gleich Wasserstoff erfolgen, wie in folgendem Schema dargestellt.

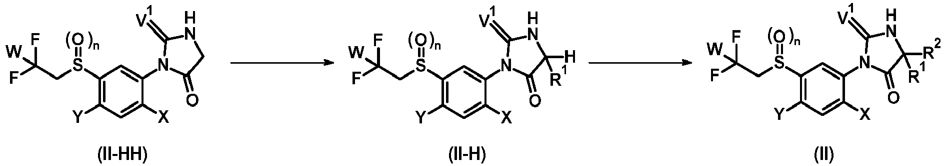

Beispiele von diesen Reaktionen für R¹ = Carbonylalkoxy finden sich in Science of Synthesis 2004, 7, 645-659; für R¹ = Benzyl oder Pyridylmethyl in Bull. Chem. Soc. Japan 2001, 74(10), 1917-1925 und WO9839303; für R¹ = Alkyl in Chirality 2002, 14(2/3), 144-150.

Bei Bedarf können die Hydantoine der Formel (II-HH) bzw. (II-H) vor diesen Umsetzungen am Stickstoffatom geschützt und anschließend entschützt zu (II-H) bzw. (II) werden. Geeignete Schutzgruppen sind z.B. Acetyl, Allyl, Benzyl oder tert-Butylcarboxylat, die nach literaturbekannten Methoden eingeführt und abgespalten werden können.

Alternativ können die erfindungsgemäßen Verbindungen der Formel (I), bei denen R⁴ für Wasserstoff steht, aus den 3-Arylhydantoinen der Formel (II) durch Umsetzung mit Isocyanaten (für V²=O) oder Isothiocyanaten (für V²=S) nach literaturbekannten Methoden hergestellt werden. Dies kann gegebenenfalls in Gegenwart eines Lösungs- und Verdünnungsmittels, gegebenfalls in Anwesenheit einer anorganischen oder organischen Base (wie z.B. eines tertiären Amines, vorzugsweise Triethylamin, Trimethylamin, Diisopropylethylamin) in katalytischen oder stöchiometrischen Mengen oder in Überschuss oder anstelle des Lösungs- oder Verdünnungsmittels erfolgen. Durch weitere Umsetzung, beispielsweise mit geeignetem Alkylierungs- oder Arylierungsmitteln bzw. Methoden, können die erfindungsgemäßen Verbindungen der Formel (I) mit R⁴ ungleich Wasserstoff erhalten werden.

Die oben beschriebenen Methoden lassen sich mit den Halogenalkylsufanylarylderivaten mit n=0 (IVa), (IIIa), (IIa) bzw. (IVa) durchführen und führen zur Herstellung der Verbindungen der allgemeinen Formel (Ia). Durch Oxidation nach literaturbekannten Methoden lassen sich die Halogenalkylsulfinylarylderivaten mit n=1 (Ib), sowie die Halogenalkylsulfonylarylderivaten mit n=2 (Ic) herstellen.

Alternativ können Verbindungen der allgemeinen Formel (Ib) und (Ic) durch ähnliche wie die hier genannten Methoden in einer anderen Reihenfolge hergestellt werden, zum Beispiel durch Oxidation der Aniline der Formel (IVa) zu Sulfoxiden der Formel (IVb) und deren weiteren Umsetzung nach einem der unter Verfahren A beschriebenen Wegen.

### Verfahren B

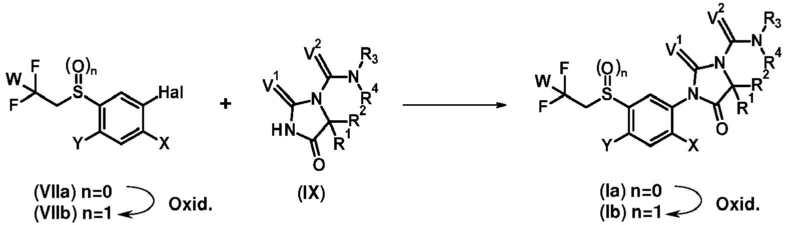

Eine alternative Herstellung der Verbindungen der Formel (I) stellt die Umsetzung von Halogeniden der Formel (VII) (vorzugsweise Hal=Br, Cl) mit Hydantoinen der Formel (IX) unter metall-vermittelten bzw. -katalysierten Reaktionsbedingungen dar. In der Literatur sind zahlreichen Methoden bekannt, zum Beispiel in WO2010/0210699 und J. Med. Chem. 2012, 55(19), 8236-8247, in denen Kupferoxid als Metallquelle bei höheren Temperaturen (beispielsweise 150 bis 160 °C in Dimethylacetamid) eingesetzt wird; oder in WO2011/136292, mit Kupferiodid als Metallquelle in Anwesenheit einer Base und eines Ligandes bei höheren Temperaturen (beispielsweise 110 °C in Toluol).

### Verfahren C

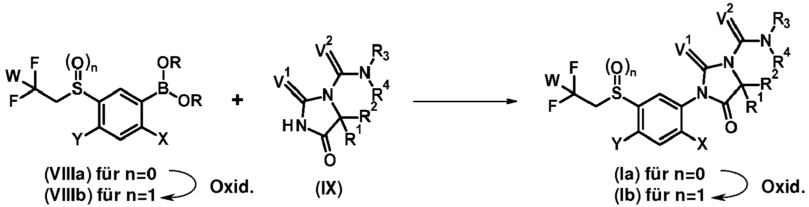

Ebenfalls wurde gefunden, dass die Umsetzung von Boronsäuren der Formel (VIII) - wobei R für Wasserstoff oder einer Alkylgruppe (bevorzugt Methyl, Ethyl) steht oder beide OR Gruppen bilden gemeinsam mit den Sauerstoffatomen und dem Boratom, an die sie gebunden sind, einen Heterocyclus (bevorzugt Pinakol) - mit Hydantoinen der Formel (IX) durch metall-vermittelten bzw. -katalysierten Reaktionen zur Herstellung der Verbindungen der Formel (I) dienen kann. Eine Übersicht solcher Reaktionen findet man in Synthesis 2011, 6, 829-856. Eine geeignete Metallquelle ist Kupfer(II)acetat in einem Gemisch aus Pyridin in Dichlormethan, bei Raumtemperatur, wie in WO2009/097997 für Hydantoine beschrieben.

Die Oxidation der Boronsäuren der Formel (VIIIa) oder deren Boronsäureester nach literaturbekannten Methoden, wie zum Beispiel mit Natriumperiodat, führt zu Sulfoxiden der Formel (VIIIb), die ebenfalls mit Verbindungen der Formel (IX) unter metall-vermittelten bzw. -katalysierten Reaktionsbedingungen umgesetzt werden können, sodass man zu den Zielverbindungen (Ib) gelangen kann.

### Reaktionen in der Mikrowelle

Bei der Durchführung der erfindungsgemäßen Verfahren kann gegebenenfalls jede für diese Reaktionen geeignete, handelsübliche Mikrowellenapparatur verwendet werden (z.B. Anton Paar Monowave 300, CEM Discover S, Biotage Initiator 60).

### Thionierung

Ein weiteres allgemeines Verfahren zur Herstellung der erfindungsgemäßen Verbindungen der allgemeinen Formel (Ia) oder (Ib), in denen V¹ und/oder V² für Schwefel steht, besteht in der Umwandlung der Carbonylgruppe entsprechender Vorstufen in die Thiocarbonylgruppe mit Hilfe geeigneter Schwefelungsreagenzien wie beispielsweise Phosphorpentasulfid oder Lawessons Reagenz in einem geeigneten Lösungsmittel, beispielsweise Pyridin, Xylol oder Cumol. Diese Variante ist in zahlreichen Publikationen beschrieben, z.B. in Biol. Med. Chem. 2012, 20(17), 5269-5276 für Hydantoine, US 3007927, DE 2554866 oder WO 2000026194 im Allgemeinen.

### Allgemeine Herstellverfahren für die Oxidation von Thioethern zu Sulfoxiden

Verbindungen der allgemeinen Formel (Ib) und (Ic) lassen sich durch Oxidation nach literaturbekannten Verfahren aus Verbindungen der allgemeinen Formel (Ia) herstellen, beispielsweise durch ein Oxidationsmittel in einem geeignetem Lösungs- und Verdünnungsmittel. Als Oxidationsmittel eignen sich zum Beispiel verdünnte Salpetersäure, Wasserstoffperoxid und Peroxycarbonsäuren, wie etwa *meta*-Chlorperbenzoesäure. Als Lösungsmittel eignen sich inerte organische Lösungsmittel, typischerweise Acetonitril und halogenierte Lösungsmittel wie Dichlormethan, Chloroform oder Dichlorethan.

Zur Erzeugung enantiomeren angereicherten Sulfoxide eignen sich eine Vielfalt von Methoden, wie von A.R. Maguire in ARKIVOC, 2011(i), 1-110, beschrieben: metall-katalysierte asymmetrische Oxidationen von Thioethern, zum Beispiel mit Titanium oder Vanadium als meistbenutzten Katalysatorquellen, in Form von Ti(OⁱPr₄) oder VO(acac)₂, zusammen mit einem chiralen Liganden und einem Oxidationsmittel wie tert-Butylwasserstoffperoxid (TBHP), 2-Phenylpropan-2-ylhydroperoxid (CHP) oder Wasserstoffperoxid; nicht-metall katalysierte asymmetrische Oxidationen durch Verwendung von chiralen Oxidationsmitteln oder chiralen Katalysatoren; elektrochemische oder biologische asymmetrische Oxidationen sowie kinetische Resolution von Sulfoxiden und nukleophilische Versetzung (nach Andersens Methode).

Die Enantiomere können auch aus dem Racemat gewonnen werden, in dem sie zum Beispiel präparativ auf einer chiralen HPLC getrennt werden.

### Erläuterung der Ausgangsstoffe und Zwischenprodukte

Aniline der Formel (IV), Halogenide der Formel (VII), Boronsäuren der Formel (VIII) und Hydantoine der Formel (IX) sind zentrale Bausteine, um die Verbindungen der Formel (I) herzustellen.

Die *Aniline der allgemeinen Formel (IV)* lassen sich in Verbindungen mit n=0 (IVa) und n=1 (IVb) unterteilen.

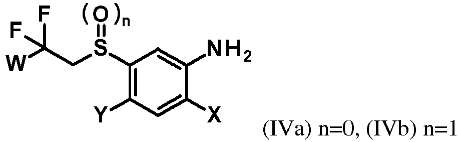

Aniline der Formel (IVa) sind literaturbekannt, z.B. aus JP 2007/284356, oder können anhand literaturbekannter Verfahren synthetisiert werden. Durch Oxidation nach literaturbekannten Methoden können Aniline der Formel (IVb) entstehen, z.B. wie in WO2013/092350 beschrieben.

### Halogenide der allgemeinen Formel (VII)

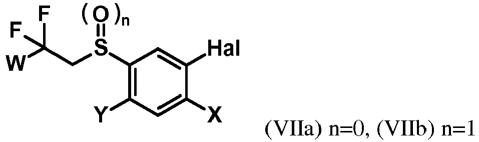

in welcher n, W, Y und X die oben angegebenen Bedeutungen haben und Hal für Chlor, Brom oder Iod steht, sind literaturbekannt aus WO 2007/034755, JP 2007/081019, JP 2007/284385, JP 2008/260706, JP 2008/308448, JP 2009/023910 oder WO 2012/176856 oder können anhand literaturbekannter Verfahren synthetisiert werden, die gegebenenfalls geringfügig modifiziert sein können.

Als Ausgangsstoffe für die Synthese der Iodide der allgemeinen Formel (VIIa) eignen sich Bromide derselben Formel, zum Beispiel in Halogenaustauschreaktionen nach literaturbekannten Methoden gegebenenfalls unter Metallkatalyse (s. H. Suzuki, Chem. Let. 1985, 3, 411-412; S. L. Buchwald, J. Amer. Chem. Soc. 2002, 124 (50), 14844-14845) . Ebenso ist die Synthese möglich ausgehend von Anilinen der Formel (IVa) unter Sandmeyers Reaktionsbedingungen, wie von E. B. Merkushev in Synthesis 1988, 12, 923-937, beschrieben.

### Boronsäuren der allgemeinen Formel (VIII)

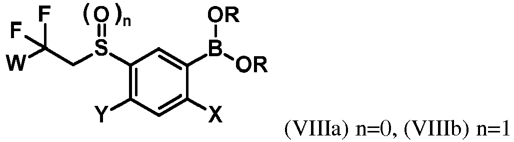

in welcher n, W, Y und X die oben angegebenen Bedeutungen haben und R für Wasserstoff oder einer Alkylgruppe (bevorzugt Methyl, Ethyl) steht oder beide OR Gruppen bilden gemeinsam mit den Sauerstoffatomen und dem Boratom, an die sie gebunden sind, einen Heterocyclus (bevorzugt Pinakol), sind literaturbekannt, z.B. aus WO2007/034755, JP2007/284385, JP2009/023910 und WO2012/176856 oder können anhand literaturbekannter Verfahren synthetisiert werden.

### Hydantoine der allgemeinen Formel (IX)

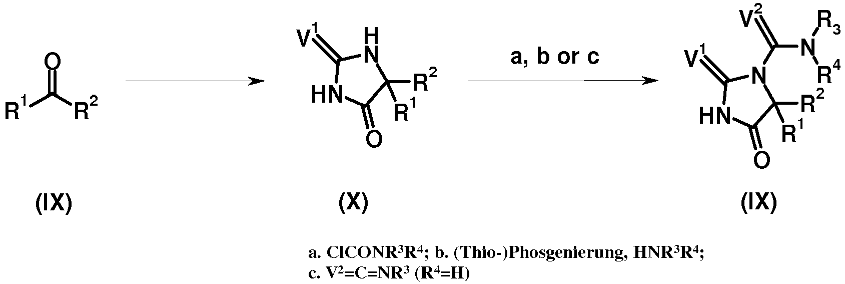

in welcher V¹, V², R¹, R², R³ und R⁴ die oben angegebenen Bedeutungen haben, sind kommerziell verfügbar, literaturbekannt oder können anhand literaturbekannten Verfahren synthetisiert werden, die gegebenenfalls geringfügig modifiziert sein können.

Als Ausgangsstoffe für die Synthese der Hydantoine der allgemeinen Formel (IX) eignen sich unsubstituierte Hydantoine der allgemeinen Formel (X), wie in WO2008/122352. Diese können mit Carbamoylchloriden acyliert werden (Methode a) oder in zwei Stufen durch Umsetzung mit Phosgen / Thiophosgen oder Äquivalenten wie Chlorcarbonsäuretrichlormethylester, Carbonsäureditrichlormethylester oder Chlorameisensäure-4-nitrophenylester und anschließender Reaktion mit Aminen (Methode b). Ebenso können die Hydantoine der Formel (X) auch mit entsprecheneden Iso(thio)cyanate umgesetzt werden (Methode c). Die Reste R⁴ (ungleich Wasserstoff) können auch nachträglich durch Alkylierung, Arylierung, etc. der Hydantoine (IX) (mit R⁴ gleich Wasserstoff) nach literaturbekannten Methoden eingeführt werden.

Hydantoine der Formel (X) mit V¹=O sind kommerziell verfügbar, literaturbekannt oder können anhand literaturbekannten Verfahren hergestellt werden. Hierfür werden häufig Ketone der allgemeinen Formel (IX) als Ausgansstoffe in einer sogenannten Bucherer-Berg modifizierten Strecker-Synthese verwendet, die über einer Kondensation mit Ammoniumcarbonat und Kaliumcyanid, meistens in einem alkoholenthaltenen Lösungsmittel oder Lösungsmittelgemisch bei erhöhter Temperatur, verläuft.

Für die Herstellung der Hydantoine der Formel (X) mit V¹=S eignen sich Hydantoine der Formel (X) mit V¹=O als Ausgangsmaterial in Thionierungsreaktionen nach literaturbekannten Methoden, wie z.B. in J. Med. Chem. 2012, 55(19), 8236-8247.

Von besonderem Interesse im Rahmen der vorliegenden Erfindung sind ferner Zwischenprodukte, die in den beschriebenen Verfahren und Methoden dargestellt sind. Diese Zwischenprodukte stellen weitere Gegenstände der Erfindung dar. Zusätzlich zu den vorstehend beschriebenen Zwischenprodukten sind weitere Zwischenprodukte im Folgenden beschrieben.

Ein weiterer Gegenstand der Erfindung ist eine Verbindung der Formel (II) 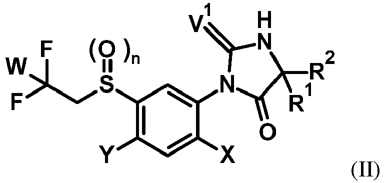 worin n, W, Y, X, V¹, R¹ und R² die vorstehend angegebenen Bedeutungen haben, insbesondere wie beschrieben in Ausgestaltung 1-1 oder I-A-1-1.

Eine weitere bevorzugte Ausgestaltung der Verbindungen der Formel (II) sind solche, in denen n gleich null ist. Hieraus ergeben sich verbindungen der Formel (IIa).

Eine weitere bevorzugte Ausgestaltung der Verbindungen der Formel (II) sind solche, in denen n gleich eins ist. Hieraus ergeben sich verbindungen der Formel (IIb).

Eine weitere Ausgestaltung der Verbindungen der Formel (II) sind solche, in denen n gleich zwei ist. Hieraus ergeben sich Verbindungen der Formel (IIc).

Bevorzugte Verbindungen der Formel (II), (IIa), (IIb) und (IIc) sind jeweils diejenigen, in welchen eine Kombination der vorstehend als bevorzugt aufgeführten Bedeutungen vorliegt, wobei jede vorstehend als bevorzugt beschriebene Ausgestaltung eine eigenständige Kombination darstellt, insbesondere wie beschrieben in Ausgestaltung 2-1, 2-2, I-A-2-1 oder I-A-2-2.

Besonders bevorzugte Verbindungen der Formel (II), (IIa), (IIb) und (IIc) sind diejenigen, in welchen eine Kombination der vorstehend als besonders bevorzugt aufgeführten Bedeutungen vorliegt, wobei jede vorstehend als besonders bevorzugt beschriebene Ausgestaltung eine eigenständige Kombination darstellt, insbesondere wie beschrieben in Ausgestaltung 3-1, 3-2, I-A-3-1 oder I-A-3-2.

Ganz besonders bevorzugte Verbindungen der Formel (II), (IIa), (IIb) und (IIc) sind diejenigen, in welchen eine Kombination der vorstehend als ganz besonders bevorzugt aufgeführten Bedeutungen vorliegt, wobei jede vorstehend als ganz besonders bevorzugt beschriebene Ausgestaltung eine eigenständige Kombination darstellt, insbesondere wie beschrieben in Ausgestaltung 4-1, 4-2, I-A-4-1 oder I-A-4-2.

Verbindungen der Formel (II), (IIa), (IIb) und (IIc) können in verschiedenen tautomeren Formen vorkommen. Diese Formen sind folglich mit umfasst, auch wenn sie nicht explizit dargestellt wurden.

Ein weiterer Gegenstand der Erfindung ist eine Verbindung der Formel (III) 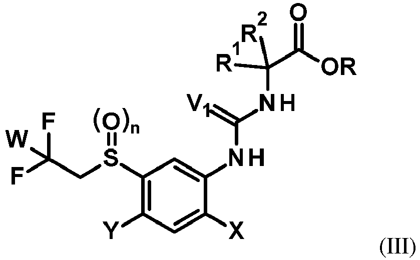 worin n, W, Y, X, V¹, R¹ und R² die vorstehend angegebenen Bedeutungen haben, insbesondere wie beschrieben in Ausgestaltung 1-1 oder I-A-1-1, und R für Wasserstoff oder einer Alkylgruppe steht (bevorzugt Methyl oder Ethyl).

Eine weitere bevorzugte Ausgestaltung der Verbindungen der Formel (III) sind solche, in denen n gleich null ist. Hieraus ergeben sich verbindungen der Formel (IIIa).

Eine weitere bevorzugte Ausgestaltung der Verbindungen der Formel (III) sind solche, in denen n gleich eins ist. Hieraus ergeben sich verbindungen der Formel (IIIb).

Eine weitere Ausgestaltung der Verbindungen der Formel (III) sind solche, in denen n gleich zwei ist. Hieraus ergeben sich Verbindungen der Formel (IIIc).

Bevorzugte Verbindungen der Formel (III), (IIIa), (IIIb) und (IIIc) sind jeweils diejenigen, in welchen eine Kombination der vorstehend als bevorzugt aufgeführten Bedeutungen vorliegt, wobei jede vorstehend als bevorzugt beschriebene Ausgestaltung eine eigenständige Kombination darstellt, insbesondere wie beschrieben in Ausgestaltung 2-1, 2-2, I-A-2-1 oder I-A-2-2.

Besonders bevorzugte Verbindungen der Formel (III), (IIIa), (IIIb) und (IIIc) sind diejenigen, in welchen eine Kombination der vorstehend als besonders bevorzugt aufgeführten Bedeutungen vorliegt, wobei jede vorstehend als besonders bevorzugt beschriebene Ausgestaltung eine eigenständige Kombination darstellt, insbesondere wie beschrieben in Ausgestaltung 3-1, 3-2, I-A-3-1 oder I-A-3-2.

Ganz besonders bevorzugte Verbindungen der Formel (III), (IIIa), (IIIb) und (IIIc) sind diejenigen, in welchen eine Kombination der vorstehend als ganz besonders bevorzugt aufgeführten Bedeutungen vorliegt, wobei jede vorstehend als ganz besonders bevorzugt beschriebene Ausgestaltung eine eigenständige Kombination darstellt, insbesondere wie beschrieben in Ausgestaltung 4-1, 4-2, I-A-4-1 oder I-A-4-2.

Verbindungen der Formel (III), (IIIa), (IIIb) und (IIIc) können in verschiedenen tautomeren Formen vorkommen. Diese Formen sind folglich mit umfasst, auch wenn sie nicht explizit dargestellt wurden.

Ein weiterer Gegenstand der Erfindung ist eine Verbindung der Formel (VI) 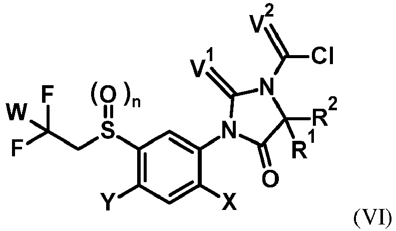 worin n, W, Y, X, V¹, V², R¹ und R² die vorstehend angegebenen Bedeutungen haben, insbesondere wie beschrieben in Ausgestaltung 1-1 oder I-A-1-1.

Eine weitere bevorzugte Ausgestaltung der Verbindungen der Formel (VI) sind solche, in denen n gleich null ist. Hieraus ergeben sich verbindungen der Formel (VIa).

Eine weitere bevorzugte Ausgestaltung der Verbindungen der Formel (VI) sind solche, in denen n gleich eins ist. Hieraus ergeben sich verbindungen der Formel (VIb).

Eine weitere Ausgestaltung der Verbindungen der Formel (VI) sind solche, in denen n gleich zwei ist. Hieraus ergeben sich Verbindungen der Formel (VIc).

Bevorzugte Verbindungen der Formel (VI), (VIa), (VIb) und (VIc) sind jeweils diejenigen, in welchen eine Kombination der vorstehend als bevorzugt aufgeführten Bedeutungen vorliegt, wobei jede vorstehend als bevorzugt beschriebene Ausgestaltung eine eigenständige Kombination darstellt, insbesondere wie beschrieben in Ausgestaltung 2-1, 2-2, I-A-2-1 oder I-A-2-2.

Besonders bevorzugte Verbindungen der Formel (VI), (VIa), (VIb) und (VIc) sind diejenigen, in welchen eine Kombination der vorstehend als besonders bevorzugt aufgeführten Bedeutungen vorliegt, wobei jede vorstehend als besonders bevorzugt beschriebene Ausgestaltung eine eigenständige Kombination darstellt, insbesondere wie beschrieben in Ausgestaltung 3-1, 3-2, I-A-3-1 oder I-A-3-2.

Ganz besonders bevorzugte Verbindungen der Formel (VI), (VIa), (VIb) und (VIc) sind diejenigen, in welchen eine Kombination der vorstehend als ganz besonders bevorzugt aufgeführten Bedeutungen vorliegt, wobei jede vorstehend als ganz besonders bevorzugt beschriebene Ausgestaltung eine eigenständige Kombination darstellt, insbesondere wie beschrieben in Ausgestaltung 4-1, 4-2, I-A-4-1 oder I-A-4-2.

Verbindungen der Formel (VI), (VIa), (VIb) und (VIc) können in verschiedenen tautomeren Formen vorkommen. Diese Formen sind folglich mit umfasst, auch wenn sie nicht explizit dargestellt wurden.

### Isomere

Die Verbindungen der Formel (I) können in Abhängigkeit von der Art der Substituenten als geometrische und/oder als optisch aktive Isomere oder entsprechende Isomerengemische in unterschiedlicher Zusammensetzung vorliegen. Diese Stereoisomere sind beispielsweise Enantiomere, Diastereomere, Atropisomere oder geometrische Isomere. Die Erfindung umfasst somit reine Stereoisomere als auch beliebige Gemische dieser Isomere.

### Verfahren und Verwendungen

Die Erfindung betrifft auch Verfahren zur Bekämpfung von tierischen Schädlingen, bei dem man Verbindungen der Formel (I) auf tierische Schädlinge und/oder ihren Lebensraum einwirken lässt. Bevorzugt wird die Bekämpfung der tierischen Schädlinge in der Land- und Forstwirtschaft und im Materialschutz durchgeführt. Hierunter vorzugsweise ausgeschlossen sind Verfahren zur chirurgischen oder therapeutischen Behandlung des menschlichen oder tierischen Körpers und Diagnostizierverfahren, die am menschlichen oder tierischen Körper vorgenommen werden.

Die Erfindung betrifft ferner die Verwendung der Verbindungen der Formel (I) als Schädlingsbekämpfungsmittel, insbesondere Pflanzenschutzmittel.

Im Rahmen der vorliegenden Anmeldung umfasst der Begriff Schädlingsbekämpfungsmittel immer auch den Begriff Pflanzenschutzmittel.

Die Verbindungen der Formel (I) eignen sich bei guter Pflanzenverträglichkeit, günstiger Warmblütertoxizität und guter Umweltverträglichkeit zum Schutz von Pflanzen und Pflanzenorganen vor biotischen und abiotischen Stressfaktoren, zur Steigerung der Ernteerträge, Verbesserung der Qualität des Erntegutes und zur Bekämpfung von tierischen Schädlingen, insbesondere Insekten, Spinnentieren, Helminthen, Nematoden und Mollusken, die in der Landwirtschaft, im Gartenbau, bei der Tierzucht, in Aquakulturen, in Forsten, in Gärten und Freizeiteinrichtungen, im Vorrats- und Materialschutz sowie auf dem Hygienesektor vorkommen. Sie können vorzugsweise als Schädlingsbekämpfungsmittel eingesetzt werden. Sie sind gegen normal sensible und resistente Arten sowie gegen alle oder einzelne Entwicklungsstadien wirksam. Zu den oben erwähnten Schädlingen gehören:
Schädlinge aus dem Stamm der Arthropoda, insbesondere aus der Klasse der Arachnida z.B. Acarus spp., z.B. Acarus siro, Aceria kuko, Aceria sheldoni, Aculops spp., Aculus spp., z.B. Aculus fockeui, Aculus schlechtendali, Amblyomma spp., Amphitetranychus viennensis, Argas spp., Boophilus spp., Brevipalpus spp., z.B. Brevipalpus phoenicis, Bryobia graminum, Bryobia praetiosa, Centruroides spp., Chorioptes spp., Dermanyssus gallinae, Dermatophagoides pteronyssinus, Dermatophagoides farinae, Dermacentor spp., Eotetranychus spp., z.B. Eotetranychus hicoriae, Epitrimerus pyri, Eutetranychus spp., z.B. Eutetranychus banksi, Eriophyes spp., z.B. Eriophyes pyri, Glycyphagus domesticus, Halotydeus destructor, Hemitarsonemus spp., z.B. Hemitarsonemus latus (=Polyphagotarsonemus latus), Hyalomma spp., Ixodes spp., Latrodectus spp., Loxosceles spp., Neutrombicula autumnalis, Nuphersa spp., Oligonychus spp., z.B. Oligonychus coniferarum, Oligonychus ilicis, Oligonychus indicus, Oligonychus mangiferus, Oligonychus pratensis, Oligonychus punicae, Oligonychus yothersi, Ornithodorus spp., Ornithonyssus spp., Panonychus spp., z.B. Panonychus citri (=Metatetranychus citri), Panonychus ulmi (=Metatetranychus ulmi), Phyllocoptruta oleivora, Platytetranychus multidigituli, Polyphagotarsonemus latus, Psoroptes spp., Rhipicephalus spp., Rhizoglyphus spp., Sarcoptes spp., Scorpio maurus, Steneotarsonemus spp., Steneotarsonemus spinki, Tarsonemus spp., z.B. Tarsonemus confusus, Tarsonemus pallidus, Tetranychus spp., z.B. Tetranychus canadensis, Tetranychus cinnabarinus, Tetranychus turkestani, Tetranychus urticae, Trombicula alfreddugesi, Vaejovis spp., Vasates lycopersici;
aus der Klasse der Chilopoda z.B. Geophilus spp., Scutigera spp.;
aus der Ordnung oder der Klasse der Collembola z.B. Onychiurus armatus; Sminthurus viridis;
aus der Klasse der Diplopoda z.B. Blaniulus guttulatus;
aus der Klasse der Insecta, z.B. aus der Ordnung der Blattodea z.B. Blatta orientalis, Blattella asahinai, Blattella germanica, Leucophaea maderae, Panchlora spp., Parcoblatta spp., Periplaneta spp., z.B. Periplaneta americana, Periplaneta australasiae, Supella longipalpa;
aus der Ordnung der Coleoptera z.B. Acalymma vittatum, Acanthoscelides obtectus, Adoretus spp., Agelastica alni, Agriotes spp., z.B. Agriotes linneatus, Agriotes mancus, Alphitobius diaperinus, Amphimallon solstitialis, Anobium punctatum, Anoplophora spp., Anthonomus spp., z.B. Anthonomus grandis, Anthrenus spp., Apion spp., Apogonia spp., Atomaria spp., z.B. Atomaria linearis, Attagenus spp., Baris caerulescens, Bruchidius obtectus, Bruchus spp., z.B. Bruchus pisorum, Bruchus rufimanus, Cassida spp., Cerotoma trifurcata, Ceutorrhynchus spp., z.B. Ceutorrhynchus assimilis, Ceutorrhynchus quadridens, Ceutorrhynchus rapae, Chaetocnema spp., z.B. Chaetocnema confinis, Chaetocnema denticulata, Chaetocnema ectypa, Cleonus mendicus, Conoderus spp., Cosmopolites spp., z.B. Cosmopolites sordidus, Costelytra zealandica, Ctenicera spp., Curculio spp., z.B. Curculio caryae, Curculio caryatrypes,Curculio obtusus, Curculio sayi, Cryptolestes ferrugineus, Cryptolestes pusillus, Cryptorhynchus lapathi, Cryptorhynchus mangiferae, Cylindrocopturus spp., Cylindrocopturus adspersus, Cylindrocopturus furnissi, Dermestes spp., Diabrotica spp., z.B. Diabrotica balteata, Diabrotica barberi, Diabrotica undecimpunctata howardi, Diabrotica undecimpunctata undecimpunctata, Diabrotica virgifera virgifera, Diabrotica virgifera zeae, Dichocrocis spp., Dicladispa armigera, Diloboderus spp., Epilachna spp., z.B. Epilachna borealis, Epilachna varivestis, Epitrix spp., z.B. Epitrix cucumeris, Epitrix fuscula, Epitrix hirtipennis, Epitrix subcrinita, Epitrix tuberis, Faustinus spp., Gibbium psylloides, Gnathocerus cornutus, Hellula undalis, Heteronychus arator, Heteronyx spp., Hylamorpha elegans, Hylotrupes bajulus, Hypera postica, Hypomeces squamosus, Hypothenemus spp., z.B. Hypothenemus hampei, Hypothenemus obscurus, Hypothenemus pubescens, Lachnosterna consanguinea, Lasioderma serricorne, Latheticus oryzae, Lathridius spp., Lema spp., Leptinotarsa decemlineata, Leucoptera spp., z.B. Leucoptera coffeella, Lissorhoptrus oryzophilus, Lixus spp., Luperomorpha xanthodera, Luperodes spp., Lyctus spp., Megascelis spp., Melanotus spp., z.B. Melanotus longulus oregonensis, Meligethes aeneus, Melolontha spp., z.B. Melolontha melolontha, Migdolus spp., Monochamus spp., Naupactus xanthographus, Necrobia spp., Niptus hololeucus, Oryctes rhinoceros, Oryzaephilus surinamensis, Oryzaphagus oryzae, Otiorhynchus spp., z.B. Otiorhynchus cribricollis, Otiorhynchus ligustici, Otiorhynchus ovatus, Otiorhynchus rugosostriarus, Otiorhynchus sulcatus, Oxycetonia jucunda, Phaedon cochleariae, Phyllophaga spp., Phyllophaga helleri, Phyllotreta spp., z.B. Phyllotreta armoraciae, Phyllotreta pusilla, Phyllotreta ramosa, Phyllotreta striolata, Popillia japonica, Premnotrypes spp., Prostephanus truncatus, Psylliodes spp., z.B. Psylliodes affinis, Psylliodes chrysocephala, Psylliodes punctulata, Ptinus spp., Rhizobius ventralis, Rhizopertha dominica, Sitophilus spp., z.B. Sitophilus granarius, Sitophilus linearis, Sitophilus oryzae, Sitophilus zeamais, Sphenophorus spp., Stegobium paniceum, Sternechus spp., z.B. Sternechus paludatus, Symphyletes spp., Tanymecus spp., z.B. Tanymecus dilaticollis, Tanymecus indicus, Tanymecus palliatus, Tenebrio molitor, Tenebrioides mauretanicus, Tribolium spp., z.B. Tribolium audax, Tribolium castaneum, Tribolium confusum, Trogoderma spp., Tychius spp., Xylotrechus spp., Zabrus spp., z.B. Zabrus tenebrioides;
aus der Ordnung der Diptera z.B. Aedes spp., z.B. Aedes aegypti, Aedes albopictus, Aedes sticticus, Aedes vexans, Agromyza spp., z.B. Agromyza frontella, Agromyza parvicornis, Anastrepha spp., Anopheles spp., z.B. Anopheles quadrimaculatus, Anopheles gambiae, Asphondylia spp., Bactrocera spp., z.B. Bactrocera cucurbitae, Bactrocera dorsalis, Bactrocera oleae, Bibio hortulanus, Calliphora erythrocephala, Calliphora vicina, Ceratitis capitata, Chironomus spp., Chrysomya spp., Chrysops spp., Chrysozona pluvialis, Cochliomya spp., Contarinia spp., z.B. Contarinia johnsoni, Contarinia nasturtii, Contarinia pyrivora, Contarinia schulzi, Contarinia sorghicola, Contarinia tritici,Cordylobia anthropophaga, Cricotopus sylvestris, Culex spp., z.B. Culex pipiens, Culex quinquefasciatus, Culicoides spp., Culiseta spp., Cuterebra spp., Dacus oleae, Dasineura spp., z.B. Dasineura brassicae, Delia spp., z.B. Delia antiqua, Delia coarctata, Delia florilega, Delia platura, Delia radicum, Dermatobia hominis, Drosophila spp., z.B. Drosphila melanogaster, Drosophila suzukii, Echinocnemus spp., Fannia spp., Gasterophilus spp., Glossina spp., Haematopota spp., Hydrellia spp., Hydrellia griseola, Hylemya spp., Hippobosca spp., Hypoderma spp., Liriomyza spp., z.B. Liriomyza brassicae, Liriomyza huidobrensis, Liriomyza sativae, Lucilia spp., z.B. Lucilia cuprina, Lutzomyia spp., Mansonia spp., Musca spp., z.B. Musca domestica, Musca domestica vicina, Oestrus spp., Oscinella frit, Paratanytarsus spp., Paralauterborniella subcincta, Pegomya spp., z.B. Pegomya betae, Pegomya hyoscyami, Pegomya rubivora, Phlebotomus spp., Phorbia spp., Phormia spp., Piophila casei, Prodiplosis spp., Psila rosae, Rhagoletis spp., z.B. Rhagoletis cingulata, Rhagoletis completa, Rhagoletis fausta, Rhagoletis indifferens, Rhagoletis mendax, Rhagoletis pomonella, Sarcophaga spp., Simulium spp., z.B. Simulium meridionale, Stomoxys spp., Tabanus spp., Tetanops spp., Tipula spp., z.B. Tipula paludosa, Tipula simplex;
aus der Ordnung der Hemiptera z.B. Acizzia acaciaebaileyanae, Acizzia dodonaeae, Acizzia uncatoides, Acrida turrita, Acyrthosipon spp., z.B. Acyrthosiphon pisum, Acrogonia spp., Aeneolamia spp., Agonoscena spp., Aleyrodes proletella, Aleurolobus barodensis, Aleurothrixus floccosus, Allocaridara malayensis, Amrasca spp., z.B. Amrasca bigutulla, Amrasca devastans, Anuraphis cardui, Aonidiella spp., z.B. Aonidiella aurantii, Aonidiella citrina, Aonidiella inornata, Aphanostigma piri, Aphis spp., z.B. Aphis citricola, Aphis craccivora, Aphis fabae, Aphis forbesi, Aphis glycines, Aphis gossypii, Aphis hederae, Aphis illinoisensis, Aphis middletoni, Aphis nasturtii, Aphis nerii, Aphis pomi, Aphis spiraecola, Aphis viburniphila, Arboridia apicalis, Arytainilla spp., Aspidiella spp., Aspidiotus spp., z.B. Aspidiotus nerii, Atanus spp., Aulacorthum solani, Bemisia tabaci, Blastopsylla occidentalis, Boreioglycaspis melaleucae, Brachycaudus helichrysi, Brachycolus spp., Brevicoryne brassicae, Cacopsylla spp., z.B. Cacopsylla pyricola, Calligypona marginata, Carneocephala fulgida, Ceratovacuna lanigera, Cercopidae, Ceroplastes spp., Chaetosiphon fragaefolii, Chionaspis tegalensis, Chlorita onukii, Chondracris rosea, Chromaphis juglandicola, Chrysomphalus ficus, Cicadulina mbila, Coccomytilus halli, Coccus spp., z.B. Coccus hesperidum, Coccus longulus, Coccus pseudomagnoliarum, Coccus viridis, Cryptomyzus ribis, Cryptoneossa spp., Ctenarytaina spp., Dalbulus spp., Dialeurodes citri, Diaphorina citri, Diaspis spp., Drosicha spp., Dysaphis spp., z.B. Dysaphis apiifolia, Dysaphis plantaginea, Dysaphis tulipae, Dysmicoccus spp., Empoasca spp., z.B. Empoasca abrupta, Empoasca fabae, Empoasca maligna, Empoasca solana, Empoasca stevensi, Eriosoma spp., z.B. Eriosoma americanum, Eriosoma lanigerum, Eriosoma pyricola, Erythroneura spp., Eucalyptolyma spp., Euphyllura spp., Euscelis bilobatus, Ferrisia spp., Geococcus coffeae, Glycaspis spp., Heteropsylla cubana, Heteropsylla spinulosa, Homalodisca coagulata, Hyalopterus arundinis, Hyalopterus pruni, Icerya spp., z.B. Icerya purchasi, Idiocerus spp., Idioscopus spp., Laodelphax striatellus, Lecanium spp., z.B. Lecanium corni (=Parthenolecanium corni), Lepidosaphes spp., z.B. Lepidosaphes ulmi, Lipaphis erysimi, Lycorma delicatula, Macrosiphum spp., z.B. Macrosiphum euphorbiae, Macrosiphum lilii, Macrosiphum rosae, Macrosteles facifrons, Mahanarva spp., Melanaphis sacchari, Metcalfiella spp., Metcalfa pruinosa, Metopolophium dirhodum, Monellia costalis, Monelliopsis pecanis, Myzus spp., z.B. Myzus ascalonicus, Myzus cerasi, Myzus ligustri, Myzus ornatus, Myzus persicae,. Myzus nicotianae, Nasonovia ribisnigri, Nephotettix spp., z.B. Nephotettix cincticeps,, Nephotettix nigropictus, Nilaparvata lugens, Oncometopia spp., Orthezia praelonga, Oxya chinensis, Pachypsylla spp., Parabemisia myricae, Paratrioza spp., z.B. Paratrioza cockerelli, Parlatoria spp., Pemphigus spp., z.B. Pemphigus bursarius, Pemphigus populivenae, Peregrinus maidis, Phenacoccus spp., z.B. Phenacoccus madeirensis, Phloeomyzus passerinii, Phorodon humuli, Phylloxera spp., z.B. Phylloxera devastatrix, Phylloxera notabilis, Pinnaspis aspidistrae, Planococcus spp., z.B. Planococcus citri, Prosopidopsylla flava, Protopulvinaria pyriformis, Pseudaulacaspis pentagona, Pseudococcus spp., z.B. Pseudococcus calceolariae, Pseudococcus comstocki, Pseudococcus longispinus, Pseudococcus maritimus, Pseudococcus viburni, Psyllopsis spp., Psylla spp., z.B. Psylla buxi, Psylla mali, Psylla pyri, Pteromalus spp., Pyrilla spp., Quadraspidiotus spp., z.B. Quadraspidiotus juglansregiae, Quadraspidiotus ostreaeformis, Quadraspidiotus perniciosus, Quesada gigas, Rastrococcus spp., Rhopalosiphum spp., z.B. Rhopalosiphum maidis, Rhopalosiphum oxyacanthae, Rhopalosiphum padi, Rhopalosiphum rufiabdominale, Saissetia spp., z.B. Saissetia coffeae, Saissetia miranda, Saissetia neglecta, Saissetia oleae, Scaphoideus titanus, Schizaphis graminum, Selenaspidus articulatus, Sitobion avenae, Sogata spp., Sogatella furcifera, Sogatodes spp., Stictocephala festina, Siphoninus phillyreae, Tenalaphara malayensis,Tetragonocephela spp., Tinocallis caryaefoliae, Tomaspis spp., Toxoptera spp., z.B. Toxoptera aurantii, Toxoptera citricidus, Trialeurodes vaporariorum, Trioza spp., z.B. Trioza diospyri, Typhlocyba spp., Unaspis spp., Viteus vitifolii, Zygina spp.;
aus der Unterordnung der Heteroptera z.B. Anasa tristis, Antestiopsis spp., Boisea spp., Blissus spp., Calocoris spp., Campylomma livida, Cavelerius spp., Cimex spp., z.B. Cimex adjunctus, Cimex hemipterus, Cimex lectularius, Cimex pilosellus, Collaria spp., Creontiades dilutus, Dasynus piperis, Dichelops furcatus, Diconocoris hewetti, Dysdercus spp., Euschistus spp., z.B. Euschistus heros, Euschistus servus, Euschistus tristigmus, Euschistus variolarius, Eurygaster spp., Halyomorpha halys, Heliopeltis spp., Horcias nobilellus, Leptocorisa spp., Leptocorisa varicornis, Leptoglossus occidentalis, Leptoglossus phyllopus, Lygocoris spp., z.B. Lygocoris pabulinus, Lygus spp., z.B. Lygus elisus, Lygus hesperus, Lygus lineolaris, Macropes excavatus, Monalonion atratum, Nezara spp., z.B. Nezara viridula, Oebalus spp., Piesma quadrata, Piezodorus spp., z.B. Piezodorus guildinii, Psallus spp., Pseudacysta persea, Rhodnius spp., Sahlbergella singularis, Scaptocoris castanea, Scotinophora spp., Stephanitis nashi, Tibraca spp., Triatoma spp.;
aus der Ordnung der Hymenoptera z.B. Acromyrmex spp., Athalia spp., z.B. Athalia rosae, Atta spp., Diprion spp., z.B. Diprion similis, Hoplocampa spp., z.B. Hoplocampa cookei, Hoplocampa testudinea, Lasius spp., Linepithema humile, Monomorium pharaonis, Sirex spp., Solenopsis invicta, Tapinoma spp., Urocerus spp., Vespa spp., z.B. Vespa crabro, Xeris spp.;
aus der Ordnung der Isopoda z.B. Armadillidium vulgare, Oniscus asellus, Porcellio scaber;
aus der Ordnung der Isoptera z.B. Coptotermes spp., z.B. Coptotermes formosanus, Cornitermes cumulans, Cryptotermes spp., Incisitermes spp., Microtermes obesi, Odontotermes spp., Reticulitermes spp., z.B. Reticulitermes flavipes, Reticulitermes hesperus;
aus der Ordnung der Lepidoptera z.B. Achroia grisella, Acronicta major, Adoxophyes spp., z.B. Adoxophyes orana, Aedia leucomelas, Agrotis spp., z.B. Agrotis segetum, Agrotis ipsilon, Alabama spp., z.B. Alabama argillacea, Amyelois transitella, Anarsia spp., Anticarsia spp., z.B. Anticarsia gemmatalis, Argyroploce spp., Barathra brassicae, Borbo cinnara, Bucculatrix thurberiella, Bupalus piniarius, Busseola spp., Cacoecia spp., Caloptilia theivora, Capua reticulana, Carpocapsa pomonella, Carposina niponensis, Cheimatobia brumata, Chilo spp., z.B. Chilo plejadellus, Chilo suppressalis, Choristoneura spp., Clysia ambiguella, Cnaphalocerus spp., Cnaphalocrocis medinalis, Cnephasia spp., Conopomorpha spp., Conotrachelus spp., Copitarsia spp., Cydia spp., z.B. Cydia nigricana, Cydia pomonella, Dalaca noctuides, Diaphania spp., Diatraea saccharalis, Earias spp., Ecdytolopha aurantium, Elasmopalpus lignosellus, Eldana saccharina, Ephestia spp., z.B. Ephestia elutella, Ephestia kuehniella, Epinotia spp., Epiphyas postvittana, Etiella spp., Eulia spp., Eupoecilia ambiguella, Euproctis spp., z.B. Euproctis chrysorrhoea, Euxoa spp., Feltia spp., Galleria mellonella, Gracillaria spp., Grapholitha spp., z.B. Grapholita molesta, Grapholita prunivora, Hedylepta spp., Helicoverpa spp., z.B. Helicoverpa armigera, Helicoverpa zea, Heliothis spp., z.B. Heliothis virescens Hofmannophila pseudospretella, Homoeosoma spp., Homona spp., Hyponomeuta padella, Kakivoria flavofasciata, Laphygma spp., Leucinodes orbonalis, Leucoptera spp., z.B. Leucoptera coffeella, Lithocolletis spp., z.B. Lithocolletis blancardella, Lithophane antennata, Lobesia spp., z.B. Lobesia botrana, Loxagrotis albicosta, Lymantria spp., z.b. Lymantria dispar, Lyonetia spp., z.B. Lyonetia clerkella, Malacosoma neustria, Maruca testulalis, Mamestra brassicae, Melanitis leda, Mocis spp., Monopis obviella, Mythimna separata, Nemapogon cloacellus, Nymphula spp., Oiketicus spp., Oria spp., Orthaga spp., Ostrinia spp., z.B. Ostrinia nubilalis, Oulema melanopus, Oulema oryzae, Panolis flammea, Parnara spp., Pectinophora spp., z.B. Pectinophora gossypiella, Perileucoptera spp., Phthorimaea spp., z.B. Phthorimaea operculella, Phyllocnistis citrella, Phyllonorycter spp., z.B. Phyllonorycter blancardella, Phyllonorycter crataegella, Pieris spp., z.B. Pieris rapae, Platynota stultana, Plodia interpunctella, Plusia spp., Plutella xylostella (=Plutella maculipennis), Prays spp., Prodenia spp., Protoparce spp., Pseudaletia spp., z.B. Pseudaletia unipuncta, Pseudoplusia includens, Pyrausta nubilalis, Rachiplusia nu, Schoenobius spp., z.B. Schoenobius bipunctifer, Scirpophaga spp., z.B. Scirpophaga innotata, Scotia segetum, Sesamia spp., z.B. Sesamia inferens, Sparganothis spp., Spodoptera spp., z.b. Spodoptera eradiana, Spodoptera exigua, Spodoptera frugiperda, Spodoptera praefica, Stathmopoda spp., Stomopteryx subsecivella, Synanthedon spp., Tecia solanivora, Thermesia gemmatalis, Tinea cloacella, Tinea pellionella, Tineola bisselliella, Tortrix spp., Trichophaga tapetzella, Trichoplusia spp., z.B. Trichoplusia ni, Tryporyza incertulas, Tuta absoluta, Virachola spp.;
aus der Ordnung der Orthoptera oder Saltatoria z.B. Acheta domesticus, Dichroplus spp., Gryllotalpa spp., z.B. Gryllotalpa gryllotalpa, Hieroglyphus spp., Locusta spp., z.B. Locusta migratoria, Melanoplus spp., z.B. Melanoplus devastator, Paratlanticus ussuriensis, Schistocerca gregaria;
aus der Ordnung der Phthiraptera z.B. Damalinia spp., Haematopinus spp., Linognathus spp., Pediculus spp., Phylloxera vastatrix, Phthirus pubis, Trichodectes spp.;
aus der Ordnung der Psocoptera z.B. Lepinotus spp., Liposcelis spp.;
aus der Ordnung der Siphonaptera z.B. Ceratophyllus spp., Ctenocephalides spp., z.B. Ctenocephalides canis, Ctenocephalides felis, Pulex irritans, Tunga penetrans, Xenopsylla cheopis;
aus der Ordnung der Thysanoptera z.B. Anaphothrips obscurus, Baliothrips biformis, Drepanothrips reuteri, Enneothrips flavens, Frankliniella spp., z.B. Frankliniella fusca, Frankliniella occidentalis, Frankliniella schultzei, Frankliniella tritici, Frankliniella vaccinii, Frankliniella williamsi, Heliothrips spp., Hercinothrips femoralis, Rhipiphorothrips cruentatus, Scirtothrips spp., Taeniothrips cardamomi, Thrips spp., z.B. Thrips palmi, Thrips tabaci;
aus der Ordnung der Zygentoma (= Thysanura), z. B. Ctenolepisma spp., Lepisma saccharina, Lepismodes inquilinus, Thermobia domestica;
aus der Klasse der Symphyla z.B. Scutigerella spp., z.B. Scutigerella immaculata;
Schädlinge aus dem Stamm der Mollusca, insbesondere aus der Klasse der Bivalvia, z.B. Dreissena spp.;
sowie aus der Klasse der Gastropoda z.B. Arion spp., z.B. Arion ater rufus, Biomphalaria spp., Bulinus spp., Deroceras spp., z.B. Deroceras laeve, Galba spp., Lymnaea spp., Oncomelania spp., Pomacea spp., Succinea spp.;
Tier- und Humanparasiten aus den Stämmen der Platyhelminthes und Nematoda, z.B. Aelurostrongylus spp., Amidostomum spp., Ancylostoma spp, Angiostrongylus spp., Anisakis spp., Anoplocephala spp., Ascaris spp., Ascaridia spp., Baylisascaris spp., Brugia spp., Bunostomum spp., Capillaria spp., Chabertia spp., Clonorchis spp., Cooperia spp., Crenosoma spp., Cyathostoma spp., Dicrocoelium spp., Dictyocaulus spp., Diphyllobothrium spp., Dipylidium spp., Dirofilaria spp., Dracunculus spp., Echinococcus spp., Echinostoma spp., Enterobius spp., Eucoleus spp., Fasciola spp., Fascioloides spp., Fasciolopsis spp., Filaroides spp., Gongylonema spp., Gyrodactylus spp., Habronema spp., Haemonchus spp., Heligmosomoides spp., Heterakis spp., Hymenolepis spp., Hyostrongylus spp., Litomosoides spp., Loa spp., Metastrongylus spp., Metorchis spp., Mesocestoides spp., Moniezia spp., Muellerius spp., Necator spp., Nematodirus spp., Nippostrongylus spp., Oesophagostomum spp., Ollulanus spp., Onchocerca spp, Opisthorchis spp., Oslerus spp., Ostertagia spp., Oxyuris spp., Paracapillaria spp., Parafilaria spp., Paragonimus spp., Paramphistomum spp., Paranoplocephala spp., Parascaris spp., Passalurus spp., Protostrongylus spp., Schistosoma spp., Setaria spp., Spirocerca spp., Stephanofilaria spp., Stephanurus spp., Strongyloides spp., Strongylus spp., Syngamus spp., Taenia spp., Teladorsagia spp., Thelazia spp., Toxascaris spp., Toxocara spp., Trichinella spp., Trichobilharzia spp., Trichostrongylus spp., Trichuris spp., Uncinaria spp., Wuchereria spp.;
Pflanzenschädlinge aus dem Stamm der Nematoda, d.h. pflanzenparasitäre Nematoden, insbesondere Aglenchus spp., z.B. Aglenchus agricola, Anguina spp., z.B. Anguina tritici, Aphelenchoides spp., z.B. Aphelenchoides arachidis, Aphelenchoides fragariae, Belonolaimus spp., z.B. Belonolaimus gracilis, Belonolaimus longicaudatus, Belonolaimus nortoni, Bursaphelenchus spp., z.B. Bursaphelenchus cocophilus, Bursaphelenchus eremus, Bursaphelenchus xylophilus, Cacopaurus spp., z.B. Cacopaurus pestis, Criconemella spp., z.B. Criconemella curvata, Criconemella onoensis, Criconemella ornata, Criconemella rusium, Criconemella xenoplax (= Mesocriconema xenoplax), Criconemoides spp., z.B. Criconemoides ferniae, Criconemoides onoense, Criconemoides ornatum, Ditylenchus spp., z.B. Ditylenchus dipsaci, Dolichodorus spp., Globodera spp., z.B. Globodera pallida, Globodera rostochiensis, Helicotylenchus spp., z.B. Helicotylenchus dihystera, Hemicriconemoides spp., Hemicycliophora spp., Heterodera spp., z.B. Heterodera avenae, Heterodera glycines, Heterodera schachtii, Hoplolaimus spp., Longidorus spp., z.B. Longidorus africanus, Meloidogyne spp., z.B. Meloidogyne chitwoodi, Meloidogyne fallax, Meloidogyne hapla, Meloidogyne incognita, Meloinema spp., Nacobbus spp., Neotylenchus spp., Paraphelenchus spp., Paratrichodorus spp., z.B. Paratrichodorus minor, Pratylenchus spp., z.B. Pratylenchus penetrans, Pseudohalenchus spp., Psilenchus spp., Punctodera spp., Quinisulcius spp., Radopholus spp., z.B. Radopholus citrophilus, Radopholus similis, Rotylenchulus spp., Rotylenchus spp., Scutellonema spp., Subanguina spp., Trichodorus spp., z.B. Trichodorus obtusus, Trichodorus primitivus, Tylenchorhynchus spp., z.B. Tylenchorhynchus annulatus, Tylenchulus spp., z.B. Tylenchulus semipenetrans, Xiphinema spp., z.B. Xiphinema index.

Weiterhin lässt sich aus dem Unterreich der Protozoa die Ordnung der Coccidia z.B. Eimeria spp. bekämpfen.

### Nematoden

Der Begriff "Nematoden" umfasst im vorliegenden Zusammenhang alle Arten des Stammes Nematoda und hierbei insbesondere Arten, die Pflanzen oder Pilze (zum Beispiel Arten der Ordnung Aphelenchida, Meloidogyne, Tylenchida und andere) oder auch Menschen und Tiere (zum Beispiel Arten der Ordnungen Trichinellida, Tylenchida, Rhabditida und Spirurida) parasitieren oder in bzw. an diesen Lebewesen Schädigungen verursachen, sowie andere parasitäre Helminthen.

Ein Nematizid im Pflanzenschutz, wie hier beschrieben, besitzt die Fähigkeit, Nematoden zu kontrollieren.

Der Begriff "Nematoden kontrollieren" bedeutet das Abtöten der Nematoden oder das Verhindern oder Erschweren ihrer Entwicklung bzw. ihres Wachstums oder das Verhindern oder Erschweren ihres Eindringens in oder ihres Saugens am pflanzlichen Gewebe.

Dabei wird die Wirksamkeit der Verbindungen durch einen Vergleich von Mortalitäten, Gallenbildung, Zystenbildung, Nematodendichte pro Bodenvolumen, Nematodendichte pro Wurzel, Anzahl von Nematodeneier pro Bodenvolumen, Mobilität (Beweglichkeit) der Nematoden zwischen einer mit der Verbindung der Formel (I) behandelten Pflanze, Pflanzenteil oder dem behandelten Boden und einer unbehandelten Pflanze, Pflanzenteil oder unbehandelten Boden (100 %) ermittelt. Vorzugsweise wird eine Verringerung um 25-50 % im Vergleich mit einer unbehandelten Pflanze, Pflanzenteil oder unbehandelten Boden, besonders bevorzugt eine Verringerung um 51 - 79 % und ganz besonders bevorzugt das vollständige Abtöten oder die vollständige Verhinderung von Entwicklung und Wachstum der Nematoden durch eine Verringerung um 80 bis 100 % erreicht. Die Kontrolle von Nematoden, wie hier beschreiben, beinhaltet ebenso die Kontrolle der Nematoden-Vermehrung (Entwicklung von Zysten und/oder Eier). Verbindungen der Formel (I) können ebenso verwendet werden, um die Pflanzen oder Tiere gesund zu erhalten und können kurativ, präventiv oder systemisch zur Nematoden-Kontrolle eingesetzt werden.

Dem Fachmann sind Methoden bekannt, wie Mortalitäten, Gallenbildung, Zystenbildung, Nematodendichte pro Bodenvolumen, Nematodendichte pro Wurzel, Anzahl von Nematodeneier pro Bodenvolumen, Mobilität (Beweglichkeit) der Nematoden bestimmt werden.

Die Verwendung einer Verbindung der Formel (I) kann die Pflanze gesund erhalten und beinhaltet ebenso eine Reduktion der von Nematoden hervorgerufenen Schäden sowie eine Erhöhung der Erntemenge.

Der Begriff "Nematoden" bezieht sich im vorliegenden Zusammenhang auf Pflanzennematoden, unter die man alle Nematoden zusammenfasst, die Pflanzen schädigen. Pflanzennematoden umfassen pflanzenparasitäre Nematoden und im Boden lebende Nematoden. Zu den pflanzenparasitären Nematoden zählen Ektoparasiten wie Xiphinema spp., Longidorus spp. und Trichodorus spp.; Halbparasiten wie Tylenchulus spp.; migratorische Endoparasiten wie Pratylenchus spp., Radopholus spp. und Scutellonema spp.; ortsgebundene Parasiten wie Heterodera spp., Globodera spp. und Meloidogyne spp., sowie Stängel- und Blattendoparasiten wie Ditylenchus spp., Aphelenchoides spp. und Hirschmaniella spp.. Besonders schädliche wurzelparasitäre Bodennematoden sind zum Beispiel zystenbildende Nematoden der Gattungen Heterodera oder Globodera, und/oder Wurzelgallennematoden der Gattung Meloidogyne. Schädliche Arten dieser Gattungen sind zum Beispiel Meloidogyne incognita, Heterodera glycines (Sojabohnenzystennematode), Globodera pallida und Globodera rostochiensis (Kartoffelzystennematode), wobei diese Arten wirksam mit dem im vorliegenden Text beschriebenen Verbindungen bekämpft werden. Die Verwendung der im vorliegenden Text beschriebenen Verbindungen ist jedoch keineswegs auf diese Gattungen oder Arten beschränkt, sondern erstreckt sich in gleicher Weise auch auf andere Nematoden.

Zu den Pflanzennematoden zählen z.B. Aglenchus agricola, Anguina tritici, Aphelenchoides arachidis, Aphelenchoides fragaria und die Stängel- und Blattendoparasiten Aphelenchoides spp., Belonolaimus gracilis, Belonolaimus longicaudatus, Belonolaimus nortoni, Bursaphelenchus cocophilus, Bursaphelenchus eremus, Bursaphelenchus xylophilus und Bursaphelenchus spp., Cacopaurus pestis, Criconemella curvata, Criconemella onoensis, Criconemella ornata, Criconemella rusium, Criconemella xenoplax (= Mesocriconema xenoplax) und Criconemella spp.,

Criconemoides ferniae, Criconemoides onoense, Criconemoides ornatum und Criconemoides spp., Ditylenchus destructor, Ditylenchus dipsaci, Ditylenchus myceliophagus sowie die Stängel- und Blattendoparasiten Ditylenchus spp., Dolichodorus heterocephalus, Globodera pallida (=Heterodera pallida), Globodera rostochiensis (Kartoffelzystennematode), Globodera solanacearum, Globodera tabacum, Globodera virginia und die ortsgebundenen zystenbildenden Parasiten Globodera spp., Helicotylenchus digonicus, Helicotylenchus dihystera, Helicotylenchus erythrine, Helicotylenchus multicinctus, Helicotylenchus nannus, Helicotylenchus pseudorobustus und Helicotylenchus spp., Hemicriconemoides, Hemicycliophora arenaria, Hemicycliophora nudata, Hemicycliophora parvana, Heterodera avenae, Heterodera cruciferae, Heterodera glycines (Sojabohnenzystennematode), Heterodera oryzae, Heterodera schachtii, Heterodera zeae und die ortsgebundenen zystenbildenden Parasiten Heterodera spp., Hirschmaniella gracilis, Hirschmaniella oryzae, Hirschmaniella spinicaudata und die Stängel- und Blattendoparasiten Hirschmaniella spp., Hoplolaimus aegyptii, Hoplolaimus californicus, Hoplolaimus columbus, Hoplolaimus galeatus, Hoplolaimus indicus, Hoplolaimus magnistylus, Hoplolaimus pararobustus, Longidorus africanus, Longidorus breviannulatus, Longidorus elongatus, Longidorus laevicapitatus, Longidorus vineacola und die Ektoparasiten Longidorus spp., Meloidogyne acronea, Meloidogyne africana, Meloidogyne arenaria, Meloidogyne arenaria thamesi, Meloidogyne artiella, Meloidogyne chitwoodi, Meloidogyne coffeicola, Meloidogyne ethiopica, Meloidogyne exigua, Meloidogyne fallax, Meloidogyne graminicola, Meloidogyne graminis, Meloidogyne hapla, Meloidogyne incognita, Meloidogyne incognita acrita, Meloidogyne javanica, Meloidogyne kikuyensis, Meloidogyne minor, Meloidogyne naasi, Meloidogyne paranaensis, Meloidogyne thamesi und die ortsgebundenen Parasiten Meloidogyne spp., Meloinema spp., Nacobbus aberrans, Neotylenchus vigissi, Paraphelenchus pseudoparietinus, Paratrichodorus allius, Paratrichodorus lobatus, Paratrichodorus minor, Paratrichodorus nanus, Paratrichodorus porosus, Paratrichodorus teres und Paratrichodorus spp., Paratylenchus hamatus, Paratylenchus minutus, Paratylenchus projectus und Paratylenchus spp., Pratylenchus agilis, Pratylenchus alleni, Pratylenchus andinus, Pratylenchus brachyurus, Pratylenchus cerealis, Pratylenchus coffeae, Pratylenchus crenatus, Pratylenchus delattrei, Pratylenchus giibbicaudatus, Pratylenchus goodeyi, Pratylenchus hamatus, Pratylenchus hexincisus, Pratylenchus loosi, Pratylenchus neglectus, Pratylenchus penetrans,Pratylenchus pratensis, Pratylenchus scribneri, Pratylenchus teres, Pratylenchus thornei, Pratylenchus vulnus, Pratylenchus zeae und die migratorischen Endoparasiten Pratylenchus spp., Pseudohalenchus minutus, Psilenchus magnidens, Psilenchus tumidus, Punctodera chalcoensis, Quinisulcius acutus, Radopholus citrophilus, Radopholus similis, die migratorischen Endoparasiten Radopholus spp., Rotylenchulus borealis, Rotylenchulus parvus, Rotylenchulus reniformis und Rotylenchulus spp., Rotylenchus laurentinus, Rotylenchus macrodoratus, Rotylenchus robustus, Rotylenchus uniformis und Rotylenchus spp., Scutellonema brachyurum, Scutellonema bradys, Scutellonema clathricaudatum und die migratorischen Endoparasiten Scutellonema spp., Subanguina radiciola, Tetylenchus nicotianae, Trichodorus cylindricus, Trichodorus minor, Trichodorus primitivus, Trichodorus proximus, Trichodorus similis, Trichodorus sparsus und die Ektoparasiten Trichodorus spp., Tylenchorhynchus agri, Tylenchorhynchus brassicae, Tylenchorhynchus clarus, Tylenchorhynchus claytoni, Tylenchorhynchus digitatus, Tylenchorhynchus ebriensis, Tylenchorhynchus maximus, Tylenchorhynchus nudus, Tylenchorhynchus vulgaris und Tylenchorhynchus spp., Tylenchulus semipenetrans und die Semiparasiten Tylenchulus spp., Xiphinema americanum, Xiphinema brevicolle, Xiphinema dimorphicaudatum, Xiphinema index und die Ektoparasiten Xiphinema spp.

Zu den Nematoden, zu deren Bekämpfung eine Verbindung der Formel (I) eingesetzt werden kann, zählen Nematoden der Gattung Meloidogyne wie der Southern Root-Knot Nematode (Meloidogyne incognita), der Javanese Root-Knot Nematode (Meloidogyne javanica, der Northern Root-Knot Nematode (Meloidogyne hapla) und der Peanut Root-Knot Nematode (Meloidogyne arenaria); Nematoden der Gattung Ditylenchus wie das Kartoffelkrätzeälchen (Ditylenchus destructor) und das Stock- und Stängelälchen (Ditylenchus dipsaci); Nematoden der Gattung Pratylenchus wie der Cob Root-Lesion Nematode (Pratylenchus penetrans), der Chrysanthemum Root-Lesion Nematode (Pratylenchus fallax), der Kaffeewurzelnematode (Pratylenchus coffeae), der Teewurzelnematode (Pratylenchus loosi) und der Walnut Root-Lesion Nematode (Pratylenchus vulnus); Nematoden der Gattung Globodera wie das Goldfarbene Kartoffelzystenälchen (Globodera rostochiensis) und das Weiße Kartoffelzystenälchen (Globodera pallida); Nematoden der Gattung Heterodera wie der Sojabohnenzystennematode (Heterodera glycines) und das Rübenzystenälchen (Heterodera schachtii); Nematoden der Gattung Aphelenchoides wie der Rice White-tip Nematode (Aphelenchoides besseyi), das Chrysanthemenälchen (Aphelenchoides ritzemabosi) und das Erdbeerälchen (Aphelenchoides fragariae); Nematoden der Gattung Aphelenchus wie der fungivore Nematode (Aphelenchus avenae); Nematoden der Gattung Radopholus, wie der Burrowing-Nematode (Radopholus similis); Nematoden der Gattung Tylenchulus wie der Orangenwurzelnematode (Tylenchulus semipenetrans); Nematoden der Gattung Rotylenchulus wie der reniforme Nematode (Rotylenchulus reniformis); in Bäumen lebende Nematoden, wie der Kiefernholznematode (Bursaphelenchus xylophilus) und der Red Ring Nematode (Bursaphelenchus cocophilus) und dergleichen.

Zu den Pflanzen, zu deren Schutz eine Verbindung der Formel (I) verwendet werden kann, zählen Pflanzen wie Getreide (zum Beispiel Reis, Gerste, Weizen, Roggen, Hafer, Mais, und dergleichen), Bohnen (Sojabohne, Azukibohne, Bohne, Dicke Bohne, Erbsen, Erdnüsse und dergleichen), Obstbäume/Früchte (Äpfel, Zitrusarten, Birnen, Trauben, Pfirsiche, japanische Aprikosen, Kirschen, Walnüsse, Mandeln, Bananen, Erdbeeren und dergleichen), Gemüsearten (Kohl, Tomate, Spinat, Brokkoli, Salat, Zwiebel, Röhrenlauch, Pfeffer und dergleichen), Hackfrüchte (Karotte, Kartoffel, Süßkartoffel, Rettich, Lotuswurzel, Steckrübe und dergleichen), Pflanzen für industrielle Rohstoffe (Baumwolle, Hanf, Papiermaulbeere, Mitsumata, Raps, Rübe, Hopfen, Zuckerrohr, Zuckerrübe, Olive, Gummi, Palmen, Kaffee, Tabak, Tee und dergleichen), Kürbisgewächse (Kürbis, Gurke, Wassermelone, Melone und dergleichen), Weidepflanzen (Knaulgras, Sorgum, Wiesenlieschgras, Klee, Luzerne und dergleichen), Rasengräser (Maskarenengras, Straußgras und dergleichen), Gewürzpflanzen usw. (Lavendel, Rosmarin, Thymian, Petersilie, Pfeffer, Ingwer und dergleichen) und Blumenpflanzen (Chrysantheme, Rose, Orchidee und dergleichen) erwähnt werden.

Die Verbindungen der Formel (I) eignen sich besonders für die Bekämpfung von Nematoden des Kaffees, insbesondere von Pratylenchus brachyurus, Pratylenchus coffeae, Meloidogyne exigua, Meloidogyne incognita, Meloidogyne coffeicola, Helicotylenchus spp. sowie aus Meloidogyne paranaensis, Rotylenchus spp., Xiphinema spp., Tylenchorhynchus spp. und Scutellonema spp..

Die Verbindungen der Formel (I) eignen sich besonders für die Bekämpfung von Nematoden der Kartoffel, insbesondere von Pratylenchus brachyurus, Pratylenchus pratensis, Pratylenchus scribneri, Pratylenchus penetrans, Pratylenchus coffeae, Ditylenchus dipsaci sowie aus Pratylenchus alleni, Pratylenchus andinus, Pratylenchus cerealis, Pratylenchus crenatus, Pratylenchus hexincisus, Pratylenchus loosi, Pratylenchus neglectus, Pratylenchus teres, Pratylenchus thornei, Pratylenchus vulnus, Belonolaimus longicaudatus, Trichodorus cylindricus, Trichodorus primitivus, Trichodorus proximus, Trichodorus similis, Trichodorus sparsus, Paratrichodorus minor, Paratrichodorus allius, Paratrichodorus nanus, Paratrichodorus teres, Meloidogyne arenaria, Meloidogyne fallax, Meloidogyne hapla, Meloidogyne thamesi, Meloidogyne incognita, Meloidogyne chitwoodi, Meloidogyne javanica, Nacobbus aberrans, Globodera rostochiensis, Globodera pallida, Ditylenchus destructor, Radopholus similis, Rotylenchulus reniformis, Neotylenchus vigissi, Paraphelenchus pseudoparietinus, Aphelenchoides fragariae und Meloinema spp.

Die Verbindungen der Formel (I) eignen sich besonders für die Bekämpfung von Nematoden der Tomate, insbesondere von Meloidogyne arenaria, Meloidogyne hapla, Meloidogyne javanica, Meloidogyne incognita, Pratylenchus penetrans und aus Pratylenchus brachyurus, Pratylenchus coffeae, Pratylenchus scribneri, Pratylenchus vulnus, Paratrichodorus minor, Meloidogyne exigua, Nacobbus aberrans, Globodera solanacearum, Dolichodorus heterocephalus und Rotylenchulus reniformis.

Die Verbindungen der Formel (I) eignen sich besonders für die Bekämpfung von Nematoden von Gurkengewächsen, insbesondere von Meloidogyne arenaria, Meloidogyne hapla, Meloidogyne javanica, Meloidogyne incognita, Rotylenchulus reniformis und Pratylenchus thornei.

Die Verbindungen der Formel (I) eignen sich besonders für die Bekämpfung von Nematoden der Baumwolle, insbesondere von Belonolaimus longicaudatus, Meloidogyne incognita, Hoplolaimus columbus, Hoplolaimus galeatus und Rotylenchulus reniformis.

Die Verbindungen der Formel (I) eignen sich besonders für die Bekämpfung von Nematoden des Maises, insbesondere von Belonolaimus longicaudatus, Paratrichodorus minor und aus Pratylenchus brachyurus, Pratylenchus delattrei, Pratylenchus hexincisus, Pratylenchus penetrans, Pratylenchus zeae, (Belonolaimus gracilis), Belonolaimus nortoni, Longidorus breviannulatus, Meloidogyne arenaria, Meloidogyne arenaria thamesi, Meloidogyne graminis, Meloidogyne incognita, Meloidogyne incognita acrita, Meloidogyne javanica, Meloidogyne naasi, Heterodera avenae, Heterodera oryzae, Heterodera zeae, Punctodera chalcoensis, Ditylenchus dipsaci, Hoplolaimus aegyptii, Hoplolaimus magnistylus, Hoplolaimus galeatus, Hoplolaimus indicus, Helicotylenchus digonicus, Helicotylenchus dihystera, Helicotylenchus pseudorobustus, Xiphinema americanum, Dolichodorus heterocephalus, Criconemella ornata, Criconemella onoensis, Radopholus similis, Rotylenchulus borealis, Rotylenchulus parvus, Tylenchorhynchus agri, Tylenchorhynchus clarus, Tylenchorhynchus claytoni, Tylenchorhynchus maximus, Tylenchorhynchus nudus, Tylenchorhynchus vulgaris, Quinisulcius acutus, Paratylenchus minutus, Hemicycliophora parvana, Aglenchus agricola, Anguina tritici, Aphelenchoides arachidis, Scutellonema brachyurum und Subanguina radiciola.

Die Verbindungen der Formel (I) eignen sich besonders für die Bekämpfung von Nematoden der Sojabohne, insbesondere vonPratylenchus brachyurus, Pratylenchus pratensis, Pratylenchus penetrans, Pratylenchus scribneri, Belonolaimus longicaudatus, Heterodera glycines, Hoplolaimus columbus und aus Pratylenchus coffeae, Pratylenchus hexincisus, Pratylenchus neglectus, Pratylenchus crenatus, Pratylenchus alleni, Pratylenchus agilis, Pratylenchus zeae, Pratylenchus vulnus, (Belonolaimus gracilis), Meloidogyne arenaria, Meloidogyne incognita, Meloidogyne javanica, Meloidogyne hapla, Hoplolaimus columbus, Hoplolaimus galeatus und Rotylenchulus reniformis.

Die Verbindungen der Formel (I) eignen sich besonders für die Bekämpfung von Nematoden des Tabaks, insbesondere von Meloidogyne incognita, Meloidogyne javanica und aus Pratylenchus brachyurus, Pratylenchus pratensis, Pratylenchus hexincisus, Pratylenchus penetrans, Pratylenchus neglectus, Pratylenchus crenatus, Pratylenchus thornei, Pratylenchus vulnus, Pratylenchus zeae, Longidorus elongatu, Paratrichodorus lobatus, Trichodorus spp., Meloidogyne arenaria, Meloidogyne hapla, Globodera tabacum, Globodera solanacearum, Globodera virginiae, Ditylenchus dipsaci, Rotylenchus spp., Helicotylenchus spp., Xiphinema americanum, Criconemella spp., Rotylenchulus reniformis, Tylenchorhynchus claytoni, Paratylenchus spp. und Tetylenchus nicotianae.

Die Verbindungen der Formel (I) eignen sich besonders für die Bekämpfung von Nematoden von Zitrusgewächsen, insbesondere von Pratylenchus coffeae und aus Pratylenchus brachyurus, Pratylenchus vulnus, Belonolaimus longicaudatus, Paratrichodorus minor, Paratrichodorus porosus, Trichodorus , Meloidogyne incognita, Meloidogyne incognita acrita, Meloidogyne javanica, Rotylenchus macrodoratus, Xiphinema americanum, Xiphinema brevicolle, Xiphinema index, Criconemella spp., Hemicriconemoides, Radopholus similis bzw. Radopholus citrophilus, Hemicycliophora arenaria, Hemicycliophora nudata und Tylenchulus semipenetrans .

Die Verbindungen der Formel (I) eignen sich besonders für die Bekämpfung von Nematoden der Banane, insbesondere von Pratylenchus coffeae, Radopholus similis und aus Pratylenchus giibbicaudatus, Pratylenchus loosi, Meloidogyne spp., Helicotylenchus multicinctus, Helicotylenchus dihystera und Rotylenchulus spp..

Die Verbindungen der Formel (I) eignen sich besonders für die Bekämpfung von Nematoden der Ananas, insbesondere von Pratylenchus zeae, Pratylenchus pratensis, Pratylenchus brachyurus, Pratylenchus goodeyi., Meloidogyne spp., Rotylenchulus reniformis und aus Longidorus elongatus, Longidorus laevicapitatus, Trichodorus primitivus, Trichodorus minor, Heterodera spp., Ditylenchus myceliophagus, Hoplolaimus californicus, Hoplolaimus pararobustus, Hoplolaimus indicus, Helicotylenchus dihystera, Helicotylenchus nannus, Helicotylenchus multicinctus, Helicotylenchus erythrine, Xiphinema dimorphicaudatum, Radopholus similis, Tylenchorhynchus digitatus, Tylenchorhynchus ebriensis, Paratylenchus minutus, Scutellonema clathricaudatum, Scutellonema bradys, Psilenchus tumidus, Psilenchus magnidens, Pseudohalenchus minutus, Criconemoides ferniae, Criconemoides onoense und Criconemoides ornatum .

Die Verbindungen der Formel (I) eignen sich besonders für die Bekämpfung von Nematoden von Trauben, insbesondere von Pratylenchus vulnus, Meloidogyne arenaria, Meloidogyne incognita, Meloidogyne javanica, Xiphinema americanum, Xiphinema index und aus Pratylenchus pratensis, Pratylenchus scribneri, Pratylenchus neglectus, Pratylenchus brachyurus, Pratylenchus thornei und Tylenchulus semipenetrans.

Die Verbindungen der Formel (I) eignen sich besonders für die Bekämpfung von Nematoden von Baumkulturen - Kernobst, insbesondere von Pratylenchus penetrans und aus Pratylenchus vulnus, Longidorus elongatus, Meloidogyne incognita und Meloidogyne hapla.

Die Verbindungen der Formel (I) eignen sich besonders für die Bekämpfung von Nematoden von Baumkulturen - Steinfrüchten, insbesondere von Pratylenchus penetrans, Pratylenchus vulnus, Meloidogyne arenaria, Meloidogyne hapla, Meloidogyne javanica, Meloidogyne incognita, Criconemella xenoplax und aus Pratylenchus brachyurus, Pratylenchus coffeae, Pratylenchus scribneri, Pratylenchus zeae, Belonolaimus longicaudatus, Helicotylenchus dihystera, Xiphinema americanum, Criconemella curvata, Tylenchorhynchus claytoni, Paratylenchus hamatus, Paratylenchus projectus, Scutellonema brachyurum und Hoplolaimus galeatus.

Die Verbindungen der Formel (I) eignen sich besonders für die Bekämpfung von Nematoden in Baumkulturen, Zuckerrohr und Reis, insbesondere von Trichodorus spp., Criconemella spp. und aus Pratylenchus spp. , Paratrichodorus spp., Meloidogyne spp. , Helicotylenchus spp., Tylenchorhynchus spp., Aphelenchoides spp. Heterodera spp, Xiphinema spp. und Cacopaurus pestis.

Ebenso bezieht sich der Begriff "Nematoden" im vorliegenden Zusammenhang auf Nematoden, die Menschen oder Tiere schädigen.

Spezifische Nematodenarten, die für den Menschen oder für Tiere schädlich sind, sind:
Trichinellida, zum Beispiel: Trichuris spp., Capillaria spp., Paracapillaria spp., Eucoleus spp., Trichomosoides spp., Trichinella spp.

Aus der Ordnung der Tylenchida zum Beispiel: Micronema spp., Strongyloides spp.

Aus der Ordnung der Rhabditida zum Beispiel: Strongylus spp., Triodontophorus spp., Oesophagodontus spp., Trichonema spp., Gyalocephalus spp., Cylindropharynx spp., Poteriostomum spp., Cyclococercus spp., Cylicostephanus spp., Oesophagostomum spp., Chabertia spp., Stephanurus spp., Ancylostoma spp., Uncinaria spp., Necator spp., Bunostomum spp., Globocephalus spp., Syngamus spp., Cyathostoma spp., Metastrongylus spp., Dictyocaulus spp., Muellerius spp., Protostrongylus spp., Neostrongylus spp., Cystocaulus spp., Pneumostrongylus spp., Spicocaulus spp., Elaphostrongylus spp., Parelaphostrongylus spp., Crenosoma spp., Paracrenosoma spp., Oslerus spp., Angiostrongylus spp., Aelurostrongylus spp., Filaroides spp., Parafilaroides spp., Trichostrongylus spp., Haemonchus spp., Ostertagia spp., Teladorsagia spp., Marshallagia spp., Cooperia spp., Nippostrongylus spp., Heligmosomoides spp., Nematodirus spp., Hyostrongylus spp., Obeliscoides spp., Amidostomum spp., Ollulanus spp.

Aus der Odnung der Spirurida zum Beispiel: Oxyuris spp., Enterobius spp., Passalurus spp., Syphacia spp., Aspiculuris spp., Heterakis spp.; Ascaris spp., Toxascaris spp., Toxocara spp., Baylisascaris spp., Parascaris spp., Anisakis spp., Ascaridia spp.; Gnathostoma spp., Physaloptera spp., Thelazia spp., Gongylonema spp., Habronema spp., Parabronema spp., Draschia spp., Dracunculus spp.; Stephanofilaria spp., Parafilaria spp., Setaria spp., Loa spp., Dirofilaria spp., Litomosoides spp., Brugia spp., Wuchereria spp., Onchocerca spp., Spirocerca spp.;
Viele bekannte Nematizide wirken gleichsam gegen andere parasitäre Helminthen und werden daher für die Bekämpfung von human- und tierparasitären Würmern, die nicht unbedingt zu der Gruppe Nematoda gehören, verwendet. Die vorliegende Erfindung bezieht sich auch auf die Verwendung der Verbindungen der Formel (I) als anthelmintische Arzneimittel. Zu den pathogenen endoparasitären Helminthen zählen Platyhelmintha (z.B. Monogenea, Cestodes und Trematodes), Acanthocephala und Pentastoma. Die folgenden Helminthen sind als bevorzugt zu erwähnen:
Monogenea: z.B.: Gyrodactylus spp., Dactylogyrus spp., Polystoma spp.
Cestodes: aus der Ordnung der Pseudophyllidea zum Beispiel: Diphyllobothrium spp., Spirometra spp., Schistocephalus spp., Ligula spp., Bothridium spp., Diplogonoporus spp.

Aus der Ordnung der Cyclophyllida zum Beispiel: Mesocestoides spp., Anoplocephala spp., Paranoplocephala spp., Moniezia spp., Thysanosoma spp., Thysaniezia spp., Avitellina spp., Stilesia spp., Cittotaenia spp., Andyra spp., Bertiella spp., Taenia spp., Echinococcus spp., Hydatigera spp., Davainea spp., Raillietina spp., Hymenolepis spp., Echinolepis spp., Echinocotyle spp., Diorchis spp., Dipylidium spp., Joyeuxiella spp., Diplopylidium spp.

Trematodes: aus der Klasse der Digenea zum Beispiel: Diplostomum spp., Posthodiplostomum spp., Schistosoma spp., Trichobilharzia spp., Ornithobilharzia spp., Austrobilharzia spp., Gigantobilharzia spp., Leucochloridium spp., Brachylaima spp., Echinostoma spp., Echinoparyphium spp., Echinochasmus spp., Hypoderaeum spp., Fasciola spp., Fascioloides spp., Fasciolopsis spp., Cyclocoelum spp., Typhlocoelum spp., Paramphistomum spp., Calicophoron spp., Cotylophoron spp., Gigantocotyle spp., Fischoederius spp., Gastrothylacus spp., Notocotylus spp., Catatropis spp., Plagiorchis spp., Prosthogonimus spp., Dicrocoelium spp., Eurytrema spp., Troglotrema spp., Paragonimus spp., Collyriclum spp., Nanophyetus spp., Opisthorchis spp., Clonorchis spp., Metorchis spp., Heterophyes spp., Metagonimus spp.

Acanthocephala: aus der Ordnung der Oligacanthorhynchida z.B.: Macracanthorhynchus spp., Prosthenorchis spp.; aus der Ordnung der Polymorphida zum Beispiel: Filicollis spp.; aus der Ordnung der Moniliformida zum Beispiel: Moniliformis spp.,
Aus der Ordnung der Echinorhynchida zum Beispiel Acanthocephalus spp., Echinorhynchus spp., Leptorhynchoides spp.

Pentastoma: aus der Ordnung der Porocephalida zum Beispiel Linguatula spp.

Auf dem Gebiet der Tiermedizin und in der Tierhaltung erfolgt die Verabreichung der Verbindungen der Formel (I) auf bekannte Weise direkt oder enteral, parenteral, dermal oder nasal in Form von geeigneten Anwendungsformen. Die Verabreichung kann prophylaktisch oder therapeutisch erfolgen.

Die Verbindungen der Formel (I) können gegebenenfalls in bestimmten Konzentrationen bzw. Aufwandmengen auch als Herbizide, Safener, Wachstumsregulatoren oder Mittel zur Verbesserung der Pflanzeneigenschaften, als Mikrobizide oder Gametozide, beispielsweise als Fungizide, Antimykotika, Bakterizide, Virizide (einschließlich Mittel gegen Viroide) oder als Mittel gegen MLO (Mycoplasmalike-organism) und RLO (Rickettsia-like-organism) verwendet werden. Sie lassen sich gegebenenfalls auch als Zwischen- oder Vorprodukte für die Synthese weiterer Wirkstoffe einsetzen.

### Formulierungen

Die vorliegende Erfindung betrifft weiterhin Formulierungen und daraus bereitete Anwendungsformen als Schädlingsbekämpfungsmittel wie z. B. Drench-, Drip- und Spritzbrühen, umfassend mindestens eine Verbindung der Formel (I). Gegebenenfalls enthalten die Anwendungsformen weitere Schädlingsbekämpfungsmittel und/oder die Wirkung verbessernde Adjuvantien wie Penetrationsförderer, z. B. vegetative Öle wie beispielsweise Rapsöl, Sonnenblumenöl, Mineralöle wie beispielsweise Paraffinöle, Alkylester vegetativer Fettsäuren wie beispielsweise Rapsöl- oder Sojaölmethylester oder Alkanol-alkoxylate und/oder Spreitmittel wie beispielsweise Alkylsiloxane und/oder Salze z.B. organische oder anorganische Ammonium- oder Phosphoniumsalze wie beispielsweise Ammoniumsulfat oder Diammonium-hydrogenphosphat und/oder die Retention fördernde Mittel wie z. B. Dioctylsulfosuccinat oder Hydroxypropyl-guar Polymere und/oder Humectants wie z.B. Glycerin und/oder Dünger wie beispielsweise Ammonium-, Kalium- oder Phosphor-enthaltende Dünger.

Übliche Formulierungen sind beispielsweise wasserlösliche Flüssigkeiten (SL), Emulsionskonzentrate (EC), Emulsionen in Wasser (EW), Suspensionskonzentrate (SC, SE, FS, OD), in Wasser dispergierbare Granulate (WG), Granulate (GR) und Kapselkonzentrate (CS); diese und weitere mögliche Formuliertypen sind beispielsweise durch Crop Life International und in Pesticide Specifications, Manual on development and use of FAO and WHO specifications for pesticides, FAO Plant Production and Protection Papers - 173, prepared by the FAO/WHO Joint Meeting on Pesticide Specifications, 2004, ISBN: 9251048576 beschrieben. Gegebenenfalls enthalten die Formulierungen neben einem oder mehreren Verbindungen der Formel (I) weitere agrochemische Wirkstoffe.

Vorzugsweise handelt es sich um Formulierungen oder Anwendungsformen, welche Hilfsstoffe wie beispielsweise Streckmittel, Lösemittel, Spontanitätsförderer, Trägerstoffe, Emulgiermittel, Dispergiermittel, Frostschutzmittel, Biozide, Verdicker und/oder weitere Hilfsstoffe wie beispielsweise Adjuvantien enthalten. Ein Adjuvant in diesem Kontext ist eine Komponente, die die biologische Wirkung der Formulierung verbessert, ohne dass die Komponente selbst eine biologische Wirkung hat. Beispiele für Adjuvantien sind Mittel, die die Retention, das Spreitverhalten, das Anhaften an der Blattoberfläche oder die Penetration fördern.

Diese Formulierungen werden in bekannter Weise hergestellt, z.B. durch Vermischen der Verbindungen der Formel (I) mit Hilfsstoffen wie beispielsweise Streckmitteln, Lösemitteln und/oder festen Trägerstoffen und/oder weiteren Hilfsstoffen wie beispielsweise oberflächenaktive Stoffe. Die Herstellung der Formulierungen erfolgt entweder in geeigneten Anlagen oder auch vor oder während der Anwendung.

Als Hilfsstoffe können solche Stoffe Verwendung finden, die geeignet sind, der Formulierung der Verbindungen der Formel (I) oder den aus diesen Formulierungen bereiteten Anwendungsformen (wie z.B. gebrauchsfähigen Schädlingsbekämpfungsmitteln wie Spritzbrühen oder Saatgutbeizen) besondere Eigenschaften, wie bestimmte physikalische, technische und/oder biologische Eigenschaften zu verleihen.

Als Streckmittel eignen sich z.B. Wasser, polare und unpolare organische chemische Flüssigkeiten z.B. aus den Klassen der aromatischen und nicht-aromatischen Kohlenwasserstoffe (wie Paraffine, Alkylbenzole, Alkylnaphthaline, Chlorbenzole), der Alkohole und Polyole (die ggf. auch substituiert, verethert und/oder verestert sein können), der Ketone (wie Aceton, Cyclohexanon), Ester (auch Fette und Öle) und (Poly-)Ether, der einfachen und substituierten Amine, Amide, Lactame (wie N-Alkylpyrrolidone) und Lactone, der Sulfone und Sulfoxide (wie Dimethylsulfoxid).

Im Falle der Benutzung von Wasser als Streckmittel können z.B. auch organische Lösemittel als Hilfslösemittel verwendet werden. Als flüssige Lösemittel kommen im Wesentlichen infrage: Aromaten wie Xylol, Toluol oder Alkylnaphthaline, chlorierte Aromaten oder chlorierte aliphatische Kohlenwasser-stoffe wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole wie Butanol oder Glykol sowie deren Ether und Ester, Ketone wie Aceton, Methylethylketon, Methylisobutylketon oder Cyclohexanon, stark polare Lösemittel wie Dimethylformamid und Dimethylsulfoxid sowie Wasser.

Grundsätzlich können alle geeigneten Lösemittel verwendet werden. Geeignete Lösemittel sind beispielsweise aromatische Kohlenwasserstoffe wie z.B. Xylol, Toluol oder Alkylnaphthaline, chlorierte aromatische oder aliphatische Kohlenwasserstoffe wie z.B. Chlorbenzol, Chlorethylen, oder Methylenchlorid, aliphatische Kohlenwasserstoffe wie z.B. Cyclohexan, Paraffine, Erdölfraktionen, mineralische und pflanzliche Öle, Alkohole wie z.B. Methanol, Ethanol, iso-Propanol, Butanol oder Glykol sowie deren Ether und Ester, Ketone wie z.B. Aceton, Methylethylketon, Methylisobutylketon oder Cyclohexanon, stark polare Lösemittel wie Dimethylsulfoxid sowie Wasser.

Grundsätzlich können alle geeigneten Trägerstoffe eingesetzt werden. Als Trägerstoffe kommen insbesondere infrage: z.B. Ammoniumsalze und natürliche Gesteinsmehle wie Kaoline, Tonerden, Talkum, Kreide, Quarz, Attapulgit, Montmorillonit oder Diatomeenerde und synthetische Gesteinsmehl, wie hochdisperse Kieselsäure, Aluminiumoxid und natürliche oder synthetische Silikate, Harze, Wachse und /oder feste Düngemittel. Mischungen solcher Trägerstoffe können ebenfalls verwendet werden. Als Trägerstoffe für Granulate kommen infrage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Papier, Kokosnussschalen, Maiskolben und Tabakstängel.

Auch verflüssigte gasförmige Streckmittel oder Lösemittel können eingesetzt werden. Insbesondere eignen sich solche Streckmittel oder Trägerstoffe, welche bei normaler Temperatur und unter Normaldruck gasförmig sind, z.B. Aerosol-Treibgase wie Halogenkohlenwasserstoffe sowie Butan, Propan, Stickstoff und Kohlendioxid.

Beispiele für Emulgier- und/oder Schaum erzeugende Mittel, Dispergiermittel oder Benetzungsmittel mit ionischen oder nicht-ionischen Eigenschaften oder Mischungen dieser oberflächenaktiven Stoffe sind Salze von Polyacrylsäure, Salze von Lignosulphonsäure, Salze von Phenolsulphonsäure oder Naphthalinsulphonsäure, Polykondensate von Ethylenoxid mit Fettalkoholen oder mit Fettsäuren oder mit Fettaminen, mit substituierten Phenolen (vorzugsweise Alkylphenole oder Arylphenole), Salze von Sulphobernsteinsäureestern, Taurinderivate (vorzugsweise Alkyltaurate), Phosphorsäureester von polyethoxylierten Alkoholen oder Phenole, Fettsäureester von Polyolen und Derivate der Verbindungen enthaltend Sulphate, Sulphonate und Phosphate, z.B. Alkylarylpolyglycolether, Alkylsulfonate, Alkylsulfate, Arylsulfonate, Eiweißhydrolysate, Lignin-Sulfitablaugen und Methylcellulose. Die Anwesenheit einer oberflächenaktiven Substanz ist vorteilhaft, wenn eine der Verbindungen der Formel (I) und/oder einer der inerten Trägerstoffe nicht in Wasser löslich ist und wenn die Anwendung in Wasser erfolgt.

Als weitere Hilfsstoffe können in den Formulierungen und den daraus abgeleiteten Anwendungsformen Farbstoffe wie anorganische Pigmente, z.B. Eisenoxid, Titanoxid, Ferrocyanblau und organische Farbstoffe wie Alizarin-, Azo- und Metallphthalocyaninfarbstoffe und Nähr- und Spurennährstoffe wie Salze von Eisen, Mangan, Bor, Kupfer, Kobalt, Molybdän und Zink vorhanden sein.

Weiterhin enthalten sein können Stabilisatoren wie Kältestabilisatoren, Konservierungsmittel, Oxidationsschutzmittel, Lichtschutzmittel oder andere die chemische und / oder physikalische Stabilität verbessernde Mittel. Weiterhin enthalten sein können schaumerzeugende Mittel oder Entschäumer.

Ferner können die Formulierungen und daraus abgeleiteten Anwendungsformen als zusätzliche Hilfsstoffe auch Haftmittel wie Carboxymethylcellulose, natürliche und synthetische pulverige, körnige oder latexförmige Polymere enthalten wie Gummiarabikum, Polyvinylalkohol, Polyvinylacetat sowie natürliche Phospholipide wie Kephaline und Lecithine und synthetische Phospholipide. Weitere Hilfsstoffe können mineralische und vegetabile Öle sein.

Gegebenenfalls können noch weitere Hilfsstoffe in den Formulierungen und den daraus abgeleiteten Anwendungsformen enthalten sein. Solche Zusatzstoffe sind beispielsweise Duftstoffe, schützende Kolloide, Bindemittel, Klebstoffe, Verdicker, thixotrope Stoffe, Penetrationsförderer, Retentionsförderer, Stabilisatoren, Sequestiermittel, Komplexbildner, Humectans, Spreitmittel. Im Allgemeinen können die Verbindungen der Formel (I) mit jedem festen oder flüssigen Zusatzstoff, welches für Formulierungszwecke gewöhnlich verwendet wird, kombiniert werden.

Als Retentionsförderer kommen alle diejenigen Substanzen in Betracht, die die dynamische Oberflächenspannung verringern wie beispielsweise Dioctylsulfosuccinat oder die die Visko-Elastizität erhöhen wie beispielsweise Hydroxypropyl-guar Polymere.

Als Penetrationsförderer kommen im vorliegenden Zusammenhang alle diejenigen Substanzen in Betracht, die üblicherweise eingesetzt werden, um das Eindringen von agrochemischen Wirkstoffen in Pflanzen zu verbessern. Penetrationsförderer werden in diesem Zusammenhang dadurch definiert, dass sie aus der (in der Regel wässerigen) Applikationsbrühe und/oder aus dem Spritzbelag in die Kutikula der Pflanze eindringen und dadurch die Stoffbeweglichkeit (Mobilität) der Wirkstoffe in der Kutikula erhöhen können. Die in der Literatur (Baur et al., 1997, Pesticide Science 51, 131-152) beschriebene Methode kann zur Bestimmung dieser Eigenschaft eingesetzt werden. Beispielhaft werden genannt Alkoholalkoxylate wie beispielsweise Kokosfettethoxylat (10) oder Isotridecylethoxylat (12), Fettsäureester wie beispielsweise Rapsöl- oder Sojaölmethylester, Fettamine Alkoxylate wie beispielsweise Tallowamine-ethoxylat (15) oder Ammonium- und/oder Phosphonium-Salze wie beispielsweise Ammoniumsulfat oder Diammonium-hydrogenphosphat.

Die Formulierungen enthalten bevorzugt zwischen 0,00000001 und 98 Gew.-% der Verbindung der Formel (I), besonders bevorzugt zwischen 0,01 und 95 Gew.-% der Verbindung der Formel (I), ganz besonders bevorzugt zwischen 0,5 und 90 Gew.-% der Verbindung der Formel (I), bezogen auf das Gewicht der Formulierung.

Der Gehalt an der Verbindung der Formel (I) in den aus den Formulierungen bereiteten Anwendungsformen (insbesondere Schädlingsbekämpfungsmittel) kann in weiten Bereichen variieren. Die Konzentration der Verbindung der Formel (I) in den Anwendungsformen kann üblicherweise zwischen 0,00000001 und 95 Gew.-% der Verbindung der Formel (I), vorzugsweise zwischen 0,00001 und 1 Gew.-%, bezogen auf das Gewicht der Anwendungsform, liegen. Die Anwendung geschieht in einer den Anwendungsformen angepaßten üblichen Weise.

### Mischungen

Die Verbindungen der Formel (I) können auch in Mischung mit einem oder mehreren geeigneten Fungiziden, Bakteriziden, Akariziden, Molluskiziden, Nematiziden, Insektiziden, Mikrobiologika, Nützlingen, Herbizide, Düngemitteln, Vogelrepellentien, Phytotonics, Sterilantien, Safenern, Semiochemicals und/oder Pflanzenwachstumsregulatoren verwendet werden, um so z.B. das Wirkungsspektrum zu verbreitern, die Wirkdauer zu verlängern, die Wirkgeschwindigkeit zu steigern, Repellenz zu verhindern oder Resistenzentwicklungen vorzubeugen. Desweiteren können solche Wirkstoffkombinationen das Pflanzenwachstum und/oder die Toleranz gegenüber abiotischen Faktoren wie z. B. hohen oder niedrigen Temperaturen, gegen Trockenheit oder gegen erhöhten Wasser- bzw. Bodensalzgehalt verbessern. Auch lässt sich das Blüh- und Fruchtverhalten verbessern, die Keimfähigkeit und Bewurzelung optimieren, die Ernte erleichtern und Ernteerträge steigern, die Reife beeinflussen, die Qualität und/oder den Ernährungswert der Ernteprodukte steigern, die Lagerfähigkeit verlängern und/oder die Bearbeitbarkeit der Ernteprodukte verbessern.

Weiterhin können die Verbindungen der Formel (I) in Mischung mit weiteren Wirkstoffen oder Semiochemicals, wie Lockstoffen und/oder Vogelrepellentien und/oder Pflanzenaktivatoren und/oder Wachstumsregulatoren und/oder Düngemitteln vorliegen. Gleichfalls können die Verbindungen der Formel (I) in Mischungen mit Mitteln zur Verbesserung der Pflanzeneigenschaften wie zum Beispiel Wuchs, Ertrag und Qualität des Erntegutes eingesetzt werden.

In einer besonderen erfindungsgemäßen Ausführungsform liegen die Verbindungen der Formel (I) in Formulierungen bzw. in den aus diesen Formulierungen bereiteten Anwendungsformen in Mischung mit weiteren Verbindungen vor, vorzugsweise solchen wie nachstehend beschrieben.

Wenn eine der im Folgenden genannten Verbindungen in verschiedenen tautomeren Formen vorkommen kann sind auch diese Formen mit umfasst, auch wenn sie sie nicht in jedem Fall explizit genannt wurden.

### Insektizide / Akarizide / Nematizide

Die hier mit ihrem "common name" genannten Wirkstoffe sind bekannt und beispielsweise im Pestizidhandbuch ("The Pesticide Manual" 16th Ed., British Crop Protection Council 2012) beschrieben oder im Internet recherchierbar (z.B. http://www.alanwood.net/pesticides).
(1) Acetylcholinesterase (AChE) Inhibitoren, wie beispielsweise Carbamate, z.B. Alanycarb, Aldicarb, Bendiocarb, Benfuracarb, Butocarboxim, Butoxycarboxim, Carbaryl, Carbofuran, Carbosulfan, Ethiofencarb, Fenobucarb, Formetanate, Furathiocarb, Isoprocarb, Methiocarb, Methomyl, Metolcarb, Oxamyl, Pirimicarb, Propoxur, Thiodicarb, Thiofanox, Triazamate, Trimethacarb, XMC und Xylylcarb oder organophosphate, z.B. Acephate, Azamethiphos, Azinphos-ethyl, Azinphos-methyl, Cadusafos, Chlorethoxyfos, Chlorfenvinphos, Chlormephos, Chlorpyrifos, Chlorpyrifos-methyl, Coumaphos, Cyanophos, Demeton-S-methyl, Diazinon, Dichlorvos/DDVP, Dicrotophos, Dimethoate, Dimethylvinphos, Disulfoton, EPN, Ethion, Ethoprophos, Famphur, Fenamiphos, Fenitrothion, Fenthion, Fosthiazate, Heptenophos, Imicyafos, Isofenphos, Isopropyl O-(methoxyaminothio-phosphoryl) salicylat, Isoxathion, Malathion, Mecarbam, Methamidophos, Methidathion, Mevinphos, Monocrotophos, Naled, Omethoate, Oxydemeton-methyl, Parathion, Parathion-methyl, Phenthoate, Phorate, Phosalone, Phosmet, Phosphamidon, Phoxim, Pirimiphos-methyl, Profenofos, Propetamphos, Prothiofos, Pyraclofos, Pyridaphenthion, Quinalphos, Sulfotep, Tebupirimfos, Temephos, Terbufos, Tetrachlorvinphos, Thiometon, Triazophos, Triclorfon und Vamidothion.
(2) GABA-gesteuerte Chlorid-Kanal-Antagonisten, wie beispielsweise Cyclodien-organochlorine, z.B. Chlordane und Endosulfan oder Phenylpyrazole (Fiprole), z.B. Ethiprole und Fipronil.
(3) Natrium-Kanal-Modulatoren / Spannungsabhängige Natrium-Kanal-Blocker, wie beispielsweise Pyrethroide, z.B. Acrinathrin, Allethrin, d-cis-trans Allethrin, d-trans Allethrin, Bifenthrin, Bioallethrin, Bioallethrin S-cyclopentenyl Isomer, Bioresmethrin, Cycloprothrin, Cyfluthrin, beta-Cyfluthrin, Cyhalothrin, lambda-Cyhalothrin, gamma-Cyhalothrin, Cypermethrin, alpha-Cypermethrin, beta-Cypermethrin, theta-Cypermethrin, zeta-Cypermethrin, Cyphenothrin [(1R)-trans-Isomere], Deltamethrin, Empenthrin [(EZ)-(1R)-Isomere), Esfenvalerate, Etofenprox, Fenpropathrin, Fenvalerate, Flucythrinate, Flumethrin, tau-Fluvalinate, Halfenprox, Imiprothrin, Kadethrin, Permethrin, Phenothrin [(1R)-trans-Isomer), Prallethrin, Pyrethrine (pyrethrum), Resmethrin, Silafluofen, Tefluthrin, Tetramethrin, Tetramethrin [(1R)- Isomere)], Tralomethrin und Transfluthrin oder DDT oder Methoxychlor.
(4) Nikotinerge Acetylcholin-Rezeptor (nAChR) Agonisten, wie beispielsweise Neonikotinoide, z.B. Acetamiprid, Clothianidin, Dinotefuran, Imidacloprid, Nitenpyram, Thiacloprid und Thiamethoxam oder Nikotin oder Sulfoxaflor oder Flupyradifurone.
(5) Nikotinerge Acetylcholin-Rezeptor (nAChR) allosterische Aktivatoren, wie beispielsweise Spinosine, z.B. Spinetoram und Spinosad.
(6) Chlorid-Kanal-Aktivatoren, wie beispielsweise Avermectine/Milbemycine, z.B. Abamectin, Emamectin-benzoat, Lepimectin und Milbemectin.
(7) Juvenilhormon-Imitatoren, wie beispielsweise Juvenilhormon-Analoge, z.B. Hydroprene, Kinoprene und Methoprene oder Fenoxycarb oder Pyriproxyfen.
(8) Wirkstoffe mit unbekannten oder nicht spezifischen Wirkmechanismen, wie beispielsweise
   Alkylhalide, z.B. Methylbromid und andere Alkylhalide; oderChloropicrin oder Sulfurylfluorid oder Borax oder Brechweinstein.
(9) Selektive Fraßhemmer, z.B. Pymetrozine oder Flonicamid.
(10) Milbenwachstumsinhibitoren, z.B. Clofentezine, Hexythiazox und Diflovidazin oder Etoxazole.
(11) Mikrobielle Disruptoren der Insektendarmmembran, z.B. Bacillus thuringiensis Subspezies israelensis, Bacillus sphaericus, Bacillus thuringiensis Subspezies aizawai, Bacillus thuringiensis Subspezies kurstaki, Bacillus thuringiensis Subspezies tenebrionis und BT Pflanzenproteine: Cry1Ab, CrylAc, Cry1Fa, Cry2Ab, mCry3A, Cry3Ab, Cry3Bb, Cry34/35Ab1.
(12) Inhibitoren der oxidativen Phosphorylierung, ATP-Disruptoren, wie beispielsweise Diafenthiuron oder Organozinnverbindungen, z.B. Azocyclotin, Cyhexatin und Fenbutatin-oxid oder Propargite oder Tetradifon.
(13) Entkoppler der oxidativen Phoshorylierung durch Unterbrechung des H-Protongradienten, wie beispielsweise Chlorfenapyr, DNOC und Sulfluramid.
(14) Nikotinerge Acetylcholin-Rezeptor-Antagonisten, wie beispielsweise Bensultap, Cartaphydrochlorid, Thiocyclam und Thiosultap-Natrium.
(15) Inhibitoren der Chitinbiosynthese, Typ 0, wie beispielsweise Bistrifluron, Chlorfluazuron, Diflubenzuron, Flucycloxuron, Flufenoxuron, Hexaflumuron, Lufenuron, Novaluron, Noviflumuron, Teflubenzuron und Triflumuron.
(16) Inhibitoren der Chitinbiosynthese, Typ 1, wie beispielsweise Buprofezin.
(17) Häutungshemmer (insbesondere bei Dipteren, d.h.Zweiflüglern), wie beispielsweise Cyromazine.
(18) Ecdyson-Rezeptor Agonisten, wie beispielsweise Chromafenozide, Halofenozide, Methoxyfenozide und Tebufenozide.
(19) Oktopaminerge Agonisten, wie beispielsweise Amitraz.
(20) Komplex-III-Elektronentransportinhibitoren, wie beispielsweise Hydramethylnon oder Acequinocyl oder Fluacrypyrim.
(21) Komplex-I-Elektronentransportinhibitoren, beispielsweise METI-Akarizide, z.B. Fenazaquin, Fenpyroximate, Pyrimidifen, Pyridaben, Tebufenpyrad und Tolfenpyrad oder Rotenone (Derris).
(22) Spannungsabhängige Natriumkanal-Blocker, z.B. Indoxacarb oder Metaflumizone.
(23) Inhibitoren der Acetyl-CoA-Carboxylase, wie beispielsweise Tetron- und Tetramsäurederivate, z.B. Spirodiclofen, Spiromesifen und Spirotetramat.
(24) Komplex-IV-Elektronentransportinhibitoren, wie beispielsweise Phosphine, z.B. Aluminiumphosphid, Calciumphosphid, Phosphin und Zinkphosphid oder Cyanid.
(25) Komplex-II-Elektronentransportinhibitoren, wie beispielsweise Cyenopyrafen und Cyflumetofen.
(28) Ryanodinrezeptor-Effektoren, wie beispielsweise Diamide, z.B. Chlorantraniliprole, Cyantraniliprole und Flubendiamide.

Weitere Wirkstoffe mit unbekanntem oder nicht eindeutigem Wirkmechanismus, wie beispielsweise Afidopyropen, Afoxolaner, Azadirachtin, Benclothiaz, Benzoximate, Bifenazate, Bromopropylate, Chinomethionat, Cryolite, Cyclaniliprole, Cycloxaprid, Cyhalodiamide Dicloromezotiaz, Dicofol, Diflovidazin, Flometoquin, Fluensulfone, Flufenerim, Flufenoxystrobin, Flufiprole, Fluhexafon, Fluopyram, Fluralaner, Fufenozide, Guadipyr, Heptafluthrin, Imidaclothiz, Iprodione, Meperfluthrin, Paichongding, Pyflubumide, Pyridalyl, Pyrifluquinazon, Pyriminostrobin, Tetramethylfluthrin, Tetraniliprole, Tetrachlorantraniliprole, Tioxazafen, Triflumezopyrim und Iodmethan; desweiteren Präparate auf Basis von Bacillus firmus (I-1582, BioNeem, Votivo), sowie folgende bekannte wirksame Verbindungen: 1-{2-Fluor-4-methyl-5-[(2,2,2-trifluorethyl)sulfinyl]phenyl}-3-(trifluormethyl)-1H-1,2,4-triazol-5-amin (bekannt aus WO2006/043635), {1'-[(2E)-3-(4-Chlorphenyl)prop-2-en-1-yl]-5-fluorspiro[indol-3,4'-piperidin]-1(2H)-yl}(2-chlorpyridin-4-yl)methanon (bekannt aus WO2003/106457), 2-Chlor-N-[2-{1-[(2E)-3-(4-chlorphenyl)prop-2-en-1-yl]piperidin-4-yl}-4-(trifluormethyl)phenyl]isonicotinamid (bekannt aus WO2006/003494), 3-(2,5-Dimethylphenyl)-4-hydroxy-8-methoxy-1,8-diazaspiro[4.5]dec-3-en-2-on (bekannt aus WO2009/049851), 3-(2,5-Dimethylphenyl)-8-methoxy-2-oxo-1,8-diazaspiro[4.5]dec-3-en-4-yl-ethylcarbonat (bekannt aus WO2009/049851), 4-(But-2-in-1-yloxy)-6-(3,5-dimethylpiperidin-1-yl)-5-fluorpyrimidin (bekannt aus WO2004/099160), 4-(But-2-in-1-yloxy)-6-(3-chlorphenyl)pyrimidin (bekannt aus WO2003/076415), PF1364 (CAS-Reg.No. 1204776-60-2), Methyl-2-[2-({[3-brom-1-(3-chlorpyridin-2-yl)-1H-pyrazol-5-yl]carbonyl}amino)-5-chlor-3-methylbenzoyl]-2-methylhydrazincarboxylat (bekannt aus WO2005/085216), Methyl-2-[2-({[3-brom-1-(3-chlorpyridin-2-yl)-1H-pyrazol-5-yl]carbonyl}amino)-5-cyan-3-methylbenzoyl]-2-ethylhydrazincarboxylat (bekannt aus WO2005/085216), Methyl-2-[2-({[3-brom-1-(3-chlorpyridin-2-yl)-1H-pyrazol-5-yl]carbonyl}amino)-5-cyan-3-methylbenzoyl]-2-methylhydrazincarboxylat (bekannt aus WO2005/085216), Methyl-2-[3,5-dibrom-2-({[3-brom-1-(3-chlorpyridin-2-yl)-1H-pyrazol-5-yl]carbonyl}amino)benzoyl]-2-ethylhydrazincarboxylat (bekannt aus WO2005/085216), N-[2-(5-Amino-1,3,4-thiadiazol-2-yl)-4-chlor-6-methylphenyl]-3-brom-1-(3-chlorpyridin-2-yl)-1H-pyrazol-5-carboxamid (bekannt aus CN102057925), 8-Chlor-N-[(2-chlor-5-methoxyphenyl)sulfonyl]-6-(trifluormethyl)imidazo[1,2-a]pyridin-2-carboxamid (bekannt aus WO2010/129500), 4-[5-(3,5-Dichlorphenyl)-5-(trifluormethyl)-4,5-dihydro-1,2-oxazol-3-yl]-2-methyl-N-(1-oxidothietan-3-yl)benzamid (bekannt aus WO2009/080250), N-[(2E)-1-[(6-Chlorpyridin-3-yl)methyl]pyridin-2(1H)-yliden]-2,2,2-trifluoracetamid (bekannt aus WO2012/029672), 1-[(2-Chlor-1,3-thiazol-5-yl)methyl]-4-oxo-3-phenyl-4H-pyrido[1,2-a]pyrimidin-1-ium-2-olat (bekannt aus WO2009/099929), 1-[(6-Chlorpyridin-3-yl)methyl]-4-oxo-3-phenyl-4H-pyrido[1,2-a]pyrimidin-1-ium-2-olat (bekannt aus WO2009/099929), 4-(3-{2,6-Dichlor-4-[(3,3-dichlorprop-2-en-1-yl)oxy]phenoxy}propoxy)-2-methoxy-6-(trifluormethyl)pyrimidin (bekannt aus CN101337940), N-[2-(tert-Butylcarbamoyl)-4-chlor-6-methylphenyl]-1-(3-chlorpyridin-2-yl)-3-(fluormethoxy)-1H-pyrazol-5-carboxamid (bekannt aus WO2008/134969, 3-[Benzoyl(methyl)amino]-N-[2-brom-4-[1,2,2,2-tetrafluor-1-(trifluormethyl)ethyl]-6-(trifluormethyl)phenyl]-2-fluor-benzamid (bekannt aus WO 2010018714), Butyl-[2-(2,4-dichlorphenyl)-3-oxo-4-oxaspiro[4.5]dec-1-en-1-yl]-carbonat (bekannt aus CN 102060818), 4-[5-(3,5-Dichlorphenyl)-5-(trifluormethyl)-4H-isoxazol-3-yl]-N-[(Z)-methoxyiminomethyl]-2-methyl-benzamid (bekannt aus WO2007/026965), 3E)-3-[1-[(6-Chlor-3-pyridyl)methyl]-2-pyridyliden]-1,1,1-trifluor-propan-2-on (bekannt aus WO2013/144213, N-(Methylsulfonyl)-6-[2-(pyridin-3-yl)-1,3-thiazol-5-yl]pyridin-2-carboxamid (bekannt aus WO2012/000896), N-[3-(Benzylcarbamoyl)-4-chlorphenyl]-1-methyl-3-(pentafluorethyl)-4-(trifluormethyl)-1H-pyrazol-5-carboxamid bekannt aus WO2010/051926).

### Fungizide

Die hier mit ihrem "common name" spezifizierten Wirkstoffe sind bekannt, beispielsweise beschrieben im "Pesticide Manual" oder im Internet (beispielsweise: http://www.alanwood.net/pesticides).

Alle aufgeführten fungiziden Mischpartner der Klassen (1) bis (15) können optional Salze mit entsprechenden Basen oder Säuren bilden, sofern geeignete funktionelle Gruppen vorliegen. Außerdem sind für die aufgeführten fungiziden Mischpartner der Klassen (1) bis (15) auch tautomere Formen eingeschlossen, sofern Tautomerie möglich ist.
1) Inhibitoren der Ergosterolbiosynthese, zum Beispiel (1.01) Aldimorph, (1.02) Azaconazol, (1.03) Bitertanol, (1.04) Bromuconazol, (1.05) Cyproconazol, (1.06) Diclobutrazol, (1.07) Difenoconazol, (1.08) Diniconazol, (1.09) Diniconazol-M, (1.10) Dodemorph, (1.11) Dodemorphacetat, (1.12) Epoxiconazol, (1.13) Etaconazol, (1.14) Fenarimol, (1.15) Fenbuconazol, (1.16) Fenhexamid, (1.17) Fenpropidin, (1.18) Fenpropimorph, (1.19) Fluquinconazol, (1.20) Flurprimidol, (1.21) Flusilazol, (1.22) Flutriafol, (1.23) Furconazol, (1.24) Furconazol-cis, (1.25) Hexaconazol, (1.26) Imazalil, (1.27) Imazalilsulfat, (1.28) Imibenconazol, (1.29) Ipconazol, (1.30) Metconazol, (1.31) Myclobutanil, (1.32) Naftifin, (1.33) Nuarimol, (1.34) Oxpoconazol, (1.35) Paclobutrazol, (1.36) Pefurazoat, (1.37) Penconazol, (1.38) Piperalin, (1.39) Prochloraz, (1.40) Propiconazol, (1.41) Prothioconazol, (1.42) Pyributicarb, (1.43) Pyrifenox, (1.44) Quinconazol, (1.45) Simeconazol, (1.46) Spiroxamin, (1.47) Tebuconazol, (1.48) Terbinafin, (1.49) Tetraconazol, (1.50) Triadimefon, (1.51) Triadimenol, (1.52) Tridemorph, (1.53) Triflumizol, (1.54) Triforin, (1.55) Triticonazol, (1.56) Uniconazol, (1.57) Uniconazol-p, (1.58) Viniconazol, (1.59) Voriconazol, (1.60) 1-(4-Chlorphenyl)-2-(1H-1,2,4-triazol-1-yl)cycloheptanol, (1.61) 1-(2,2-Dimethyl-2,3-dihydro-1H-inden-1-yl)-1H-imidazo1-5-carbonsäuremethylester, (1.62) N'-{5-(Difluormethyl)-2-methyl-4-[3-(trimethylsilyl)propoxy]phenyl}-N-ethyl-N-methylimidoformamid, (1.63) N-Ethyl-N-methyl-N'- {2-methyl-5-(trifluormethyl)-4-[3-(trimethylsilyl)propoxy]phenyl}imidoformamid, (1.64) O-[1-(4-Methoxyphenoxy)-3,3-dimethylbutan-2-yl]-1H-imidazol-1-carbothioat, (1.65) Pyrisoxazol, (1.66) 2-{[3-(2-Chlorphenyl)-2-(2,4-difluorphenyl)oxiran-2-yl]methyl}-2,4-dihydro-3H-1,2,4-triazol-3-thion, (1.67) 1-{[3-(2-Chlorphenyl)-2-(2,4-difluorphenyl)oxiran-2-yl]methyl}-1H-1 ,2,4-triazol-5-ylthiocyanat, (1.68) 5-(Allylsulfanyl)-1-{[3-(2-chlorphenyl)-2-(2,4-difluorphenyl)oxiran-2-yl]methyl}-1H-1,2,4-triazol, (1.69) 2-[1-(2,4-Dichlorphenyl)-5-hydroxy-2,6,6-trimethylheptan-4-yl]-2,4-dihydro-3H-1,2,4-triazol-3-thion, (1.70) 2-{[rel(2R,3S)-3-(2-Chlorphenyl)-2-(2,4-difluorphenyl)oxiran-2-yl]methyl}-2,4-dihydro-3H-1,2,4-triazol-3-thion, (1.71) 2-{[rel(2R,3R)-3-(2-Chlorphenyl)-2-(2,4-difluorphenyl)oxiran-2-yl]methyl}-2,4-dihydro-3H-1,2,4-triazol-3-thion, (1.72) 1-{[rel(2R,3S)-3-(2-Chlorphenyl)-2-(2,4-difluorphenyl)oxiran-2-yl]methyl}-1H-1,2,4-triazol-5-ylthiocyanat, (1.73) 1-{ [rel(2R,3R)-3-(2-Chlorphenyl)-2-(2,4-difluorphenyl)oxiran-2-yl]methyl}-1H-1,2,4-triazol-5-ylthiocyanat, (1.74) 5-(Allylsulfanyl)-1-{[rel(2R,3S)-3-(2-chlorphenyl)-2-(2,4-difluorphenyl)oxiran-2-yl]methyl}-1H-1,2,4-triazol, (1.75) 5-(Allylsulfanyl)-1-{[rel(2R,3R)-3-(2-chlorphenyl)-2-(2,4-difluorphenyl)oxiran-2-yl]methyl}-1H-1,2,4-triazol, (1.76) 2-[(2S,4S,5S)-1-(2,4-Dichlorphenyl)-5-hydroxy-2,6,6-trimethylheptan-4-yl]-2,4-dihydro-3H-1,2,4-triazol-3-thion, (1.77) 2-[(2R,4S,5S)-1-(2,4-Dichlorphenyl)-5-hydroxy-2,6,6-trimethylheptan-4-yl]-2,4-dihydro-3H-1,2,4-triazol-3-thion, (1.78) 2-[(2R,4R,5R)-1-(2,4-Dichlorphenyl)-5-hydroxy-2,6,6-trimethylheptan-4-yl]-2,4-dihydro-3H-1,2,4-triazol-3-thion, (1.79) 2-[(2S,4R,5R)-1-(2,4-Dichlorphenyl)-5-hydroxy-2,6,6-trimethylheptan-4-yl]-2,4-dihydro-3H-1,2,4-triazol-3-thion, (1.80) 2-[(2S,4S,5R)-1-(2,4-Dichlorphenyl)-5-hydroxy-2,6,6-trimethylheptan-4-yl]-2,4-dihydro-3H-1,2,4-triazol-3-thion, (1.81) 2-[(2R,4S,5R)-1-(2,4-Dichlorphenyl)-5-hydroxy-2,6,6-trimethylheptan-4-yl]-2,4-dihydro-3H-1,2,4-triazol-3-thion, (1.82) 2-[(2R,4R,5S)-1-(2,4-Dichlorphenyl)-5-hydroxy-2,6,6-trimethylheptan-4-yl]-2,4-dihydro-3H-1,2,4-triazol-3-thion, (1.83) 2-[(2S,4R,5S)-1-(2,4-Dichlorphenyl)-5-hydroxy-2,6,6-trimethylheptan-4-yl]-2,4-dihydro-3H-1,2,4-triazol-3-thion, (1.84) 2-[4-(4-Chlorphenoxy)-2-(trifluormethyl)phenyl]-1-(1H-1,2,4-triazol-1-yl)propan-2-ol, (1.85) 2-[4-(4-Chlorphenoxy)-2-(trifluormethyl)phenyl]-1-(1H-1,2,4-triazol-1-yl)butan-2-ol, (1.86) 2-[4-(4-Chlorphenoxy)-2-(trifluormethyl)phenyl]-1-(1H-1,2,4-triazol-1-yl)pentan-2-ol, (1.87) 2-[2-Chlor-4-(4-chlorphenoxy)phenyl]-1-(1H-1,2,4-triazol-1-yl)butan-2-ol, (1.88) 2-[2-Chlor-4-(2,4-dichlorphenoxy)phenyl]-1-(1H-1,2,4-triazol-1-yl)propan-2-ol, (1.89) (2R)-2-(1-Chlorcyclopropyl)-4-[(1R)-2,2-dichlorcyclopropyl]-1-(1H-1,2,4-triazol-1-yl)butan-2-ol, (1.90) (2R)-2-(1-Chlorcyclopropyl)-4-[(1S)-2,2-dichlorcyclopropyl]-1-(1H-1,2,4-triazol-1-yl)butan-2-ol, (1.91) (2S)-2-(1-Chlorcyclopropyl)-4-[(1S)-2,2-dichlorcyclopropyl]-1-(1H-1,2,4-triazol-1-yl)butan-2-ol, (1.92) (2S)-2-(1-Chlorcyclopropyl)-4-[(1R)-2,2-dichlorcyclopropyl]-1-(1H-1,2,4-triazol-1-yl)butan-2-ol, (1.93) (1S,2R,5R)-5-(4-Chlorbenzyl)-2-(chlormethyl)-2-methyl-1-(1H-1,2,4-triazol-1-ylmethyl)cyclopentanol, (1.94) (1R,2S,5S)-5-(4-Chlorbenzyl)-2-(chlormethyl)-2-methyl-1-(1H-1,2,4-triazol-1-ylmethyl)cyclopentanol, (1.95) 5-(4-Chlorbenzyl)-2-(chlormethyl)-2-methyl-1-(1H-1,2,4-triazol-1-ylmethyl)cyclopentanol.
2) Inhibitoren der Atmungskette am Komplex I oder II, zum Beispiel (2.01) Bixafen, (2.02) Boscalid, (2.03) Carboxin, (2.04) Diflumetorim, (2.05) Fenfuram, (2.06) Fluopyram, (2.07) Flutolanil, (2.08) Fluxapyroxad, (2.09) Furametpyr, (2.10) Furmecyclox, (2.11) Isopyrazam (Mischung von syn-epimerem Racemat 1RS,4SR,9RS und anti-epimerem Racemat 1RS,4SR,9SR), (2.12) Isopyrazam (anti-epimeres Racemat 1RS,4SR,9SR), (2.13) Isopyrazam (anti-epimeres Enantiomer 1R,4S,9S), (2.14) Isopyrazam (anti-epimeres Enantiomer 1S,4R,9R), (2.15) Isopyrazam (syn-epimeres Racemat 1RS,4SR,9RS), (2.16) Isopyrazam (syn-epimeres Enantiomer 1R,4S,9R), (2.17) Isopyrazam (syn-epimeres Enantiomer 1S,4R,9S), (2.18) Mepronil, (2.19) Oxycarboxin, (2.20) Penflufen, (2.21) Penthiopyrad, (2.22) Sedaxan, (2.23) Thifluzamid, (2.24) 1-Methyl-N-[2-(1,1,2,2-tetrafluorethoxy)phenyl]-3-(trifluormethyl)-1H-pyrazol-4-carboxamid, (2.25) 3-(Difluormethyl)-1-methyl-N-[2-(1,1,2,2-tetrafluorethoxy)phenyl]-1H-pyrazol-4-carboxamid, (2.26) 3-(Difluormethyl)-N-[4-fluor-2-(1,1,2,3,3,3-hexafluorpropoxy)phenyl]-1-methyl-1H-pyrazol-4-carboxamid, (2.27) N-[1-(2,4-Dichlorphenyl)-1-methoxypropan-2-yl]-3-(difluormethyl)-1-methyl-1H-pyrazol-4-carboxamid, (2.28) 5,8-Difluor-N-[2-(2-fluor-4-{ [4-(trifluormethyl)pyridin-2-yl]oxy}phenyl)ethyl]chinazolin-4-amin, (2.29) Benzovindiflupyr, (2.30) N-[(1S,4R)-9-(Dichlormethylen)-1,2,3,4-tetrahydro-1,4-methanonaphthalin-5-yl]-3-(difluormethyl)-1-methyl-1H-pyrazol-4-carboxamid, (2.31) N-[(1R,4S)-9-(Dichlormethylen)-1 ,2,3,4-tetrahydro-1 ,4-methanonaphthalin-5-yl]-3-(difluormethyl)-1-methyl-1H-pyrazol-4-carboxamid, (2.32) 3-(Difluormethyl)-1-methyl-N-(1,1,3-trimethyl-2,3-dihydro-1H-inden-4-yl)-1H-pyrazol-4-carboxamid, (2.33) 1,3,5-Trimethyl-N-(1,1,3-trimethyl-2,3-dihydro-1H-inden-4-yl)-1H-pyrazol-4-carboxamid, (2.34) 1-Methyl-3-(trifluormethyl)-N-(1,1,3-trimethyl-2,3-dihydro-1H-inden-4-yl)-1H-pyrazol-4-carboxamid, (2.35) 1-Methyl-3-(trifluormethyl)-N-[(3R)-1,1,3-trimethyl-2,3-dihydro-1H-inden-4-yl]-1H-pyrazol-4-carboxamid, (2.36) 1-Methyl-3-(trifluormethyl)-N-[(3S)-1,1,3-trimethyl-2,3-dihydro-1H-inden-4-yl]-1H-pyrazol-4-carboxamid, (2.37) 3-(Difluormethyl)-1-methyl-N-[(3S)-1,1,3-trimethyl-2,3-dihydro-1H-inden-4-yl]-1H-pyrazol-4-carboxamid, (2.38) 3-(Difluormethyl)-1-methyl-N-[(3R)-1,1,3-trimethyl-2,3-dihydro-1H-inden-4-yl]-1H-pyrazol-4-carboxamid, (2.39) 1,3,5-Trimethyl-N-[(3R)-1,1,3-trimethyl-2,3-dihydro-1H-inden-4-yl]-1H-pyrazol-4-carboxamid, (2.40) 1,3,5-Trimethyl-N-[(3S)-1,1,3-trimethyl-2,3-dihydro-1H-inden-4-yl]-1H-pyrazol-4-carboxamid, (2.41) Benodanil, (2.42) 2-Chlor-N-(1,1,3-trimethyl-2,3-dihydro-1H-inden-4-yl)pyridin-3-carboxamid, (2.43) Isofetamid, (2.44) 1-Methyl-3-(trifluormethyl)-N-[2'-(trifluormethyl)biphenyl-2-yl]-1H-pyrazol-4-carboxamid, (2.45) N-(4'-Chlorbiphenyl-2-yl)-3-(difluormethyl)-1-methyl-1H -pyrazol-4-carboxamid, (2.46) N-(2',4'-Dichlorbiphenyl-2-yl)-3-(difluormethyl)-1-methyl-1H-pyrazol-4-carboxamid, (2.47) 3-(Difluormethyl)-1-methyl-N-[4'-(trifluormethyl)biphenyl-2-yl]-1H-pyrazol-4-carboxamid, (2.48) N-(2',5'-Difluorbiphenyl-2-yl)-1-methyl-3-(trifluormethyl)-1H-pyrazol-4-carboxamid, (2.49) 3-(Difluormethyl)-1-methyl-N-[4'-(prop-1-in-1-yl)biphenyl-2-yl]-1H-pyrazol-4-carboxamid, (2.50) 5-Fluor-1,3-dimethyl-N-[4'-(prop-1-in-1-yl)biphenyl-2-yl] -1H-pyrazol-4-carboxamid, (2.51) 2-Chlor-N- [4'-(prop-1 -in-1 -yl)biphenyl-2-yl]nicotinamid, (2.52) 3-(Difluormethyl)-N-[4'-(3,3-dimethylbut-1-in-1-yl)biphenyl-2-yl]-1-methyl-1H-pyrazol-4-carboxamid, (2.53) N-[4'-(3,3-Dimethylbut-1-in-1-yl)biphenyl-2-yl]-5-fluor-1,3-dimethyl-1H-pyrazol-4-carboxamid, (2.54) 3-(Difluormethyl)-N-(4'-ethinylbiphenyl-2-yl)-1-methyl-1H-pyrazol-4-carboxamid, (2.55) N-(4'-Ethinylbiphenyl-2-yl)-5-fluor-1,3-dimethyl-1H-pyrazol-4-carboxamid, (2.56) 2-Chlor-N-(4'-ethinylbiphenyl-2-yl)nicotinamid, (2.57) 2-Chlor-N-[4'-(3,3-dimethylbut-1-in-1-yl)biphenyl-2-yl]nicotinamid, (2.58) 4-(Difluormethyl)-2-methyl-N-[4'-(trifluormethyl)biphenyl-2-yl]-1,3-thiazol-5-carboxamid, (2.59) 5-Fluor-N-[4'-(3-hydroxy-3-methylbut-1-in-1-yl)biphenyl-2-yl]-1,3-dimethyl-1H-pyrazol-4-carboxamid, (2.60) 2-Chlor-N-[4'-(3-hydroxy-3-methylbut-1-in-1-yl)biphenyl-2-yl]nicotinamid, (2.61) 3-(Difluormethyl)-N-[4'-(3-methoxy-3-methylbut-1-in-1-yl)biphenyl-2-yl]-1-methyl-1H-pyrazol-4-carboxamid, (2.62) 5-Fluor-N-[4'-(3-methoxy-3-methylbut-1-in-1-yl)biphenyl-2-yl]-1,3-dimethyl-1H-pyrazol-4-carboxamid, (2.63) 2-Chlor-N-[4'-(3-methoxy-3-methylbut-1-in-1-yl)biphenyl-2-yl]nicotinamid, (2.64) 1,3-Dimethyl-N-(1,1,3-trimethyl-2,3-dihydro-1H-inden-4-yl)-1H-pyrazol-4-carboxamid, (2.65) 1,3-Dimethyl-N-[(3R)-1,1,3-trimethyl-2,3-dihydro-1H-inden-4-yl]-1H-pyrazol-4-carboxamid, (2.66) 1,3-Dimethyl-N-[(3S)-1,1,3-trimethyl-2,3-dihydro-1H-inden-4-yl]-1H-pyrazol-4-carboxamid, (2.67) 3-(Difluormethyl)-N-methoxy-1-methyl-N-[1-(2,4,6-trichlorphenyl)propan-2-yl]-1H-pyrazol-4-carboxamid, (2.68) 3-(Difluormethyl)-N-(7-fluor-1,1,3-trimethyl-2,3-dihydro-1H-inden-4-yl)-1-methyl-1H-pyrazol-4-carboxamid, (2.69) 3-(Difluormethyl)-N-[(3R)-7-fluor-1,1,3-trimethyl-2,3-dihydro-1H-inden-4-yl]-1-methyl-1H-pyrazol-4-carboxamid, (2.70) 3-(Difluormethyl)-N-[(3S)-7-fluor-1,1,3-trimethyl-2,3-dihydro-1H-inden-4-yl]-1-methyl-1H-pyrazol-4-carboxamid.
3) Inhibitoren der Atmungskette am Komplex III, zum Beispiel (3.01) Ametoctradin, (3.02) Amisulbrom, (3.03) Azoxystrobin, (3.04) Cyazofamid, (3.05) Coumethoxystrobin, (3.06) Coumoxystrobin, (3.07) Dimoxystrobin, (3.08) Enoxastrobin, (3.09) Famoxadon, (3.10) Fenamidon, (3.11) Flufenoxystrobin, (3.12) Fluoxastrobin, (3.13) Kresoxim-methyl, (3.14) Metominostrobin, (3.15) Orysastrobin, (3.16) Picoxystrobin, (3.17) Pyraclostrobin, (3.18) Pyrametostrobin, (3.19) Pyraoxystrobin, (3.20) Pyribencarb, (3.21) Triclopyricarb, (3.22) Trifloxystrobin, (3.23) (2E)-2-(2-{ [6-(3-Chlor-2-methylphenoxy)-5-fluorpyrimidin-4-yl]oxy}phenyl)-2-(methoxyimino)-N-methylacetamid, (3.24) (2E)-2-(Methoxyimino)-N-methyl-2-(2-{[({(1E)-1-[3-(trifluormethyl)phenyl]ethyliden}-amino)oxy]methyl}phenyl)acetamid, (3.25) (2E)-2-(Methoxyimino)-N-methyl-2-{2-[(E)-({1-[3-(trifluormethyl)phenyl]ethoxy}imino)methyl]phenyl}acetamid, (3.26) (2E)-2-{2-[({[(1E)-1-(3-1[(E)-1-Fluor-2-phenylvinyl]oxy}phenyl)ethyliden]amino}oxy)methyl]phenyl}-2-(methoxyimino)-N-methylacetamid, (3.27) Fenaminostrobin, (3.28) 5-Methoxy-2-methyl-4-(2-{[({(1E)-1-[3-(trifluormethyl)phenyl]ethyliden}amino)oxy]methyl}phenyl)-2,4-dihydro-3H-1,2,4-triazol-3-on, (3.29) (2E)-2-{2-[({Cyclopropyl[(4-methoxyphenyl)imino]methyl}sulfanyl)methyl]phenyl}-3-methoxyacrylsäuremethylester, (3.30) N-(3-Ethyl-3,5,5-trimethylcyclohexyl)-3-formamido-2-hydroxybenzamid, (3.31) 2-{2-[(2,5-Dimethylphenoxy)methyl]phenyl}-2-methoxy-N-methylacetamid, (3.32) 2-{2-[(2,5-Dimethylphenoxy)methyl]phenyl}-2-methoxy-N-methylacetamid, (3.33) (2E,3Z)-5-{[1-(4-Chlorphenyl)-1H-pyrazol-3-yl]oxy}-2-(methoxyimino)-N,3-dimethylpent-3-enamid.
4) Inhibitoren der Mitose und Zellteilung, zum Beispiel (4.01) Benomyl, (4.02) Carbendazim, (4.03) Chlorfenazol, (4.04) Diethofencarb, (4.05) Ethaboxam, (4.06) Fluopicolid, (4.07) Fuberidazol, (4.08) Pencycuron, (4.09) Thiabendazol, (4.10) Thiophanat-methyl, (4.11) Thiophanat, (4.12) Zoxamid, (4.13) 5-Chlor-7-(4-methylpiperidin-1-yl)-6-(2,4,6-trifluorphenyl)[1,2,4]triazolo[1,5-a]pyrimidin, (4.14) 3-Chlor-5-(6-chlorpyridin-3-yl)-6-methyl-4-(2,4,6-trifluorphenyl)pyridazin.
5) Verbindungen, die dazu fähig sind, an mehreren Stellen anzugreifen ("Multisite Action"), zum Beispiel (5.01) Bordeaux-Mischung, (5.02) Captafol, (5.03) Captan, (5.04) Chlorothalonil, (5.05) Kupferhydroxid, (5.06) Kupfernaphthenat, (5.07) Kupferoxid, (5.08) Kupferoxychlorid, (5.09) Kupfer(2+)-sulfat, (5.10) Dichlofluanid, (5.11) Dithianon, (5.12) Dodin, (5.13) Dodin freie Base, (5.14) Ferbam, (5.15) Fluorofolpet, (5.16) Folpet, (5.17) Guazatin, (5.18) Guazatinacetat, (5.19) Iminoctadin, (5.20) Iminoctadinalbesilat, (5.21) Iminoctadintriacetat, (5.22) Mancopper, (5.23) Mancozeb, (5.24) Maneb, (5.25) Metiram, (5.26) Metiram-Zink, (5.27) Oxin-Kupfer, (5.28) Propamidin, (5.29) Propineb, (5.30) Schwefel und Schwefelzubereitungen einschließlich Calciumpolysulfid, (5.31) Thiram, (5.32) Tolylfluanid, (5.33) Zineb, (5.34) Ziram, (5.35) Anilazin.
6) Verbindungen, die dazu fähig sind, eine Abwehrreaktion des Wirtes zu induzieren, zum Beispiel (6.01) Acibenzolar-S-methyl, (6.02) Isotianil, (6.03) Probenazol, (6.04) Tiadinil, (6.05) Laminarin.
7) Inhibitoren der Aminosäure- und/oder Proteinbiosynthese, zum Beispiel (7.01) Andoprim, (7.02) Blasticidin-S, (7.03) Cyprodinil, (7.04) Kasugamycin, (7.05) Kasugamycinhydrochlorid-hydrat, (7.06) Mepanipyrim, (7.07) Pyrimethanil, (7.08) 3-(5-Fluor-3,3,4,4-tetramethyl-3,4-dihydroisochinolin-1-yl)chinolin, (7.09) Oxytetracyclin, (7.10) Streptomycin.
8) Inhibitoren der ATP-Produktion, zum Beispiel (8.01) Fentinacetat, (8.02) Fentinchlorid, (8.03) Fentinhydroxid, (8.04) Silthiofam.
9) Inhibitoren der Zellwandsynthese, zum Beispiel (9.01) Benthiavalicarb, (9.02) Dimethomorph, (9.03) Flumorph, (9.04) Iprovalicarb, (9.05) Mandipropamid, (9.06) Polyoxine, (9.07) Polyoxorim, (9.08) Validamycin A, (9.09) Valifenalat, (9.10) Polyoxin B, (9.11) (2E)-3-(4-tert.-Butylphenyl)-3-(2-chlorpyridin-4-yl)-1-(morpholin-4-yl)prop-2-en-1-on, (9.12) (2Z)-3-(4-tert.-Butylphenyl)-3-(2-chlorpyridin-4-yl)-1-(morpholin-4-yl)prop-2-en-1-on.
10) Inhibitoren der Lipid- und Membransynthese, zum Beispiel (10.01) Biphenyl, (10.02) Chloroneb, (10.03) Dicloran, (10.04) Edifenphos, (10.05) Etridiazol, (10.06) Iodocarb, (10.07) Iprobenfos, (10.08) Isoprothiolan, (10.09) Propamocarb, (10.10) Propamocarbhydrochlorid, (10.11) Prothiocarb, (10.12) Pyrazophos, (10.13) Quintozen, (10.14) Tecnazen, (10.15) Tolclofos-methyl.
11) Inhibitoren der Melaninbiosynthese, zum Beispiel (11.01) Carpropamid, (11.02) Diclocymet, (11.03) Fenoxanil, (11.04) Phthalid, (11.05) Pyroquilon, (11.06) Tricyclazol, (11.07) 2,2,2-Trifluorethyl {3-methyl-1-[(4-methylbenzoyl)amino]butan-2-yl}carbamat.
12) Inhibitoren der Nukleinsäuresynthese, zum Beispiel (12.01) Benalaxyl, (12.02) Benalaxyl-M (Kiralaxyl), (12.03) Bupirimat, (12.04) Clozylacon, (12.05) Dimethirimol, (12.06) Ethirimol, (12.07) Furalaxyl, (12.08) Hymexazol, (12.09) Metalaxyl, (12.10) Metalaxyl-M (Mefenoxam), (12.11) Ofurace, (12.12) Oxadixyl, (12.13) Oxolinsäure, (12.14) Octhilinon.
13) Inhibitoren der Signalvermittlung, zum Beispiel (13.01) Chlozolinat, (13.02) Fenpiclonil, (13.03) Fludioxonil, (13.04) Iprodion, (13.05) Procymidon, (13.06) Quinoxyfen, (13.07) Vinclozolin, (13.08) Proquinazid.
14) Verbindungen, die als Entkoppler wirken können, zum Beispiel (14.01) Binapacryl, (14.02) Dinocap, (14.03) Ferimzon, (14.04) Fluazinam, (14.05) Meptyldinocap.
15) Weitere Verbindungen, zum Beispiel (15.001) Benthiazol, (15.002) Bethoxazin, (15.003) Capsimycin, (15.004) Carvon, (15.005) Chinomethionat, (15.006) Pyriofenon (Chlazafenon), (15.007) Cufraneb, (15.008) Cyflufenamid, (15.009) Cymoxanil, (15.010) Cyprosulfamid, (15.011) Dazomet, (15.012) Debacarb, (15.013) Dichlorophen, (15.014) Diclomezin, (15.015) Difenzoquat, (15.016) Difenzoquatmetilsulfat, (15.017) Diphenylamin, (15.018) Ecomat, (15.019) Fenpyrazamin, (15.020) Flumetover, (15.021) Fluoroimid, (15.022) Flusulfamid, (15.023) Flutianil, (15.024) Fosetyl-Aluminium, (15.025) Fosetyl-Calcium, (15.026) Fosetyl-Natrium, (15.027) Hexachlorbenzol, (15.028) Irumamycin, (15.029) Methasulfocarb, (15.030) Methylisothiocyanat, (15.031) Metrafenon, (15.032) Mildiomycin, (15.033) Natamycin, (15.034) Nickeldimethyldithiocarbamat, (15.035) Nitrothalisopropyl, (15.036) Oxamocarb, (15.037) Oxyfenthiin, (15.038) Pentachlorphenol und Salze, (15.039) Phenothrin, (15.040) phosphorige Säure und deren Salze, (15.041) Propamocarb-fosetylat, (15.042) Propanosin-Natrium, (15.043) Pyrimorph, (15.044) Pyrrolnitrin, (15.045) Tebufloquin, (15.046) Tecloftalam, (15.047) Tolnifanid, (15.048) Triazoxid, (15.049) Trichlamid, (15.050) Zarilamid, (15.051) 2-Methylpropansäure-(3S,6S,7R,8R)-8-benzyl-3-[({3-[(isobutyryloxy)methoxy]-4-methoxypyridin-2-yl}carbonyl)amino]-6-methyl-4,9-dioxo-1,5-dioxonan-7-ylester, (15.052) 1-(4-{4-[(5R)-5-(2,6-Difluorphenyl)-4,5-dihydro-1,2-oxazol-3-yl]-1,3-thiazol-2-yl}piperidin-1-yl)-2-[5-methyl-3-(trifluormethyl)-1H-pyrazol-1-yl]ethanon, (15.053) 1-(4-{4-[(5S)-5-(2,6-Difluorphenyl)-4,5-dihydro-1,2-oxazol-3-yl]-1,3-thiazol-2-yl}piperidin-1-yl)-2-[5-methyl-3-(trifluormethyl)-1H-pyrazol-1-yl]ethanon, (15.054) Oxathiapiprolin, (15.055) 1H-Imidazol-1-carbonsäure-1-(4-methoxyphenoxy)-3,3-dimethylbutan-2-ylester, (15.056) 2,3,5,6-Tetrachlor-4-(methylsulfonyl)pyridin, (15.057) 2,3-Dibutyl-6-chlorthieno[2,3-d]pyrimidin-4(3H)-on, (15.058) 2,6-Dimethyl-1H,5H-[1,4]dithiino[2,3-c:5,6-c']dipyrrol-1,3,5,7(2H,6H)-tetron, (15.059) 2-[5-Methyl-3-(trifluormethyl)-1H-pyrazol-1-yl]-1-(4-{4-[(5R)-5-phenyl-4,5-dihydro-1,2-oxazol-3-yl]-1,3-thiazol-2-yl}piperidin-1-yl)ethanon, (15.060) 2-[5-Methyl-3-(trifluormethyl)-1H-pyrazol-1-yl]-1-(4-{4-[(5S)-5-phenyl-4,5-dihydro-1,2-oxazol-3-yl]-1,3-thiazol-2-yl}piperdin-1-yl)ethanon, (15.061) 2-[5-Methyl-3-(trifluormethyl)-1H-pyrazol-1-yl]-1-{4-[4-(5-phenyl-4,5-dihydro-1,2-oxazol-3-yl)-1,3-thiazol-2-yl]piperidin-1-yl}ethanon, (15.062) 2-Butoxy-6-iod-3-propyl-4H-chromen-4-on, (15.063) 2-Chlor-5-[2-chlor-1-(2,6-difluor-4-methoxyphenyl)-4-methyl-1H-imidazol-5-yl]pyridin, (15.064) 2-Phenylphenol und Salze, (15.065) 3-(4,4,5-Trifluor-3,3-dimethyl-3,4-dihydroisochinolin-1-yl)chinolin, (15.066) 3,4,5-Trichlorpyridin-2,6-dicarbonsäurenitril, (15.067) 3-Chlor-5-(4-chlorphenyl)-4-(2,6-difluorphenyl)-6-methylpyridazin, (15.068) 4-(4-Chlorphenyl)-5-(2,6-difluorphenyl)-3,6-dimethylpyridazin, (15.069) 5-Amino-1,3,4-thiadiazol-2-thiol, (15.070) 5-Chlor-N'-phenyl-N'-(prop-2-in-1-yl)thiophen-2-sulfonohydrazid, (15.071) 5-Fluor-2-[(4-fluorbenzyl)oxy]pyrimidin-4-amin, (15.072) 5-Fluor-2-[(4-methylbenzyl)oxy]pyrimidin-4-amin, (15.073) 5-Methyl-6-octyl[1,2,4]triazolo[1,5-a]pyrimidin-7-amin, (15.074) (2Z)-3-Amino-2-cyano-3-phenylacrylsäureethylester, (15.075) N'-(4-{[3-(4-Chlorbenzyl)-1,2,4-thiadiazol-5-yl]oxy}-2,5-dimethylphenyl)-N-ethyl-N-methylimidoformamid, (15.076) N-(4-Chlorbenzyl)-3-[3-methoxy-4-(prop-2-in-1-yloxy)phenyl]propanamid, (15.077) N-[(4-Chlorphenyl)(cyano)methyl]-3-[3-methoxy-4-(prop-2-in-1-yloxy)phenyl]propanamid, (15.078) N-[(5-Brom-3-chlorpyridin-2-yl)methyl]-2,4-dichlornicotinamid, (15.079) N-[1 -(5-Brom-3-chlorpyridin-2-yl)ethyl] -2,4-dichlornicotinamid, (15.080) N-[1-(5-Brom-3 -chlorpyridin-2-yl)ethyl] -2-fluor-4-iodnicotinamid, (15.081) N-{(E)-[(Cyclopropylmethoxy)imino][6-(difluormethoxy)-2,3-difluorphenyl]methyl}-2-phenylacetamid, (15.082) N-{(Z)-[(Cyclopropylmethoxy)imino][6-(difluormethoxy)-2,3-difluorphenyl]methyl}-2-phenylacetamid, (15.083) N'-{4-[(3-tert.-Butyl-4-cyano-1,2-thiazol-5-yl)oxy]-2-chlor-5-methylphenyl}-N-ethyl-N-methylimidoformamid, (15.084) N-Methyl-2-(1-{[5-methyl-3-(trifluormethyl)-1H-pyrazol-1-yl]acetyl}piperidin-4-yl)-N-(1,2,3,4-tetrahydronaphthalin-1-yl)-1,3-thiazol-4-carboxamid, (15.085) N-Methyl-2-(1-{[5-methyl-3-(trifluormethyl)-1H-pyrazol-1-yl]acetyl}piperidin-4-yl)-N-[(1R)-1,2,3,4-tetrahydronaphthalin-1-yl]-1,3-thiazol-4-carboxamid, (15.086) N-Methyl-2-(1-{[5-methyl-3-(trifluormethyl)-1H-pyrazol-1-yl]acetyl}piperidin-4-yl)-N-[(1S)-1,2,3,4-tetrahydronaphthalin-1-yl]-1,3-thiazol-4-carboxamid, (15.087) {6-[({[(1-Methyl-1H-tetrazol-5-yl)(phenyl)methylen]amino}oxy)methyl]pyridin-2-yl}carbamidsäurepentylester, (15.088) Phenazin-1-carbonsäure, (15.089) Chinolin-8-ol, (15.090) Chinolin-8-olsulfat (2:1), (15.091) {6-[({[(1-Methyl-1H-tetrazol-5-yl)(phenyl)methylen]amino}oxy)methyl]pyridin-2-yl}carbamidsäure-tert.-butylester, (15.092) (5-Brom-2-methoxy-4-methylpyridin-3-yl)(2,3,4-trimethoxy-6-methylphenyl)methanon, (15.093) N-[2-(4-{[3-(4-Chlorphenyl)prop-2-in-1-yl]oxy}-3-methoxyphenyl)ethyl]-N2-(methylsulfonyl)valinamid, (15.094) 4-Oxo-4-[(2-phenylethyl)amino]butansäure, (15.095) {6-[({[(Z)-(1-Methyl-1H-tetrazol-5-yl)(phenyl)methylen]amino}oxy)methyl]pyridin-2-yl}carbamidsäurebut-3-in-1-ylester, (15.096) 4-Amino-5-fluorpyrimidin-2-ol (tautomere Form: 4-Amino-5-fluorpyrimidin-2(1H)-on), (15.097) 3,4,5-Trihydroxybenzoesäurepropylester, (15.098) [3-(4-Chlor-2-fluorphenyl)-5-(2,4-difluorphenyl)-1,2-oxazol-4-yl](pyridin-3-yl)methanol, (15.099) (S)-[3-(4-Chlor-2-fluorphenyl)-5-(2,4-difluorphenyl)-1,2-oxazol-4-yl](pyridin-3-yl)methanol, (15.100) (R)-[3-(4-Chlor-2-fluorphenyl)-5-(2,4-difluorphenyl)-1,2-oxazol-4-yl](pyridin-3-yl)methanol, (15.101) 2-Fluor-6-(trifluormethyl)-N-(1,1,3-trimethyl-2,3-dihydro-1H-inden-4-yl)benzamid, (15.102) 2-(6-Benzylpyridin-2-yl)chinazolin, (15.103) 2-[6-(3-Fluor-4-methoxyphenyl)-5-methylpyridin-2-yl]chinazolin, (15.104) 3-(4,4-Difluor-3,3-dimethyl-3,4-dihydroisochinolin-1-yl)chinolin, (15.105) Abscisinsäure, (15.106) N'-[5-Brom-6-(2,3-dihydro-1H-inden-2-yloxy)-2-methylpyridin-3-yl]-N-ethyl-N-methylimidoformamid, (15.107) N'-{5-Brom-6-[1-(3,5-difluorphenyl)ethoxy] -2-methylpyridin-3-yl} -N-ethyl-N-methylimidoformamid, (15.108) N'-{5-Brom-6-[(1R)-1-(3,5-difluorphenyl)ethoxy]-2-methylpyridin-3-yl}-N-ethyl-N-methylimidoformamid, (15.109) N'-{5-Brom-6-[(1S)-1-(3,5-difluorphenyl)ethoxy] -2-methylpyridin-3 -yl} -N-ethyl-N-methylimidoformamid, (15.110) N'-{5-Brom-6-[(cis-4-isopropylcyclohexyl)oxy]-2-methylpyridin-3-yl}-N-ethyl-N-methylimidoformamid, (15.111) N'-{5-Brom-6-[(trans-4-isopropylcyclohexyl)oxy]-2-methylpyridin-3-yl}-N-ethyl-N-methylimidoformamid, (15.112) N-Cyclopropyl-3-(difluormethyl)-5-fluor-N-(2-isopropylbenzyl)-1-methyl-1H-pyrazol-4-carboxamid, (15.113) N-Cyclopropyl-N-(2-cyclopropylbenzyl)-3-(difluormethyl)-5-fluor-1-methyl-1H-pyrazol-4-carboxamid, (15.114) N-(2-tert.-Butylbenzyl)-N-cyclopropyl-3-(difluormethyl)-5-fluor-1-methyl-1H-pyrazol-4-carboxamid, (15.115) N-(5-Chlor-2-ethylbenzyl)-N-cyclopropyl-3-(difluormethyl)-5-fluor-1-methyl-1H-pyrazol-4-carboxamid, (15.116) N-(5-Chlor-2-isopropylbenzyl)-N-cyclopropyl-3-(difluormethyl)-5-fluor-1-methyl-1H-pyrazol-4-carboxamid, (15.117) N-Cyclopropyl-3-(difluormethyl)-N-(2-ethyl-5-fluorbenzyl)-5-fluor-1-methyl-1H-pyrazol-4-carboxamid, (15.118) N-Cyclopropyl-3-(difluormethyl)-5-fluor-N-(5-fluor-2-isopropylbenzyl)-1-methyl-1H-pyrazol-4-carboxamid, (15.119) N-Cyclopropyl-N-(2-cyclopropyl-5-fluorbenzyl)-3 -(difluormethyl)-5-fluor-1-methyl-1H-pyrazol-4-carboxamid, (15.120) N-(2-Cyclopentyl-5-fluorbenzyl)-N-cyclopropyl-3-(difluormethyl)-5-fluor-1-methyl-1H-pyrazol-4-carboxamid, (15.121) N-Cyclopropyl-3-(difluormethyl)-5-fluor-N-(2-fluor-6-isopropylbenzyl)-1-methyl-1H-pyrazol-4-carboxamid, (15.122) N-Cyclopropyl-3-(difluormethyl)-N-(2-ethyl-5-methylbenzyl)-5-fluor-1-methyl-1H-pyrazol-4-carboxamid, (15.123) N-Cyclopropyl-3-(difluormethyl)-5-fluor-N-(2-isopropyl-5-methylbenzyl)-1 -methyl-1H-pyrazol-4-carboxamid, (15.124) N-Cyclopropyl-N-(2-cyclopropyl-5-methylbenzyl)-3-(difluormethyl)-5-fluor-1-methyl-1H-pyrazol-4-carboxamid, (15.125) N-(2-tert.-Butyl-5-methylbenzyl)-N-cyclopropyl-3-(difluormethyl)-5-fluor-1-methyl-1H-pyrazol-4-carboxamid, (15.126) N-[5-Chlor-2-(trifluormethyl)benzyl]-N-cyclopropyl-3-(difluormethyl)-5-fluor-1-methyl-1H-pyrazol-4-carboxamid, (15.127) N-Cyclopropyl-3-(difluormethyl)-5-fluor-1-methyl-N-[5-methyl-2-(trifluormethyl)benzyl]-1H-pyrazol-4-carboxamid, (15.128) N- [2-Chlor-6-(trifluormethyl)benzyl] -N-cyclopropyl-3-(difluormethyl)-5-fluor-1-methyl-1H-pyrazol-4-carboxamid, (15.129) N-[3-Chlor-2-fluor-6-(trifluormethyl)benzyl]-N-cyclopropyl-3-(difluormethyl)-5-fluor-1-methyl-1H-pyrazol-4-carboxamid, (15.130) N-Cyclopropyl-3-(difluormethyl)-N-(2-ethyl-4,5-dimethylbenzyl)-5-fluor-1-methyl-1H-pyrazol-4-carboxamid, (15.131) N-Cyclopropyl-3-(difluormethyl)-5-fluor-N-(2-isopropylbenzyl)-1-methyl-1H-pyrazol-4-carbothioamid, (15.132) N'-(2,5-Dimethyl-4-phenoxyphenyl)-N-ethyl-N-methylimidoformamid, (15.133) N'-{4-[(4,5-Dichlor-1,3-thiazol-2-yl)oxy]-2,5-dimethylphenyl}-N-ethyl-N-methylimidoformamid, (15.134) N-(4-Chlor-2,6-difluorphenyl)-4-(2-chlor-4-fluorphenyl)-1,3-dimethyl-1H-pyrazol-5-amin, (15.135) 9-Fluor-2,2-dimethyl-5-(chinolin-3-yl)-2,3-dihydro-1,4-benzoxazepin, (15.136) 2-{2-Fluor-6-[(8-fluor-2-methylchinolin-3-yl)oxy]phenyl}propan-2-ol, (15.137) 2-{2-[(7,8-Difluor-2-methylchinolin-3-yl)oxy]-6-fluorphenyl}propan-2-ol, (15.138) 4-(2-Chlor-4-fluorphenyl)-N-(2-fluorphenyl)-1,3-dimethyl-1H-pyrazol-5 -amin, (15.139) 4-(2-Chlor-4-fluorphenyl)-N-(2,6-difluorphenyl)-1,3-dimethyl-1H-pyrazol-5-amin, (15.140) 4-(2-Chlor-4-fluorphenyl)-N-(2-chlor-6-fluorphenyl)-1,3-dimethyl-1H-pyrazol-5-amin, (15.141) 4-(2-Brom-4-fluorphenyl)-N-(2-chlor-6-fluorphenyl)-1,3-dimethyl-1H-pyrazol-5-amin, (15.142) N-(2-Brom-6-fluorphenyl)-4-(2-chlor-4-fluorphenyl)-1,3-dimethyl-1H-pyrazol-5-amin, (15.143) 4-(2-Brom-4-fluorphenyl)-N-(2-bromphenyl)-1,3-dimethyl-1H-pyrazol-5-amin, (15.144) 4-(2-Brom-4-fluorphenyl)-N-(2-brom-6-fluorphenyl)-1,3-dimethyl-1H-pyrazol-5-amin, (15.145) 4-(2-Brom-4-fluorphenyl)-N-(2-chlorphenyl)-1,3-dimethyl-1H-pyrazol-5-amin, (15.146) N-(2-Bromphenyl)-4-(2-chlor-4-fluorphenyl)-1,3-dimethyl-1H-pyrazol-5-amin, (15.147) 4-(2-Chlor-4-fluorphenyl)-N-(2-chlorphenyl)-1,3-dimethyl-1H-pyrazol-5-amin, (15.148) 4-(2-Brom-4-fluorphenyl)-N-(2,6-difluorphenyl)-1,3-dimethyl-1H-pyrazol-5-amin, (15.149) 4-(2-Brom-4-fluorphenyl)-N-(2-fluorphenyl)-1,3-dimethyl-1H-pyrazol-5-amin, (15.150) N'-(4-{3-[(Difluormethyl)sulfanyl]phenoxy}-2,5-dimethylphenyl)-N-ethyl-N-methylimidoformamid, (15.151) N'-(2,5-Dimethyl-4-{3-[(1,1,2,2-tetrafluorethyl)sulfanyl]phenoxy}phenyl)-N-ethyl-N-methylimidoformamid, (15.152) N'-(2,5-Dimethyl-4-{3-[(2,2,2-trifluorethyl)sulfanyl]phenoxy}phenyl)-N-ethyl-N-methylimidoformamid, (15.153) N'-(2,5-Dimethyl-4-{3-[(2,2,3,3-tetrafluorpropyl)sulfanyl]phenoxy}phenyl)-N-ethyl-N-methylimidoformamid, (15.154) N'-(2,5-Dimethyl-4-{3-[(pentafluorethyl)sulfanyl]phenoxy}phenyl)-N-ethyl-N-methylimidoformamid, (15.155) N'-(4-{[3-(Difluormethoxy)phenyl]sulfanyl}-2,5-dimethylphenyl)-N-ethyl-N-methylimidoformamid, (15.156) N'-(2,5-Dimethyl-4-{ [3-(1,1,2,2-tetrafluorethoxy)phenyl]sulfanyl}phenyl)-N-ethyl-N-methylimidoformamid, (15.157) N'-(2,5-Dimethyl-4-{[3-(2,2,2-trifluorethoxy)phenyl]sulfanyl}phenyl)-N-ethyl-N-methylimidoformamid, (15.158) N'-(2,5-Dimethyl-4-{[3-(2,2,3,3-tetrafluorpropoxy)phenyl]sulfanyl}phenyl)-N-ethyl-N-methylimidoformamid, (15.159) N'-(2,5-Dimethyl-4-{[3-(pentafluorethoxy)phenyl]sulfanyl}phenyl)-N-ethyl-N-methylimidoformamid, (15.160) 2-[3,5-Bis(difluormethyl)-1H-pyrazol-1-yl]-1-[4-(4-{5-[2-(prop-2-in-1-yloxy)phenyl]-4,5-dihydro-1,2-oxazol-3-yl}-1,3-thiazol-2-yl)piperidin-1-yl]ethanon, (15.161) 2-[3,5-Bis(difluormethyl)-1H-pyrazol-1-yl]-1-[4-(4-{5-[2-fluor-6-(prop-2-in-1-yloxy)phenyl]-4,5-dihydro-1,2-oxazol-3-yl}-1,3-thiazol-2-yl)piperidin-1-yl]ethanon, (15.162) 2-[3,5-Bis(difluormethyl)-1H-pyrazol-1-yl]-1-[4-(4-{5-[2-chlor-6-(prop-2-in-1-yloxy)phenyl]-4,5-dihydro-1,2-oxazol-3-yl}-1,3-thiazol-2-yl)piperidin-1-yl]ethanon, (15.163) 2-{3-[2-(1-{[3,5-Bis(difluormethyl)-1H-pyrazol-1-yl]acetyl}piperidin-4-yl)-1,3-thiazol-4-yl]-4,5-dihydro-1,2-oxazol-5-yl}phenylmethansulfonat, (15.164) 2-{3-[2-(1-{[3,5-Bis(difluormethyl)-1H-pyrazol-1-yl]acetyl}piperidin-4-yl)-1,3-thiazol-4-yl]-4,5-dihydro-1,2-oxazol-5-yl}-3-chlorphenylmethansulfonat, (15.165) 2-[3,5-Bis(difluormethyl)-1H-pyrazol-1-yl]-1-[4-(4-{(5S)-5-[2-(prop-2-in-1-yloxy)phenyl]-4,5-dihydro-1,2-oxazol-3-yl}-1,3-thiazol-2-yl)piperidin-1-yl]ethanon, (15.166) 2-[3,5-Bis(difluormethyl)-1H-pyrazol-1-yl]-1-[4-(4-{(5R)-5-[2-(prop-2-in-1-yloxy)phenyl]-4,5-dihydro-1,2-oxazol-3-yl}-1,3-thiazol-2-yl)piperidin-1-yl]ethanon, (15.167) 2-[3,5-Bis(difluormethyl)-1H-pyrazol-1-yl]-1-[4-(4-{(5S)-5-[2-fluor-6-(prop-2-in-1-yloxy)phenyl]-4,5-dihydro-1,2-oxazol-3-yl}-1,3-thiazol-2-yl)piperidin-1-yl]ethanon, (15.168) 2-[3,5-Bis(difluormethyl)-1H-pyrazol-1-yl]-1-[4-(4-{(5R)-5-[2-fluor-6-(prop-2-in-1-yloxy)phenyl]-4,5-dihydro-1,2-oxazol-3-yl}-1,3-thiazol-2-yl)piperidin-1-yl]ethanon, (15.169) 2- [3,5-Bis(difluormethyl)-1H-pyrazol-1-yl]-1-[4-(4-{(5S)-5-[2-chlor-6-(prop-2-in-1-yloxy)phenyl]-4,5-dihydro-1,2-oxazol-3-yl}-1,3-thiazol-2-yl)piperidin-1-yl]ethanon, (15.170) 2-[3,5-Bis(difluormethyl)-1H-pyrazol-1-yl]-1-[4-(4-{(5R)-5-[2-chlor-6-(prop-2-in-1-yloxy)phenyl]-4,5-dihydro-1,2-oxazol-3-yl}-1,3-thiazol-2-yl)piperidin-1-yl]ethanon, (15.171) 2-{(5S)-3-[2-(1-{[3,5-Bis(difluormethyl)-1H-pyrazol-1-yl]acetyl}piperidin-4-yl)-1,3-thiazol-4-yl]-4,5-dihydro-1,2-oxazol-5-yl}phenylmethansulfonat, (15.172) 2-{(5R)-3-[2-(1-{[3,5-Bis(difluormethyl)-1H-pyrazol-1-yl]acetyl}piperidin-4-yl)-1,3-thiazol-4-yl]-4,5-dihydro-1,2-oxazol-5-yl}phenylmethansulfonat, (15.173) 2-{(5S)-3-[2-(1-{[3,5-Bis(difluormethyl)-1H-pyrazol-1-yl]acetyl}piperidin-4-yl)-1,3-thiazol-4-yl]-4,5-dihydro-1,2-oxazol-5-yl}-3-chlorphenylmethansulfonat, (15.174) 2-{(5R)-3-[2-(1-{[3,5-Bis(difluormethyl)-1H-pyrazol-1-yl]acetyl}piperidin-4-yl)-1,3-thiazol-4-yl] -4,5-dihydro-1 ,2-oxazol-5-yl} -3-chlorphenylmethansulfonat.

### Biologische Schädlingsbekämpfungsmittel als Mischungspartner

Die Verbindungen der Formel (I) können mit biologischen Schädlingsbekämpfungsmitteln kombiniert werden.

Biologische Schädlingsbekämpfungsmittel umfassen insbesondere Bakterien, Pilze, Hefen, Pflanzenextrakte, und solche Produkte, die von Mikroorganismen gebildet wurden inklusive Proteine und sekundäre Stoffwechselprodukte.

Biologische Schädlingsbekämpfungsmittel umfassen Bakterien wie sporenbildende Bakterien, wurzelbesiedelnde Bakterien und Bakterien, die als biologische Insektizide, Fungizide oder Nematizide wirken.

Beispiele für solche Bakterien, die als biologische Schädlingsbekämpfungsmittel eingesetzt werden bzw. verwendet werden können, sind:
*Bacillus amyloliquefaciens,* Stamm FZB42 (DSM 231179), oder *Bacillus cereus,* insbesondere *B. cereus* Stamm CNCM I-1562 oder *Bacillus firmus,* Stamm I-1582 (Accession number CNCM I-1582) oder
*Bacillus pumilus,* insbesondere Stamm GB34 (Accession No. ATCC 700814) und Stamm QST2808 (Accession No. NRRL B-30087), oder *Bacillus subtilis,* insbesondere Stamm GB03 (Accession No. ATCC SD-1397), oder *Bacillus subtilis* Stamm QST713 (Accession No. NRRL B-21661) oder *Bacillus subtilis* Stamm OST 30002 (Accession No. NRRL B-50421) *Bacillus thuringiensis,* insbesondere *B. thuringiensis* subspecies *israelensis* (serotype H-14), Stamm AM65-52 (Accession No. ATCC 1276), oder *B. thuringiensis subsp. aizawai,* insbesondere Stamm ABTS-1857 (SD-1372), oder *B. thuringiensis subsp. kurstaki* Stamm HD-1, oder *B. thuringiensis subsp. tenebrionis* Stamm NB 176 (SD-5428), *Pasteuria penetrans, Pasteuria spp.* (Rotylenchulus reniformis nematode)-PR3 (Accession Number ATCC SD-5834), *Streptomyces microflavus* Stamm AQ6121 (= QRD 31.013, NRRL B-50550), *Streptomyces galbus* Stamm AQ 6047 (Acession Number NRRL 30232).

Beispiele für Pilze und Hefen, die als biologische Schädlingsbekämpfungsmittel eingesetzt werden bzw. verwendet werden können, sind:
*Beauveria bassiana,* insbesondere Stamm ATCC 74040, *Coniothyrium minitans,* insbesondere Stamm CON/M/91-8 (Accession No. DSM-9660), *Lecanicillium spp.,* insbesondere Stamm HRO LEC 12, *Lecanicillium lecanii,* (ehemals bekannt als *Verticillium lecanii*), insbesondere Stamm KV01, *Metarhizium anisopliae,* insbesondere Stamm F52 (DSM3884/ ATCC 90448), *Metschnikowia fructicola,* insbesondere Stamm NRRL Y-30752, *Paecilomyces fumosoroseus* (neu: *Isaria fumosorosea*), insbesondere Stamm IFPC 200613, oder Stamm Apopka 97 (Accesion No. ATCC 20874), *Paecilomyces lilacinus,* insbesondere *P. lilacinus* Stamm 251 (AGAL 89/030550), *Talaromyces flavus,* insbesondere Stamm V117b, *Trichoderma atroviride,* insbesondere Stamm SC1 (Accession Number CBS 122089), *Trichoderma harzianum,* insbesondere *T. harzianum rifai T39.* (Accession Number CNCM I-952).

Beispiele für Viren, die als biologische Schädlingsbekämpfungsmittel eingesetzt werden bzw. verwendet werden können, sind:
*Adoxophyes orana* (Apfelschalenwickler) Granulosevirus (GV), *Cydia pomonella* (Apfelwickler) Granulosevirus (GV), *Helicoverpa armigera* (Baumwollkapselwurm) Nuklear Polyhedrosis Virus (NPV), *Spodoptera exigua* (Zuckerrübeneule) mNPV, *Spodoptera frugiperda* (Heerwurm) mNPV, *Spodoptera littoralis* (Afrikanischer Baumwollwurm) NPV.

Es sind auch Bakterien und Pilze umfasst, die als 'Inokulant' Pflanzen oder Pflanzenteilen oder Pflanzenorganen beigegeben werden und durch ihre besonderen Eigenschaften das Pflanzenwachstum und die Pflanzengesundheit fördern. Als Beispiele sind genannt:
*Agrobacterium spp., Azorhizobium caulinodans, Azospirillum spp., Azotobacter spp., Bradyrhizobium spp., Burkholderia spp.,* insbesondere *Burkholderia cepacia* (ehemals bekannt als *Pseudomonas cepacia), Gigaspora spp.,* oder *Gigaspora monosporum, Glomus spp., Laccaria spp., Lactobacillus buchneri, Paraglomus spp., Pisolithus tinctorus, Pseudomonas spp., Rhizobium spp.,* insbesondere *Rhizobium trifolii, Rhizopogon spp., Scleroderma spp., Suillus spp., Streptomyces spp..*

Beispiele für Pflanzenextrakte und solche Produkte, die von Mikroorganismen gebildet wurden inklusive Proteine und sekundäre Stoffwechselprodukte, die als biologische Schädlingsbekämpfungsmittel eingesetzt werden bzw. verwendet werden können, sind:
Allium sativum, Artemisia absinthium, Azadirachtin, Biokeeper WP, Cassia nigricans, Celastrus angulatus, Chenopodium anthelminticum, Chitin, Armour-Zen, Dryopteris filix-mas, Equisetum arvense,Fortune Aza, Fungastop, Heads Up (Chenopodium quinoa-Saponinextrakt), Pyrethrum/Pyrethrins, Quassia amara, Quercus, Quillaja, Regalia,"Requiem™ Insecticide", Rotenon, Ryania/Ryanodine, Symphytum officinale, Tanacetum vulgare, Thymol, Triact 70, TriCon, Tropaeulum majus, Urtica dioica, Veratrin, Viscum album, Brassicacaeen-Extrakt, insbesondere Raps- oder Senfpulver.

### Safener als Mischpartner

Die Verbindungen der Formel (I) können mit Safenern kombiniert werden, wie zum Beispiel Benoxacor, Cloquintocet (-mexyl), Cyometrinil, Cyprosulfamide, Dichlormid, Fenchlorazole (-ethyl), Fenclorim, Flurazole, Fluxofenim, Furilazole, Isoxadifen (-ethyl), Mefenpyr (-diethyl), Naphthalic anhydride, Oxabetrinil, 2-Methoxy-N-({4-[(methylcarbamoyl)amino]phenyl}sulfonyl)benzamid (CAS 129531-12-0), 4-(Dichloroacetyl)-1-oxa-4-azaspiro[4.5]decane (CAS 71526-07-3), 2,2,5-Trimethyl-3-(dichloroacetyl)-1,3-oxazolidine (CAS 52836-31-4).

### Pflanzen und Pflanzenteile

Erfindungsgemäß können alle Pflanzen und Pflanzenteile behandelt werden. Unter Pflanzen werden hierbei alle Pflanzen und Pflanzenpopulationen verstanden wie erwünschte und unerwünschte Wildpflanzen oder Kulturpflanzen (einschließlich natürlich vorkommender Kulturpflanzen), beispielsweise Getreide (Weizen, Reis, Triticale, Gerste, Roggen, Hafer), Mais, Soja, Kartoffel, Zuckerrüben, Zuckerrohr, Tomaten, Erbsen und andere Gemüsesorten, Baumwolle, Tabak, Raps, sowie Obstpflanzen (mit den Früchten Äpfel, Birnen, Zitrusfrüchten und Weintrauben). Kulturpflanzen können Pflanzen sein, die durch konventionelle Züchtungs- und Optimierungsmethoden oder durch biotechnologische und gentechnologische Methoden oder Kombinationen dieser Methoden erhalten werden können, einschließlich der transgenen Pflanzen und einschließlich der durch Sortenschutzrechte schützbaren oder nicht schützbaren Pflanzensorten. Unter Pflanzenteilen sollen alle oberirdischen und unterirdischen Teile und Organe der Pflanzen wie Spross, Blatt, Blüte und Wurzel verstanden werden, wobei beispielhaft Blätter, Nadeln, Stängel, Stämme, Blüten, Fruchtkörper, Früchte und Samen sowie Wurzeln, Knollen und Rhizome aufgeführt werden. Zu den Pflanzenteilen gehört auch Erntegut sowie vegetatives und generatives Vermehrungsmaterial, beispielsweise Stecklinge, Knollen, Rhizome, Ableger und Samen.

Die erfindungsgemäße Behandlung der Pflanzen und Pflanzenteile mit den Verbindungen der Formel (I) erfolgt direkt oder durch Einwirkung auf deren Umgebung, Lebensraum oder Lagerraum nach den üblichen Behandlungsmethoden, z.B. durch Tauchen, Sprühen, Verdampfen, Vernebeln, Streuen, Aufstreichen, Injizieren und bei Vermehrungsmaterial, insbesondere bei Saatgut, weiterhin durch ein- oder mehrschichtiges Umhüllen.

Wie bereits oben erwähnt können erfindungsgemäß alle Pflanzen und deren Teile behandelt werden. In einer bevorzugten Ausführungsform werden wild vorkommende oder durch konventionelle biologische Zuchtmethoden wie Kreuzung oder Protoplastenfusion erhaltene Pflanzenarten und Pflanzensorten sowie deren Teile behandelt. In einer weiteren bevorzugten Ausführungsform werden transgene Pflanzen und Pflanzensorten, die durch gentechnologische Methoden gegebenenfalls in Kombination mit konventionellen Methoden erhalten wurden (Genetically Modified Organisms) und deren Teile behandelt. Der Begriff "Teile" bzw. "Teile von Pflanzen" oder "Pflanzenteile" wurde oben erläutert. Besonders bevorzugt werden erfindungsgemäß Pflanzen der jeweils handelsüblichen oder in Gebrauch befindlichen Pflanzensorten behandelt. Unter Pflanzensorten versteht man Pflanzen mit neuen Eigenschaften ("Traits"), die sowohl durch konventionelle Züchtung, durch Mutagenese oder durch rekombinante DNA-Techniken gezüchtet worden sind. Dies können Sorten, Rassen, Bio- und Genotypen sein.

### Transgene Pflanze, Saatgutbehandlung und Integrationsereignisse

Zu den bevorzugten erfindungsgemäß zu behandelnden transgenen (gentechnologisch erhaltenen) Pflanzen bzw. Pflanzensorten gehören alle Pflanzen, die durch die gentechnologische Modifikation genetisches Material erhielten, welches diesen Pflanzen besondere vorteilhafte wertvolle Eigenschaften ("Traits") verleiht. Beispiele für solche Eigenschaften sind besseres Pflanzenwachstum, erhöhte Toleranz gegenüber hohen oder niedrigen Temperaturen, erhöhte Toleranz gegen Trockenheit oder gegen Wasser- bzw. Bodensalzgehalt, erhöhte Blühleistung, erleichterte Ernte, Beschleunigung der Reife, höhere Ernteerträge, höhere Qualität und/oder höherer Ernährungswert der Ernteprodukte, höhere Lagerfähigkeit und/oder Bearbeitbarkeit der Ernteprodukte. Weitere und besonders hervorgehobene Beispiele für solche Eigenschaften sind eine erhöhte Abwehrfähigkeit der Pflanzen gegen tierische und mikrobielle Schädlinge, wie-Insekten, Spinnentiere, Nematoden, Milben, Schnecken, bewirkt z.B. durch in den Pflanzen entstehende Toxine, insbesondere solche, die durch das genetische Material aus Bacillus Thuringiensis (z.B. durch die Gene CryIA(a), CryIA(b), CryIA(c), CryIIA, CryIIIA, CryIIIB2, Cry9c Cry2Ab, Cry3Bb und CryIF sowie deren Kombinationen) in den Pflanzen erzeugt werden, ferner eine erhöhte Abwehrfähigkeit der Pflanzen gegen pflanzenpathogene Pilze, Bakterien und/oder Viren, bewirkt z.B. durch Systemisch Akquirierte Resistenz (SAR), Systemin, Phytoalexine, Elicitoren sowie Resistenzgene und entsprechend exprimierte Proteine und Toxine, sowie eine erhöhte Toleranz der Pflanzen gegen bestimmte herbizide Wirkstoffe, beispielsweise Imidazolinonen, Sulfonyl-harnstoffen, Glyphosate oder Phosphinotricin (z.B. "PAT"-Gen). Die jeweils die gewünschten Eigenschaften ("Traits") verleihenden Gene können auch in Kombinationen miteinander in den transgenen Pflanzen vorkommen. Als Beispiele transgener Pflanzen werden die wichtigen Kulturpflanzen, wie Getreide (Weizen, Reis, Triticale, Gerste, Roggen, Hafer), Mais, Soja, Kartoffel, Zuckerrüben, Zuckerrohr, Tomaten, Erbsen und andere Gemüsesorten, Baumwolle, Tabak, Raps, sowie Obstpflanzen (mit den Früchten Äpfel, Birnen, Zitrusfrüchten und Weintrauben) erwähnt, wobei Mais, Soja, Weizen, Reis, Kartoffel, Baumwolle, Zuckerrohr, Tabak und Raps besonders hervorgehoben werden. Als Eigenschaften ("Traits") werden besonders hervorgehoben die erhöhte Abwehrfähigkeit der Pflanzen gegen Insekten, Spinnentiere, Nematoden und Schnecken.

### Pflanzenschutz - Behandlungsarten

Die Behandlung der Pflanzen und Pflanzenteile mit den Verbindungen der Formel (I) erfolgt direkt oder durch Einwirkung auf deren Umgebung, Lebensraum oder Lagerraum nach den üblichen Behandlungsmethoden, z.B. durch Tauchen, (Ver-) Spritzen, (Ver-)Sprühen, Berieseln, Verdampfen, Zerstäuben, Vernebeln, (Ver-)Streuen, Verschäumen, Bestreichen, Verstreichen, Injizieren, Gießen (drenchen), Tröpfchenbewässerung und bei Vermehrungsmaterial, insbesondere bei Saatgut, weiterhin durch Trockenbeizen, Nassbeizen, Schlämmbeizen, Inkrustieren, ein- oder mehrschichtiges Umhüllen, usw. Es ist ferner möglich, die Verbindungen der Formel (I) nach dem Ultra-Low-Volume-Verfahren auszubringen oder die Anwendungsform oder die Verbindung der Formel (I) selbst in den Boden zu injizieren.

Eine bevorzugte direkte Behandlung der Pflanzen ist die Blattapplikation, d.h. Verbindungen der Formel (I) werden auf das Blattwerk aufgebracht, wobei die Behandlungsfrequenz und die Aufwandmenge auf den Befallsdruck des jeweiligen Schädlings abgestimmt sein sollte.

Bei systemisch wirksamen Verbindungen gelangen die Verbindungen der Formel (I) auch über das Wurzelwerk in die Pflanzen. Die Behandlung der Pflanzen erfolgt dann durch Einwirkung der Verbindungen der Formel (I) auf den Lebensraum der Pflanze. Das kann beispielsweise durch Drenchen, Einmischen in den Boden oder die Nährlösung sein, d.h. der Standort der Pflanze (z.B. Boden oder hydroponische Systeme) wird mit einer flüssigen Form der Verbindungen der Formel (I) getränkt, oder durch die Bodenapplikation, d.h. die Verbindungen der Formel (I) werden in fester Form, (z.B. in Form eines Granulats) in den Standort der Pflanzen eingebracht. Bei Wasserreiskulturen kann das auch durch Zudosieren der Verbindung der Formel (I) in einer festen Anwendungsform (z.B. als Granulat) in ein überflutetes Reisfeld sein.

### Saatgutbehandlung

Die Bekämpfung von tierischen Schädlingen durch die Behandlung des Saatguts von Pflanzen ist seit langem bekannt und ist Gegenstand ständiger Verbesserungen. Dennoch ergeben sich bei der Behandlung von Saatgut eine Reihe von Problemen, die nicht immer zufriedenstellend gelöst werden können. So ist es erstrebenswert, Verfahren zum Schutz des Saatguts und der keimenden Pflanze zu entwickeln, die das zusätzliche Ausbringen von Schädlingsbekämpfungsmitteln bei der Lagerung, nach der Saat oder nach dem Auflaufen der Pflanzen überflüssig machen oder zumindest deutlich verringern. Es ist weiterhin erstrebenswert, die Menge des eingesetzten Wirkstoffs dahingehend zu optimieren, dass das Saatgut und die keimende Pflanze vor dem Befall durch tierische Schädlinge bestmöglich geschützt werden, ohne jedoch die Pflanze selbst durch den eingesetzten Wirkstoff zu schädigen. Insbesondere sollten Verfahren zur Behandlung von Saatgut auch die intrinsischen insektiziden bzw. nematiziden Eigenschaften schädlingsresistenter bzw. - toleranter transgener Pflanzen einbeziehen, um einen optimalen Schutz des Saatguts und der keimenden Pflanze bei einem minimalen Aufwand an Schädlingsbekämpfungsmitteln zu erreichen.

Die vorliegende Erfindung bezieht sich daher insbesondere auch auf ein Verfahren zum Schutz von Saatgut und keimenden Pflanzen vor dem Befall von Schädlingen, indem das Saatgut mit einer der Verbindungen der Formel (I) behandelt wird. Das erfindungsgemäße Verfahren zum Schutz von Saatgut und keimenden Pflanzen vor dem Befall von Schädlingen umfasst ferner ein Verfahren, in dem das Saatgut gleichzeitig in einem Vorgang oder sequentiell mit einer Verbindung der Formel (I) und Mischungspartner behandelt wird. Es umfasst ferner auch ein Verfahren, in dem das Saatgut zu unterschiedlichen Zeiten mit einer Verbindung der Formel (I) und Mischungspartner behandelt wird.

Die Erfindung bezieht sich ebenfalls auf die Verwendung der Verbindungen der Formel (I) zur Behandlung von Saatgut zum Schutz des Saatguts und der daraus entstehenden Pflanze vor tierischen Schädlingen.

Einer der auftretenden Vorteile, wenn eine der Verbindungen der Formel (I) systemisch wirkt, ist es, dass die Behandlung des Saatguts nicht nur das Saatgut selbst, sondern auch die daraus hervorgehenden Pflanzen nach dem Auflaufen vor tierischen Schädlingen schützt. Auf diese Weise kann die unmittelbare Behandlung der Kultur zum Zeitpunkt der Aussaat oder kurz danach entfallen.

Ein weiterer Vorteil ist darin zu sehen, dass durch die Behandlung des Saatguts mit einer Verbindung der Formel (I) Keimung und Auflauf des behandelten Saatguts gefördert werden können.

Ebenso ist es als vorteilhaft anzusehen, dass Verbindungen der Formel (I) insbesondere auch bei transgenem Saatgut eingesetzt werden können.

Verbindungen der Formel (I) können ferner in Kombination mit Mitteln der Signaltechnologie eingesetzt werden, wodurch eine bessere Besiedlung mit Symbionten, wie zum Beispiel Rhizobien, Mycorrhiza und/oder endophytischen Bakterien oder Pilzen, stattfindet und/oder es zu einer optimierten Stickstofffixierung kommt.

Die Verbindungen der Formel (I) eignen sich zum Schutz von Saatgut jeglicher Pflanzensorte, die in der Landwirtschaft, im Gewächshaus, in Forsten oder im Gartenbau eingesetzt wird. Insbesondere handelt es sich dabei um Saatgut von Getreide (z. B. Weizen, Gerste, Roggen, Hirse und Hafer), Mais, Baum-wolle, Soja, Reis, Kartoffeln, Sonnenblume, Kaffee, Tabak, Canola, Raps, Rübe (z.B. Zuckerrübe und Futterrübe), Erdnuss, Gemüse (z. B. Tomate, Gurke, Bohne, Kohlgewächse, Zwiebeln und Salat), Obstpflanzen, Rasen und Zierpflanzen. Besondere Bedeutung kommt der Behandlung des Saatguts von Getreide (wie Weizen, Gerste, Roggen und Hafer), Mais, Soja, Baumwolle, Canola, Raps und Reis zu.

Wie vorstehend bereits erwähnt, kommt auch der Behandlung von transgenem Saatgut mit einer Verbindung der Formel (I) eine besondere Bedeutung zu. Dabei handelt es sich um das Saatgut von Pflanzen, die in der Regel zumindest ein heterologes Gen enthalten, das die Expression eines Polypeptids mit insbesondere insektiziden bzw. nematiziden Eigenschaften steuert. Die heterologen Gene in transgenem Saatgut können dabei aus Mikro-organismen wie Bacillus, Rhizobium, Pseudomonas, Serratia, Trichoderma, Clavibacter, Glomus oder Gliocladium stammen. Die vorliegende Erfindung eignet sich besonders für die Behandlung von trans-genem Saatgut, das zumindest ein heterologes Gen enthält, das aus Bacillus sp. stammt. Besonders bevorzugt handelt es sich dabei um ein heterologes Gen, das aus Bacillus thuringiensis stammt.

Im Rahmen der vorliegenden Erfindung wird die Verbindung der Formel (I) auf das Saatgut aufgebracht. Vorzugsweise wird das Saatgut in einem Zustand behandelt, in dem es so stabil ist, dass keine Schäden bei der Behandlung auftreten. Im Allgemeinen kann die Behandlung des Saatguts zu jedem Zeitpunkt zwischen der Ernte und der Aussaat erfolgen. Üblicherweise wird Saatgut verwendet, das von der Pflanze getrennt und von Kolben, Schalen, Stängeln, Hülle, Wolle oder Fruchtfleisch befreit wurde. So kann zum Beispiel Saatgut verwendet werden, das geerntet, gereinigt und bis zu einem lagerfähigen Feuchtigkeitsgehalt getrocknet wurde. Alternativ kann auch Saatgut verwendet werden, das nach dem Trocknen z.B. mit Wasser behandelt und dann erneut getrocknet wurde, zum Beispiel Priming. Im Falle von Reissaatgut ist es auch möglich Saatgut zu verwenden, das zum Beispiel in Wasser bis zu einem bestimmten Stadium vorgequollen wurde (pigeon breast Stadium), was zu einer verbesserten Keimung und zu einem gleichmäßigeren Auflaufen führt.

Im Allgemeinen muss bei der Behandlung des Saatguts darauf geachtet werden, dass die Menge der auf das Saatgut aufgebrachten Verbindung der Formel (I) und/oder weiterer Zusatzstoffe so gewählt wird, dass die Keimung des Saatguts nicht beeinträchtigt bzw. die daraus hervorgehende Pflanze nicht geschädigt wird. Dies ist vor allem bei Wirkstoffen zu beachten, die in bestimmten Aufwandmengen phytotoxische Effekte zeigen können.

Die Verbindungen der Formel (I) werden in der Regel in Form einer geeigneten Formulierung auf das Saatgut aufgebracht. Geeignete Formulierungen und Verfahren für die Saatgutbehandlung sind dem Fachmann bekannt.

Die Verbindungen der Formel (I) können in die üblichen Beizmittel-Formulierungen überführt werden, wie Lösungen, Emulsionen, Suspensionen, Pulver, Schäume, Slurries oder andere Hüllmassen für Saatgut, sowie ULV-Formulierungen.

Diese Formulierungen werden in bekannter Weise hergestellt, indem man Verbindungen der Formel (I) mit üblichen Zusatzstoffen vermischt, wie zum Beispiel übliche Streckmittel sowie Lösungs- oder Verdünnungsmittel, Farbstoffe, Netzmittel, Dispergiermittel, Emulgatoren, Entschäumer, Konservierungsmittel, sekundäre Verdickungsmittel, Kleber, Gibberelline und auch Wasser.

Als Farbstoffe, die in den erfindungsgemäß verwendbaren Beizmittel-Formulierungen enthalten sein können, kommen alle für derartige Zwecke üblichen Farbstoffe in Betracht. Dabei sind sowohl in Wasser wenig lösliche Pigmente als auch in Wasser lösliche Farbstoffe verwendbar. Als Beispiele genannt seien die unter den Bezeichnungen Rhodamin B, C.I. Pigment Red 112 und C.I. Solvent Red 1 bekannten Farbstoffe.

Als Netzmittel, die in den erfindungsgemäß verwendbaren Beizmittel-Formulierungen enthalten sein können, kommen alle zur Formulierung von agrochemischen Wirkstoffen üblichen, die Benetzung fördernden Stoffe in Frage. Vorzugsweise verwendbar sind Alkylnaphthalin-Sulfonate, wie Diisopropyl- oder Diisobutyl-naphthalin-Sulfonate.

Als Dispergiermittel und/oder Emulgatoren, die in den erfindungsgemäß verwendbaren Beizmittel-Formulierungen enthalten sein können, kommen alle zur Formulierung von agrochemischen Wirkstoffen üblichen nichtionischen, anionischen und kationischen Dispergiermittel in Betracht. Vor-zugsweise verwendbar sind nichtionische oder anionische Dispergiermittel oder Gemische von nichtionischen oder anionischen Dispergiermitteln. Als geeignete nichtionische Dispergiermittel sind insbesondere Ethylenoxid-Propylenoxid Blockpolymere, Alkylphenolpolyglykolether sowie Tristryrylphenolpolyglykolether und deren phosphatierte oder sulfatierte Derivate zu nennen. Geeignete anionische Dispergiermittel sind insbesondere Ligninsulfonate, Polyacrylsäuresalze und Arylsulfonat-Formaldehydkondensate.

Als Entschäumer können in den erfindungsgemäß verwendbaren Beizmittel-Formulierungen alle zur Formulierung von agrochemischen Wirkstoffen üblichen schaumhemmenden Stoffe enthalten sein. Vorzugsweise verwendbar sind Silikonentschäumer und Magnesiumstearat.

Als Konservierungsmittel können in den erfindungsgemäß verwendbaren Beizmittel-Formulierungen alle für derartige Zwecke in agrochemischen Mitteln einsetzbaren Stoffe vorhanden sein. Beispielhaft genannt seien Dichlorophen und Benzylalkoholhemiformal.

Als sekundäre Verdickungsmittel, die in den erfindungsgemäß verwendbaren Beizmittel-Formulierungen enthalten sein können, kommen alle für derartige Zwecke in agrochemischen Mitteln einsetzbaren Stoffe in Frage. Vorzugsweise in Betracht kommen Cellulosederivate, Acrylsäurederivate, Xanthan, modifizierte Tone und hochdisperse Kieselsäure.

Als Kleber, die in den erfindungsgemäß verwendbaren Beizmittel-Formulierungen enthalten sein können, kommen alle üblichen in Beizmitteln einsetzbaren Bindemittel in Frage. Vorzugsweise genannt seien Polyvinylpyrrolidon, Polyvinylacetat, Polyvinylalkohol und Tylose.

Als Gibberelline, die in den erfindungsgemäß verwendbaren Beizmittel-Formulierungen enthalten sein können, kommen vorzugsweise die Gibberelline A1, A3 (= Gibberellinsäure), A4 und A7 infrage, besonders bevorzugt verwendet man die Gibberellinsäure. Die Gibberelline sind bekannt (vgl. R. Wegler "Chemie der Pflanzenschutz- und Schädlingsbekämpfungsmittel", Bd. 2, Springer Verlag, 1970, S. 401-412).

Die erfindungsgemäß verwendbaren Beizmittel-Formulierungen können entweder direkt oder nach vorherigem Verdünnen mit Wasser zur Behandlung von Saatgut der verschiedensten Art eingesetzt werden. So lassen sich die Konzentrate oder die daraus durch Verdünnen mit Wasser erhältlichen Zubereitungen einsetzen zur Beizung des Saatgutes von Getreide, wie Weizen, Gerste, Roggen, Hafer und Triticale, sowie des Saatgutes von Mais, Reis, Raps, Erbsen, Bohnen, Baumwolle, Sonnenblumen, Soja und Rüben oder auch von Gemüsesaatgut der verschiedensten Natur. Die erfindungsgemäß verwendbaren Beizmittel-Formulierungen oder deren verdünnte Anwendungsformen können auch zum Beizen von Saatgut transgener Pflanzen eingesetzt werden.

Zur Behandlung von Saatgut mit den erfindungsgemäß verwendbaren Beizmittel-Formulierungen oder daraus hergestellten Anwendungsformen kommen alle üblicherweise für die Beizung einsetzbaren Mischgeräte in Betracht. Im einzelnen geht man bei der Beizung so vor, dass man das Saatgut in einen Mischer im diskontinuierlichem oder kontinuierlichem Betrieb gibt, die jeweils gewünschte Menge an Beizmittel-Formulierungen entweder als solche oder nach vorherigem Verdünnen mit Wasser hinzufügt und bis zur gleichmäßigen Verteilung der Formulierung auf dem Saatgut mischt. Gegebenenfalls schließt sich ein Trocknungsvorgang an.

Die Aufwandmenge an den erfindungsgemäß verwendbaren Beizmittel-Formulierungen kann inner-halb eines größeren Bereiches variiert werden. Sie richtet sich nach dem jeweiligen Gehalt der Verbindungen der Formel (I) in den Formulierungen und nach dem Saatgut. Die Aufwandmengen bei der Verbindung der Formel (I) liegen im Allgemeinen zwischen 0,001 und 50 g pro Kilogramm Saatgut, vorzugsweise zwischen 0,01 und 15 g pro Kilogramm Saatgut.

### Tiergesundheit

Auf dem Gebiet der Tiergesundheit, d.h. dem Gebiet der Tiermedizin, sind die Verbindungen der Formel (I) gegen Tierparasiten, insbesondere Ektoparasiten oder Endoparasiten, wirksam. Der Begriff Endoparasiten umfasst insbesondere Helminthen und Protozoa wie Kokzidien. Ektoparasiten sind typischerweise und bevorzugt Arthropoden, insbesondere Insekten und Akariden.

Auf dem Gebiet der Tiermedizin eignen sich die Verbindungen der Formel (I), die eine günstige Toxizität gegenüber Warmblütern aufweisen, für die Bekämpfung von Parasiten, die in der Tierzucht und Tierhaltung bei Nutztieren, Zuchttieren, Zootieren, Laboratoriumstieren, Versuchstieren und Haustieren auftreten. Sie sind gegen alle oder einzelne Entwicklungsstadien der Parasiten wirksam.

Zu den landwirtschaftlichen Nutztieren zählen zum Beispiel Säugetiere wie Schafe, Ziegen, Pferde, Esel, Kamele, Büffel, Kaninchen, Rentiere, Damhirsche und insbesondere Rinder und Schweine; Geflügel wie Truthähne, Enten, Gänse und insbesondere Hühner; Fische und Krustentiere, z.B. in der Aquakultur und auch Insekten wie Bienen.

Zu den Haustieren zählen zum Beispiel Säugetiere wie Hamster, Meerschweinchen, Ratten, Mäuse, Chinchillas, Frettchen und insbesondere Hunde, Katzen, Stubenvögel, Reptilien, Amphibien und Aquariumfische.

Gemäß einer bevorzugten Ausführungsform werden die Verbindungen der Formel (I) an Säugetiere verabreicht.

Gemäß einer weiteren bevorzugten Ausführungsform werden die Verbindungen der Formel (I) an Vögel, nämlich Stubenvögel und insbesondere Geflügel, verabreicht.

Durch Verwendung der Verbindungen der Formel (I) für die Bekämpfung von Tierparasiten sollen Krankheit, Todesfälle und Leistungsminderungen (bei Fleisch, Milch, Wolle, Häuten, Eiern, Honig und dergleichen) verringert bzw. vorgebeugt werden, so dass eine wirtschaftlichere und einfachere Tierhaltung ermöglicht wird und ein besseres Wohlbefinden der Tiere erzielbar ist.

In Bezug auf das Gebiet der Tiergesundheit bedeutet der Begriff "Bekämpfung" oder "bekämpfen", dass durch die Verbindungen der Formel (I) wirksam das Auftreten des jeweiligen Parasiten in einem Tier, das mit solchen Parasiten in einem harmlosen Ausmaß infiziert ist, reduziert werden kann. Genauer gesagt bedeutet "bekämpfen" im vorliegenden Zusammenhang, dass die Verbindung der Formel (I) den jeweiligen Parasiten abtöten, sein Wachstum verhindern oder seine Vermehrung verhindern kann.

Zu den Arthropoden zählen:
aus der Ordnung Anoplurida, zum Beispiel Haematopinus spp., Linognathus spp., Pediculus spp., Phtirus spp., Solenopotes spp.; aus der Ordnung Mallophagida und den Unterordnungen Amblycerina and Ischnocerina, zum Beispiel Trimenopon spp., Menopon spp., Trinoton spp., Bovicola spp., Werneckiella spp., Lepikentron spp., Damalina spp., Trichodectes spp., Felicola spp.; aus der Ordnung Diptera und den Unterordnungen Nematocerina und Brachycerina, zum Beispiel Aedes spp., Anopheles spp., Culex spp., Simulium spp., Eusimulium spp., Phlebotomus spp., Lutzomyia spp., Culicoides spp., Chrysops spp., Odagmia spp., Wilhelmia spp., Hybomitra spp., Atylotus spp., Tabanus spp., Haematopota spp., Philipomyia spp., Braula spp., Musca spp., Hydrotaea spp., Stomoxys spp., Haematobia spp., Morellia spp., Fannia spp., Glossina spp., Calliphora spp., Lucilia spp., Chrysomyia spp., Wohlfahrtia spp., Sarcophaga spp., Oestrus spp., Hypoderma spp., Gasterophilus spp., Hippobosca spp., Lipoptena spp., Melophagus spp., Rhinoestrus spp., Tipula spp.; aus der Ordnung Siphonapterida, zum Beispiel Pulex spp., Ctenocephalides spp., Tunga spp., Xenopsylla spp., Ceratophyllus spp.;
aus der Ordnung Heteropterida, zum Beispiel Cimex spp., Triatoma spp., Rhodnius spp., Panstrongylus spp.; sowie Lästlinge und Hygieneschädlinge aus der Ordnung Blattarida.

Weiterhin zählen zu den Arthropoden:
Aus der Unterklasse Akari (Acarina) und der Ordnung Metastigmata, zum Beispiel aus der Familie Argasidae, wie Argas spp., Ornithodorus spp., Otobius spp., aus der Familie Ixodidae, wie Ixodes spp., Amblyomma spp., Rhipicephalus (Boophilus) spp. Dermacentor spp., Haemophysalis spp., Hyalomma spp., Rhipicephalus spp. (die ursprüngliche Gattung der mehrwirtigen Zecken); aus der Ordnung Mesostigmata, wie Dermanyssus spp., Ornithonyssus spp., Pneumonyssus spp., Raillietia spp., Pneumonyssus spp., Sternostoma spp., Varroa spp., Acarapis spp.; aus der Ordnung Actinedida (Prostigmata), zum Beispiel Acarapis spp., Cheyletiella spp., Ornithocheyletia spp., Myobia spp., Psorergates spp., Demodex spp., Trombicula spp., Neotrombiculla spp., Listrophorus spp.; und aus der Ordnung Acaridida (Astigmata), zum Beispiel Acarus spp., Tyrophagus spp., Caloglyphus spp., Hypodectes spp., Pterolichus spp., Psoroptes spp., Chorioptes spp., Otodectes spp., Sarcoptes spp., Notoedres spp., Knemidocoptes spp., Cytodites spp., Laminosioptes spp..

Zu parasitären Protozoen zählen:
Mastigophora (Flagellata), wie zum Beispiel Trypanosomatidae, zum Beispiel Trypanosoma b. brucei, T.b. gambiense, T.b. rhodesiense, T. congolense, T. cruzi, T. evansi, T. equinum, T. lewisi, T. percae, T. simiae, T. vivax, Leishmania brasiliensis, L. donovani, L. tropica, wie zum Beispiel Trichomonadidae, zum Beispiel Giardia lamblia, G. canis;

Sarcomastigophora (Rhizopoda), wie Entamoebidae, zum Beispiel Entamoeba histolytica, Hartmanellidae, zum Beispiel Acanthamoeba sp., Harmanella sp.;
Apicomplexa (Sporozoa), wie Eimeridae, zum Beispiel Eimeria acervulina, E. adenoides, E. alabamensis, E. anatis, E. anserina, E. arloingi, E. ashata, E. auburnensis, E. bovis, E. brunetti, E. canis, E. chinchillae, E. clupearum, E. columbae, E. contorta, E. crandalis, E. debliecki, E. dispersa, E. ellipsoidales, E. falciformis, E. faurei, E. flavescens, E. gallopavonis, E. hagani, E. intestinalis, E. iroquoina, E. irresidua, E. labbeana, E. leucarti, E. magna, E. maxima, E. media, E. meleagridis, E. meleagrimitis, E. mitis, E. necatrix, E. ninakohlyakimovae, E. ovis, E. parva, E. pavonis, E. perforans, E. phasani, E. piriformis, E. praecox, E. residua, E. scabra, E. spec., E. stiedai, E. suis, E. tenella, E. truncata, E. truttae, E. zuernii, Globidium spec., Isospora belli, I. canis, I. felis, I. ohioensis, I. rivolta, I. spec., I. suis, Cystisospora spec., Cryptosporidium spec., insbesondere C. parvum; wie Toxoplasmadidae, zum Beispiel Toxoplasma gondii, Hammondia heydornii, Neospora caninum, Besnoitia besnoitii; wie Sarcocystidae, zum Beispiel Sarcocystis bovicanis, S. bovihominis, S. ovicanis, S. ovifelis, S. neurona, S. spec., S. suihominis, wie Leucozoidae, zum Beispiel Leucozytozoon simondi, wie Plasmodiidae, zum Beispiel Plasmodium berghei, P. falciparum, P. malariae, P. ovale, P. vivax, P. spec., wie Piroplasmea, zum Beispiel Babesia argentina, B. bovis, B. canis, B. spec., Theileria parva, Theileria spec., wie Adeleina, zum Beispiel Hepatozoon canis, H. spec..

Zu pathogenen Endoparasiten, bei denen es sich um Helminthen handelt, zählen Plattwürmer (z.B. Monogenea, Cestodes und Trematodes), Rundwürmer, Acanthocephala und Pentastoma. Dazu zählen:
Monogenea: z.B.: Gyrodactylus spp., Dactylogyrus spp., Polystoma spp.;
Cestodes: aus der Ordnung Pseudophyllidea zum Beispiel: Diphyllobothrium spp., Spirometra spp., Schistocephalus spp., Ligula spp., Bothridium spp., Diplogonoporus spp.;
aus der Ordnung Cyclophyllida zum Beispiel: Mesocestoides spp., Anoplocephala spp., Paranoplocephala spp., Moniezia spp., Thysanosoma spp., Thysaniezia spp., Avitellina spp., Stilesia spp., Cittotaenia spp., Andyra spp., Bertiella spp., Taenia spp., Echinococcus spp., Hydatigera spp., Davainea spp., Raillietina spp., Hymenolepis spp., Echinolepis spp., Echinocotyle spp., Diorchis spp., Dipylidium spp., Joyeuxiella spp., Diplopylidium spp.;
Trematodes: aus der Klasse Digenea zum Beispiel: Diplostomum spp., Posthodiplostomum spp., Schistosoma spp., Trichobilharzia spp., Ornithobilharzia spp., Austrobilharzia spp., Gigantobilharzia spp., Leucochloridium spp., Brachylaima spp., Echinostoma spp., Echinoparyphium spp., Echinochasmus spp., Hypoderaeum spp., Fasciola spp., Fascioloides spp., Fasciolopsis spp., Cyclocoelum spp., Typhlocoelum spp., Paramphistomum spp., Calicophoron spp., Cotylophoron spp., Gigantocotyle spp., Fischoederius spp., Gastrothylacus spp., Notocotylus spp., Catatropis spp., Plagiorchis spp., Prosthogonimus spp., Dicrocoelium spp., Eurytrema spp., Troglotrema spp., Paragonimus spp., Collyriclum spp., Nanophyetus spp., Opisthorchis spp., Clonorchis spp. Metorchis spp., Heterophyes spp., Metagonimus spp.;
Rundwürmer: Trichinellida zum Beispiel: Trichuris spp., Capillaria spp., Paracapillaria spp., Eucoleus spp., Trichomosoides spp., Trichinella spp.;
aus der Ordnung Tylenchida zum Beispiel: Micronema spp., Strongyloides spp.;
aus der Ordnung Rhabditida zum Beispiel: Strongylus spp., Triodontophorus spp., Oesophagodontus spp., Trichonema spp., Gyalocephalus spp., Cylindropharynx spp., Poteriostomum spp., Cyclococercus spp., Cylicostephanus spp., Oesophagostomum spp., Chabertia spp., Stephanurus spp., Ancylostoma spp., Uncinaria spp., Necator spp., Bunostomum spp., Globocephalus spp., Syngamus spp., Cyathostoma spp., Metastrongylus spp., Dictyocaulus spp., Muellerius spp., Protostrongylus spp., Neostrongylus spp., Cystocaulus spp., Pneumostrongylus spp., Spicocaulus spp., Elaphostrongylus spp. Parelaphostrongylus spp., Crenosoma spp., Paracrenosoma spp., Oslerus spp., Angiostrongylus spp., Aelurostrongylus spp., Filaroides spp., Parafilaroides spp., Trichostrongylus spp., Haemonchus spp., Ostertagia spp., Teladorsagia spp., Marshallagia spp., Cooperia spp., Nippostrongylus spp., Heligmosomoides spp., Nematodirus spp., Hyostrongylus spp., Obeliscoides spp., Amidostomum spp., Ollulanus spp.;
aus der Ordnung Spirurida zum Beispiel: Oxyuris spp., Enterobius spp., Passalurus spp., Syphacia spp., Aspiculuris spp., Heterakis spp.; Ascaris spp., Toxascaris spp., Toxocara spp., Baylisascaris spp., Parascaris spp., Anisakis spp., Ascaridia spp.; Gnathostoma spp., Physaloptera spp., Thelazia spp., Gongylonema spp., Habronema spp., Parabronema spp., Draschia spp., Dracunculus spp.; Stephanofilaria spp., Parafilaria spp., Setaria spp., Loa spp., Dirofilaria spp., Litomosoides spp., Brugia spp., Wuchereria spp., Onchocerca spp., Spirocerca spp.;
Acanthocephala: aus der Ordnung Oligacanthorhynchida z.B: Macracanthorhynchus spp., Prosthenorchis spp.; aus der Ordnung Polymorphida zum Beispiel: Filicollis spp.; aus der Ordnung Moniliformida zum Beispiel: Moniliformis spp.;
aus der Ordnung Echinorhynchida zum Beispiel Acanthocephalus spp., Echinorhynchus spp., Leptorhynchoides spp.;
Pentastoma: aus der Ordnung Porocephalida zum Beispiel Linguatula spp..

Auf dem Gebiet der Tiermedizin und der Tierhaltung erfolgt die Verabreichung der Verbindungen der Formel (I) nach allgemein fachbekannten Verfahren, wie enteral, parenteral, dermal oder nasal in Form von geeigneten Präparaten. Die Verabreichung kann prophylaktisch oder therapeutisch erfolgen.

So bezieht sich eine Ausführungsform der vorliegenden Erfindung auf die Verwendung einer Verbindung der Formel (I) als Arzneimittel.

Ein weiterer Aspekt bezieht sich auf die Verwendung einer Verbindung der Formel (I) als Antiendoparasitikum, insbesondere als ein Helminthizid oder ein Mittel gegen Protozoen. Verbindungen der Formel (I) eignen sich für die Verwendung als Antiendoparasitikum, insbesondere als ein Helminthizid oder Mittel gegen Protozoen, beispielsweise in der Tierzucht, in der Tierhaltung, in Ställen und auf dem Hygienesektor.

Ein weiterer Aspekt wiederum betrifft die Verwendung einer Verbindung der Formel (I) als Antiektoparasitikum, insbesondere ein Arthropodizid wie ein Insektizid oder ein Akarizid Ein weiterer Aspekt betrifft die Verwendung einer Verbindung der Formel (I) als Antiektoparasitikum, insbesondere ein Arthropodizid wie ein Insektizid oder Akarizid, zum Beispiel in der Tierhaltung, in der Tierzucht, in Ställen oder auf dem Hygienesektor.

### Anthelminthische Mischungspartner

Beispielhaft seien folgende anthelmintische Mischungspartner genannt:
Anthelminthische Wirkstoffe, einschließlich trematicide und cestocide Wirkstoffe:
aus der Klasse der **macrocyclischen Lactone,** z. B.: Abamectin, Doramectin, Emamectin, Eprinomectin, Ivermectin, Milbemycin, Moxidectin, Nemadectin, Selamectin;
aus der Klasse der **Benzimidazole** und **Probenzimidazole,** z. B.: Albendazol, Albendazol- Sulfoxid, Cambendazol, Cyclobendazol, Febantel, Fenbendazol, Flubendazol, Mebendazol, Netobimin, Oxfendazol, Oxibendazol, Parbendazol, Thiabendazol, Thiophanat, Triclabendazol;
aus der Klasse der **Cyclooctadepsipeptide,** z. B.: Emodepsid, PF1022;
aus der Klasse der **Aminoacetonitril-Derivate,** z. B.: Monepantel;
aus der Klasse der **Tetrahydropyrimidine,** z. B.: Morantel, Pyrantel, Oxantel;
aus der Klasse der **Imidazothiazole**, z. B.: Butamisol, Levamisol, Tetramisol;
aus der Klasse der **Salicylanilide,** z. B.: Bromoxanid, Brotianid, Clioxanid, Closantel, Niclosamid, Oxyclozanid, Rafoxanid, Tribromsalan;
aus der Klasse der **Paraherquamide,** z. B.: Derquantel, Paraherquamid;
aus der Klasse der **Aminophenylamidine,** z. B.: Amidantel, deacyliertes Amidantel (dAMD), Tribendimidin;
aus der Klasse der **Organophosphate,** z. B.: Coumaphos, Crufomat, Dichlorvos, Haloxon, Naphthalofos, Trichlorfon;
aus der Klasse der **substituierten Phenole,** z. B.: Bithionol, Disophenol, Hexachlorophen, Niclofolan, Meniclopholan, Nitroxynil;
aus der Klasse der **Piperazinone,** z. B.: Praziquantel, Epsiprantel;
aus anderen **diversen Klassen,** z. B.: Amoscanat, Bephenium, Bunamidin, Clonazepam, Clorsulon, Diamfenetid, Dichlorophen, Diethylcarbamazin, Emetin, Hetolin, Hycanthon, Lucanthon, Miracil, Mirasan, Niclosamid, Niridazol, Nitroxynil, Nitroscanat, Oltipraz, Omphalotin, Oxamniquin, Paromomycin, Piperazin, Resorantel.

### Vektorkontrolle

Die Verbindungen der Formel (I) können auch in der Vektorkontrolle eingesetzt werden. Ein Vektor im Sinne der vorliegenden Erfindung ist ein Arthropode, insbesondere ein Insekt oder Arachnide, der in der Lage ist, Krankheitserreger wie z. B. Viren, Würmer, Einzeller und Bakterien aus einem Reservoir (Pflanze, Tier, Mensch, etc.) auf einen Wirt zu übertragen. Die Krankheitserreger können entweder mechanisch (z.B. Trachoma durch nicht-stechende Fliegen) auf einem Wirt, oder nach Injektion (z.B. Malaria-Parasiten durch Mücken) in einen Wirt übertragen werden.

Beispiele für Vektoren und die von ihnen übertragenen Krankheiten bzw. Krankheitserreger sind:
1) Mücken
   - Anopheles: Malaria, Filariose;
   - Culex: Japanische Encephalitis, Filariasis, weitere virale Erkrankungen, Übertragung von Würmern;
   - Aedes: Gelbfieber, Dengue-Fieber, Filariasis, weitere virale Erkrankungen;
   - Simulien: Übertragung von Würmern insbesondere Onchocerca volvulus;
2) Läuse: Hautinfektionen, Fleckfieber (epidemic typhus);
3) Flöhe: Pest, endemisches Fleckfieber;
4) Fliegen: Schlafkrankheit (Trypanosomiasis); Cholera, weitere bakterielle Erkrankungen;
5) Milben: Acariose, Fleckfieber, Rickettsipocken, Tularämie, Saint-Louis-Enzephalitis, virale Hirnhautentzündung (FSME), Krim-Kongo-Fieber, Borreliose;
6) Zecken: Borelliosen wie Borrelia duttoni, Frühsommer-Meningoenzephalitis, Q-Fieber (Coxiella burnetii), Babesien (Babesia canis canis).

Beispiele für Vektoren im Sinne der vorliegenden Erfindung sind Insekten wie Aphiden, Fliegen, Zikaden oder Thripse, die Pflanzenviren auf Pflanzen übertragen können. Weitere Vektoren, die Pflanzenviren übertragen können, sind Spinnmilben, Läuse, Käfer und Nematoden.

Weitere Beispiele für Vektoren im Sinne der vorliegenden Erfindung sind Insekten und Arachniden wie Mücken, insbesondere der Gattungen Aedes, Anopheles, z.B. A. gambiae, A. arabiensis, A. funestus, A. dirus (Malaria) und Culex, Läuse, Flöhe, Fliegen, Milben und Zecken, die Krankheitserreger auf Tiere und/oder Menschen übertragen können.

Eine Vektorkontrolle ist auch möglich, wenn die Verbindungen der Formel (I) Resistenz-brechend sind.

Verbindungen der Formel (I) sind zur Verwendung in der Prävention von Krankheiten bzw. vor Krankheitserregern, die durch Vektoren übertragen werden, geeignet. Somit ist ein weiterer Aspekt der vorliegenden Erfindung die Verwendung von Verbindungen der Formel (I) zur Vektorkontrolle, z.B. in der Landwirtschaft, im Gartenbau, in Forsten, in Gärten und Freizeiteinrichtungen sowie im Vorrats- und Materialschutz.

### Schutz von technischen Materialen

Die Verbindungen der Formel (I) eignen sich zum Schutz von technischen Materialien gegen Befall oder Zerstörung durch Insekten, z.B. aus der Ordnung Coleoptera, Hymenoptera, Isoptera, Lepidoptera, Psocoptera und Zygentoma.

Unter technischen Materialien sind im vorliegenden Zusammenhang nicht lebende Materialien zu verstehen, wie vorzugsweise Kunststoffe, Klebstoffe, Leime, Papiere und Kartone, Leder, Holz, Holzverarbeitungsprodukte und Anstrichmittel. Die Anwendung der Erfindung zum Schutz von Holz ist besonders bevorzugt.

In einer weiteren Ausführungsform werden die Verbindungen der Formel (I) zusammen mit mindestens einem weiteren Insektizid und/oder mindestens einem Fungizid eingesetzt.

In einer weiteren Ausführungsform liegen die Verbindungen der Formel (I) als ein anwendungsfertiges (ready-to-use) Schädlingsbekämpfungsmittel vor, d.h., es kann ohne weitere Änderungen auf das entsprechende Material aufgebracht werden. Als weitere Insektizide oder als Fungizide kommen insbesondere die oben genannten in Frage.

Überraschenderweise wurde auch gefunden, dass die Verbindungen der Formel (I) zum Schutz vor Bewuchs von Gegenständen, insbesondere von Schiffskörpern, Sieben, Netzen, Bauwerken, Kaianlagen und Signalanlagen, welche mit See- oder Brackwasser in Verbindung kommen, verwendet werden können. Gleichfalls können die Verbindungen der Formel (I) allein oder in Kombinationen mit anderen Wirkstoffen als Antifouling-Mittel eingesetzt werden.

### Bekämpfung von tierischen Schädlingen auf dem Hygienesektor

Die Verbindungen der Formel (I) eignen sich zur Bekämpfung von tierischen Schädlingen auf dem Hygienesektor. Insbesondere kann die Erfindung im Haushalts-, Hygiene- und Vorratsschutz verwendet werden, vor allem zur Bekämpfung von Insekten, Spinnentieren und Milben, die in geschlossenen Räumen, wie beispielsweise Wohnungen, Fabrikhallen, Büros, Fahrzeugkabinen vorkommen. Zur Bekämpfung der tierischen Schädlinge werden die Verbindungen der Formel (I) allein oder in Kombination mit anderen Wirk- und/oder Hilfsstoffen verwendet. Bevorzugt werden sie in Haushaltsinsektizid-Produkten verwendet. Die Verbindungen der Formel (I) sind gegen sensible und resistente Arten sowie gegen alle Entwicklungsstadien wirksam.

Zu diesen Schädlingen gehören beispielsweise Schädlinge aus der Klasse Arachnida, aus den Ordnungen Scorpiones, Araneae und Opiliones, aus den Klassen Chilopoda und Diplopoda, aus der Klasse Insecta die Ordnung Blattodea, aus den Ordnungen Coleoptera, Dermaptera, Diptera, Heteroptera, Hymenoptera, Isoptera, Lepidoptera, Phthiraptera, Psocoptera, Saltatoria oder Orthoptera, Siphonaptera und Zygentoma und aus der Klasse Malacostraca die Ordnung Isopoda.

Die Anwendung erfolgt beispielsweise in Aerosolen, drucklosen Sprühmitteln, z.B. Pump- und Zerstäubersprays, Nebelautomaten, Foggern, Schäumen, Gelen, Verdampferprodukten mit Verdampferplättchen aus Cellulose oder Kunststoff, Flüssigverdampfern, Gel- und Membranverdampfern, propellergetriebenen Verdampfern, energielosen bzw. passiven Verdampfungssystemen, Mottenpapieren, Mottensäckchen und Mottengelen, als Granulate oder Stäube, in Streuködern oder Köderstationen.

### Beispiele:

Die folgenden Herstellungs- und Verwendungsbeispiele illustrieren die Erfindung, ohne sie zu beschränken. Die Produkte wurden mittels 1H-NMR Spektroskopie und/oder LC-MS (Liquid Chromatography Mass Spectrometry) und/oder GC-MS (Gas Chromatography-Mass Spectrometry) charakterisiert.

Die Bestimmung der logP Werte erfolgte analog OECD Guideline 117 (EC Directive 92/69/EEC) durch HPLC (High Performance Liquid Chromatography) an reversed-phase Säulen (C 18), mit nachfolgenden Methoden:
[a] Die Bestimmung mit der LC-MS im sauren Bereich erfolgt bei pH 2,7 mit 0,1 % wässriger Ameisensäure und Acetonitril (enthält 0,1% Ameisensäure) als Eluenten; linearer Gradient von 10% Acetonitril bis 95% Acetonitril. LogP[a] wird auch logP(HCOOH) genannt.
[b] Die Bestimmung mit der LC-MS im neutralen Bereich erfolgt bei pH 7.8 mit 0,001 molarer wässriger Ammoniumhydrogencarbonat-Lösung und Acetonitril als Eluenten; linearer Gradient von 10 % Acetonitril bis 95 % Acetonitril. LogP[b] wird auch logP(neutral) genannt.

Die Eichung erfolgt mit Lösungen einer homologen Reihe unverzweigter Alkan-2-onen (mit 3 bis 16 Kohlenstoffatomen), deren logP-Werte bekannt sind (Bestimmung der logP-Werte anhand der Retentionszeiten durch lineare Interpolation zwischen zwei aufeinander folgenden Alkanonen).

Die NMR-Spektren wurden mit einem Bruker II Avance 400, ausgestattet mit einem 1,7 mm TCI-Probenkopf, gemessen. In Einzelfällen wurden die NMR Spektren mit einem Bruker Avance II 600 bestimmt.

Die NMR-Daten ausgewählter Beispiele werden in klassischer Form (δ-Werte, Multiplettaufspaltung, Anzahl der H-Atome) aufgeführt. Die Aufspaltung der Signale wurde wie folgt beschrieben: s (Singulett), d (Duplett), t (Triplett), q (Quartett), m (Multiplett), breit (für breite Signale). Als Lösungsmittel wurden CD₃CN, CDCl₃ oder D6-DMSO verwendet, wobei als Referenz Tetramethylsilan (0.00 ppm) eingesetzt wurde.

Die GC-MS-Spektren werden mit einem Agilent 6890 GC, HP 5973 MSD an Dimethylsilikonphase bestimmt, mit einem Temperaturgradient von 50 °C bis 320 °C. GC-MS-Indices werden als Kovats-Indices mit Lösung einer homologen Reihe von n-Alkanen (mit geradzahliger Anzahl von 8 bis 38 Kohlenstoffatomen) bestimmt.

### Herstellbeispiel 1: N-Cyclopropyl-3-[2-fluor-4-methyl-5-(2,2,2-Trifluorethylsulfinyl)phenyl]-2,4-dioxo-imidazolidin-1-carboxamid (Bsp Nr. 3)

### Stufe 1: N-Cyclopropyl-3-[2-fluor-4-methyl-5-(2,2,2-Trifluorethylsulfanyl)phenyl]-2,4-dioxo-imidazolidin-1-carboxamid (Bsp Nr. 1)

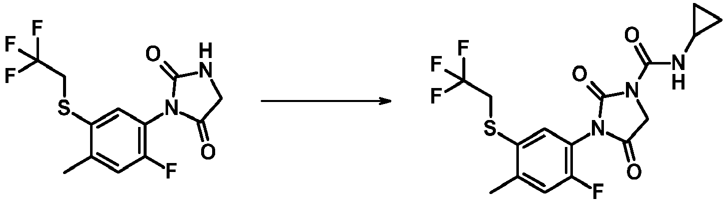

100 mg (0,31 mmol) 3-[2-Fluor-4-methyl-5-(2,2,2)-trifluorethylsulfanyl)phenyl]imidazolidin-2,4-dion (IIa-1) wurden in 5 ml Dichlormethan vorgelegt. Dazu wurden 52 mg (0,62 mmol) Cyclopropylisocyanat gegeben und die Suspension wurde 1 h bei Raumtemperatur gerührt. Anschließend wurden 130 µl (0,93 mmol) Triethylamin hinzugetropft und das Reaktionsgemisch wurde 1 h nachgerührt. Nach DC-Kontrolle (Cyclohexan/Aceton 3:1) war die Reaktion vollständig. Das Lösungsmittel wurde unter vermindertem Druck am Rotationsverdampfer entfernt und der Rückstand wurde mittels einer MPLC über einer RP-18 Säule chromatographiert mit einem Gradienten aus Wasser / Acetonitril / 0.1% Ameisensäure. Es wurden 95 mg (Reinheit 98% nach LC/MS, 74% der Theorie) der Titelverbindung isoliert.
logP(HCOOH): 3,07; logP(neutral): 2,98
¹H-NMR(400,0 MHz, D6-DMSO) δ ppm: 7,79-7,78(m,1H), 7,67(d,1H), 7,44(d,1H), 4,47(breit,2H), 3,86(q,2H), 2,70-2,66(m,1H), 2,45(s,3H), 0,73-0,68(m,2H), 0,56-0,53(m,2H)

### Stufe 2: N-Cyclopropyl-3-[2-fluor-4-methyl-5-(2,2,2-Trifluorethylsulfinyl)phenyl]-2,4-dioxo-imidazolidin-1-carboxamid (Bsp Nr. 3)

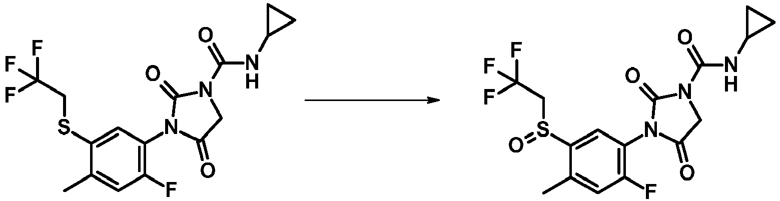

60 mg (0,15 mmol) *N*-Cyclopropyl-3-[2-fluor-4-methyl-5-(2,2,2-Trifluorethylsulfanyl)phenyl]-2,4-dioxo-imidazolidin-1-carboxamid wurden in 6 ml Dichlormethan vorgelegt, 37 mg (75%ig, 0,16 mmol) meta-Chlorperbenzoesäure wurden zugegeben und das Reaktionsgemisch wurde über Nacht bei Raumtemperatur gerührt und anschließend mit Natriumthiosulfat- und Natriumhydrogencarbonatlösung gewaschen. Die organische Phase wurde über trockenem Natriumsulfat getrocknet und filtriert. Nach Entfernen des Lösungsmittels unter vermindertem Druck wurde der Rückstand mittels einer MPLC über einer RP-18 Säule chromatographiert mit einem Gradienten aus Wasser / Acetonitril / 0.1% Ameisensäure. Es wurden 49 mg (Reinheit 100% nach LC/MS, 78% der Theorie) der Titelverbindung isoliert.
logP(HCOOH): 2,15; logP(neutral): 2,12
¹H-NMR(400,0 MHz, D6-DMSO) δ ppm: 7,99(d,1H), 7,78-7,77(m,1H), 7,55(d,1H), 4,46(breit,2H), 4,37-4,25(m,1H), 3,88-3,82(m,1H), 2,71-2,66(m,1H), 2,43(s,3H), 0,73-0,69(m,2H), 0,56-0,52(m,2H)

### Herstellbeispiel 2: N-Cyclopropyl-3-[2-fluor-4-methyl-5-(2,2,2-Trifluorethylsulfinyl)phenyl]-4-oxo-2-thioxo-imidazolidin-1-carboxamid (Bsp Nr. 4)

### Stufe 1: N-Cyclopropyl-3-[2-fluor-4-methyl-5-(2,2,2-Trifluorethylsulfanyl)phenyl]-4-oxo-2-thioxo-imidazolidin-1-carboxamid (Bsp Nr. 2)

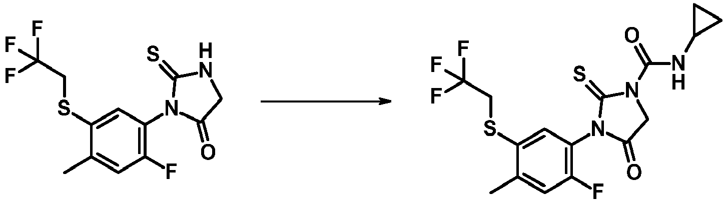

100 mg (0,30 mmol) 3-[2-Fluor-4-methyl-5-(2,2,2-Trifluorethylsulfanyl)phenyl]-2-thioxo-imidazolidin-4-on (IIa-2) wurden in 5 ml Dichlormethan vorgelegt. Dazu wurden 49 mg (0,59 mmol) Cyclopropylisocyanat gegeben und die Suspension wurde 1 h bei Raumtemperatur gerührt. Anschließend wurden 124 µl (0,89 mmol) Triethylamin hinzugetropft und das Reaktionsgemisch wurde 1 h nachgerührt. Das Lösungsmittel wurde unter vermindertem Druck am Rotationsverdampfer entfernt und der Rückstand wurde mittels einer MPLC über einer RP-18 Säule chromatographiert mit einem Gradienten aus Wasser / Acetonitril / 0.1% Ameisensäure. Es wurden 57 mg (Reinheit 93% nach LC/MS, 42% der Theorie) der Titelverbindung isoliert.
logP(HCOOH): 3,66; logP(neutral): 3,56
¹H-NMR(400,0 MHz, D6-DMSO) δ ppm: 9,50-9,49(m,1H), 7,65(d,1H), 7,45(d,1H), 4,86-4,60(m,2H), 3,86(q,2H), 2,77-2,72(m,1H), 2,46(s,3H), 0,79-0,74(m,2H), 0,60-0,52(m,2H)

### Stufe 2: N-Cyclopropyl-3-[2-fluor-4-methyl-5-(2,2,2-Trifluorethylsulfinyl)phenyl]-4-oxo-2-thioxo-imidazolidin-1-carboxamid (Bsp Nr. 4)

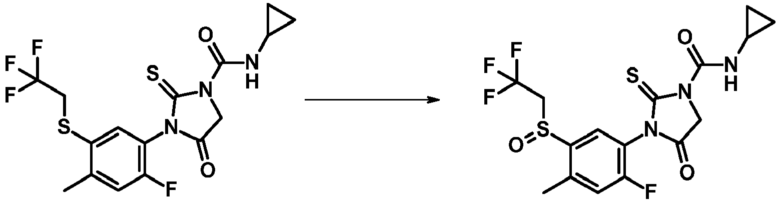

40 mg (0,095 mmol) *N*-Cyclopropyl-3-[2-fluor-4-methyl-5-(2,2,2-Trifluorethylsulfanyl)phenyl]-4-oxo-2-thioxo-imidazolidin-1-carboxamid wurden in 4 ml Dichlormethan vorgelegt, 24 mg (75%ig, 0,10 mmol) meta-Chlorperbenzoesäure wurden zugegeben und das Reaktionsgemisch wurde über Nacht bei Raumtemperatur gerührt und anschließend mit Natriumthiosulfat- und Natriumhydrogencarbonatlösung gewaschen. Die organische Phase wurde über trockenem Natriumsulfat getrocknet und filtriert. Nach Entfernen des Lösungsmittels unter vermindertem Druck wurde der Rückstand mittels einer MPLC über einer RP-18 Säule chromatographiert mit einem Gradienten aus Wasser / Acetonitril / 0.1% Ameisensäure. 18 mg (Reinheit 100% nach LC/MS, 44% der Theorie) der Titelverbindung wurden isoliert.
logP(HCOOH): 2,64; logP(neutral): 2,65
¹H-NMR(400,0 MHz, D6-DMSO) δ ppm: 9,50-9,46(m,1H), 7,99-7,97(m,1H), 7,56(d,1H), 4,84-4,78(m,1H), 4,63-4,58(m,1H), 4,39-4,29(m,1H), 3,88-3,77(m,1H), 2,78-2,73(m,1H), 2,44(s,3H), 0,80-0,74(m,2H), 0,57-0,55(m,2H)

### Herstellbeispiel 3: 3-[2-Fluor-4-methyl-5-(2,2,2-Trifluorethylsulfinyl)phenyl]-N-isobutyl-2,4-dioxo-imidazolidin-1-carboxamid (Bsp Nr. 15)

### Stufe 1: 3-[2-Fluor-4-methyl-5-(2,2,2-Trifluorethylsulfanyl)phenyl]-N-isobutyl-2,4-dioxo-imidazolidin-1-carboxamid (Bsp Nr. 12)

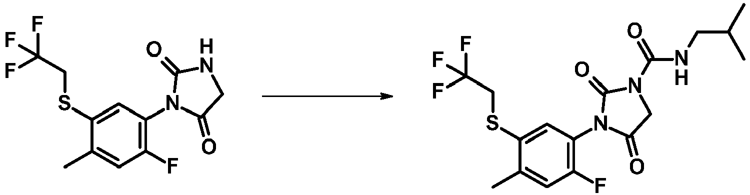

100 mg (0,31 mmol) 3-[2-Fluor-4-methyl-5-(2,2,2)-trifluorethylsulfanyl)phenyl]imidazolidin-2,4-dion (IIa-1) wurden in 5 ml Dichlormethan vorgelegt. Dazu wurden 62 mg (0,62 mmol) Isobutylisocyanat gegeben und die Suspension wurde 1 h bei Raumtemperatur gerührt. Anschließend wurden 130 µl (0,93 mmol) Triethylamin hinzugetropft und das Reaktionsgemisch wurde 1 h nachgerührt. Das Lösungsmittel wurde unter vermindertem Druck am Rotationsverdampfer entfernt und der Rückstand wurde mittels einer MPLC über einer Kieselgel-Säule chromatographiert mit einem Gradienten aus Cyclohexan / Aceton. Es wurden 80 mg (Reinheit 97% nach LC/MS, 60% der Theorie) der Titelverbindung isoliert.
logP(HCOOH): 3,67; logP(neutral): 3,58
¹H-NMR(400,0 MHz, D6-DMSO) δ ppm: 7,85(t,1H), 7,70(d,1H), 7,45(d,1H), 4,46(breit,2H), 3,87(q,2H), 3,09-3,05(m,2H), 2,46(s,3H), 1,80-1,77(m,1H), 0,87(d,6H)

### Stufe 2: 3-[2-Fluor-4-methyl-5-(2,2,2-Trifluorethylsuffinyl)phenyl]-N-isobutyl-2,4-dioxo-imidazolidin-1-carboxamid (Bsp Nr. 15)

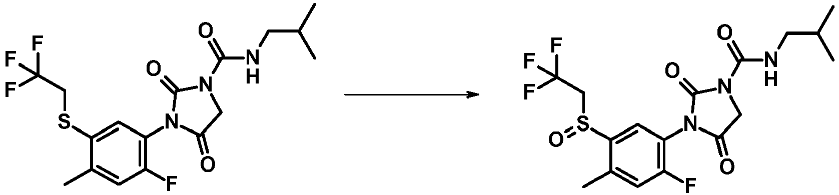

45 mg (0,11 mmol) 3-[2-Fluor-4-methyl-5-(2,2,2-Trifluorethylsulfanyl)phenyl]-*N*-isobutyl-2,4-dioxo-imidazolidin-1-carboxamid wurden in 4,5 ml Dichlormethan vorgelegt, 27 mg (75%ig, 0,12 mmol) meta-Chlorperbenzoesäure wurden zugegeben und das Reaktionsgemisch wurde über Nacht bei Raumtemperatur gerührt und anschließend mit Natriumthiosulfat- und Natriumhydrogencarbonatlösung gewaschen. Die organische Phase wurde über Natriumsulfat getrocknet und filtriert. Nach Entfernen des Lösungsmittels unter vermindertem Druck wurde der Rückstand mittels einer MPLC über einer RP-18 Säule chromatographiert mit einem Gradienten aus Wasser / Acetonitril / 0.1% Ameisensäure. Es wurden 32 mg (Reinheit 100% nach LC/MS, 69% der Theorie) der Titelverbindung isoliert.
logP(HCOOH): 2,72; logP(neutral): 2,66
¹H-NMR(400,0 MHz, D6-DMSO) δ ppm: 8,01(d,1H), 7,85(t,1H), 7,56(d,1H), 4,55(breit,2H), 4,35-4,28(m,1H), 3,90-3,83(m,1H), 3,07(t,2H), 2,43(s,3H), 1,82-1,75(m,1H), 0,88(d,6H)

### Herstellbeispiel 4: N-Ethyl-3-[2-fluor-4-methyl-5-(2,2,2-trifluorethylsulfinyl)phenyl]-5-methyl-2,4-dioxo-imidazolidin-1-carboxamid (Bsp Nr. 27)

### Stufe 1: N-Ethyl-3-[2-fluor-4-methyl-5-(2,2,2-trifluorethylsulfanyl)phenyl]-5-methyl-2,4-dioxo-imidazolidin-1-carboxamid (Bsp Nr. 24)

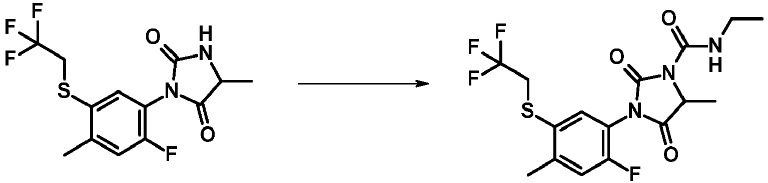

200 mg (0,59 mmol) 3-[2-Fluor-4-methyl-5-(2,2,2-trifluorethylsulfanyl)phenyl]-5-methyl-imidazolidin-2,4-dion (IIa-3) wurden in 10 ml Dichlormethan vorgelegt. Dazu wurden 85 mg (1,18 mmol) Ethylisocyanat und 249 µl (1,78 mmol) Triethylamin hinzugetropft und das Reaktionsgemisch wurde 1 h nachgerührt. Die flüchtigen Bestandteile wurden unter vermindertem Druck am Rotationsverdampfer entfernt. Der Rückstand wurde mittels einer MPLC über einer RP-18 Säule chromatographiert mit einem Gradienten aus Wasser / Acetonitril / 0.1% Ameisensäure. 153 mg (Reinheit 99% nach LC/MS, 63% der Theorie) der Titelverbindung wurden isoliert.
logP(HCOOH): 3,38; logP(neutral): 3,30
¹H-NMR(400,0 MHz, D6-DMSO) δ ppm: 7,91-7,88(m,1H), 7,74(breit,1H), 7,45(d,1H), 4,73(breit,1H), 3,88(q,2H), 3,28-3,21(m,2H), 2,46(s,3H), 1,56(d,3H), 1,10(t,3H)

### Stufe 2: N-Ethyl-3-[2-fluor-4-methyl-5-(2,2,2-trifluorethylsulfinyl)phenyl]-5-methyl-2,4-dioxo-imidazolidin-1-carboxamid (Bsp Nr. 27)

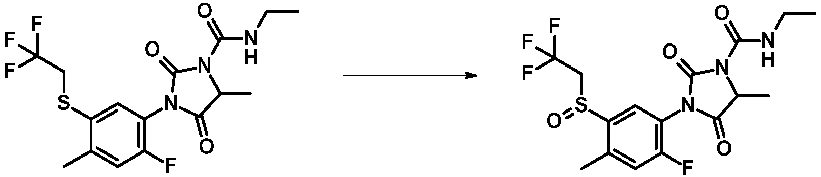

100 mg (0,24 mmol) *N*-Ethyl-3-[2-fluor-4-methyl-5-(2,2,2-trifluorethylsulfanyl)phenyl]-5-methyl-2,4-dioxo-imidazolidin-1-carboxamid wurden in 10 ml Dichlormethan vorgelegt, 62 mg (75%ig, 0,27 mmol) meta-Chlorperbenzoesäure wurden zugegeben und das Reaktionsgemisch wurde über Nacht bei Raumtemperatur gerührt und anschließend mit Natriumthiosulfat- und Natriumhydrogencarbonatlösung gewaschen. Die organische Phase wurde über trockenem Natriumsulfat getrocknet und filtriert. Nach Entfernen des Lösungsmittels unter vermindertem Druck wurde der Rückstand mittels einer MPLC über einer RP-18 Säule chromatographiert mit einem Gradienten aus Wasser / Acetonitril / 0.1% Ameisensäure. 110 mg (Reinheit 93% nach LC/MS, 98% der Theorie) der Titelverbindung wurden isoliert.
logP(HCOOH): 2,42; logP(neutral): 2,39
¹H-NMR(400,0 MHz, D6-DMSO) δ ppm: 8,06(d,1H), 7,90-7,87(m,1H), 7,56(d,1H), 4,71(breit,1H), 4,35-4,28(m,1H), 3,89-3,82(m,1H), 3,29-3,21(m,2H), 2,43(s,3H), 1,57(d,3H), 1,10(t,3H).

### Herstellbeispiel 5: 3-[2-Fluor-4-methyl-5-(2,2,2-Trifluorethylsulfinyl)phenyl]-2,4-dioxo-N-(2,2,2-Trifluorethyl)imidazolidin-1-carboxamid (Bsp Nr. 36)

### Stufe 1: 3-[2-Fluor-4-methyl-5-(2,2,2-Trifluorethylsulfanyl)phenyl]-2,4-dioxo-N-(2,2,2-Trifluorethyl)imidazolidin-1-carboxamid (Bsp Nr. 30)

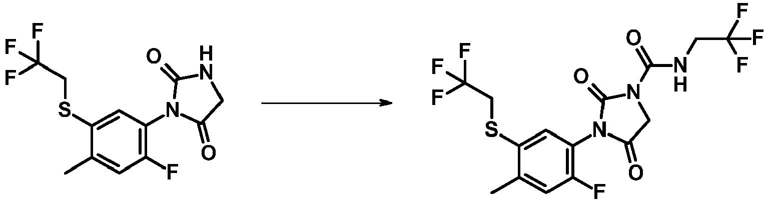

300 mg (0,93 mmol) 3-[2-Fluor-4-methyl-5-(2,2,2)-trifluorethylsulfanyl)phenyl]imidazolidin-2,4-dion (IIa-1) wurden in 15 ml Dichlormethan vorgelegt. Dazu wurden 175 mg (1,40 mmol) Trifluorethylisocyanat und 259 µl (1,86 mmol) Triethylamin hinzugetropft und das Reaktionsgemisch wurde 1,5 h nachgerührt. Anschließend wurde das Reaktionsgemisch zweimal mit gesättigter Natriumhydrogencarbonat-Lösung ausgeschüttelt. Die wässrige Phase wurde einmal mit Dichlormethan extrahiert. Die organischen Phasen wurden vereint, über trockenem Natriumsulfat getrocknet und filtriert. Das Lösungsmittel wurde unter vermindertem Druck am Rotationsverdampfer entfernt. Der Rückstand betrug 376 mg (Reinheit 100% nach LC/MS, 90% der Theorie) der Titelverbindung.
logP(HCOOH): 3,28; logP(neutral): 3,21
¹H-NMR(400,0 MHz, D6-DMSO) δ ppm: 8,32(t,1H), 7,71(d,1H), 7,46(d,1H), 4,58-4,40(breit,2H), 4,10-4,05(m,2H), 3,91-3,83(m,2H), 2,46(s,3H)

### Stufe 2: 3-[2-Fluor-4-methyl-5-(2,2,2-Trifluorethylsulfinyl)phenyl]-2,4-dioxo-N-(2,2,2-Trifluorethyl)imidazolidin-1-carboxamid (Bsp Nr. 36)

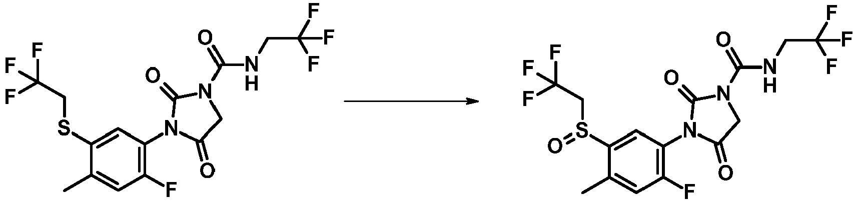

234 mg (0,50 mmol) 3-[2-Fluor-4-methyl-5-(2,2,2-Trifluorethylsulfanyl)phenyl]-2,4-dioxo-*N*-(2,2,2-Trifluorethyl)imidazolidin-1-carboxamid wurden in 10 ml Dichlormethan vorgelegt, 126 mg (75%ig, 0,55 mmol) meta-Chlorperbenzoesäure wurden zugegeben und das Reaktionsgemisch wurde über Nacht bei Raumtemperatur gerührt und anschließend mit Natriumthiosulfat- und Natriumhydrogencarbonatlösung gewaschen. Die organische Phase wurde über trockenem Natriumsulfat getrocknet und filtriert. Nach Entfernen des Lösungsmittels unter vermindertem Druck wurde der Rückstand mittels einer MPLC über einer RP-18 Säule chromatographiert mit einem Gradienten aus Wasser / Acetonitril / 0.1% Ameisensäure. 93 mg (Reinheit 100% nach LC/MS, 40% der Theorie) der Titelverbindung wurden isoliert.
logP(HCOOH): 2,46; logP(neutral): 2,42
¹H-NMR(400,0 MHz, D6-DMSO) δ ppm: 8,32(t,1H), 8,03(d,1H), 7,57(d,1H), 4,54-4,40(breit,2H), 4,39-4,27(m,1H), 4,13-4,04(m,2H), 3,88-3,82(m,1H), 2,43(s,3H)

Die Enantiomere wurden aus dem Racemat gewonnen, in dem sie präparativ mittels HPLC über einer chiralen Säule (ChiralCel OJ-H z.B. 5nm 250 x4.6 mm) mit Laufmittel Heptan / Methanol / Ethanol getrennt wurden.

Die Bestimmung der Drehwerte erfolgte an einem Perkin Elmer 341, Seriennummer 9123, bei einer Wellenlänge von 589 nm und einer Temperatur von 20 °C.

Die unten stehenden spezifischen Drehwerte sind als Durchschnitt aus 5 unterschiedlichen Messungen zu verstehen:
Enantiomer 1 (Bsp. Nr. 135): -35,6 in Acetonitril (c=0,010)
Enantiomer 2 (Bsp Nr. 136): 36,4 in Acetonitril (c=0,010)

### Herstellbeispiel 6: N-cyclopropyl-3-[2-fluor-4-methyl-5-(2,2,2-trifluorethylsulfinyl)phenyl]-5,5-dimethyl-2,4-dioxo-imidazolidin-1-carboxamid (Bsp Nr. 84)

### Stufe 1: N-cyclopropyl-3-[2-fluor-4-methyl-5-(2,2,2-trifluorethylsulfanyl)phenyl]-5,5-dimethyl-2,4-dioxo-imidazolidin-1-carboxamid (Bsp Nr. 74)

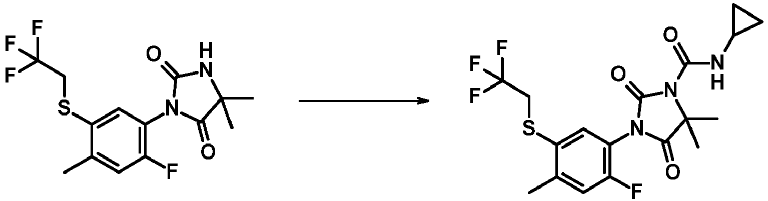

100 mg (0,28 mmol) 3-[2-Fluor-4-methyl-5-(2,2,2-trifluorethylsulfanyl)phenyl]-5,5-dimethyl-imidazolidin-2,4-dion (IIa-5) wurden in 5 ml Dichlormethan vorgelegt. Dazu wurden 47 mg (0,57 mmol) Cyclopropylisocyanat und 119 µl (0,85 mmol) Triethylamin hinzugetropft und das Reaktionsgemisch wurde 1 h nachgerührt. Die flüchtigen Bestandteile wurden unter vermindertem Druck am Rotationsverdampfer entfernt. Der Rückstand wurde mittels einer MPLC über einer RP-18 Säule chromatographiert mit einem Gradienten aus Wasser / Acetonitril / 0.1% Ameisensäure. 102 mg (Reinheit 100% nach LC/MS, 82% der Theorie) der Titelverbindung wurden isoliert.
logP(HCOOH): 3,76; logP(neutral): 3,72
¹H-NMR(400,0 MHz, D6-DMSO) δ ppm: 8,01-8,00(m,1H), 7,81(d,1H), 7,45(d,1H), 3,90(q,2H), 2,71-2,67(m,1H), 2,45(s,3H), 1,70(s,6H), 0,71-0,69(m,2H), 0,53(breit,2H)

### Stufe 2: N-cyclopropyl-3-[2-fluor-4-methyl-5-(2,2,2-trifluorethylsulfinyl)phenyl]-5,5-dimethyl-2,4-dioxo-imidazolidin-1-carboxamid (Bsp Nr. 84)

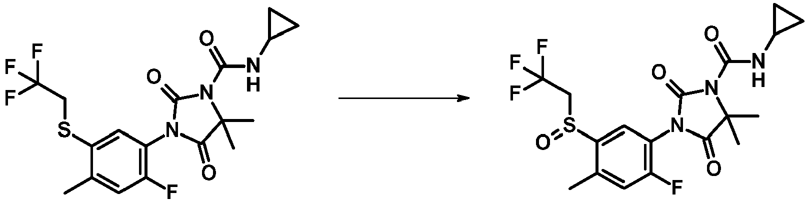

70 mg (0,16 mmol) *N*-cyclopropyl-3-[2-fluor-4-methyl-5-(2,2,2-trifluorethylsulfanyl)phenyl]-5,5-dimethyl-2,4-dioxo-imidazolidin-1-carboxamid wurden in 7 ml Dichlormethan vorgelegt, 41 mg (75%ig, 0,17 mmol) meta-Chlorperbenzoesäure wurden zugegeben und das Reaktionsgemisch wurde über Nacht bei Raumtemperatur gerührt und anschließend mit Natriumthiosulfat- und Natriumhydrogencarbonatlösung gewaschen. Die organische Phase wurde über trockenem Natriumsulfat getrocknet, filtriert und eingeengt. 74 mg (Reinheit 94% nach LC/MS, 96% der Theorie) der Titelverbindung wurden isoliert.
logP(HCOOH): 2,73; logP(neutral): 2,73
¹H-NMR(400,0 MHz, D6-DMSO) δ ppm: 8,14(d,1H), 8,00-7,99(m,1H), 7,56(d,1H), 4,43-4,18(m,1H), 4,04-3,72(m,1H), 2,72-2,67(m,1H), 2,44(s,3H), 1,71(s,6H), 0,71-0,70(m,2H), 0,53(breit,2H).

### Herstellbeispiel 7: 3-[2-Fluor-4-methyl-5-(2,2,2-trifluorethylsulfinyl)phenyl]-1-(morpholin-4-carbonyl)imidazolidin-2,4-dion (Bsp Nr. 125)

### Stufe 1: 3-[2-Fluor-4-methyl-5-(2,2,2-trifluorethylsulfanyl)phenyl]-1-(morpholin-4-carbonyl)imidazolidin-2,4-dion (Bsp Nr. 122)

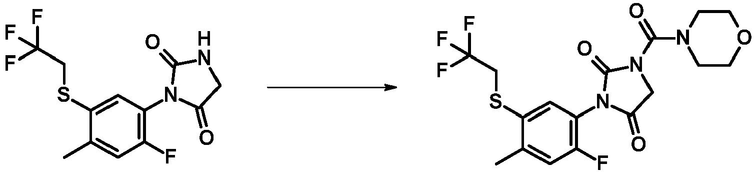

300 mg (0,93 mmol) 3-[2-Fluor-4-methyl-5-(2,2,2-trifluorethylsulfanyl)phenyl]imidazolidin-2,4-dion (IIa-1) wurden in 10 ml Toluol vorgelegt. Dazu wurden 0,26 ml (1,86 mmol) Triethylamin un 0,12 ml (0,93 mmol) Trichlormethylchlorformiat gegeben. Das Reaktionsgemisch wurde 1 h bei Rückflusstemperatur gerührt und anschließend auf 0 °C abgekühlt. Nach der Zugabe von 0,25 ml (2,78 mmol) Morpholin wurde das Reaktionsgemisch über Nacht bei Raumtemperatur nachgerührt. Die entstandene Suspension wurde filtriert, das Filtrat wurde vom Lösungsmittel unter vermindertem Druck am Rotationsverdampfer befreit. Der Rückstand wurde in Dichlormethan gelöst und zweimal mit einer gesättigten Natriumbicarbonat-Lösung und zweimal mit einer 10%-iger Salzsäure-Lösung gewaschen. Die organische Phase wurde über trockenem Natriumsulfat getrocknet, filtriert und eingeengt. Der Rückstand wurde mittels einer MPLC über einer RP-18 Säule chromatographiert mit einem Gradienten aus Wasser / Acetonitril. 178 mg (Reinheit 100% nach LC/MS, 44% der Theorie) der Titelverbindung wurden isoliert.
logP(HCOOH): 2,61; logP(neutral): 2,58
¹H-NMR(400,0 MHz, D6-DMSO) δ ppm: 7,72(d,1H), 7,42(d,1H), 4,53(s,2H), 3,87(q,2H), 3,64-3,62(m,4H), 3,48-3,46(m,4H), 2,45(s,3H).

### Stufe 2: 3-[2-Fluor-4-methyl-5-(2,2,2-trifluorethylsuffinyl)phenyl]-1-(morpholin-4-carbonyl)imidazolidin-2,4-dion (Bsp Nr. 125)

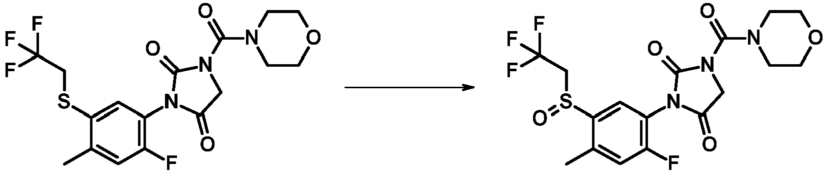

128 mg (0,29 mmol) 3-[2-Fluor-4-methyl-5-(2,2,2-trifluorethylsulfanyl)phenyl]-1-(morpholin-4-carbonyl)imidazolidin-2,4-dion wurden in 5 ml Dichlormethan vorgelegt, 71 mg (75%ig, 0,31 mmol) meta-Chlorperbenzoesäure wurden zugegeben. Das Reaktionsgemisch wurde über Nacht bei Raumtemperatur gerührt und anschließend mit Natriumthiosulfat- und Natriumhydrogencarbonatlösung gewaschen. Die organische Phase wurde über trockenem Natriumsulfat getrocknet, filtriert und eingeengt. 137 mg (Reinheit 98% nach LC/MS, quantitativ) der Titelverbindung wurden isoliert.
logP(HCOOH): 1,79; logP(neutral): 1,75
¹H-NMR(400,0 MHz, D6-DMSO) δ ppm: 8,04(d,1H), 7,53(d,1H), 4,53(s,2H), 4,33-4,24(m,1H), 3,88-3,79(m,1H), 3,64-3,62(m,4H), 3,49-3,47(m,4H), 2,43(s,3H).

### Syntheseintermediate

### 3-[2-Fluor-4-methyl-5-(2,2,2)-trifluorethylsulfanyl)phenyl]imidazolidin-2,4-dion (IIa-1)

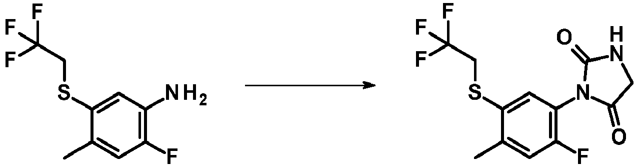

9,72 g (75,2 mmol) Isocyanatoessigsäureethylester wurden in 270 ml Chloroform vorgelegt. 18,00 g (75,2 mmol) 2-Fluor-4-methyl-5-(2,2,2-trifluorethylsulfanyl)anilin wurden in 270 ml Chloroform vorgelegt und langsam hinzugetropft. Die Lösung wurde über Nacht bei Raumtemperatur gerührt und dann wurde das Lösungsmittel unter vermindertem Druck entfernt. Der Rückstand wurde in 540 ml von einem Gemisch aus Ethanol / 35%iger Salzsäure 1:1 aufgenommen, 3 h refluxiert und übers Wochenende stehen gelassen. Der entstandene, weiße Feststoff wurde abgenutscht unt mit wenig eiskaltem Wasser bis pH5 gewaschen. Es wurden 18,55 g (Reinheit 100% nach LC/MS, 77% der Theorie) der Titelverbindung isoliert.
logP(HCOOH): 2,11; logP(neutral): 2,08
¹H-NMR(400,0 MHz, D6-DMSO) δ ppm: 8,42(bs,1H), 7,62(d,1H), 7,39(d,1H), 4,15(breit,2H), 3,91(q,2H), 2,43(s,3H)

### 3-[2-Fluor-4-methyl-5-(2,2,2-trifluorethylsulfanyl)phenyl]-2-thioxo-imidazolidin-4-on (IIa-2)

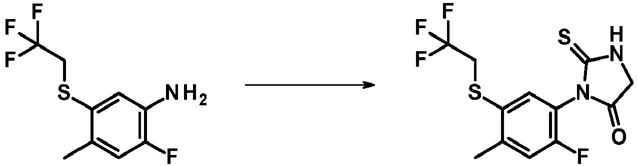

303 mg (2,09 mmol) Isothiocyanatoessigsäureethylester wurden in 7,5 ml Chloroform vorgelegt. 500 mg (2,09 mmol) 2-Fluor-4-methyl-5-(2,2,2-trifluorethylsulfanyl)anilin wurden in 7,5 ml Chloroform vorgelegt und langsam hinzugetropft. Die Lösung wurde über Nacht bei Raumtemperatur gerührt und dann wurde das Lösungsmittel unter vermindertem Druck entfernt. Der Rückstand wurde in 15 ml von einem Gemisch aus Ethanol / 35%iger Salzsäure 1:1 aufgenommen und 3 h refluxiert. Nach dem Abkühlen wurde das Lösungsmittel unter vermindertem Druck entfernt. Der Rückstand wurde mittels einer MPLC über einer RP-18 Säule chromatographiert mit einem Gradienten aus Wasser / Acetonitril / 0.1% Ameisensäure. 576 mg (Reinheit 99% nach LC/MS, 81% der Theorie) der Titelverbindung wurden isoliert.
logP(HCOOH): 2,53; logP(neutral): 2,50
¹H-NMR(400,0 MHz, D6-DMSO) δ ppm: 10,56(s,1H), 7,61(d,1H), 7,39(d,1H), 4,48-4,30(m,2H), 3,93-3,85(m,2H), 2,44(s,3H)

### 3-[2-Fluor-4-methyl-5-(2,2,2)-trifluorethylsulfinyl)phenyl]imidazolidin-2,4-dion (IIb-1)

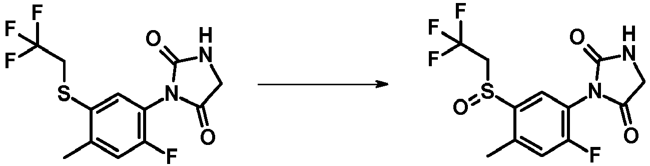

50 mg (0,15 mmol) 3-[2-Fluor-4-methyl-5-(2,2,2)-trifluorethylsulfanyl)phenyl]imidazolidin-2,4-dion (IIa-1) wurden in 5 ml Dichlormethan vorgelegt, 38 mg (75%ig, 0,16 mmol) meta-Chlorperbenzoesäure wurden zugegeben und das Reaktionsgemisch wurde 2 h bei Raumtemperatur gerührt und anschließend mit Natriumthiosulfat- und Natriumhydrogencarbonatlösung gewaschen. Die organische Phase wurde über trockenem Natriumsulfat getrocknet und filtriert. Nach Entfernen des Lösungsmittels unter vermindertem Druck wurde der Rückstand mittels einer MPLC über einer RP-18 Säule chromatographiert mit einem Gradienten aus Wasser / Acetonitril / 0.1% Ameisensäure. 25 mg (Reinheit 100% nach LC/MS, 49% der Theorie) der Titelverbindung wurden isoliert.
logP(HCOOH): 1,33; logP(neutral): 1,31
¹H-NMR(400,0 MHz, D6-DMSO) δ ppm: 8,47(bs,1H), 7,89(d,1H), 7,50(d,1H), 4,31-4,21(m,1H), 4,16(breit,2H), 3,96-3,87(m,1H), 2,42(s,3H).

### 3-[2-Fluor-4-methyl-5-(2,2,2-trifluorethylsulfanyl)phenyl]-5-methyl-imidazolidin-2,4-dion (IIa-3)

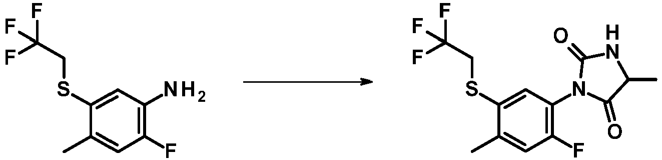

598 mg (4,18 mmol) Ethyl-2-isocyanatopropionat wurden in 15 ml Chloroform vorgelegt. 1,00 g (4,18 mmol) 2-Fluor-4-methyl-5-(2,2,2-trifluorethylsulfanyl)anilin wurden in 15 ml Chloroform vorgelegt und langsam hinzugetropft. Die Lösung wurde über Nacht bei Raumtemperatur gerührt und dann wurde das Lösungsmittel unter vermindertem Druck entfernt. Der Rückstand wurde in 30 ml von einem Gemisch aus Ethanol / 35%iger Salzsäure 1:1 aufgenommen und 3 h refluxiert. Nach dem Abkühlen wurde das Lösungsmittel unter vermindertem Druck entfernt. Der Rückstand wurde mittels einer MPLC über einer RP-18 Säule chromatographiert mit einem Gradienten aus Wasser / Acetonitril / 0.1% Ameisensäure. 395 mg (Reinheit 99% nach LC/MS, 28% der Theorie) der Titelverbindung wurden isoliert.
logP(HCOOH): 2,34; logP(neutral): 2,31
¹H-NMR(400,0 MHz, D6-DMSO) δ ppm: 8,56(s,1H), 7,64(d,1H), 7,39(d,1H), 4,35-4,33(m,1H), 3,96-3,88(m,2H), 2,43(s,3H), 1,36 (d,3H).

### 3-[2-Fluor-4-methyl-5-(2,2,2-trifluorethylsulfanyl)phenyl]-5,5-dimethyl-imidazolidin-2,4-dion (IIa-5)

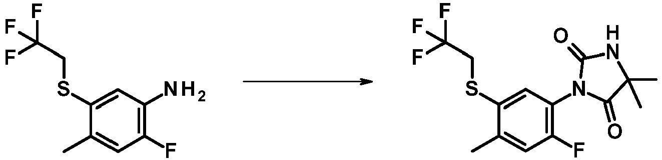

657 mg (4,18 mmol) Ethyl-2-isocyanato-2-methylpropionat wurden in 15 ml Chloroform vorgelegt. 1,00 g (4,18 mmol) 2-Fluor-4-methyl-5-(2,2,2-trifluorethylsulfanyl)anilin wurden in 15 ml Chloroform vorgelegt und langsam hinzugetropft. Die Lösung wurde über Nacht bei Raumtemperatur gerührt und dann wurde das Lösungsmittel unter vermindertem Druck entfernt. Der Rückstand wurde in 30 ml von einem Gemisch aus Ethanol / 35%iger Salzsäure 1:1 aufgenommen und 3 h refluxiert. Nach dem Abkühlen wurde das Lösungsmittel unter vermindertem Druck entfernt. Der Rückstand wurde mittels einer MPLC über einer RP-18 Säule chromatographiert mit einem Gradienten aus Wasser / Acetonitril / 0.1% Ameisensäure. 402 mg (Reinheit 100% nach LC/MS, 27% der Theorie) der Titelverbindung wurden isoliert.
logP(HCOOH): 2,61; logP(neutral): 2,53
¹H-NMR(400,0 MHz, D6-DMSO) δ ppm: 8,66(s,1H), 7,67(d,1H), 7,38(d,1H), 3,96-3,91(m,2H), 2,43(s,3H), 1,41(s,6H).

Gemäß den zuvor beschriebenen Verfahren wurden die folgenden Verbindungen der Formel (I) hergestellt (vgl. Tabelle 1).

**Tabelle 1: Verbindungen der Formel (I)**

| | | | | | | | | | |
|---|---|---|---|---|---|---|---|---|---|
| 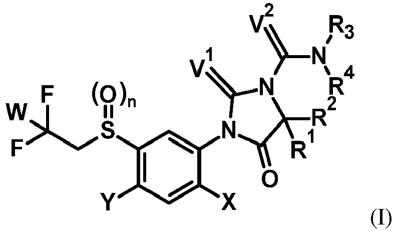 | | | | | | | | | |
| mit W=F | | | | | | | | | |

| BspNr | n | Y | X | V¹ | V² | R¹ | R² | R³ | R⁴ |
|---|---|---|---|---|---|---|---|---|---|
| 1 | 0 | CH₃ | F | O | O | H | H | Cyclopropyl | H |
| 2 | 0 | CH₃ | F | S | O | H | H | Cyclopropyl | H |
| 3 | 1 | CH₃ | F | O | O | H | H | Cyclopropyl | H |
| 4 | 1 | CH₃ | F | S | O | H | H | Cyclopropyl | H |
| 5 | 0 | CH₃ | F | S | O | H | H | Isopropyl | H |
| 6 | 0 | CH₃ | F | O | O | H | H | Ethyl | H |
| 7 | 0 | CH₃ | F | O | O | H | H | Isopropyl | H |
| 8 | 0 | CH₃ | F | S | O | H | H | Ethyl | H |
| 9 | 1 | CH₃ | F | O | O | H | H | Ethyl | H |
| 10 | 1 | CH₃ | F | O | O | H | H | Isopropyl | H |
| 11 | 0 | CH₃ | F | S | O | H | H | Isobutyl | H |
| 12 | 0 | CH₃ | F | O | O | H | H | Isobutyl | H |
| 13 | 1 | CH₃ | F | S | O | H | H | Isobutyl | H |
| 14 | 1 | CH₃ | F | S | O | H | H | Isopropyl | H |
| 15 | 1 | CH₃ | F | O | O | H | H | Isobutyl | H |
| 16 | 0 | CH₃ | F | O | O | H | H | Phenyl | H |
| 17 | 0 | CH₃ | F | O | O | H | H | 3-Pyridyl | H |
| 18 | 0 | CH₃ | F | O | O | H | H | Benzyl | H |
| 19 | 1 | CH₃ | F | O | O | H | H | Phenyl | H |
| 20 | 1 | CH₃ | F | O | O | H | H | 3-Pyridyl | H |
| 21 | 1 | CH₃ | F | O | O | H | H | Benzyl | H |
| 22 | 0 | CH₃ | CH₃ | O | O | H | H | Isopropyl | H |
| 23 | 1 | CH₃ | CH₃ | O | O | H | H | Isopropyl | H |
| 24 | 0 | CH₃ | F | O | O | CH₃ | H | Ethyl | H |
| 25 | 0 | CH₃ | F | O | O | CH₃ | H | Cyclopropyl | H |
| 26 | 0 | CH₃ | F | O | O | CH₃ | H | Isopropyl | H |
| 27 | 1 | CH₃ | F | O | O | CH₃ | H | Ethyl | H |
| 28 | 1 | CH₃ | F | O | O | CH₃ | H | Cyclopropyl | H |
| 29 | 1 | CH₃ | F | O | O | CH₃ | H | Isopropyl | H |
| 30 | 0 | CH₃ | F | O | O | H | H | 2,2,2-Trifluorethyl | H |
| 31 | 0 | CH₃ | CH₃ | O | O | H | H | Ethyl | H |
| 32 | 1 | CH₃ | CH₃ | O | O | H | H | Ethyl | H |
| 33 | 0 | CH₃ | CH₃ | O | O | H | H | tert-Butyl | H |
| 34 | 0 | CH₃ | CH₃ | O | O | H | H | Isobutyl | H |
| 35 | 1 | CH₃ | CH₃ | O | O | H | H | Isobutyl | H |
| 36 | 1 | CH₃ | F | O | O | H | H | 2,2,2-Trifluorethyl | H |
| 37 | 0 | CH₃ | F | O | O | H | H | 3-Trifluorethylp henyl | H |
| 38 | 0 | CH₃ | CH₃ | O | O | H | H | 2,2,2-Trifluorethyl | H |
| 39 | 0 | CH₃ | F | O | O | H | H | 4-Fluorphenyl | H |
| 40 | 1 | CH₃ | CH₃ | O | O | H | H | tert-Butyl | H |
| 41 | 1 | CH₃ | F | O | O | H | H | 4-Fluorphenyl | H |
| 42 | 0 | CH₃ | F | O | O | H | H | 3-Fluorphenyl | H |
| 43 | 1 | CH₃ | F | O | O | H | H | 3-Trifluorethylp henyl | H |
| 44 | 0 | CH₃ | F | O | O | H | H | sec-Butyl | H |
| 45 | 0 | CH₃ | F | O | O | H | H | 3-Chlorphenyl | H |
| 46 | 1 | CH₃ | F | O | O | H | H | 3-Fluorphenyl | H |
| 47 | 1 | CH₃ | F | O | O | H | H | sec-Butyl | H |
| 48 | 1 | CH₃ | F | O | O | H | H | 3-Chlorphenyl | H |
| 49 | 0 | CH₃ | CH₃ | O | O | H | H | Cyclopropyl | H |
| 50 | 0 | CH₃ | Cl | O | O | H | H | Cyclopropyl | H |
| 51 | 0 | CH₃ | Cl | O | O | H | H | Ethyl | H |
| 52 | 0 | CH₃ | Cl | O | O | H | H | Isobutyl | H |
| 53 | 0 | CH₃ | Cl | O | O | H | H | 2,2,2-Trifluorethyl | H |
| 54 | 0 | CH₃ | F | O | O | H | H | tert-Butyl | H |
| 55 | 0 | CH₃ | CH₃ | O | O | H | H | 3-Trifluorethylp henyl | H |
| 56 | 0 | CH₃ | CH₃ | O | O | H | H | 3-Chlorphenyl | H |
| 57 | 0 | CH₃ | Cl | O | O | H | H | Benzyl | H |
| 58 | 0 | CH₃ | Cl | O | O | H | H | 3-Trifluorethylp henyl | H |
| 59 | 0 | CH₃ | Cl | O | O | H | H | 3-Chlorphenyl | H |
| 60 | 1 | CH₃ | CH₃ | O | O | H | H | Cyclopropyl | H |
| 61 | 1 | CH₃ | CH₃ | O | O | H | H | 2,2,2-Trifluorethyl | H |
| 62 | 1 | CH₃ | Cl | O | O | H | H | Cyclopropyl | H |
| 63 | 1 | CH₃ | Cl | O | O | H | H | 2,2,2-Trifluorethyl | H |
| 64 | 1 | CH₃ | Cl | O | O | H | H | Ethyl | H |
| 65 | 1 | CH₃ | Cl | O | O | H | H | Isobutyl | H |
| 66 | 0 | CH₃ | F | O | O | H | H | 2-Methoxyethyl | H |
| 67 | 0 | CH₃ | F | O | O | H | H | 2-Tetrahydrofur ylmethyl | H |
| 68 | 1 | CH₃ | F | O | O | H | H | tert-Butyl | H |
| 69 | 1 | CH₃ | CH₃ | O | O | H | H | 3-Chlorphenyl | H |
| 70 | 1 | CH₃ | Cl | O | O | H | H | Benzyl | H |
| 71 | 1 | CH₃ | Cl | O | O | H | H | 3-Trifluorethylp henyl | H |
| 72 | 1 | CH₃ | Cl | O | O | H | H | 3-Chlorphenyl | H |
| 73 | 0 | CH₃ | F | O | O | CH₃ | CH₃ | Ethyl | H |
| 74 | 0 | CH₃ | F | O | O | CH₃ | CH₃ | Cyclopropyl | H |
| 75 | 0 | CH₃ | F | O | O | CH₃ | CH₃ | 2,2,2-Trifluorethyl | H |
| 76 | 0 | CH₃ | F | O | O | CH₃ | CH₃ | Isobutyl | H |
| 77 | 0 | CH₃ | F | O | O | CH₃ | CH₃ | Benzyl | H |
| 78 | 0 | CH₃ | F | O | O | H | H | 2-Pyridyl | H |
| 79 | 0 | CH₃ | F | O | O | CH₃ | H | 2,2,2-Trifluorethyl | H |
| 80 | 0 | CH₃ | F | O | O | CH₃ | H | Isobutyl | H |
| 81 | 0 | CH₃ | F | O | O | CH₃ | H | Benzyl | H |
| 82 | 1 | CH₃ | F | O | O | H | H | 2-Methoxyethyl | H |
| 83 | 1 | CH₃ | F | O | O | CH₃ | CH₃ | Ethyl | H |
| 84 | 1 | CH₃ | F | O | O | CH₃ | CH₃ | Cyclopropyl | H |
| 85 | 1 | CH₃ | F | O | O | CH₃ | CH₃ | 2,2,2-Trifluorethyl | H |
| 86 | 1 | CH₃ | F | O | O | CH₃ | CH₃ | Isobutyl | H |
| 87 | 1 | CH₃ | F | O | O | CH₃ | CH₃ | Benzyl | H |
| 88 | 1 | CH₃ | F | O | O | CH₃ | H | 2,2,2-Trifluorethyl | H |
| 89 | 1 | CH₃ | F | O | O | CH₃ | H | Isobutyl | H |
| 90 | 1 | CH₃ | F | O | O | CH₃ | H | Benzyl | H |
| 91 | 1 | CH₃ | F | O | O | H | H | 2-Tetrahydrofur ylmethyl | H |
| 92 | 0 | CH₃ | CH₃ | O | O | H | H | Benzyl | H |
| 93 | 0 | CH₃ | CH₃ | O | O | H | H | 4-Fluorphenyl | H |
| 94 | 0 | CH₃ | Cl | O | O | H | H | 4-Fluorphenyl | H |
| 95 | 0 | CH₃ | CH₃ | O | O | H | H | 2-Pyridyl | H |
| 96 | 0 | CH₃ | Cl | O | O | H | H | 2-Pyridyl | H |
| 97 | 1 | CH₃ | CH₃ | O | O | H | H | 3-Trifluorethylp henyl | H |
| 98 | 1 | CH₃ | CH₃ | O | O | H | H | Benzyl | H |
| 99 | 1 | CH₃ | CH₃ | O | O | H | H | 4-Fluorphenyl | H |
| 100 | 1 | CH₃ | Cl | O | O | H | H | 4-Fluorphenyl | H |
| 101 | 0 | CH₃ | F | O | O | H | H | 1,1-Dimethylprop yl | H |
| 102 | 0 | CH₃ | F | O | S | H | H | 2,2,2-Trifluorethyl | H |
| 103 | 0 | Cl | Cl | O | O | H | H | Ethyl | H |
| 104 | 0 | Cl | Cl | O | O | H | H | Cyclopropyl | H |
| 105 | 0 | Cl | Cl | O | O | H | H | Isobutyl | H |
| 106 | 0 | Cl | Cl | O | O | H | H | 2,2,2-Trifluorethyl | H |
| 107 | 0 | Cl | Cl | O | O | H | H | Benzyl | H |
| 108 | 0 | Cl | Cl | O | O | H | H | 4-Fluorphenyl | H |
| 109 | 0 | Cl | Cl | O | O | H | H | 3-Chlorphenyl | H |
| 110 | 0 | Cl | Cl | O | O | H | H | 3-Trifluormethyl phenyl | H |
| 111 | 1 | Cl | Cl | O | O | H | H | Ethyl | H |
| 112 | 1 | Cl | Cl | O | O | H | H | Cyclopropyl | H |
| 113 | 1 | Cl | Cl | O | O | H | H | Isobutyl | H |
| 114 | 1 | Cl | Cl | O | O | H | H | 2,2,2-Trifluorethyl | H |
| 115 | 1 | Cl | Cl | O | O | H | H | Benzyl | H |
| 116 | 1 | Cl | Cl | O | O | H | H | 4-Fluorphenyl | H |
| 117 | 1 | Cl | Cl | O | O | H | H | 3-Chlorphenyl | H |
| 118 | 1 | CH₃ | CH₃ | O | O | H | H | 2-Pyridyl | H |
| 119 | 1 | CH₃ | Cl | O | O | H | H | 2-Pyridyl | H |
| 120 | 1 | Cl | Cl | O | O | H | H | 3-Trifluormethyl phenyl | H |
| 121 | 0 | CH₃ | F | O | O | H | H | Cyclopropyl | CH₃ |
| 122 | 0 | CH₃ | F | O | O | H | H | 1-Morpholin | |
| 123 | 0 | CH₃ | F | O | O | H | H | 1-(4-Methylpiperazin) | |
| 124 | 0 | CH₃ | F | O | O | H | H | Isobutyl | CH₃ |
| 125 | 1 | CH₃ | F | O | O | H | H | 1-Morpholin | |
| 126 | 1 | CH₃ | F | O | O | H | H | Isobutyl | CH₃ |
| 127 | 1 | CH₃ | F | O | O | H | H | 1-(4-Methylpiperazin) | |
| 128 | 0 | CH₃ | F | O | O | H | H | 1-(1,1-Dioxo-1,4-thiazinan) | |
| 129 | 0 | CH₃ | F | O | O | H | H | 1-(4,4-Difluorpiperidin) | |
| 130 | 0 | CH₃ | F | O | O | H | H | CH₃ | CH₃ |
| 131 | 1 | CH₃ | F | O | O | H | H | Cyclopropyl | CH₃ |
| 132 | 1 | CH₃ | F | O | O | H | H | 1-(4,4-Difluorpiperidin) | |
| 133 | 1 | CH₃ | F | O | O | H | H | CH₃ | CH₃ |
| 134 | 1 | CH₃ | F | O | O | H | H | 1-(1,1-Dioxo-1,4-thiazinan) | |
| 135 | 1 | CH₃ | F | O | O | H | H | 2,2,2-Trifluorethyl | H |
| 136 | 1 | CH₃ | F | O | O | H | H | 2,2,2-Trifluorethyl | H |

### NMR-Peak-Listenverfahren

Die ¹H-NMR-Daten ausgewählter Beispiele werden in Form von ¹H-NMR-Peaklisten notiert. Zu jedem Signalpeak wird erst der δ-Wert in ppm und dann die Signalintensität in runden Klammern aufgeführt. Die δ-Wert - Signalintensitäts- Zahlenpaare von verschiedenen Signalpeaks werden durch Semikolons voneinander getrennt aufgelistet.

Die Peakliste eines Beispieles hat daher die Form:
δ₁ (Intensität₁⁾; δ₂ (Intensität₂);........; δᵢ (Intensitätᵢ⁾;......; δₙ (Intensitätₙ)

Die Intensität scharfer Signale korreliert mit der Höhe der Signale in einem gedruckten Beispiel eines NMR-Spektrums in cm und zeigt die wirklichen Verhältnisse der Signalintensitäten. Bei breiten Signalen können mehrere Peaks oder die Mitte des Signals und ihre relative Intensität im Vergleich zum intensivsten Signal im Spektrum gezeigt werden.

Zur Kalibrierung der chemischen Verschiebung von ¹H-NMR-Spektren benutzen wir Tetramethylsilan und/oder die chemische Verschiebung des Lösungsmittels, besondern im Falle von Spektren, die in DMSO gemessen werden. Daher kann in NMR-Peaklisten der Tetramethylsilan-Peak vorkommen, muss es aber nicht.

Die Listen der ¹H-NMR-Peaks sind ähnlich den klassischen ¹H-NMR-Ausdrucken und enthalten somit gewöhnlich alle Peaks, die bei einer klassischen NMR-Interpretation aufgeführt werden.

Darüber hinaus können sie wie klassische ¹H-NMR-Ausdrucke Lösungsmittelsignale, Signale von Stereoisomeren der Zielverbindungen, die ebenfalls Gegenstand der Erfindung sind, und/oder Peaks von Verunreinigungen zeigen.

Bei der Angabe von Verbindungssignalen im Delta-Bereich von Lösungsmitteln und/oder Wasser sind in unseren Listen von ¹H-NMR-Peaks die gewöhnlichen Lösungsmittelpeaks, zum Beispiel Peaks von DMSO in D₆-DMSO und der Peak von Wasser, gezeigt, die gewöhnlich im Durchschnitt eine hohe Intensität aufweisen.

Die Peaks von Stereoisomeren der Targetverbindungen und/oder Peaks von Verunreinigungen haben gewöhnlich im Durchschnitt eine geringere Intensität als die Peaks der Zielverbindungen (zum Beispiel mit einer Reinheit von >90%).

Solche Stereoisomere und/oder Verunreinigungen können typisch für das jeweilige Herstellungsverfahren sein. Ihre Peaks können somit dabei helfen, die Reproduktion unseres Herstellungsverfahrens anhand von "Nebenprodukt-Fingerabdrücken" zu erkennen.

Einem Experten, der die Peaks der Zielverbindungen mit bekannten Verfahren (MestreC, ACD-Simulation, aber auch mit empirisch ausgewerteten Erwartungswerten) berechnet, kann je nach Bedarf die Peaks der Zielverbindungen isolieren, wobei gegebenenfalls zusätzliche Intensitätsfilter eingesetzt werden. Diese Isolierung wäre ähnlich dem betreffenden Peak-Picking bei der klassischen ¹H-NMR-Interpretation.

Weitere Details zu ¹H-NMR-Peaklisten können der Research Disclosure Database Number 564025 entnommen werden.

**Tabelle 2: Spektroskopische Daten der Verbindungen gemäß Tabelle 1:**

| Bsp Nr. | logP[b] | logP[a] | ¹H-NMR |
|---|---|---|---|
| 1 | 2,98 | 3,07 | (400,0 MHz, d₆-DMSO): δ= 7,789(2,2);7,781(2,3);7,683(2,9);7,665(3,0);7,458(2,6); 7,431(2,6);5,756(5,5);4,467(0,6);3,902(1,3);3,876(3,9);3,850(4,0);3,825(1,4);3,323( 23,3);2,700(0,6);2,691(1,0);2,682(1,5);2,673(1,6);2,664(1,3);2,656(0,7);2,524(0,8);2 ,510(18,6);2,506(38,1);2,502(51,1);2,497(38,6);2,453(16,0);2,328(0,3);0,730(0,7);0, 716(2,4);0,712(3,2);0,699(3,0);0,694(2,7);0,683(1,0);0,564(1,0);0,553(2,9);0,546(3, 2);0,538(2,6);0,525(0,8);0,008(0,6);0,000(17,2);-0,008(0,8) |
| 2 | 3,56 | 3,66 | (400,0 MHz, d₆-DMSO): δ= 9,503(2,1);9,495(2,2);7,659(2,8);7,641(2,9);7,462(2,6); 7,435(2,5);5,756(3,9);4,845(2,8);4,796(4,2);4,652(4,2);4,603(2,7);4,414(0,6);3,898( 1,2);3,872(3,8);3,846(3,9);3,820(1,4);3,323(57,0);2,770(0,6);2,761(1,0);2,752(1,4);2 ,743(1,5);2,734(1,1);2,726(0,7);2,717(0,3);2,675(0,5);2,671(0,8);2,666(0,6);2,523(1, 9);2,510(41,4);2,506(84,0);2,502(112,6);2,497(85,2);2,459(16,0);2,407(0,8);2,333(0 ,5);2,328(0,7);2,324(0,6);0,789(0,3);0,770(2,9);0,757(2,5);0,753(2,8);0,739(0,6);0,5 97(0,4);0,588(0,4);0,571(2,0);0,568(2,1);0,559(2,2);0,554(1,9);0,550(2,0);0,545(1,7) ;0,525(0,4);0,519(0,4);0,008(1,2);0,000(36,9);-0,008(1,9) |
| 3 | 2,12 | 2,15 | (600,1 MHz, d₆-DMSO): δ= 7,993(2,6);7,981(2,6);7,782(2,2);7,777(2,2);7,562(2,2); 7,545(2,2);4,484(0,3);4,449(0,4);4,331(0,8);4,324(0,4);4,313(1,0);4,306(1,0);4,294( 0,4);4,288(1,0);4,270(0,3);3,903(12,1);3,870(0,5);3,852(0,6);3,846(0,5);3,828(0,5);3 ,321(142,8);2,698(0,6);2,692(1,0);2,686(1,4);2,681(1,4);2,675(1,0);2,669(0,6);2,616 (0,4);2,613(0,6);2,610(0,4);2,522(1,1);2,519(1,3);2,516(1,5);2,507(34,0);2,504(71,6) ;2,501(98,2);2,498(71,7);2,495(34,3);2,425(16,0);2,388(0,5);2,385(0,6);2,382(0,5);0 ,724(0,6);0,715(2,4);0,713(2,9);0,704(2,7);0,701(2,5);0,693(0,8);0,556(0,9);0,549(2, 7);0,545(2,9);0,539(2,4);0,531(0,7);0,000(5,6) |
| 4 | 2,65 | 2,64 | (400,0 MHz, d₆-DMSO): δ= 9,495(1,6);9,487(1,7);9,463(1,8);9,455(1,9);8,315(0,6); 7,991(2,4);7,983(2,7);7,973(2,5);7,965(2,6);7,576(3,2);7,550(3,2);5,755(7,9);4,841( 2,5);4,830(2,3);4,792(3,5);4,782(3,3);4,632(5,1);4,584(3,3);4,389(0,8);4,379(0,5);4, 362(0,9);4,352(1,6);4,342(0,4);4,335(0,4);4,325(1,8);4,315(1,0);4,298(0,6);4,288(1, 0);3,878(0,9);3,867(0,4);3,862(0,3);3,851(1,1);3,841(1,0);3,836(1,0);3,824(0,7);3,81 4(1,0);3,809(1,1);3,798(0,8);3,787(0,4);3,782(0,4);3,772(0,8);3,321(105,4);2,780(0,6);2,771(1,3);2,762(1,9);2,753(2,3);2,744(2,0);2,735(1,4);2,727(0,8);2,675(1,1);2,67 0(1,5);2,666(1,1);2,541(0,8);2,524(3,9);2,519(6,0);2,510(80,6);2,506(166,8);2,501(2 22,8);2,497(164,9);2,492(82,4);2,435(16,0);2,337(0,5);2,333(1,1);2,328(1,5);2,324( 1,1);0,796(0,4);0,781(2,1);0,778(3,2);0,771(3,5);0,765(3,1);0,760(3,3);0,757(3,1);0, 754(2,7);0,749(1,7);0,740(0,7);0,594(0,4);0,572(2,1);0,563(4,0);0,553(3,9);0,528(0, 4);0,521(0,3);0,146(1,0);0,008(8,0);0,000(238,8);-0,009(10,0);-0,150(1,0) |
| 5 | 3,87 | 3,98 | (600,1 MHz, d₆-DMSO): δ= 9,419(1,8);9,407(1,9);7,671(2,5);7,659(2,5);7,461(2,3); 7,444(2,2);4,832(3,2);4,800(4,0);4,638(4,2);4,605(3,1);3,937(0,9);3,926(1,4);3,914( 1,5);3,903(3,1);3,891(1,2);3,873(3,2);3,856(3,3);3,839(1,2);3,318(38,7);2,616(0,4);2 ,613(0,5);2,610(0,4);2,522(1,0);2,519(1,2);2,516(1,3);2,507(30,2);2,504(62,2);2,501 (84,2);2,498(61,2);2,495(29,4);2,461(16,0);2,388(0,4);2,385(0,5);2,382(0,4);1,908(0 ,4);1,238(0,7);1,227(0,7);1,212(9,9);1,206(10,4);1,201(10,5);1,195(9,9);0,000(5,7) |
| 6 | 2,93 | 2,98 | (400,0 MHz, d₆-DMSO): δ= 7,874(0,9);7,860(1,9);7,846(1,0);7,701(2,9);7,683(2,9); 7,462(2,6);7,435(2,5);4,466(0,6);4,418(0,7);3,908(1,2);3,882(3,7);3,856(3,9);3,831( 1,3);3,324(26,4);3,283(0,7);3,265(2,2);3,248(2,9);3,232(2,3);3,215(0,7);2,671(0,4);2 ,506(45,6);2,502(60,0);2,498(45,4);2,457(16,0);2,375(0,8);2,329(0,4);1,113(5,1);1,0 95(10,7);1,077(4,9);1,066(0,7);0,008(1,3);0,000(35,5) |
| 7 | 3,29 | 3,34 | (400,0 MHz, d₆-DMSO): δ= 7,700(1,9);7,682(1,9);7,616(1,2);7,597(1,2);7,460(1,6); 7,433(1,6);5,488(0,7);5,469(0,7);4,466(0,3);3,925(0,6);3,907(1,5);3,890(1,0);3,880( 2,6);3,855(2,7);3,829(0,9);3,659(0,6);3,643(0,9);3,624(0,9);3,608(0,6);3,324(7,9);2, 525(0,3);2,511(8,7);2,507(17,7);2,502(23,4);2,498(17,1);2,493(8,4);2,458(10,3);2,4 37(0,6);2,086(0,6);1,176(12,8);1,160(12,7);1,010(16,0);0,993(15,7);0,008(0,5);0,00 0(15,5);-0,008(0,6) |
| 8 | 3,86 | 3,91 | (400,0 MHz, d₆-DMSO): δ= 11,476(2,2);8,315(0,9);7,975(2,7);7,956(2,7);7,332(2,5) ;7,303(2,5);4,524(12,8);3,905(1,3);3,879(4,1);3,853(4,2);3,827(1,5);3,523(1,2);3,50 5(4,1);3,487(4,1);3,469(1,3);3,323(71,4);2,675(0,9);2,671(1,3);2,666(0,9);2,524(2,8) ;2,511(70,8);2,506(145,7);2,502(193,9);2,497(140,0);2,493(67,1);2,408(16,0);2,338( 0,4);2,333(0,9);2,329(1,3);2,324(0,9);2,086(0,8);1,181(4,6);1,163(10,5);1,145(4,5);0 ,146(0,6);0,008(4,5);0,000(146,5);-0,008(4,9);-0,150(0,7) |
| 9 | 2,05 | 2,05 | (400,0 MHz, d₆-DMSO): δ= 8,317(0,5);8,016(3,0);7,998(3,0);7,867(1,1);7,854(2,1); 7,839(1,0);7,574(2,5);7,547(2,6);4,450(0,9);4,379(0,4);4,352(1,0);4,341(0,5);4,325( 1,1);4,315(1,1);4,297(0,5);4,287(1,1);4,260(0,4);3,884(0,8);3,857(0,9);3,847(0,8);3, 831(0,4);3,820(0,7);3,326(158,4);3,324(144,6);3,284(0,8);3,267(2,5);3,249(3,1);3,2 34(2,5);3,216(0,7);2,719(0,8);2,675(1,5);2,671(2,0);2,666(1,5);2,506(247,6);2,502(3 16,7);2,498(233,6);2,431(16,0);2,333(1,5);2,329(2,0);2,324(1,5);2,086(3,9);1,115(5, 3);1,097(11,3);1,079(5,2);0,008(1,5);0,000(32,5);-0,007(1,4) |
| 10 | 2,38 | 2,40 | (400,0 MHz, d₆-DMSO): δ= 8,317(0,5);8,012(2,4);7,994(2,4);7,614(1,6);7,596(1,7); 7,572(2,1);7,546(2,0);5,487(0,3);5,469(0,3);4,445(0,7);4,372(0,4);4,345(0,8);4,335( 0,4);4,318(0,9);4,308(1,0);4,290(0,4);4,281(1,0);3,923(0,8);3,907(1,2);3,891(1,5);3, 872(0,9);3,867(1,0);3,856(1,0);3,840(0,4);3,830(0,7);3,640(0,4);3,622(0,3);3,325(18 3,3);2,719(0,7);2,675(1,2);2,671(1,7);2,666(1,3);2,524(4,0);2,506(206,5);2,502(274, 3);2,497(205,2);2,430(13,0);2,333(1,3);2,328(1,8);2,324(1,3);2,086(1,8);1,237(0,3); 1,176(16,0);1,160(15,9);1,140(0,4);1,008(6,4);0,992(6,4);0,008(0,9);0,000(26,0);-0,008(1,2) |
| 11 | 4,18 | 4,27 | (400,0 MHz, d₆-DMSO): δ= 9,451(0,7);9,437(1,4);9,422(0,7);7,685(2,1);7,667(2,1); 7,470(1,9);7,443(1,8);4,853(2,2);4,804(3,2);4,654(3,3);4,606(2,1);3,906(0,9);3,881( 2,7);3,855(2,8);3,829(1,0);3,324(27,8);3,198(0,4);3,181(1,1);3,165(1,6);3,160(1,2);3 ,149(1,3);3,146(1,6);3,129(1,1);3,111(0,4);2,675(0,3);2,671(0,5);2,666(0,3);2,524(1, 1);2,511(25,5);2,506(52,7);2,502(70,3);2,497(51,3);2,493(25,1);2,464(11,8);2,442(0 ,4);2,333(0,3);2,328(0,4);2,324(0,3);1,844(0,4);1,827(0,9);1,811(1,1);1,794(0,9);1,7 78(0,5);0,935(0,5);0,923(16,0);0,906(15,5);0,000(4,7) |
| 12 | 3,58 | 3,67 | (400,0 MHz, d₆-DMSO): δ= 7,868(0,4);7,853(0,8);7,838(0,4);7,707(1,2);7,688(1,2); 7,466(1,1);7,439(1,1);5,757(16,0);3,911(0,5);3,885(1,6);3,860(1,7);3,834(0,6);3,086 (0,8);3,070(1,5);3,055(0,9);2,809(0,6);2,792(0,6);2,511(5,1);2,506(10,7);2,502(14,3) ;2,497(10,5);2,493(5,2);2,459(6,5);2,086(5,0);1,802(0,5);1,786(0,6);1,769(0,5);1,590(0,3);1,398(0,8);1,352(0,5);1,229(0,5);0,883(8,9);0,866(8,7);0,824(4,8);0,807(4,6); 0,000(1,8) |
| 13 | 3,24 | 3,33 | (400,0 MHz, d₆-DMSO): δ= 9,446(0,6);9,432(1,2);9,417(1,2);9,403(1,3);9,388(0,6); 8,316(0,8);8,011(3,4);7,993(3,4);7,585(2,6);7,559(2,6);5,757(0,6);4,848(1,9);4,837( 1,8);4,800(2,6);4,789(2,5);4,633(5,1);4,584(3,3);4,392(0,6);4,365(0,7);4,356(0,9);4, 332(0,9);4,328(0,9);4,322(0,8);4,301(0,4);4,295(0,8);3,884(0,8);3,858(1,1);3,847(0, 8);3,833(0,9);3,821(1,0);3,807(0,3);3,796(0,7);3,328(347,3);3,326(339,5);3,208(0,6) ;3,192(1,0);3,181(1,2);3,176(1,4);3,163(2,4);3,148(2,6);3,132(1,5);3,115(0,6);3,100( 0,4);2,675(2,4);2,671(3,3);2,666(2,4);2,662(1,2);2,524(8,5);2,519(12,7);2,511(186,9 );2,506(383,3);2,502(508,7);2,497(370,2);2,493(180,3);2,442(16,0);2,378(0,5);2,337 (1,1);2,333(2,4);2,329(3,3);2,324(2,4);1,850(0,4);1,844(0,4);1,833(0,8);1,827(0,9);1 ,817(1,0);1,811(1,1);1,801(0,9);1,794(0,9);1,784(0,5);1,777(0,5);1,148(0,4);0,930(1 3,3);0,922(14,1);0,913(13,4);0,905(13,3);0,893(0,6);0,886(0,5);0,866(0,6);0,849(0,5 );0,146(2,4);0,008(16,6);0,000(509,8);-0,009(17,5);-0,150(2,4) |
| 14 | 2,93 | 3,01 | (400,0 MHz, d₆-DMSO): δ= 9,420(1,4);9,402(1,5);9,383(1,5);9,365(1,6);8,316(0,7); 8,004(2,4);7,996(2,6);7,986(2,5);7,978(2,6);7,581(2,8);7,555(2,8);5,757(2,1);4,838( 2,4);4,828(2,3);4,790(3,4);4,779(3,2);4,623(6,5);4,574(4,4);4,397(0,7);4,386(0,4);4, 370(0,8);4,360(1,0);4,356(0,9);4,343(0,5);4,333(1,0);4,329(1,0);4,319(0,9);4,305(0, 4);4,301(0,4);4,291(0,9);3,945(0,9);3,929(1,5);3,912(1,5);3,898(1,0);3,895(0,9);3,88 1(1,2);3,870(0,4);3,854(1,0);3,841(1,1);3,828(0,6);3,817(1,1);3,814(1,2);3,803(0,8); 3,788(0,5);3,777(0,8);3,336(208,6);3,335(210,7);3,327(183,7);2,680(0,9);2,675(2,0) ;2,671(2,7);2,666(2,0);2,662(1,0);2,524(6,7);2,519(10,4);2,511(154,0);2,506(315,8); 2,502(418,7);2,497(305,0);2,493(148,5);2,439(15,6);2,378(0,6);2,333(2,0);2,329(2,7 );2,324(2,0);2,320(0,9);1,215(15,8);1,207(10,0);1,198(16,0);1,191(8,9);1,175(0,5);1, 147(0,4);1,140(0,5);1,119(0,4);0,146(2,2);0,008(16,6);0,000(503,2);-0,009(17,8);-0,150(2,2) |
| 15 | 2,66 | 2,72 | (400,0 MHz, d₆-DMSO): δ= 8,021(2,3);8,003(2,3);7,861(0,7);7,847(1,5);7,832(0, 8);7,575(1,9);7,548(1,9);5,757(0,9);4,455(0,5);4,348(0,8);4,338(0,4);4,320(0,9);4,31 1(0,9);4,293(0,4);4,283(0,9);3,897(0,7);3,870(0,8);3,860(0,7);3,833(0,7);3,328(42,9) ;3,325(42,8);3,089(1,5);3,073(2,7);3,057(1,6);2,675(0,5);2,671(0,6);2,666(0,5);2,52 4(1,8);2,510(37,6);2,506(76,1);2,502(100,7);2,497(75,4);2,493(38,4);2,431(11,9);2, 333(0,5);2,328(0,6);2,324(0,5);1,820(0,4);1,803(0,9);1,786(1,1);1,770(0,9);1,753(0, 5);0,885(16,0);0,868(15,5);0,146(0,4);0,008(3,1);0,000(92,2);-0,008(3,9);-0,150(0,4) |
| 16 | 3,71 | 3,82 | (400,0 MHz, d₆-DMSO): δ= 9,824(4,1);7,739(3,1);7,721(3,1);7,556(3,9);7,537(4,6); 7,499(2,6);7,472(2,6);7,457(0,4);7,438(0,4);7,385(3,0);7,380(1,1);7,366(4,4);7,345( 2,9);7,276(0,3);7,149(1,5);7,130(2,6);7,112(1,1);5,757(6,0);4,587(0,6);3,921(1,2);3, 896(3,9);3,870(4,0);3,844(1,4);3,327(20,3);2,524(0,7);2,511(14,1);2,506(28,8);2,50 2(38,4);2,497(28,3);2,493(14,0);2,477(16,0);2,086(1,8);1,398(1,1);0,000(0,7) |
| 17 | 2,75 | 2,37 | (400,0 MHz, d₆-DMSO): δ= 9,901(4,1);8,747(3,1);8,741(3,1);8,345(2,1);8,342(2,3); 8,334(2,3);8,330(2,4);8,027(1,2);8,023(1,4);8,021(1,4);8,017(1,3);8,006(1,3);8,002( 1,5);8,000(1,5);7,996(1,3);7,743(2,9);7,725(2,9);7,504(2,5);7,477(2,5);7,421(1,7);7, 409(1,7);7,400(1,7);7,388(1,6);4,591(0,7);3,921(1,2);3,895(3,7);3,870(3,9);3,844(1, 3);3,326(24,0);2,671(0,4);2,666(0,3);2,524(0,9);2,519(1,5);2,511(24,9);2,506(51,5); 2,502(68,7);2,497(50,6);2,493(25,2);2,479(16,0);2,389(0,4);2,333(0,3);2,328(0,5);2, 324(0,4);2,086(4,9);0,008(1,5);0,000(47,9);-0,009(1,8) |
| 18 | 3,53 | 3,64 | (600,1 MHz, d₆-DMSO): δ= 8,346(1,0);8,336(2,1);8,326(1,0);7,701(2,6);7,689(2,6); 7,460(2,3);7,442(2,3);7,336(14,4);7,329(11,6);7,319(0,4);7,316(0,4);7,314(0,5);7,26 7(0,8);7,260(1,4);7,253(1,6);7,246(1,0);7,239(0,4);4,435(3,3);4,425(3,3);3,891(1,0); 3,874(3,2);3,857(3,4);3,840(1,2);3,319(13,8);2,522(0,4);2,519(0,5);2,515(0,6);2,504 (25,5);2,501(34,5);2,498(25,1);2,456(16,0);0,000(2,5) |
| 19 | 2,83 | 2,89 | (400,0 MHz, d₆-DMSO): δ= 9,825(4,2);8,057(3,0);8,039(3,0);7,607(2,5);7,580(2,5); 7,557(4,1);7,537(4,9);7,386(2,8);7,367(4,5);7,347(2,7);7,149(1,5);7,131(2,6);7,112( 1,1);5,756(1,7);4,569(0,8);4,366(0,9);4,356(0,4);4,339(1,0);4,329(1,1);4,312(0,5);4, 302(1,1);4,275(0,4);4,163(0,3);3,913(0,8);3,902(0,4);3,886(0,9);3,875(0,8);3,859(0, 4);3,849(0,7);3,327(129,4);2,675(0,8);2,671(1,0);2,666(0,8);2,524(2,5);2,506(126,0);2,502(163,2);2,497(122,9);2,445(16,0);2,424(1,5);2,333(0,9);2,328(1,1);2,324(0,9); 2,086(5,5);0,146(0,5);0,008(4,9);0,000(112,1);-0,008(5,6);-0,149(0,5) |
| 20 | 1,98 | 1,51 | (400,0 MHz, d₆-DMSO): δ= 9,904(4,2);8,746(3,1);8,740(3,1);8,343(2,4);8,331(2,4); 8,060(2,9);8,042(3,1);8,029(1,6);8,007(1,7);7,611(2,6);7,584(2,6);7,423(1,6);7,411( 1,6);7,402(1,6);7,391(1,5);4,589(0,9);4,371(0,9);4,362(0,4);4,343(1,0);4,334(1,1);4, 316(0,5);4,306(1,1);4,279(0,4);3,902(0,6);3,876(0,7);3,868(0,7);3,840(0,5);3,326(35 ,7);2,671(0,8);2,506(103,9);2,502(133,3);2,447(16,0);2,423(1,1);2,328(0,9);2,086(1 1,0);1,140(0,6);0,146(0,4);0,000(82,7);-0,149(0,4) |
| 21 | 2,71 | 2,77 | (400,0 MHz, d₆-DMSO): δ= 8,347(1,2);8,333(2,2);8,317(1,3);8,021(2,8);8,003(2,6); 7,575(2,5);7,548(2,4);7,338(12,4);7,328(13,5);7,273(1,3);7,263(1,9);7,252(1,8);7,24 2(1,0);7,230(0,4);5,757(1,7);4,442(5,1);4,427(4,5);4,375(0,5);4,348(1,1);4,339(0,6); 4,320(1,2);4,311(1,2);4,284(1,1);4,256(0,4);3,884(0,8);3,857(1,0);3,847(0,8);3,821( 0,7);3,330(199,9);2,717(0,3);2,675(1,9);2,671(2,0);2,506(272,3);2,502(287,5);2,429 (16,0);2,333(2,1);2,328(2,2);2,267(0,3);1,234(0,4);0,146(0,7);0,000(138,7);-0,008(7,4);-0,150(0,8) |
| 22 | 3,38 | 3,48 | (400,0 MHz, d₆-DMSO): δ= 8,317(0,7);8,076(1,3);7,657(1,8);7,638(1,9);7,500(5,9); 7,289(4,5);7,021(1,1);6,441(0,4);6,423(0,4);4,505(2,7);4,461(4,3);4,351(4,4);4,307( 2,6);3,924(0,8);3,907(1,3);3,889(1,5);3,885(1,4);3,873(1,0);3,858(3,6);3,833(3,6);3, 807(1,3);3,748(0,4);3,728(1,1);3,702(1,1);3,676(0,4);3,326(209,4);2,675(1,5);2,671( 2,1);2,666(1,6);2,524(5,6);2,519(8,7);2,511(114,0);2,506(231,6);2,502(307,3);2,497 (229,0);2,493(115,7);2,450(0,5);2,389(15,1);2,345(0,3);2,333(1,7);2,329(2,3);2,324( 1,8);2,288(3,8);2,169(0,4);2,118(4,5);2,110(16,0);2,075(0,8);1,175(14,5);1,159(14,5 );1,123(0,5);1,104(5,5);1,088(5,2);1,074(0,3);0,146(1,7);0,008(13,4);0,000(380,9);-0,009(15,4);-0,150(1,8) |
| 23 | 2,45 | 2,52 | (400,0 MHz, d₆-DMSO): δ= 8,438(0,3);8,317(0,5);7,835(3,2);7,827(3,3);7,676(0,3); 7,651(2,2);7,632(2,3);7,392(5,0);5,757(0,6);4,499(1,4);4,493(1,4);4,455(2,3);4,450( 2,5);4,355(2,1);4,344(2,2);4,312(1,3);4,301(1,5);4,275(1,0);4,248(1,1);4,238(1,2);4, 211(1,1);4,185(0,4);3,921(0,7);3,904(1,1);3,892(1,1);3,875(0,8);3,842(0,3);3,832(0, 3);3,816(0,7);3,805(0,9);3,789(0,8);3,778(1,3);3,769(0,8);3,752(0,8);3,742(0,7);3,32 8(256,1);2,671(2,1);2,502(313,7);2,379(16,0);2,329(2,3);2,281(0,4);2,252(1,4);2,20 9(16,0);1,334(0,4);1,318(0,3);1,235(0,6);1,173(13,2);1,160(12,5);1,156(12,9);1,120( 1,6);1,104(1,6);1,082(0,4);0,146(1,8);0,000(342,6);-0,150(1,9) |
| 24 | 3,30 | 3,38 | (400,0 MHz, d₆-DMSO): δ= 7,905(1,0);7,891(1,9);7,877(1,0);7,739(0,6);7,464(2,2); 7,437(2,2);4,731(0,4);4,722(0,4);3,923(0,7);3,898(1,9);3,872(1,9);3,846(0,7);3,325( 25,0);3,283(0,5);3,265(1,8);3,247(2,6);3,230(1,9);3,214(0,6);2,506(32,8);2,502(42,1 );2,497(31,5);2,456(16,0);1,572(3,8);1,555(3,8);1,114(5,4);1,096(11,3);1,078(5,2);0, 007(1,4);0,000(28,0);-0,008(1,3) |
| 25 | 3,35 | 3,43 | (400,0 MHz, d₆-DMSO): = 7,831(2,3);7,824(2,3);7,720(0,6);7,459(2,2);7,432(2,2) ;5,757(0,8);4,732(0,4);3,915(0,7);3,890(2,0);3,864(2,0);3,839(0,7);3,326(26,0);2,70 9(0,7);2,701(1,1);2,692(1,6);2,683(1,6);2,674(1,3);2,666(0,9);2,506(30,7);2,502(40, 0);2,498(30,5);2,452(16,0);1,572(3,7);1,555(3,7);0,726(0,7);0,710(3,2);0,699(2,7);0, 693(2,8);0,682(1,1);0,563(1,0);0,552(3,0);0,547(3,2);0,527(0,8);0,000(26,8);-0,008(1,4) |
| 26 | 3,68 | 3,78 | (400,0 MHz, d₆-DMSO): δ= 7,740(0,6);7,664(1,6);7,645(1,7);7,463(2,1);7,436(2,1); 4,739(0,3);4,730(0,3);3,944(0,4);3,927(1,3);3,911(1,8);3,895(2,9);3,876(2,1);3,872( 2,1);3,846(0,7);3,325(46,1);2,671(0,4);2,524(1,0);2,511(24,1);2,506(47,7);2,502(62, 6);2,497(46,3);2,493(23,1);2,456(16,0);2,328(0,4);1,575(3,5);1,558(3,5);1,178(10,3) ;1,167(11,9);1,162(11,6);1,150(10,6);1,009(0,7);0,992(0,7);0,008(2,0);0,000(48,8);-0,009(1,9) |
| 27 | 2,39 | 2,42 | (400,0 MHz, d₆-DMSO): δ= 8,318(0,3);8,066(0,8);8,051(0,8);7,896(1,0);7,882(1,9); 7,868(1,0);7,575(2,5);7,548(2,5);4,725(0,4);4,711(0,4);4,353(0,6);4,343(0,5);4,325( 0,8);4,316(1,0);4,298(0,4);4,288(0,9);4,281(0,4);3,887(0,6);3,877(0,4);3,861(0,7);3, 851(0,6);3,834(0,4);3,824(0,5);3,327(104,9);3,287(0,6);3,279(0,6);3,269(1,6);3,262( 1,6);3,249(2,2);3,237(1,6);3,230(1,7);3,218(0,6);3,212(0,6);2,719(0,9);2,675(0,9);2, 671(1,2);2,666(0,9);2,524(3,4);2,511(68,9);2,506(140,0);2,502(185,5);2,497(136,9);2,493(68,9);2,433(16,0);2,337(0,4);2,333(0,9);2,328(1,2);2,324(0,9);1,581(4,1);1,56 5(4,2);1,116(6,5);1,099(14,1);1,081(6,4);0,146(0,9);0,008(7,5);0,000(203,6);-0,009(9,2);-0,150(0,9) |
| 28 | 2,42 | 2,46 | (400,0 MHz, d₆-DMSO): δ= 8,046(0,8);7,831(1,5);7,824(2,3);7,569(2,4);7,543(2,4); 5,758(3,8);4,727(0,4);4,718(0,4);4,346(0,6);4,336(0,5);4,319(0,7);4,309(1,0);4,299( 0,5);4,292(0,4);4,282(0,8);4,272(0,4);3,885(0,5);3,876(0,4);3,859(0,6);3,848(0,5);3, 833(0,4);3,821(0,4);3,335(44,8);3,330(105,2);2,714(1,1);2,705(1,0);2,697(1,4);2,68 8(1,4);2,679(1,3);2,671(1,4);2,662(0,5);2,524(1,8);2,510(42,6);2,506(86,4);2,502(11 4,5);2,497(84,0);2,493(41,5);2,428(16,0);2,333(0,6);2,329(0,8);2,324(0,5);1,582(3,9 );1,566(3,9);0,730(0,7);0,714(3,1);0,697(2,8);0,686(1,0);0,560(0,9);0,543(3,4);0,524 (0,8);0,146(0,5);0,008(3,1);0,000(104,8);-0,008(4,0);-0,150(0,5) |
| 29 | 2,73 | 2,77 | (400,0 MHz, d₆-DMSO): δ= 8,064(0,8);8,049(0,8);7,664(1,4);7,645(1,5);7,574(2,6); 7,547(2,6);5,758(1,8);4,714(0,4);4,349(0,6);4,336(0,5);4,322(0,8);4,311(0,9);4,298( 0,6);4,284(0,8);4,271(0,5);3,943(0,4);3,926(1,0);3,910(1,6);3,894(2,1);3,877(1,2);3, 867(0,8);3,858(0,9);3,842(0,4);3,831(0,5);3,335(97,8);3,333(109,6);3,329(141,7);2, 680(0,4);2,675(0,9);2,671(1,2);2,666(0,9);2,524(3,0);2,519(4,7);2,511(68,5);2,506(1 40,3);2,502(186,0);2,497(136,0);2,493(67,3);2,433(16,0);2,333(0,9);2,329(1,3);2,32 4(0,9);1,585(4,2);1,568(4,2);1,179(11,3);1,168(13,4);1,163(12,9);1,152(12,0);1,008( 0,8);0,992(0,8);0,146(0,8);0,008(5,8);0,000(179,7);-0,009(6,9);-0,150(0,8) |
| 30 | 3,21 | 3,28 | (400,0 MHz, d₆-DMSO): δ= 8,336(1,1);8,320(2,2);8,304(1,1);7,714(2,8);7,696(2,9); 7,478(2,6);7,451(2,6);5,757(3,8);4,505(0,6);4,491(0,6);4,100(1,2);4,079(1,5);4,061( 1,2);3,909(1,2);3,883(3,8);3,857(4,0);3,842(0,6);3,832(1,4);3,330(65,9);2,671(0,5);2 ,506(56,6);2,502(76,4);2,498(63,3);2,463(16,0);2,329(0,5);2,086(4,9);2,075(0,6);0,0 00(4,4) |
| 31 | 3,04 | 3,14 | (400,0 MHz, d₆-DMSO): δ= 7,894(0,9);7,880(1,8);7,866(1,0);7,500(5,7);7,291(4,6); 4,507(2,7);4,463(4,2);4,351(4,4);4,307(2,6);4,190(0,9);3,885(1,2);3,860(3,6);3,834( 3,8);3,808(1,3);3,434(0,4);3,416(0,4);3,328(123,2);3,288(0,6);3,282(0,5);3,269(1,7); 3,264(1,4);3,251(2,3);3,237(1,6);3,232(1,8);3,220(0,7);3,214(0,6);2,676(0,8);2,671( 1,1);2,667(0,9);2,524(2,8);2,510(65,2);2,506(130,5);2,502(171,3);2,497(126,9);2,38 9(15,3);2,338(0,5);2,333(0,9);2,329(1,2);2,324(0,9);2,111(16,0);1,398(7,6);1,236(0, 3);1,125(0,5);1,115(5,3);1,108(1,3);1,097(11,3);1,079(5,2);1,065(0,5);0,146(0,9);0,0 08(6,9);0,000(185,7);-0,008(8,4);-0,150(0,9) |
| 32 | 2,12 | 2,14 | (400,0 MHz, d₆-DMSO): δ= 7,881(1,0);7,867(2,0);7,853(1,1);7,833(4,6);7,394(5,0); 5,757(1,8);4,501(1,5);4,495(1,5);4,457(2,4);4,451(2,5);4,359(2,3);4,346(2,4);4,315( 1,4);4,303(1,5);4,289(0,5);4,279(0,7);4,262(0,6);4,252(1,1);4,242(0,7);4,235(0,3);4, 225(0,8);4,215(0,6);4,190(1,3);3,814(0,7);3,787(1,2);3,778(0,7);3,760(0,9);3,750(1, 0);3,723(0,7);3,434(0,4);3,416(0,5);3,329(89,8);3,292(0,4);3,274(1,1);3,265(1,5);3,2 62(1,5);3,256(1,9);3,251(1,8);3,248(2,0);3,238(1,6);3,231(1,4);3,224(0,8);3,206(0,3) ;2,675(0,5);2,671(0,7);2,666(0,5);2,524(1,6);2,506(78,4);2,502(103,4);2,498(78,6);2 ,381(14,6);2,333(0,5);2,329(0,7);2,324(0,6);2,211(16,0);1,183(0,4);1,119(3,1);1,114 (3,3);1,107(1,8);1,102(6,9);1,096(6,9);1,084(4,0);1,078(3,3);1,065(0,6);0,000(7,1) |
| 33 | 3,89 | 3,86 | (400,0 MHz, d₆-DMSO): δ= 7,754(1,3);7,500(1,9);7,285(1,5);4,493(0,9);4,449(1,4); 4,337(1,4);4,293(0,9);3,887(0,4);3,861(1,1);3,835(1,2);3,809(0,4);3,331(14,8);2,511 (4,9);2,506(9,9);2,502(13,1);2,497(9,6);2,493(4,8);2,386(4,9);2,107(5,2);2,074(0,6); 1,340(16,0);0,000(0,5) |
| 34 | 3,69 | 3,80 | (400,0 MHz, d₆-DMSO): = 7,902(0,8);7,887(1,6);7,873(0,8);7,507(4,4);7,293(3,6) ;4,515(2,0);4,471(3,1);4,358(3,2);4,314(1,9);4,239(0,6);3,887(0,9);3,861(2,8);3,835( 2,9);3,810(1,0);3,334(230,1);3,114(0,4);3,097(1,2);3,080(2,0);3,064(2,2);3,046(1,4); 3,029(0,5);2,936(0,3);2,676(0,6);2,671(0,8);2,667(0,6);2,507(93,3);2,502(119,7);2,4 98(89,3);2,451(0,4);2,390(11,7);2,333(0,7);2,329(0,9);2,325(0,7);2,289(0,5);2,131(0 ,5);2,115(12,3);1,989(0,4);1,816(0,4);1,799(0,9);1,782(1,2);1,766(1,0);1,749(0,6);1, 398(2,0);0,885(15,6);0,869(16,0);0,852(1,5);0,846(2,0);0,829(1,6);0,818(0,8);0,802( 0,7);0,008(0,8);0,000(19,0);-0,008(0,9) |
| 35 | 2,76 | 2,77 | (400,0 MHz, d₆-DMSO): δ= 7,878(1,3);7,847(2,0);7,836(1,7);7,821(0,5);7,806(0,5); 7,397(2,2);7,376(0,3);7,364(0,3);4,508(0,8);4,464(1,5);4,434(0,4);4,363(1,2);4,351(1,1);4,319(0,9);4,308(0,9);4,282(0,8);4,254(0,9);4,245(0,8);4,220(0,7);4,192(0,4);3, 819(0,6);3,813(0,5);3,792(0,7);3,757(0,6);3,730(0,4);3,388(0,5);3,328(19,8);3,305(2 ,7);3,286(3,0);3,258(2,2);3,086(1,4);3,070(1,5);3,060(1,2);3,043(1,0);2,674(1,0);2,6 52(0,7);2,506(89,1);2,502(88,1);2,468(16,0);2,382(8,4);2,335(1,8);2,314(1,1);2,216( 7,0);2,180(1,0);2,144(0,7);1,800(0,7);1,787(0,8);1,784(0,8);1,768(0,8);1,753(0,5);0, 891(7,1);0,884(7,0);0,874(7,0);0,867(5,8);0,000(1,8) |
| 36 | 2,41 | 2,46 | (400,0 MHz, d₆-DMSO): δ= 8,333(1,0);8,317(2,5);8,300(1,1);8,037(3,1);8,019(3,1); 7,586(2,6);7,559(2,5);4,481(0,7);4,388(0,4);4,361(1,0);4,351(0,5);4,333(1,1);4,323( 1,1);4,306(0,5);4,296(1,1);4,269(0,4);4,127(0,4);4,105(1,3);4,083(1,5);4,066(1,3);4, 041(0,5);3,884(0,8);3,875(0,3);3,858(0,9);3,847(0,8);3,831(0,4);3,821(0,7);3,331(10 5,1);2,675(0,4);2,671(0,6);2,667(0,4);2,524(1,4);2,511(32,5);2,506(65,6);2,502(86,5 );2,497(64,0);2,493(32,3);2,434(16,0);2,333(0,4);2,329(0,6);2,324(0,5);2,075(3,5);0, 000(6,8) |
| 37 | 4,24 | 4,32 | (400,0 MHz, d₆-DMSO): δ= 10,047(3,7);8,240(0,3);8,061(2,8);7,877(0,4);7,818(2,0) ;7,797(1,8);7,763(0,4);7,742(2,4);7,724(2,2);7,618(1,0);7,598(2,0);7,578(1,1);7,505( 2,1);7,492(2,2);7,477(2,9);7,351(0,3);4,598(0,8);4,502(1,4);3,919(0,9);3,894(2,8);3, 868(2,9);3,854(0,8);3,843(1,1);3,828(0,6);3,332(99,3);2,671(0,7);2,502(101,7);2,47 9(16,0);2,434(0,5);2,391(2,0);2,329(0,7);2,075(1,7);0,000(10,9) |
| 38 | 3,33 | 3,46 | (400,0 MHz, d₆-DMSO): δ= 8,346(1,0);8,330(2,2);8,313(1,0);7,521(6,0);7,301(4,7); 4,551(3,0);4,508(4,5);4,391(4,6);4,348(2,9);4,141(0,4);4,119(1,0);4,100(1,3);4,096( 1,2);4,083(1,1);4,078(1,3);4,059(1,0);4,036(0,4);3,881(1,2);3,856(3,9);3,830(4,0);3, 804(1,4);3,329(29,2);2,524(0,5);2,519(0,7);2,511(9,5);2,506(19,8);2,502(26,4);2,49 7(19,6);2,493(9,7);2,395(15,1);2,127(16,0);2,073(3,5);0,008(0,6);0,000(19,0);-0,009(0,7) |
| 39 | 3,75 | 3,85 | (400,0 MHz, d₆-DMSO): δ= 9,811(4,2);7,736(2,9);7,718(2,9);7,594(2,8);7,589(1,1); 7,582(3,0);7,577(1,8);7,572(3,2);7,565(1,2);7,559(3,0);7,498(2,5);7,471(2,6);7,227( 3,1);7,221(1,0);7,205(5,3);7,188(0,9);7,183(2,8);4,570(0,6);4,566(0,6);3,920(1,1);3, 894(3,7);3,868(3,8);3,842(1,3);3,329(39,5);2,676(0,4);2,671(0,6);2,667(0,4);2,524(1 ,3);2,511(33,5);2,507(67,5);2,502(88,0);2,498(63,4);2,493(30,7);2,475(16,0);2,333( 0,4);2,329(0,6);2,324(0,5);2,075(1,9);0,008(0,9);0,000(29,4);-0,009(1,1) |
| 40 | 2,83 | 2,90 | (400,0 MHz, d₆-DMSO): δ= 7,835(1,8);7,823(1,7);7,754(1,4);7,749(1,4);7,389(2,5); 4,485(0,7);4,479(0,7);4,441(1,2);4,435(1,2);4,341(1,2);4,331(1,2);4,298(0,7);4,287( 0,7);4,266(0,3);4,249(0,3);4,239(0,6);4,229(0,3);4,212(0,4);3,819(0,6);3,792(0,6);3, 782(0,5);3,755(0,5);3,329(53,1);2,671(0,5);2,506(57,1);2,502(72,7);2,498(53,9);2,3 79(8,2);2,329(0,5);2,206(8,5);2,075(1,4);1,344(15,7);1,338(16,0);1,304(0,4);0,008(0 ,5);0,000(10,4) |
| 41 | 2,86 | 2,92 | (400,0 MHz, d₆-DMSO): δ= 9,813(4,7);8,054(3,2);8,036(3,3);7,606(2,8);7,597(3,1); 7,591(1,4);7,584(3,7);7,579(4,4);7,574(3,9);7,567(1,4);7,562(3,3);7,553(0,4);7,468( 0,4);7,464(0,3);7,455(0,6);7,445(0,5);7,433(0,4);7,229(3,2);7,223(1,1);7,207(5,3);7, 190(1,0);7,184(2,8);7,166(0,4);7,144(0,6);5,758(6,2);4,561(0,7);4,368(0,9);4,358(0, 4);4,341(1,1);4,331(1,1);4,314(0,4);4,304(1,1);4,276(0,3);3,904(0,6);3,878(0,7);3,86 8(0,6);3,842(0,5);3,344(0,6);3,329(63,1);2,676(0,5);2,671(0,7);2,667(0,6);2,524(2,0) ;2,520(3,0);2,511(40,1);2,507(82,9);2,502(110,2);2,498(81,4);2,493(40,6);2,473(0,5 );2,444(16,0);2,333(0,6);2,329(0,8);2,324(0,6);0,014(0,3);0,008(1,5);0,000(47,3);-0,009(1,9);-0,013(0,4) |
| 42 | 3,94 | 3,90 | (400,0 MHz, d₆-DMSO): δ= 9,951(3,8);8,975(0,4);8,318(1,4);7,736(3,0);7,718(3,0); 7,628(0,3);7,610(0,3);7,573(1,1);7,568(1,7);7,562(1,1);7,544(1,1);7,539(1,7);7,534( 1,1);7,502(2,6);7,474(2,6);7,463(0,3);7,423(0,7);7,403(2,1);7,387(1,9);7,383(1,7);7, 374(0,5);7,367(1,5);7,354(2,1);7,351(2,8);7,347(2,1);7,334(1,0);7,329(1,2);7,326(0, 9);7,319(0,4);7,301(0,3);6,997(0,4);6,987(0,8);6,985(0,8);6,979(1,1);6,965(1,3);6,95 9(1,5);6,946(0,7);6,943(0,8);6,939(0,7);6,936(0,6);5,758(1,3);5,372(0,4);4,582(0,6); 4,487(0,7);3,919(1,3);3,893(3,9);3,868(4,0);3,850(0,4);3,842(1,4);3,332(49,8);3,308 (0,6);2,671(0,4);2,525(1,0);2,511(23,0);2,507(46,6);2,502(60,9);2,498(44,4);2,494(2 1,9);2,477(16,0);2,433(1,8);2,392(0,9);2,329(0,4);2,075(10,9);0,008(0,6);0,000(19,1 );-0,009(0,7) |
| 43 | 3,41 | 3,45 | (400,0 MHz, d₆-DMSO): δ= 10,229(0,4);10,223(0,4);10,051(4,3);8,594(0,5);8,574(0 ,5);8,472(0,8);8,317(1,1);8,061(5,6);8,042(3,2);7,903(1,2);7,884(1,5);7,824(1,9);7,8 02(1,7);7,775(0,7);7,752(0,5);7,732(0,3);7,716(0,3);7,620(1,4);7,614(2,7);7,601(2,5) ;7,587(2,6);7,520(0,8);7,494(2,8);7,474(1,5);7,451(0,4);7,421(0,4);7,196(0,6);7,176( 0,3);6,829(0,8);6,798(0,4);6,778(0,4);6,764(0,4);6,744(0,4);5,552(0,9);5,453(0,4);4, 590(0,7);4,518(1,8);4,505(0,5);4,373(0,8);4,363(0,4);4,346(1,0);4,336(1,1);4,319(0, 4);4,309(1,2);4,280(0,6);4,270(0,4);4,243(0,4);4,177(0,4);4,164(1,5);4,078(0,8);4,07 6(0,8);3,966(0,4);3,954(0,4);3,939(0,5);3,928(0,5);3,902(0,8);3,874(0,7);3,849(0,4); 3,839(0,4);3,330(177,4);2,680(0,5);2,676(1,1);2,671(1,5);2,667(1,1);2,662(0,5);2,52 5(3,6);2,520(5,7);2,511(84,5);2,507(174,6);2,502(231,0);2,498(167,9);2,493(81,3);2 ,447(16,0);2,424(5,6);2,350(2,7);2,338(0,7);2,334(1,3);2,329(1,8);2,324(1,6);2,188( 1,3);2,075(1,2);0,008(2,1);0,000(73,0);-0,009(2,7) |
| 44 | 3,58 | 3,64 | (400,0 MHz, d₆-DMSO): δ= 7,705(3,0);7,687(3,0);7,597(1,9);7,577(1,9);7,462(2,6); 7,435(2,5);5,755(1,5);5,465(0,4);5,445(0,4);4,470(0,5);4,465(0,5);3,909(1,2);3,883( 3,9);3,857(4,1);3,832(1,4);3,765(0,4);3,749(1,0);3,732(1,1);3,729(1,2);3,712(1,0);3, 696(0,5);3,471(0,3);3,329(107,8);2,671(0,4);2,525(1,1);2,520(1,7);2,511(23,7);2,50 7(49,0);2,502(66,1);2,498(49,6);2,493(24,6);2,458(16,0);2,333(0,3);2,329(0,5);2,32 4(0,3);2,086(1,6);1,536(0,6);1,518(2,3);1,500(3,5);1,482(2,8);1,464(0,9);1,342(1,0); 1,324(1,3);1,307(1,1);1,289(0,4);1,228(1,5);1,212(1,5);1,147(10,3);1,130(10,2);1,05 9(0,4);1,042(0,4);1,017(0,4);1,001(0,4);0,990(0,6);0,980(4,3);0,964(4,3);0,924(0,7); 0,906(1,5);0,890(5,2);0,872(11,0);0,853(4,6);0,829(2,1);0,810(3,6);0,792(1,6);0,000 (8,0) |
| 45 | 4,16 | 4,23 | (400,0 MHz, d₆-DMSO): δ= 9,926(4,2);8,317(0,8);7,773(2,2);7,768(4,2);7,763(2,4); 7,736(3,0);7,718(3,0);7,702(0,3);7,501(2,6);7,490(1,5);7,488(1,4);7,486(1,4);7,473( 3,8);7,467(2,3);7,403(2,4);7,383(3,9);7,363(1,9);7,289(0,3);7,203(1,7);7,201(1,9);7, 198(1,8);7,196(1,7);7,183(1,4);7,181(1,5);7,178(1,6);7,176(1,4);4,580(0,6);3,918(1, 2);3,893(3,7);3,867(3,9);3,841(1,3);3,331(40,0);3,307(0,4);2,671(0,3);2,525(0,9);2,5 11(20,1);2,507(40,7);2,502(53,4);2,498(39,0);2,494(19,1);2,477(16,0);2,433(1,5);2, 329(0,4);2,075(9,7);0,000(6,6) |
| 46 | 2,97 | 3,02 | (400,0 MHz, d₆-DMSO): δ= 9,951(4,1);8,974(0,4);8,467(0,4);8,315(1,6);8,054(3,1); 8,036(3,2);7,902(0,5);7,884(0,5);7,607(2,6);7,580(2,6);7,572(1,3);7,567(1,9);7,561( 1,3);7,544(1,2);7,538(1,9);7,533(1,2);7,518(0,4);7,491(0,7);7,461(0,4);7,424(0,7);7, 404(1,9);7,387(2,0);7,384(1,8);7,368(1,6);7,351(2,7);7,339(0,5);7,334(1,0);7,330(1, 3);7,318(0,6);7,301(0,5);7,138(0,3);7,135(0,3);6,996(0,6);6,984(0,9);6,978(1,3);6,96 4(1,5);6,959(1,7);6,945(0,7);6,942(0,8);6,939(0,7);6,936(0,7);6,363(0,4);6,360(0,4); 6,358(0,4);6,342(0,3);6,339(0,4);6,337(0,4);6,306(0,4);6,276(0,4);6,227(0,4);5,756( 2,1);5,366(0,7);4,576(0,8);4,500(0,8);4,367(0,9);4,357(0,4);4,340(1,1);4,330(1,2);4, 312(0,5);4,303(1,2);4,277(0,5);4,163(0,8);3,927(0,3);3,901(0,7);3,875(0,7);3,866(0, 6);3,839(0,5);3,506(0,4);3,328(181,6);2,736(0,7);2,676(0,8);2,671(1,1);2,667(0,9);2, 524(2,7);2,520(4,1);2,511(55,3);2,507(114,3);2,502(155,2);2,497(119,3);2,493(62,3 );2,445(16,0);2,424(2,8);2,349(0,8);2,333(0,8);2,329(1,1);2,324(0,8);2,086(14,3);1,2 34(0,6);0,008(0,6);0,000(17,8);-0,008(0,8) |
| 47 | 2,65 | 2,70 | (400,0 MHz, d₆-DMSO): δ= 8,018(2,5);8,000(2,6);7,595(2,0);7,573(4,2);7,545(2,6); 5,756(0,7);5,465(0,6);5,445(0,6);5,004(0,3);4,979(0,3);4,450(0,9);4,371(0,4);4,343( 0,9);4,334(0,5);4,315(1,0);4,306(1,0);4,287(0,5);4,279(1,0);4,252(0,3);3,901(0,7);3, 893(0,6);3,874(0,7);3,865(0,9);3,857(0,5);3,848(0,3);3,837(0,7);3,831(0,5);3,768(0, 5);3,751(1,1);3,733(1,4);3,716(1,2);3,699(0,5);3,486(0,4);3,471(0,5);3,455(0,4);3,32 8(108,1);2,720(1,5);2,671(0,6);2,506(71,2);2,502(91,7);2,498(70,0);2,431(16,0);2,3 33(0,5);2,329(0,6);2,324(0,5);1,537(0,7);1,518(2,6);1,500(4,0);1,483(2,9);1,465(0,9) ;1,360(0,4);1,342(1,2);1,324(1,8);1,307(1,4);1,288(0,5);1,148(9,8);1,132(9,7);1,058( 0,4);1,042(0,4);0,980(4,8);0,963(4,8);0,893(5,3);0,874(10,8);0,856(4,8);0,828(2,7);0 ,810(4,6);0,792(2,1);0,146(0,6);0,008(5,8);0,000(119,1);-0,150(0,6) |
| 48 | 3,25 | 3,31 | (400,0 MHz, d₆-DMSO): δ= 9,927(4,5);8,316(0,4);8,054(3,2);8,036(3,2);7,772(2,4); 7,767(4,4);7,762(2,4);7,608(2,5);7,581(2,5);7,493(1,4);7,490(1,3);7,488(1,2);7,474( 1,9);7,472(2,0);7,470(2,0);7,404(2,4);7,384(4,1);7,364(2,0);7,203(1,8);7,201(2,0);7, 198(1,9);7,196(1,7);7,183(1,5);7,181(1,6);7,178(1,6);7,176(1,4);5,756(2,3);4,572(0,7);4,369(0,9);4,359(0,4);4,342(1,0);4,332(1,1);4,315(0,4);4,305(1,1);4,278(0,3);3,90 1(0,5);3,874(0,6);3,865(0,5);3,838(0,5);3,326(59,8);2,737(1,0);2,676(0,6);2,671(0,8) ;2,666(0,6);2,524(2,0);2,520(2,9);2,511(42,5);2,506(88,1);2,502(117,6);2,497(85,8); 2,493(41,6);2,445(16,0);2,333(0,6);2,329(0,8);2,324(0,6);0,146(0,9);0,008(6,7);0,00 0(200,4);-0,009(7,5);-0,025(0,3);-0,063(0,3);-0,150(0,9) |
| 49 | 2,81 | 1,36 | (400,0 MHz, d₆-DMSO): δ= 7,827(2,2);7,820(2,3);7,482(5,9);7,285(4,7);4,510(2,8); 4,466(4,4);4,358(4,5);4,314(2,7);3,876(1,2);3,850(3,6);3,824(3,7);3,799(1,3);3,327( 23,0);2,699(0,6);2,691(1,1);2,681(1,5);2,673(1,6);2,664(1,2);2,655(0,7);2,524(0,7);2 ,510(14,1);2,506(28,5);2,502(37,9);2,497(29,0);2,385(15,3);2,099(16,0);2,074(1,3); 0,731(0,6);0,727(0,7);0,718(2,1);0,714(2,9);0,710(2,1);0,702(2,6);0,697(2,6);0,685( 0,8);0,552(1,4);0,544(3,3);0,535(2,9);0,528(1,9);0,522(1,0);0,008(1,0);0,000(26,3);-0,008(1,4) |
| 50 | 3,19 | 3,29 | (400,0 MHz, d₆-DMSO): δ= 7,794(2,3);7,786(2,3);7,716(6,6);7,637(5,5);4,625(3,1); 4,580(4,5);4,444(4,7);4,399(3,0);3,989(1,2);3,963(3,7);3,938(3,9);3,912(1,4);3,329( 52,5);2,702(0,6);2,693(1,1);2,684(1,5);2,676(1,7);2,667(1,3);2,658(0,7);2,525(0,6);2 ,520(1,0);2,511(14,2);2,507(29,1);2,502(38,7);2,498(28,6);2,493(14,3);2,412(16,0); 2,075(2,4);0,730(0,5);0,723(0,6);0,715(2,0);0,712(2,9);0,708(1,8);0,699(2,6);0,694( 2,5);0,681(0,7);0,567(1,4);0,557(3,1);0,549(2,8);0,541(1,7);0,535(1,0);0,008(1,0);0, 000(29,5);-0,008(1,1) |
| 51 | 3,13 | 3,19 | (400,0 MHz, d₆-DMSO): δ= 8,317(0,3);7,878(0,9);7,863(1,9);7,849(1,0);7,734(6,5); 7,641(5,5);4,618(3,0);4,573(4,5);4,435(4,6);4,390(2,9);3,996(1,2);3,971(3,7);3,945( 3,9);3,920(1,4);3,326(34,9);3,288(0,6);3,281(0,5);3,270(1,5);3,263(1,4);3,252(1,8);3 ,249(1,9);3,238(1,4);3,231(1,6);3,220(0,5);3,213(0,6);2,675(0,5);2,671(0,7);2,666(0, 6);2,524(1,7);2,519(2,6);2,511(39,3);2,506(81,8);2,502(110,1);2,497(82,6);2,493(41 ,9);2,415(16,0);2,333(0,7);2,329(0,8);2,324(0,6);2,074(4,5);1,114(5,4);1,096(11,8);1 ,078(5,3);0,146(0,7);0,008(5,3);0,000(158,0);-0,009(6,4);-0,150(0,7) |
| 52 | 3,78 | 3,93 | (400,0 MHz, d₆-DMSO): δ= 7,869(0,7);7,855(1,5);7,840(0,7);7,739(4,8);7,643(4,0); 4,629(2,3);4,584(3,3);4,444(3,4);4,399(2,2);3,999(0,9);3,973(2,7);3,948(2,9);3,922( 1,0);3,328(33,5);3,121(0,5);3,104(1,2);3,087(1,5);3,072(1,4);3,059(1,3);3,042(1,2);3 ,025(0,5);2,524(0,6);2,519(1,0);2,511(13,0);2,507(27,0);2,502(36,2);2,497(27,1);2,4 93(13,7);2,417(11,7);2,075(1,2);1,821(0,4);1,804(0,9);1,787(1,2);1,770(0,9);1,754(0 ,5);0,882(16,0);0,865(15,4);0,846(1,5);0,830(1,4);0,819(0,6);0,802(0,6);0,008(0,8);0 ,000(26,5);-0,009(1,1) |
| 53 | 3,40 | 3,51 | (400,0 MHz, d₆-DMSO): δ= 8,336(1,0);8,320(2,2);8,304(1,1);7,749(6,5);7,655(5,6); 4,687(3,0);4,643(4,2);4,479(4,4);4,434(2,9);4,148(0,5);4,141(0,4);4,126(0,9);4,110( 0,8);4,103(1,1);4,086(1,0);4,080(1,0);4,063(1,0);4,056(0,8);4,040(1,0);4,032(0,4);4, 026(0,4);4,017(0,6);3,994(1,3);3,969(3,7);3,943(3,9);3,918(1,4);3,327(23,5);2,671(0 ,3);2,524(1,0);2,506(36,5);2,502(48,4);2,497(37,0);2,422(16,0);2,333(0,4);2,329(0,4 );2,075(0,8);0,008(1,2);0,000(31,0);-0,008(1,6) |
| 54 | 3,71 | 3,84 | (400,0 MHz, d₆-DMSO): δ= 7,697(2,1);7,678(0,9);7,459(0,8);7,432(0,8);3,909(0,4); 3,884(1,2);3,858(1,2);3,832(0,4);3,330(37,9);2,506(29,3);2,502(38,3);2,498(30,9);2, 455(5,1);2,336(0,8);1,339(16,0);1,283(2,5);0,000(11,9) |
| 55 | 4,35 | 4,43 | (400,0 MHz, d₆-DMSO): δ= 10,107(4,3);8,069(3,4);7,811(1,7);7,790(2,0);7,617(1,2) ;7,597(2,5);7,577(1,4);7,547(5,8);7,488(2,2);7,468(1,6);7,329(4,9);4,638(2,3);4,594( 3,8);4,499(4,1);4,455(2,2);3,892(1,1);3,866(3,4);3,840(3,5);3,815(1,2);3,329(32,0);2 ,671(0,5);2,502(68,3);2,412(15,3);2,344(0,3);2,329(0,5);2,161(16,0);0,000(6,0) |
| 56 | 4,19 | 4,32 | (400,0 MHz, d₆-DMSO): δ= 9,992(4,3);7,782(2,2);7,777(4,2);7,772(2,4);7,540(6,0); 7,479(1,3);7,476(1,2);7,474(1,2);7,460(1,8);7,458(2,0);7,456(2,1);7,453(1,8);7,401( 2,4);7,381(3,9);7,361(1,9);7,324(4,7);7,198(1,6);7,195(1,9);7,193(1,8);7,190(1,7);7, 178(1,4);7,176(1,5);7,173(1,5);7,171(1,4);4,622(2,7);4,579(4,3);4,484(4,5);4,440(2, 6);3,890(1,1);3,864(3,4);3,838(3,4);3,813(1,2);3,330(33,7);2,524(0,6);2,511(14,7);2, 506(30,6);2,502(40,8);2,497(30,1);2,493(15,1);2,409(15,1);2,155(16,0);0,000(5,8) |
| 57 | 3,61 | 3,81 | (400,0 MHz, d₆-DMSO): δ= 8,358(1,0);8,343(2,2);8,328(1,1);8,317(0,4);7,734(6,6); 7,643(5,6);7,340(13,3);7,329(15,0);7,310(0,5);7,308(0,5);7,284(0,4);7,275(1,1);7,26 4(1,8);7,254(1,8);7,243(1,1);7,232(0,4);4,645(3,0);4,600(4,3);4,485(0,6);4,470(0,7);4,454(4,6);4,448(2,6);4,432(2,3);4,425(2,3);4,410(5,2);4,387(0,7);4,372(0,6);3,991( 1,2);3,965(3,7);3,940(3,9);3,914(1,3);3,329(75,1);2,675(0,4);2,671(0,6);2,666(0,4);2 ,524(1,5);2,506(68,5);2,502(90,4);2,497(66,7);2,414(16,0);2,333(0,5);2,329(0,6);2,3 24(0,5);2,075(0,4);0,008(0,4);0,000(11,5);-0,009(0,5) |
| 58 | 4,38 | 4,51 | (400,0 MHz, d₆-DMSO): δ= 10,045(4,4);8,400(0,4);8,064(3,1);7,822(1,6);7,801(1,7) ;7,771(6,6);7,681(5,7);7,619(1,2);7,599(2,4);7,579(1,4);7,494(2,1);7,474(1,5);4,763( 2,7);4,719(4,0);4,587(4,2);4,542(2,7);4,516(0,6);4,003(1,2);3,978(3,6);3,952(3,8);3, 927(1,4);3,329(47,8);2,676(0,4);2,672(0,5);2,667(0,4);2,525(1,3);2,507(58,6);2,502( 77,5);2,498(57,2);2,438(16,0);2,393(0,4);2,356(0,9);2,333(0,4);2,329(0,5);2,325(0,4 );2,075(1,3);0,000(9,6);-0,008(0,4) |
| 59 | 4,28 | 4,46 | (400,0 MHz, d₆-DMSO): δ= 9,923(4,6);7,769(4,8);7,764(8,6);7,677(5,8);7,491(1,6); 7,489(1,5);7,470(2,3);7,468(2,3);7,403(2,1);7,383(3,6);7,363(1,8);7,202(2,1);7,199( 2,1);7,182(1,7);7,180(1,7);4,751(2,7);4,706(4,0);4,572(4,1);4,528(2,6);4,002(1,2);3, 976(3,7);3,951(3,9);3,925(1,4);3,330(17,4);2,506(30,8);2,502(38,6);2,499(29,1);2,4 36(16,0);2,075(2,0);0,000(4,3) |
| 60 | 2,17 | 2,24 | (400,0 MHz, d₆-DMSO): δ= 7,814(8,9);7,388(4,9);5,756(11,8);4,506(1,5);4,499(1,4) ;4,463(2,4);4,455(2,4);4,363(2,3);4,352(2,4);4,319(1,3);4,309(1,5);4,278(0,6);4,274( 0,7);4,251(0,7);4,246(0,8);4,242(0,9);4,237(0,8);4,224(0,3);4,214(0,8);4,210(0,8);3, 811(0,6);3,784(0,7);3,778(0,8);3,775(0,8);3,751(0,8);3,748(0,8);3,742(0,6);3,715(0, 6);3,327(25,0);2,707(0,4);2,699(0,9);2,690(1,3);2,681(1,6);2,672(1,6);2,664(1,1);2,6 55(0,5);2,506(32,4);2,501(42,5);2,497(32,9);2,375(14,8);2,328(0,3);2,199(16,0);0,7 38(0,4);0,722(2,0);0,719(1,8);0,711(3,1);0,705(2,4);0,701(1,9);0,693(1,9);0,682(0,5) ;0,550(1,5);0,541(3,3);0,532(3,2);0,525(2,2);0,000(7,1) |
| 61 | 2,47 | 2,54 | (400,0 MHz, d₆-DMSO): δ= 8,337(1,1);8,321(2,3);8,304(1,2);7,867(3,4);7,859(3,4); 7,402(5,1);5,756(8,6);4,544(1,5);4,539(1,5);4,501(2,3);4,495(2,4);4,391(2,1);4,375( 2,1);4,347(1,2);4,331(1,4);4,298(0,5);4,286(0,7);4,271(0,7);4,260(1,0);4,248(0,7);4, 234(0,7);4,222(0,6);4,123(0,9);4,106(1,5);4,084(1,7);4,066(1,2);4,041(0,4);3,808(0, 7);3,781(1,1);3,771(0,8);3,754(0,9);3,744(1,0);3,717(0,7);3,328(31,1);2,670(0,4);2,5 02(44,7);2,384(15,3);2,328(0,3);2,226(16,0);0,000(5,4) |
| 62 | 2,29 | 2,38 | (400,0 MHz, d₆-DMSO): δ= 8,035(7,3);7,774(7,7);5,757(7,5);4,610(1,7);4,606(1,6); 4,566(2,5);4,561(2,5);4,420(2,2);4,407(2,4);4,375(2,0);4,363(1,9);4,348(0,7);4,340( 1,1);4,331(0,8);4,321(0,3);4,312(0,8);4,304(0,7);3,915(0,7);3,904(0,4);3,888(0,7);3, 877(1,1);3,861(0,4);3,851(1,1);3,840(0,6);3,824(0,4);3,814(0,5);3,328(31,6);2,709(0 ,5);2,701(0,8);2,692(1,3);2,682(1,5);2,673(1,5);2,665(1,1);2,656(0,5);2,506(34,8);2, 502(44,4);2,498(35,0);2,416(16,0);1,214(0,4);1,183(0,3);1,175(0,4);0,734(0,4);0,72 1(2,1);0,708(3,3);0,696(2,2);0,691(2,0);0,679(0,5);0,551(3,7);0,544(3,6);0,000(6,7) |
| 63 | 2,58 | 2,66 | (400,0 MHz, d₆-DMSO): δ= 8,318(1,2);8,303(2,3);8,286(1,2);8,084(3,9);8,076(3,8); 7,790(5,6);5,756(9,8);4,672(1,8);4,666(1,7);4,628(2,4);4,622(2,4);4,449(2,1);4,429( 2,1);4,405(1,6);4,393(0,7);4,385(1,9);4,366(0,8);4,355(1,0);4,343(0,8);4,329(0,8);4, 316(0,7);4,153(0,5);4,135(0,8);4,115(1,1);4,093(1,4);4,070(1,3);4,054(0,9);4,047(0, 9);4,032(0,5);4,023(0,4);3,917(0,8);3,891(1,2);3,880(0,8);3,864(0,9);3,853(1,1);3,82 7(0,7);3,327(17,7);2,506(35,4);2,502(44,3);2,497(34,6);2,425(16,0);2,329(0,5);1,21 4(0,3);0,000(6,6) |
| 64 | 2,24 | 2,23 | (400,0 MHz, d₆-DMSO): δ= 8,317(0,4);8,054(5,4);8,053(5,4);7,858(1,1);7,844(2,3); 7,830(1,1);7,779(5,9);4,602(1,9);4,597(1,8);4,557(2,6);4,553(2,7);4,414(2,5);4,399( 2,5);4,385(0,6);4,369(1,8);4,355(1,9);4,347(1,2);4,336(0,7);4,321(0,8);4,309(0,7);3, 915(0,7);3,888(1,2);3,878(0,7);3,861(0,9);3,851(1,0);3,824(0,7);3,331(266,7);3,293( 0,5);3,275(1,1);3,265(1,6);3,261(1,7);3,255(1,6);3,247(2,1);3,236(1,3);3,232(1,3);3, 229(1,4);2,676(0,8);2,671(1,2);2,667(0,9);2,524(3,0);2,507(133,5);2,502(176,0);2,4 98(131,1);2,421(16,0);2,333(0,9);2,329(1,2);2,324(0,9);1,119(3,1);1,112(3,4);1,101( 6,7);1,094(7,0);1,083(3,2);1,076(3,2);0,146(1,4);0,008(10,6);0,000(291,1);-0,009(13,3);-0,150(1,4) |
| 65 | 2,88 | 2,95 | (400,0 MHz, d₆-DMSO): δ= 8,317(0,5);8,066(4,1);8,056(4,0);7,850(0,8);7,841(1,4); 7,836(1,5);7,826(0,8);7,782(5,6);4,611(1,8);4,607(1,7);4,567(2,5);4,563(2,6);4,419( 2,2);4,405(2,4);4,375(2,3);4,360(1,7);4,348(1,1);4,339(1,1);4,321(0,4);4,311(1,1);4,284(0,4);3,920(0,6);3,912(0,7);3,893(0,7);3,884(1,0);3,875(0,6);3,857(0,7);3,848(0, 6);3,329(229,6);3,129(0,4);3,121(0,5);3,113(0,9);3,105(1,0);3,096(1,1);3,088(1,2);3, 080(1,2);3,072(0,8);3,065(1,3);3,048(1,3);3,035(0,9);3,017(0,5);2,676(1,1);2,671(1, 6);2,667(1,2);2,524(4,3);2,511(89,8);2,507(181,1);2,502(237,5);2,498(175,1);2,494( 88,4);2,422(16,0);2,333(1,1);2,329(1,6);2,324(1,2);1,818(0,4);1,804(0,8);1,801(0,8); 1,788(1,0);1,784(1,0);1,771(0,8);1,767(0,8);1,754(0,5);0,888(11,5);0,880(12,5);0,87 1(11,5);0,864(11,7);0,146(1,8);0,008(14,1);0,000(373,3);-0,008(16,8);-0,150(1,8) |
| 66 | 2,76 | 2,77 | (400,0 MHz, d₆-DMSO): δ= 7,926(0,9);7,913(1,8);7,901(0,9);7,701(2,9);7,683(2,9); 7,467(2,5);7,440(2,5);5,756(0,6);4,470(0,5);3,912(1,2);3,886(3,8);3,860(4,0);3,834( 1,4);3,445(0,5);3,439(1,0);3,427(3,7);3,417(6,6);3,405(3,1);3,393(2,0);3,329(58,9);3 ,287(0,4);3,269(0,5);3,256(27,7);3,236(1,1);3,231(0,4);2,675(0,4);2,671(0,5);2,667( 0,4);2,524(1,5);2,511(31,0);2,507(63,3);2,502(83,7);2,498(62,0);2,459(16,0);2,333( 0,4);2,329(0,6);2,324(0,4);0,146(0,6);0,016(0,3);0,015(0,3);0,008(4,4);0,000(124,7); -0,009(5,5);-0,150(0,6) |
| 67 | 2,98 | 2,99 | (400,0 MHz, d₆-DMSO): δ= 7,923(1,0);7,909(2,1);7,895(1,1);7,701(2,9);7,683(3,0); 7,467(2,7);7,440(2,7);5,756(2,0);4,470(0,5);3,964(0,4);3,948(1,2);3,937(1,3);3,931( 1,4);3,920(1,4);3,913(1,8);3,903(0,7);3,887(4,0);3,861(4,2);3,835(1,5);3,779(0,9);3, 762(1,7);3,759(1,7);3,742(2,2);3,726(1,2);3,649(1,0);3,631(2,1);3,612(1,7);3,595(0, 8);3,408(0,5);3,393(0,8);3,382(0,6);3,374(0,9);3,362(1,1);3,348(0,9);3,329(12,4);3,2 58(0,8);3,243(1,2);3,226(1,1);3,208(0,8);3,193(0,6);2,506(18,6);2,502(24,7);2,498(1 8,8);2,459(16,0);2,436(0,4);1,930(0,6);1,918(0,8);1,912(0,8);1,907(0,7);1,898(1,1);1 ,886(1,0);1,869(1,0);1,856(0,7);1,840(1,3);1,830(1,8);1,820(1,8);1,813(2,0);1,796(1, 3);1,776(0,7);1,576(0,5);1,559(1,0);1,543(0,7);1,537(0,9);1,529(1,0);1,521(0,6);1,50 9(0,9);1,492(0,4);0,008(1,0);0,000(25,8);-0,008(1,5) |
| 68 | 2,75 | 2,84 | (400,0 MHz, d₆-DMSO): δ= 8,008(0,9);7,990(0,9);7,699(1,3);7,569(0,8);7,543(0,8); 4,312(0,3);4,302(0,4);4,275(0,4);3,901(0,3);3,874(0,4);3,329(49,9);2,506(34,0);2,50 2(45,1);2,498(34,1);2,454(0,3);2,429(4,8);2,276(0,7);1,341(16,0);1,296(2,4);0,000(5 ,1) |
| 69 | 3,33 | 3,37 | (400,0 MHz, d₆-DMSO): δ= 9,994(2,5);9,984(2,8);7,878(6,9);7,780(1,4);7,775(3,3); 7,770(3,3);7,765(1,3);7,483(0,9);7,478(1,1);7,470(0,8);7,463(1,3);7,457(1,8);7,424( 5,1);7,408(1,3);7,397(1,3);7,388(2,0);7,377(2,2);7,367(1,1);7,357(1,1);7,199(1,1);7, 193(1,5);7,188(1,2);7,182(1,0);7,179(1,0);7,174(1,2);7,168(0,9);5,757(1,2);4,624(1, 6);4,616(1,4);4,581(2,4);4,572(2,3);4,482(2,3);4,472(2,5);4,439(1,3);4,428(1,5);4,31 0(0,5);4,297(0,6);4,284(0,7);4,273(0,8);4,270(0,8);4,259(0,8);4,246(0,7);4,232(0,7); 3,836(0,6);3,810(0,7);3,799(0,6);3,790(0,6);3,782(0,4);3,773(0,6);3,763(0,7);3,753( 0,6);3,726(0,5);3,329(21,8);2,506(36,4);2,502(46,8);2,497(34,4);2,393(14,2);2,376( 0,6);2,328(0,3);2,253(16,0);1,215(0,4);1,188(0,4);1,183(0,5);1,174(0,4);0,008(1,8);0 ,000(46,6);-0,008(2,1) |
| 70 | 2,91 | 2,95 | (400,0 MHz, d₆-DMSO): δ= 8,342(0,7);8,328(1,6);8,316(1,6);8,301(0,7);8,064(3,9); 8,055(3,8);7,780(5,3);7,342(7,0);7,336(9,5);7,332(11,1);7,325(9,1);7,291(0,5);7,279 (0,9);7,269(1,6);7,258(2,3);7,248(1,5);7,239(1,0);7,227(0,3);7,219(0,4);5,757(3,5);4 ,629(2,0);4,584(3,0);4,498(0,4);4,483(0,5);4,470(0,5);4,460(1,3);4,447(2,0);4,434(4, 1);4,417(3,8);4,390(1,9);4,373(2,4);4,349(1,1);4,339(1,1);4,321(0,7);4,311(1,2);4,28 5(0,4);4,238(0,3);3,916(0,8);3,889(1,2);3,879(0,8);3,863(0,9);3,852(1,0);3,826(0,7); 3,329(42,0);2,675(0,4);2,671(0,6);2,666(0,4);2,506(68,2);2,502(85,0);2,497(64,2);2, 419(16,0);2,329(0,6);2,324(0,5);2,320(0,4);0,146(0,4);0,000(72,7);-0,150(0,3) |
| 71 | 3,64 | 3,68 | (400,0 MHz, d₆-DMSO): δ= 10,049(2,7);10,039(2,9);8,772(0,4);8,100(6,4);8,064(2, 6);7,815(7,9);7,798(2,1);7,636(0,4);7,625(0,7);7,614(0,8);7,605(1,5);7,594(1,6);7,58 5(0,9);7,574(0,9);7,491(1,5);7,474(1,1);5,757(1,5);4,754(1,9);4,747(1,7);4,710(2,6); 4,703(2,4);4,563(2,3);4,552(2,5);4,530(0,7);4,519(1,6);4,508(1,7);4,410(0,5);4,392( 0,6);4,382(0,8);4,372(0,7);4,365(0,7);4,355(0,9);4,345(0,7);4,338(0,4);4,327(0,7);3, 946(0,7);3,919(0,8);3,909(0,8);3,892(0,4);3,882(1,1);3,856(0,8);3,846(0,6);3,820(0, 6);3,332(21,9);2,525(0,6);2,511(12,2);2,507(24,5);2,503(31,9);2,498(23,7);2,494(12 ,0);2,438(16,0);2,416(0,8);2,333(1,1);0,008(1,3);0,000(37,8);-0,008(1,7) |
| 72 | 3,50 | 3,55 | (400,0 MHz, d₆-DMSO): δ= 9,925(2,6);9,914(2,8);8,091(6,8);7,812(6,0);7,765(3,6); 7,487(1,4);7,467(2,1);7,410(1,2);7,399(1,3);7,390(2,1);7,379(2,3);7,369(1,0);7,359( 1,1);7,206(1,1);7,200(1,6);7,195(1,3);7,189(1,0);7,187(1,0);7,181(1,3);7,175(1,0);5, 757(1,7);4,741(1,8);4,734(1,6);4,696(2,4);4,690(2,3);4,545(2,2);4,532(2,4);4,500(1, 5);4,488(1,6);4,405(0,5);4,388(0,6);4,378(0,8);4,368(0,7);4,362(0,7);4,351(0,9);4,34 1(0,7);4,324(0,7);3,944(0,6);3,917(0,8);3,907(0,6);3,889(0,8);3,880(0,7);3,862(0,7); 3,852(0,7);3,825(0,5);3,329(66,7);2,675(0,5);2,671(0,7);2,667(0,6);2,506(80,3);2,50 2(104,7);2,498(78,6);2,434(16,0);2,329(0,9);1,215(0,5);1,188(0,4);1,183(0,6);1,174( 0,5);0,146(0,5);0,008(3,8);0,000(99,2);-0,008(4,9);-0,150(0,5) |
| 73 | 3,70 | 3,72 | (400,0 MHz, d₆-DMSO): δ= 8,317(1,0);8,067(0,8);8,053(1,7);8,039(0,9);7,841(2,8); 7,823(2,8);7,469(2,4);7,442(2,4);3,949(1,1);3,924(3,6);3,898(3,7);3,872(1,3);3,328( 342,7);3,257(1,4);3,241(1,9);3,224(1,4);2,675(2,0);2,671(2,8);2,667(2,1);2,524(6,9); 2,511(156,5);2,506(322,1);2,502(427,9);2,498(318,4);2,455(16,0);2,333(2,1);2,329( 2,9);2,324(2,2);1,698(6,9);1,351(0,4);1,297(0,3);1,235(0,8);1,148(0,4);1,110(5,1);1, 092(10,9);1,074(5,0);0,827(0,5);0,146(3,3);0,008(26,0);0,000(717,9);-0,009(30,0);-0,031(0,6);-0,150(3,3) |
| 74 | 3,72 | 3,76 | (400,0 MHz, d₆-DMSO): δ= 8,005(2,4);7,998(2,5);7,820(2,9);7,802(2,9);7,464(2,6); 7,437(2,6);5,757(1,5);3,941(1,2);3,915(3,7);3,889(3,8);3,864(1,3);3,330(61,4);2,708 (0,6);2,700(1,1);2,691(1,5);2,682(1,6);2,673(1,4);2,665(0,9);2,506(44,6);2,502(56,1) ;2,452(16,0);2,329(0,4);1,701(8,5);0,709(3,1);0,692(3,0);0,528(3,0);0,146(0,4);0,00 0(73,5);-0,150(0,4) |
| 75 | 3,95 | 4,02 | (400,0 MHz, d₆-DMSO): δ= 8,912(0,4);8,522(1,0);8,506(2,1);8,489(1,0);7,868(2,9); 7,850(2,9);7,483(2,5);7,456(2,5);6,824(0,3);6,808(0,6);6,792(0,4);5,756(0,9);4,754( 0,3);4,731(0,4);4,672(0,4);4,649(1,3);4,627(1,3);4,605(0,5);4,080(0,8);4,065(0,8);4, 048(0,7);4,040(0,8);4,024(0,6);4,016(0,6);4,001(0,5);3,942(1,2);3,917(3,7);3,891(4, 2);3,874(0,6);3,866(2,4);3,850(1,2);3,842(1,3);3,826(1,2);3,817(0,5);3,801(0,4);3,32 8(55,1);2,676(0,4);2,671(0,6);2,667(0,5);2,525(1,5);2,511(33,8);2,507(67,4);2,502(8 8,0);2,498(64,3);2,494(32,0);2,463(16,0);2,334(0,4);2,329(0,6);2,325(0,4);1,708(8,2 );0,146(0,5);0,008(4,0);0,000(109,1);-0,009(4,5);-0,150(0,5) |
| 76 | 4,42 | 4,51 | (400,0 MHz, d₆-DMSO): δ= 8,086(0,7);8,071(1,5);8,057(0,8);7,846(2,2);7,828(2,3); 7,472(2,0);7,445(2,0);3,950(0,9);3,925(2,9);3,899(3,0);3,873(1,0);3,331(113,4);3,07 7(1,1);3,063(1,9);3,049(1,2);2,676(0,4);2,672(0,5);2,667(0,4);2,525(1,4);2,507(65,6) ;2,502(84,5);2,498(62,4);2,458(12,4);2,334(0,4);2,329(0,5);2,325(0,4);1,809(0,5);1, 792(0,9);1,775(1,2);1,759(1,1);1,742(0,7);1,697(5,6);0,884(16,0);0,867(15,5);0,146( 0,6);0,008(4,7);0,000(119,0);-0,008(5,4);-0,150(0,6) |
| 77 | 4,33 | 4,37 | (400,0 MHz, d₆-DMSO): δ= 8,524(1,0);8,509(2,0);8,494(1,0);7,846(2,9);7,828(2,9); 7,470(2,5);7,443(2,4);7,362(0,7);7,359(0,5);7,349(1,1);7,343(4,6);7,335(5,1);7,328( 16,0);7,319(1,2);7,314(1,2);7,278(0,9);7,272(1,2);7,263(1,2);7,257(1,3);7,253(0,8);7 ,248(0,7);7,242(0,6);7,235(0,3);5,757(4,8);4,436(2,0);4,425(2,0);3,939(1,1);3,914(3, 5);3,888(3,7);3,862(1,3);3,332(36,6);2,541(0,4);2,511(14,6);2,507(28,4);2,502(37,0) ;2,498(27,4);2,493(14,1);2,455(15,1);1,709(9,2);0,008(3,0);0,000(63,8);-0,009(3,4) |
| 78 | 3,29 | 3,37 | (400,0 MHz, d₆-DMSO): δ= 10,275(4,2);8,342(1,8);8,339(1,9);8,330(1,9);8,327(1,8) ;8,005(2,3);7,984(3,3);7,900(1,2);7,895(1,3);7,877(1,8);7,861(0,9);7,856(0,9);7,729( 2,9);7,711(2,9);7,507(2,8);7,480(2,6);7,198(1,4);7,196(1,6);7,184(1,7);7,180(1,6);7, 178(1,5);7,168(1,4);7,166(1,4);4,589(0,6);4,547(1,1);3,927(1,2);3,901(3,9);3,875(4, 2);3,849(1,6);3,330(19,4);2,525(0,7);2,511(15,1);2,507(30,7);2,503(40,7);2,498(30, 5);2,494(15,7);2,480(16,0);2,390(1,1);2,075(0,4);0,000(5,5) |
| 79 | 3,56 | 3,55 | (400,0 MHz, d₆-DMSO): δ= 8,377(1,0);8,361(2,2);8,345(1,1);7,756(0,5);7,478(2,1); 7,451(2,1);5,757(0,8);4,773(0,4);4,765(0,4);4,119(0,6);4,101(0,7);4,080(0,8);4,059( 0,7);3,922(0,8);3,896(2,1);3,871(2,1);3,845(0,8);3,329(17,9);2,507(35,9);2,502(47,3 );2,498(36,2);2,462(16,0);2,420(0,4);1,587(4,5);1,570(4,5);0,008(2,1);0,000(51,1);-0,008(2,8) |
| 80 | 3,98 | 3,99 | (400,0 MHz, d₆-DMSO): δ= 7,906(0,7);7,891(1,4);7,877(0,7);7,745(0,4);7,466(1,6); 7,439(1,6);5,757(0,9);3,924(0,5);3,899(1,4);3,874(1,4);3,848(0,5);3,328(19,6);3,112 (0,4);3,095(0,8);3,079(1,2);3,071(0,9);3,063(1,0);3,056(1,2);3,039(0,8);3,023(0,3);2,524(0,8);2,507(29,8);2,502(38,8);2,498(28,7);2,458(12,2);1,818(0,5);1,801(1,0);1,7 84(1,2);1,768(1,0);1,751(0,6);1,570(2,8);1,553(2,7);0,884(16,0);0,867(15,4);0,846(1 ,4);0,830(1,3);0,819(0,6);0,802(0,6);0,008(1,7);0,000(43,9);-0,008(2,0) |
| 81 | 3,91 | 3,94 | (400,0 MHz, d₆-DMSO): δ= 8,388(1,0);8,373(2,0);8,358(1,0);7,745(0,5);7,465(2,0); 7,438(2,0);7,360(0,3);7,339(10,0);7,328(16,0);7,284(0,4);7,275(1,1);7,264(1,9);7,25 3(1,8);7,242(1,3);7,232(0,4);7,215(0,4);7,147(0,5);7,130(0,4);5,757(5,1);5,052(0,4); 4,746(0,3);4,495(0,5);4,480(0,5);4,458(1,4);4,443(1,4);4,425(1,4);4,410(1,5);4,387( 0,5);4,373(0,5);4,322(0,5);4,307(0,5);3,917(0,6);3,891(1,7);3,866(1,7);3,840(0,7);3, 332(18,8);2,506(22,0);2,502(28,6);2,497(21,7);2,456(14,5);2,074(0,4);1,583(4,0);1, 566(4,0);0,008(1,2);0,000(32,3) |
| 82 | 1,94 | 1,97 | (400,0 MHz, d₆-DMSO): δ= 8,158(0,6);8,020(3,1);8,001(3,1);7,926(1,0);7,913(2,0); 7,901(1,0);7,576(2,5);7,549(2,5);4,452(0,7);4,377(0,4);4,349(1,0);4,339(0,5);4,322( 1,1);4,312(1,2);4,295(0,5);4,285(1,2);4,258(0,4);3,898(1,1);3,888(0,4);3,871(1,2);3, 861(1,0);3,845(0,5);3,834(1,0);3,807(0,3);3,441(1,1);3,430(4,1);3,420(7,0);3,407(3, 5);3,395(2,3);3,331(5,9);3,258(27,9);2,524(0,7);2,506(34,2);2,502(45,1);2,497(33,4) ;2,432(16,0);0,008(0,9);0,000(27,0);-0,008(1,2) |
| 83 | 2,70 | 2,73 | (400,0 MHz, d₆-DMSO): δ= 20,011(0,4);8,317(4,0);8,171(2,8);8,152(2,7);8,059(1,1) ;8,044(2,1);8,030(1,0);7,581(2,4);7,554(2,5);5,758(0,4);4,345(0,6);4,308(0,8);4,282( 0,7);4,252(0,4);3,872(0,5);3,328(955,0);3,260(2,0);3,244(2,6);3,228(2,0);3,212(0,7); 2,676(7,3);2,671(10,1);2,667(7,6);2,524(27,3);2,507(1169,7);2,502(1521,2);2,498(1 110,4);2,442(16,0);2,333(7,2);2,329(9,8);2,324(7,2);1,710(11,7);1,351(0,8);1,297(0, 6);1,260(0,9);1,234(3,2);1,114(5,3);1,096(11,0);1,078(5,1);0,854(0,7);0,826(0,6);0,1 46(2,3);0,008(18,1);0,000(502,1);-0,008(19,2);-0,150(2,2) |
| 84 | 2,73 | 2,73 | (400,0 MHz, d₆-DMSO): δ= 8,317(0,7);8,146(2,9);8,128(2,9);8,004(2,3);7,996(2,4); 7,576(2,5);7,549(2,5);5,757(2,3);4,337(0,5);4,310(0,7);4,300(0,7);4,273(0,6);3,903( 0,3);3,879(0,4);3,857(0,3);3,568(0,6);3,328(178,0);2,717(1,4);2,707(1,2);2,698(1,6); 2,689(1,6);2,680(1,8);2,676(1,9);2,671(2,7);2,667(1,8);2,506(234,8);2,502(310,2);2, 498(230,6);2,435(16,0);2,333(1,4);2,329(2,0);2,324(1,5);1,712(12,2);1,235(0,5);0,7 13(3,0);0,700(2,7);0,696(2,8);0,526(2,9);0,146(0,4);0,008(3,3);0,000(101,0);-0,008(4,6);-0,150(0,5) |
| 85 | 3,05 | 3,07 | (400,0 MHz, d₆-DMSO): δ= 8,911(0,4);8,518(1,1);8,502(2,4);8,486(1,2);8,317(0,8); 8,212(2,1);8,194(2,2);7,591(2,5);7,565(2,5);6,806(0,4);5,757(1,1);4,752(0,3);4,730( 0,4);4,671(0,4);4,649(1,2);4,627(1,2);4,605(0,4);4,355(0,5);4,320(0,6);4,291(0,6);4, 088(1,4);4,041(0,7);4,025(0,6);4,015(0,5);4,000(0,4);3,891(0,5);3,866(1,0);3,850(1, 0);3,842(1,0);3,826(0,8);3,800(0,4);3,331(487,4);2,727(1,1);2,676(1,6);2,671(2,3);2, 667(1,8);2,524(5,5);2,507(258,6);2,502(344,8);2,498(264,5);2,444(16,0);2,333(1,6); 2,329(2,2);2,324(1,7);1,718(13,8);1,233(0,9);0,146(0,5);0,008(3,8);0,000(103,2);-0,150(0,4) |
| 86 | 3,39 | 3,41 | (400,0 MHz, d₆-DMSO): δ= 8,318(0,8);8,180(1,6);8,161(1,6);8,080(0,8);8,066(1,6); 8,051(0,8);7,583(1,8);7,557(1,8);4,343(0,5);4,315(0,6);4,307(0,6);4,278(0,6);3,897( 0,4);3,328(183,7);3,082(1,2);3,067(1,9);2,725(0,4);2,675(2,0);2,671(2,7);2,667(2,1); 2,564(0,4);2,506(328,5);2,502(417,8);2,498(309,0);2,442(12,1);2,333(2,1);2,329(2,7 );2,324(2,0);1,811(0,5);1,794(0,9);1,777(1,2);1,761(1,1);1,744(0,8);1,708(9,1);1,351 (0,4);1,235(1,0);0,887(16,0);0,871(15,7);0,146(0,6);0,000(126,5);-0,008(6,6);-0,150(0,6) |
| 87 | 3,38 | 3,48 | (400,0 MHz, d₆-DMSO): δ= 8,516(0,9);8,502(1,9);8,486(0,9);8,180(2,2);8,162(2,2); 7,581(2,4);7,555(2,4);7,363(0,7);7,343(4,4);7,335(4,8);7,328(16,0);7,314(1,2);7,278 (0,9);7,272(1,1);7,263(1,1);7,256(1,3);7,248(0,7);7,242(0,6);4,444(2,4);4,429(2,5);4 ,342(0,6);4,314(0,7);4,304(0,7);4,277(0,7);3,872(0,4);3,331(119,1);2,676(0,5);2,671 (0,6);2,666(0,5);2,524(1,6);2,511(35,7);2,506(73,5);2,502(97,4);2,498(71,5);2,493(3 5,3);2,438(15,1);2,333(0,5);2,329(0,6);2,324(0,5);2,086(13,9);1,929(0,9);1,768(1,0); 1,720(13,2);0,008(0,6);0,000(18,1);-0,009(0,6) |
| 88 | 2,71 | 2,75 | (400,0 MHz, d₆-DMSO): δ= 8,357(0,3);8,139(2,4);7,582(0,4);7,555(0,4);3,420(34,4) ;3,170(0,3);2,511(16,9);2,507(21,9);2,503(16,8);2,438(2,7);2,088(16,0);1,598(0,8);1 ,582(0,8);0,000(3,9) |
| 89 | 3,02 | 3,10 | (400,0 MHz, d₆-DMSO): δ= 8,317(0,6);8,071(0,6);8,059(0,6);7,886(1,1);7,872(0,6); 7,576(1,8);7,550(1,8);4,351(0,4);4,340(0,4);4,324(0,5);4,314(0,7);4,301(0,4);4,287( 0,6);4,275(0,4);3,895(0,5);3,869(0,5);3,859(0,5);3,832(0,4);3,328(165,7);3,118(0,5); 3,102(0,9);3,086(1,3);3,070(1,2);3,056(1,1);3,041(0,8);3,024(0,4);2,676(1,0);2,671( 1,4);2,667(1,1);2,524(3,7);2,511(80,2);2,507(163,7);2,502(215,7);2,497(156,7);2,49 3(76,7);2,434(12,0);2,333(1,0);2,329(1,4);2,324(1,0);1,819(0,5);1,802(1,0);1,785(1, 4);1,768(1,1);1,752(0,6);1,580(3,1);1,564(3,0);0,886(16,0);0,869(15,6);0,846(1,5);0, 829(1,5);0,818(0,7);0,801(0,6);0,008(2,0);0,000(65,0);-0,009(2,4) |
| 90 | 3,05 | 3,12 | (400,0 MHz, d₆-DMSO): δ= 8,377(0,6);8,363(1,2);8,347(0,6);8,074(0,5);8,060(0,5); 7,575(1,4);7,548(1,4);7,339(6,2);7,328(11,2);7,274(0,6);7,264(1,0);7,253(1,0);7,241 (0,5);4,486(0,3);4,464(0,9);4,449(0,8);4,425(1,0);4,411(1,1);4,388(0,4);4,374(0,4);4 ,351(0,3);4,324(0,4);4,314(0,5);4,301(0,4);4,287(0,4);3,880(0,3);3,854(0,3);3,845(0, 3);3,330(19,7);2,524(0,5);2,510(11,2);2,506(22,8);2,502(30,2);2,497(22,5);2,493(11 ,5);2,431(8,5);2,086(16,0);1,593(2,7);1,576(2,7);0,000(6,0) |
| 91 | 2,13 | 2,19 | (400,0 MHz, d₆-DMSO): δ= 8,317(0,5);8,147(1,0);8,018(3,1);8,000(3,1);7,920(1,0); 7,906(1,9);7,892(1,0);7,577(2,5);7,550(2,4);4,455(0,6);4,376(0,4);4,349(1,0);4,339( 0,5);4,321(1,1);4,312(1,2);4,294(0,5);4,284(1,2);4,257(0,4);3,963(0,4);3,946(1,2);3, 935(1,3);3,929(1,6);3,918(1,3);3,912(0,6);3,903(1,2);3,894(0,4);3,877(1,2);3,866(1, 0);3,850(0,5);3,840(1,0);3,813(0,3);3,779(0,8);3,762(1,5);3,759(1,4);3,742(1,9);3,72 6(1,0);3,649(1,0);3,631(2,0);3,612(1,6);3,594(0,7);3,415(0,6);3,400(0,8);3,389(0,8); 3,381(1,0);3,366(1,3);3,355(1,4);3,329(78,8);3,257(0,8);3,242(1,2);3,225(1,1);3,208 (0,7);3,192(0,5);2,676(0,7);2,671(1,0);2,667(0,8);2,524(2,7);2,511(57,4);2,507(117, 9);2,502(156,1);2,498(114,7);2,493(56,8);2,432(16,0);2,333(0,8);2,329(1,0);2,324(0 ,8);1,932(0,6);1,920(0,7);1,916(0,7);1,909(0,6);1,900(1,0);1,887(0,9);1,871(0,9);1,8 57(0,6);1,841(1,1);1,837(1,0);1,831(1,5);1,821(1,5);1,814(1,6);1,794(1,0);1,777(0,4) ;1,578(0,5);1,561(0,9);1,545(0,6);1,539(0,9);1,531(1,0);1,523(0,5);1,511(0,8);0,008( 0,9);0,000(29,2);-0,009(1,1) |
| 92 | 3,66 | 3,75 | (400,0 MHz, d₆-DMSO): δ= 8,365(1,0);8,350(2,1);8,334(1,0);7,505(5,8);7,342(12,5) ;7,331(10,2);7,316(0,5);7,309(0,7);7,291(4,8);7,275(1,0);7,263(1,5);7,253(1,6);7,24 2(1,0);7,232(0,4);4,529(2,5);4,485(4,2);4,468(0,5);4,446(2,4);4,432(3,7);4,418(2,4); 4,396(0,4);4,372(4,2);4,328(2,5);3,879(1,1);3,853(3,5);3,827(3,7);3,802(1,3);3,335( 108,4);2,671(0,4);2,506(50,0);2,502(65,5);2,498(49,8);2,388(15,1);2,333(0,4);2,329 (0,4);2,117(16,0);2,074(1,2);0,008(0,6);0,000(16,5) |
| 93 | 3,86 | 3,95 | (400,0 MHz, d₆-DMSO): δ= 9,875(4,3);7,594(2,7);7,589(1,1);7,582(2,9);7,577(1,8); 7,571(3,2);7,565(1,2);7,559(3,0);7,550(0,4);7,540(5,9);7,321(4,7);7,226(3,0);7,220( 1,0);7,203(5,2);7,187(0,9);7,181(2,7);4,615(2,5);4,571(4,2);4,474(4,3);4,430(2,5);3, 891(1,1);3,866(3,4);3,840(3,5);3,814(1,2);3,330(35,0);2,671(0,4);2,524(1,0);2,507(4 3,1);2,502(56,4);2,498(41,9);2,408(15,1);2,329(0,4);2,154(16,0);2,075(0,4);0,008(2, 1);0,000(57,2);-0,008(2,6) |
| 94 | 3,96 | 4,05 | (400,0 MHz, d₆-DMSO): δ= 9,807(4,5);7,764(6,7);7,674(5,7);7,596(2,8);7,591(1,2); 7,584(3,1);7,578(1,9);7,573(3,4);7,566(1,3);7,561(3,2);7,552(0,4);7,227(3,2);7,222( 1,1);7,205(5,5);7,188(1,0);7,183(2,9);4,741(3,0);4,696(4,3);4,561(4,4);4,516(2,8);4, 003(1,2);3,978(3,7);3,952(3,9);3,927(1,3);3,329(47,9);2,676(0,4);2,671(0,5);2,667(0 ,4);2,524(1,4);2,511(30,5);2,507(61,0);2,502(79,9);2,498(59,1);2,493(30,1);2,434(1 6,0);2,333(0,5);2,329(0,6);2,325(0,4);0,146(0,4);0,008(3,0);0,000(80,2);-0,008(3,7);-0,150(0,4) |
| 95 | 3,42 | 3,47 | (400,0 MHz, d₆-DMSO): δ= 10,346(4,1);8,333(1,8);8,323(1,8);8,321(1,8);8,319(1,8) ;8,016(2,3);7,995(3,1);7,898(1,1);7,893(1,2);7,875(1,7);7,858(0,9);7,854(0,9);7,532( 5,8);7,328(4,6);7,189(1,5);7,177(1,6);7,173(1,5);7,158(1,4);4,632(2,5);4,588(4,1);4, 495(4,3);4,451(2,4);3,895(1,2);3,870(3,8);3,844(3,9);3,818(1,4);3,330(245,6);2,675( 1,1);2,671(1,5);2,667(1,1);2,524(3,5);2,510(83,5);2,506(171,1);2,502(227,6);2,498( 168,6);2,412(15,3);2,374(0,6);2,333(1,1);2,329(1,5);2,324(1,2);2,156(16,0);2,061(0, 5);0,146(1,2);0,008(9,1);0,000(268,8);-0,009(11,0);-0,150(1,3) |
| 96 | 3,51 | 3,55 | (400,0 MHz, d₆-DMSO): δ= 10,405(0,8);10,263(4,3);8,415(1,1);8,341(1,8);8,339(1, 9);8,329(1,9);8,327(1,8);8,318(0,4);8,084(0,4);8,079(0,4);8,007(2,3);7,986(3,4);7,904(1,2);7,899(1,2);7,881(1,8);7,864(0,9);7,860(0,9);7,750(6,4);7,681(5,5);7,531(0,5); 7,513(1,2);7,201(1,5);7,199(1,5);7,187(1,6);7,183(1,6);7,181(1,5);7,170(1,4);7,168( 1,4);4,774(2,9);4,730(4,1);4,580(4,3);4,555(1,9);4,535(2,9);4,008(1,1);3,982(3,6);3, 957(3,8);3,931(1,4);3,920(0,8);3,895(0,7);3,330(94,1);2,676(0,6);2,671(0,9);2,667(0 ,6);2,524(2,2);2,511(49,3);2,507(99,2);2,502(129,9);2,498(95,1);2,493(47,2);2,439( 16,0);2,365(0,6);2,354(2,7);2,333(1,1);2,329(1,1);2,325(0,7);2,206(0,4);0,146(0,7);0 ,008(5,6);0,000(152,0);-0,008(6,5);-0,150(0,7) |
| 97 | 3,47 | 3,57 | (400,0 MHz, d₆-DMSO): δ= 10,110(2,6);10,102(2,9);8,073(1,8);8,067(1,6);8,061(1, 7);7,885(7,1);7,809(1,5);7,789(1,9);7,623(0,6);7,612(0,7);7,604(1,3);7,592(1,4);7,58 4(0,8);7,572(0,8);7,483(1,3);7,466(1,0);7,428(5,1);4,639(1,6);4,631(1,4);4,596(2,4); 4,587(2,2);4,498(2,3);4,488(2,5);4,455(1,3);4,445(1,5);4,314(0,5);4,299(0,6);4,287( 0,7);4,277(0,7);4,272(0,8);4,262(0,8);4,250(0,7);4,235(0,7);3,839(0,6);3,812(0,8);3, 802(0,6);3,785(0,8);3,775(0,8);3,758(0,6);3,748(0,7);3,721(0,5);3,330(34,0);2,524(0 ,6);2,511(14,7);2,506(30,5);2,502(40,6);2,497(30,0);2,493(15,1);2,396(13,5);2,312( 0,3);2,295(0,4);2,259(16,0);2,075(8,8);0,008(1,2);0,000(35,4);-0,009(1,4) |
| 98 | 2,77 | 2,85 | (400,0 MHz, d₆-DMSO): δ= 8,359(0,5);8,344(1,7);8,329(1,7);8,314(0,6);7,847(3,4); 7,838(3,4);7,393(4,8);7,344(7,0);7,337(8,3);7,333(8,4);7,326(6,5);7,312(0,5);7,304( 0,4);7,278(0,7);7,268(1,3);7,257(1,7);7,247(1,3);7,236(0,7);5,757(1,7);4,522(1,6);4, 518(1,6);4,478(2,8);4,475(2,8);4,455(1,3);4,440(2,9);4,425(2,1);4,420(1,7);4,402(0, 3);4,380(2,3);4,365(2,3);4,336(1,3);4,322(1,6);4,306(0,5);4,280(0,9);4,270(0,4);4,25 2(1,0);4,243(1,1);4,225(0,4);4,216(1,1);4,188(0,3);3,813(0,7);3,786(1,1);3,776(0,7); 3,759(0,8);3,749(1,0);3,722(0,7);3,328(45,0);2,675(0,4);2,671(0,6);2,666(0,5);2,662 (0,5);2,524(1,2);2,506(63,5);2,501(84,5);2,497(63,5);2,378(15,3);2,333(0,5);2,328(0 ,6);2,324(0,5);2,249(0,4);2,217(16,0);0,000(0,9) |
| 99 | 2,92 | 3,01 | (400,0 MHz, d₆-DMSO): δ= 9,875(2,6);9,868(2,8);7,877(7,1);7,597(1,7);7,591(2,0); 7,585(2,2);7,579(2,5);7,574(2,8);7,568(2,4);7,562(2,4);7,556(1,8);7,422(5,0);7,232( 1,7);7,222(1,9);7,216(1,1);7,210(3,0);7,205(1,4);7,200(3,2);7,188(1,7);7,184(0,7);7, 178(1,7);5,757(2,9);4,617(1,6);4,608(1,4);4,573(2,4);4,565(2,3);4,473(2,3);4,463(2, 5);4,430(1,3);4,419(1,5);4,306(0,5);4,296(0,7);4,279(0,6);4,269(1,2);4,259(0,7);4,24 2(0,8);4,232(0,7);3,837(0,6);3,826(0,3);3,810(0,7);3,799(1,0);3,783(0,3);3,772(1,1); 3,761(0,6);3,745(0,4);3,735(0,5);3,329(37,6);2,679(0,4);2,671(0,4);2,524(0,9);2,510 (21,5);2,506(43,9);2,502(58,1);2,497(43,0);2,393(13,9);2,328(0,4);2,285(0,3);2,251( 16,0);0,000(0,6) |
| 100 | 3,09 | 3,17 | (400,0 MHz, d₆-DMSO): δ= 9,808(2,6);9,799(2,8);8,317(0,3);8,091(8,0);7,810(6,2); 7,593(2,2);7,583(2,2);7,580(2,5);7,573(2,7);7,570(2,4);7,560(2,4);7,234(1,8);7,224( 2,0);7,217(1,1);7,212(3,2);7,207(1,4);7,202(3,4);7,190(1,7);7,179(1,7);5,757(1,8);4, 731(1,8);4,724(1,6);4,686(2,5);4,680(2,4);4,534(2,2);4,522(2,4);4,490(1,5);4,478(1, 7);4,400(0,5);4,388(0,7);4,373(0,7);4,362(1,0);4,351(0,7);4,335(0,8);4,323(0,7);3,94 3(0,6);3,916(0,7);3,905(0,6);3,896(0,6);3,889(0,4);3,879(0,6);3,869(0,7);3,859(0,6); 3,832(0,5);3,329(73,0);2,676(0,6);2,671(0,9);2,667(0,6);2,524(2,2);2,506(100,5);2,5 02(132,5);2,498(98,0);2,434(16,0);2,333(0,6);2,329(0,9);2,324(0,7);2,320(0,5);0,00 0(1,5) |
| 101 | 3,96 | 4,09 | (400,0 MHz, d₆-DMSO): δ= 7,702(1,3);7,684(1,4);7,649(1,8);7,460(1,2);7,434(1,2); 3,912(0,6);3,886(1,8);3,860(1,8);3,835(0,6);3,328(23,5);2,671(0,4);2,506(47,9);2,50 2(62,7);2,498(47,1);2,456(7,5);2,335(0,5);2,329(0,4);1,692(0,5);1,673(1,6);1,655(1, 7);1,636(0,6);1,292(16,0);1,230(0,7);0,861(2,0);0,843(4,3);0,824(1,9);0,008(1,2);0,0 00(34,6) |
| 102 | 3,84 | 3,71 | (400,0 MHz, d₆-DMSO): δ= 10,263(0,9);10,248(1,8);10,232(0,9);7,730(2,9);7,712(2 ,9);7,491(2,5);7,464(2,5);4,755(0,5);4,714(0,6);4,644(0,9);3,897(1,2);3,871(3,7);3,8 45(3,9);3,820(1,3);3,328(46,7);2,675(0,4);2,671(0,5);2,666(0,4);2,524(1,5);2,511(30 ,7);2,506(62,1);2,502(81,4);2,497(59,1);2,493(28,8);2,468(16,0);2,333(0,4);2,329(0, 5);2,324(0,4);2,075(5,8);0,008(0,5);0,000(14,2);-0,009(0,5) |
| 103 | 3,26 | 3,25 | (400,0 MHz, d₆-DMSO): δ= 8,318(0,6);8,030(12,2);7,904(9,6);7,878(1,4);7,864(2,8) ;7,850(1,4);4,656(4,3);4,611(6,1);4,461(6,2);4,416(4,1);4,132(1,5);4,107(4,8);4,082( 5,0);4,056(1,8);3,328(73,7);3,307(0,4);3,291(0,8);3,282(0,7);3,274(2,1);3,265(1,9);3 ,256(2,4);3,250(2,4);3,241(2,0);3,232(2,1);3,224(0,7);3,215(0,8);2,676(1,0);2,671(1,4);2,667(1,0);2,524(3,3);2,510(79,0);2,506(160,6);2,502(212,5);2,498(156,1);2,493( 78,0);2,333(1,0);2,329(1,4);2,324(1,0);1,989(0,7);1,398(8,4);1,175(0,4);1,114(7,4);1 ,096(16,0);1,078(7,3);1,068(0,6);0,146(0,4);0,008(2,7);0,000(85,3);-0,008(3,5);-0,150(0,4) |
| 104 | 3,37 | 3,3 | (400,0 MHz, d₆-DMSO): δ= 8,313(0,4);8,018(16,0);7,882(15,1);7,780(6,1);7,773(6, 6);4,658(6,3);4,613(9,5);4,469(9,7);4,424(6,3);4,120(2,8);4,095(8,5);4,070(8,8);4,04 4(3,1);4,020(0,5);3,318(112,5);2,715(0,6);2,706(1,5);2,698(2,6);2,688(3,6);2,680(4, 0);2,671(3,7);2,663(2,2);2,501(154,6);2,498(125,0);2,328(1,0);1,988(1,7);1,398(9,9) ;1,193(0,5);1,175(0,9);1,158(0,4);0,732(1,1);0,714(7,8);0,701(7,3);0,697(7,2);0,684( 1,9);0,609(0,4);0,591(0,7);0,582(0,9);0,570(4,4);0,561(8,2);0,553(8,1);0,518(0,6);0, 146(0,5);0,000(101,9);-0,150(0,5) |
| 105 | 3,99 | 3,91 | (400,0 MHz, d₆-DMSO): δ= 8,025(5,8);7,906(5,5);7,857(1,0);7,842(2,1);7,828(1,2); 4,663(2,2);4,618(3,4);4,470(3,4);4,425(2,2);4,130(1,0);4,105(3,2);4,080(3,3);4,055( 1.2);3,321(68,8);3,143(0,3);3,128(0,7);3,111(1,3);3,095(1,8);3,078(1,7);3,060(1,7);3 ,044(1,3);3,028(0,7);3,012(0,4);2,671(0,3);2,502(48,4);1,825(0,5);1,808(1,0);1,792( 1,3);1,775(1,1);1,758(0,6);1,398(9,9);0,883(16,0);0,867(15,7);0,002(22,6);0,000(26, 7) |
| 106 | 3,53 | 3,5 | (400,0 MHz, d₆-DMSO): δ= 10,398(0,4);8,485(0,6);8,330(2,4);8,314(5,2);8,298(2,5) ;8,038(16,0);8,024(0,8);7,913(14,0);7,876(0,7);4,718(6,0);4,674(8,5);4,617(0,4);4,5 09(8,6);4,490(1,1);4,464(5,9);4,171(0,7);4,154(1,2);4,148(1,1);4,125(3,5);4,117(2,6) ;4,100(8,8);4,075(9,5);4,062(2,9);4,050(4,0);4,038(3,1);4,021(1,3);4,015(1,4);4,001( 0,8);3,347(0,7);3,321(211,5);3,292(0,5);2,671(0,8);2,502(123,2);2,498(97,3);2,329( 0,8);1,988(1,8);1,398(8,1);1,193(0,5);1,175(1,0);1,158(0,5);0,883(1,7);0,867(1,7);0, 146(0,4);0,000(79,7);-0,150(0,4) |
| 107 | 3,90 | 3,86 | (400,0 MHz, d₆-DMSO): δ= 8,353(1,5);8,339(2,9);8,324(1,5);8,025(7,6);8,016(1,2); 7,901(7,4);7,340(16,0);7,330(16,0);7,275(1,6);7,264(2,5);7,254(2,3);7,243(1,6);7,15 0(0,3);4,678(3,0);4,633(4,4);4,480(5,0);4,452(2,9);4,435(5,7);4,427(3,4);4,411(2,8); 4,388(0,8);4,374(0,7);4,322(0,3);4,122(1,4);4,097(4,2);4,072(4,4);4,046(1,5);3,318( 50,8);3,309(7,3);2,670(0,7);2,505(91,2);2,501(99,6);2,328(0,7);1,988(0,4);1,398(2,7 );0,000(51,9);-0,008(8,6) |
| 108 | 4,20 | 4,09 | (400,0 MHz, d₆-DMSO): δ= 9,783(7,7);8,313(0,6);8,056(16,0);7,952(0,5);7,929(12, 4);7,831(0,4);7,605(0,5);7,596(5,1);7,591(2,0);7,584(5,5);7,579(3,3);7,573(6,1);7,56 7(2,1);7,561(5,8);7,552(0,6);7,236(0,6);7,227(5,9);7,222(1,9);7,215(0,9);7,205(9,8); 7,189(1,7);7,183(5,3);7,174(0,5);4,770(5,5);4,725(7,8);4,590(8,1);4,545(5,3);4,495( 0,4);4,136(1,9);4,110(6,0);4,085(6,2);4,060(2,2);3,343(0,5);3,318(251,1);2,675(1,0); 2,671(1,5);2,666(1,1);2,662(0,5);2,524(3,5);2,519(5,3);2,510(79,0);2,506(165,5);2,5 01(222,7);2,497(164,1);2,492(81,0);2,333(1,1);2,328(1,5);2,324(1,1);2,319(0,6);1,9 88(0,5);1,398(7,4);0,146(0,9);0,008(6,3);0,000(204,6);-0,009(8,0);-0,150(0,9) |
| 109 | 4,56 | 4,49 | (400,0 MHz, d₆-DMSO): δ= 9,897(9,1);8,520(0,4);8,313(0,5);8,059(16,0);7,927(13, 6);7,871(0,6);7,773(4,4);7,768(8,3);7,763(5,0);7,593(0,5);7,494(3,0);7,492(2,9);7,47 4(4,3);7,471(4,5);7,406(4,5);7,385(7,6);7,365(3,7);7,206(4,1);7,203(4,1);7,186(3,3); 7,183(3,4);4,780(5,6);4,735(8,2);4,601(8,5);4,556(5,5);4,507(0,6);4,134(2,2);4,109( 6,9);4,084(7,2);4,059(2,5);4,039(0,3);4,021(0,3);3,371(0,4);3,365(0,4);3,357(0,8);3, 321(307,7);2,675(0,9);2,671(1,2);2,667(0,9);2,506(147,4);2,502(194,8);2,497(148,1 );2,333(0,9);2,329(1,2);1,988(1,2);1,398(9,4);1,193(0,3);1,175(0,7);1,157(0,3);0,146 (0,7);0,016(0,6);0,008(5,7);0,000(149,8);-0,008(6,5);-0,150(0,7) |
| 110 | 4,61 | 4,56 | (400,0 MHz, d₆-DMSO): δ= 10,023(6,2);8,313(0,4);8,064(16,0);7,951(0,7);7,937(9, 5);7,827(2,0);7,805(2,4);7,621(1,6);7,601(3,4);7,581(1,9);7,497(2,9);7,478(2,1);4,79 3(4,1);4,748(5,8);4,617(6,1);4,572(3,9);4,137(1,5);4,112(4,7);4,086(4,9);4,061(1,7); 3,317(49,9);2,675(0,5);2,671(0,7);2,666(0,5);2,524(1,8);2,511(40,2);2,506(81,9);2,5 02(109,0);2,497(81,2);2,493(41,2);2,333(0,5);2,329(0,7);2,324(0,5);2,073(0,5);0,00 8(0,4);0,000(11,8);-0,008(0,5) |
| 111 | 2,59 | 2,56 | (400,0 MHz, d₆-DMSO): δ= 8,209(5,0);8,207(4,7);8,127(4,5);8,123(4,6);7,848(1,0); 7,834(2,1);7,820(1,1);4,617(1,8);4,613(1,8);4,572(2,5);4,569(2,5);4,494(0,5);4,471( 0,7);4,467(0,7);4,456(0,7);4,444(0,6);4,433(0,8);4,430(0,8);4,404(2,6);4,396(2,4);4,359(1,5);4,352(1,6);4,075(0,6);4,064(0,3);4,048(0,7);4,037(1,0);4,022(0,4);4,010(1, 1);3,999(0,6);3,983(0,4);3,972(0,5);3,316(21,1);3,295(0,4);3,277(1,0);3,268(1,5);3,2 64(1,5);3,250(2,1);3,237(1,4);3,232(1,3);3,220(0,5);2,670(0,3);2,506(40,8);2,501(53 ,5);2,497(41,0);2,328(0,4);1,398(16,0);1,236(0,4);1,120(2,9);1,115(3,2);1,102(6,3);1 ,097(6,7);1,085(3,0);1,079(3,1);0,008(0,5);0,000(13,7) |
| 112 | 2,65 | 2,61 | (400,0 MHz, d₆-DMSO): δ= 8,203(3,5);8,202(3,3);8,107(3,3);8,105(3,2);7,762(1,3); 7,756(0,9);4,624(1,1);4,620(1,1);4,580(1,5);4,576(1,5);4,466(0,3);4,455(0,4);4,444( 0,4);4,440(0,4);4,429(0,5);4,417(0,5);4,411(1,6);4,406(1,6);4,367(1,0);4,361(1,0);4, 073(0,3);4,046(0,4);4,038(0,4);4,035(0,4);4,028(0,4);4,020(0,4);4,009(0,4);4,002(0, 5);3,991(0,3);3,316(10,5);2,704(0,5);2,695(0,8);2,686(0,9);2,677(0,8);2,669(0,7);2,6 60(0,3);2,524(0,5);2,519(0,8);2,510(11,9);2,506(24,4);2,501(32,4);2,497(23,8);2,49 2(11,8);1,988(0,8);1,398(16,0);1,175(0,4);0,722(1,1);0,718(1,1);0,711(1,4);0,704(1, 1);0,700(1,2);0,694(0,9);0,565(0,8);0,555(1,9);0,548(1,7);0,539(1,1);0,533(0,5);0,00 0(10,0);-0,009(0,4) |
| 113 | 3,28 | 3,21 | (400,0 MHz, d₆-DMSO): δ= 8,210(3,5);8,137(2,6);8,128(2,5);7,836(0,4);7,825(0,8); 7,822(0,8);7,810(0,4);4,625(1,1);4,622(1,0);4,581(1,5);4,578(1,4);4,477(0,3);4,473( 0,3);4,450(0,4);4,446(0,4);4,439(0,4);4,436(0,4);4,410(1,6);4,404(1,4);4,365(0,9);4, 359(0,9);4,078(0,3);4,062(0,3);4,052(0,5);4,041(0,3);4,035(0,4);4,025(0,4);4,014(0, 3);3,316(17,1);3,116(0,5);3,110(0,5);3,100(0,6);3,093(0,6);3,084(0,4);3,078(0,6);3,0 68(0,4);3,064(0,6);3,053(0,5);3,047(0,5);3,037(0,5);2,524(0,5);2,510(13,7);2,506(28 ,1);2,501(37,5);2,497(27,7);2,492(13,7);1,808(0,4);1,805(0,4);1,792(0,5);1,788(0,5); 1,775(0,4);1,771(0,4);1,398(16,0);0,888(6,3);0,882(6,8);0,872(6,2);0,866(6,5);0,000 (10,8);-0,009(0,4) |
| 114 | 2,91 | 2,88 | (400,0 MHz, d₆-DMSO): δ= 8,801(0,4);8,310(1,0);8,294(2,0);8,278(1,0);8,222(5,2); 8,219(5,1);8,152(4,6);8,144(4,6);8,052(0,4);4,686(1,8);4,680(1,8);4,641(2,3);4,636( 2,4);4,500(0,9);4,477(0,7);4,474(0,8);4,463(0,8);4,450(0,8);4,440(3,0);4,431(2,4);4, 413(0,9);4,396(1,6);4,386(1,9);4,156(0,4);4,134(0,6);4,117(1,0);4,111(0,8);4,101(0, 8);4,094(1,3);4,079(1,4);4,070(1,4);4,053(1,5);4,045(1,4);4,034(0,7);4,026(0,7);4,01 8(1,2);4,008(0,7);3,991(0,4);3,981(0,6);3,318(61,4);2,675(0,3);2,670(0,4);2,666(0,3) ;2,523(1,1);2,510(24,8);2,506(49,8);2,501(65,5);2,115497(49,0);2,492(25,0);2,328(0 ,4);1,988(0,9);1,398(16,0);1,175(0,5);0,888(0,4);0,882(0,4);0,872(0,4);0,865(0,4);0, 008(0,6);0,000(17,7) |
| 115 | 3,29 | 3,23 | (400,0 MHz, d₆-DMSO): δ= 8,335(1,0);8,320(2,3);8,310(2,3);8,296(1,0);8,209(7,9); 8,206(7,7);8,135(7,5);8,125(7,4);7,342(11,9);7,336(13,8);7,331(16,0);7,326(14,1);7, 304(0,4);7,278(1,1);7,268(1,9);7,257(2,4);7,248(1,9);7,238(1,0);7,236(0,9);7,227(0, 4);4,642(3,9);4,597(5,4);4,499(0,8);4,489(0,8);4,482(1,4);4,471(1,6);4,462(2,5);4,45 2(3,0);4,445(3,8);4,434(4,5);4,429(3,7);4,424(5,1);4,416(7,3);4,407(1,6);4,396(0,9); 4,391(1,1);4,380(3,5);4,371(2,8);4,075(1,0);4,065(0,5);4,055(0,4);4,048(1,2);4,038( 2,1);4,029(0,5);4,020(1,1);4,013(1,6);4,002(1,2);3,986(0,6);3,976(0,9);3,317(35,3);2 ,674(0,4);2,670(0,5);2,666(0,4);2,523(1,2);2,509(28,8);2,505(58,2);2,501(77,1);2,49 6(57,8);2,332(0,4);2,328(0,5);2,323(0,4);1,988(2,6);1,398(4,1);1,193(0,7);1,175(1,4) ;1,157(0,7);0,008(0,8);0,000(22,4);-0,008(0,9) |
| 116 | 3,46 | 3,39 | (400,0 MHz, d₆-DMSO): δ= 9,782(2,5);9,778(2,7);8,239(7,3);8,160(7,9);7,598(1,6); 7,595(1,8);7,585(1,8);7,582(2,0);7,575(2,2);7,572(1,9);7,563(2,0);7,560(1,8);7,232( 1,6);7,224(1,7);7,219(0,7);7,210(2,6);7,202(2,8);7,194(0,7);7,188(1,5);7,180(1,5);4, 743(1,6);4,739(1,6);4,699(2,2);4,694(2,1);4,531(2,1);4,526(2,3);4,504(0,5);4,487(1, 8);4,481(1,8);4,467(0,6);4,458(0,6);4,448(0,7);4,440(0,6);4,421(0,6);4,099(0,5);4,07 3(0,7);4,062(0,5);4,049(0,6);4,035(0,6);4,022(0,6);4,011(0,5);3,985(0,5);3,317(29,7) ;2,670(0,4);2,524(0,9);2,519(1,4);2,510(21,4);2,506(44,2);2,501(58,9);2,497(43,4);2 ,492(21,4);2,328(0,4);1,398(16,0);0,008(0,6);0,000(17,9);-0,008(0,6) |
| 117 | 3,89 | 3,81 | (400,0 MHz, d₆-DMSO): δ= 9,897(0,7);9,892(0,7);8,241(2,2);8,159(2,2);7,771(0,4); 7,767(0,8);7,763(0,8);7,758(0,4);7,494(0,3);7,492(0,4);7,473(0,5);7,471(0,5);7,390( 0,5);7,381(0,5);7,202(0,5);7,183(0,4);4,752(0,5);4,748(0,4);4,708(0,6);4,704(0,6);4, 542(0,6);4,537(0,6);4,498(0,4);4,492(0,5);3,317(6,4);2,524(0,4);2,510(7,3);2,506(14 ,1);2,501(18,0);2,497(13,2);2,493(6,5);1,398(16,0);0,000(5,2) |
| 118 | 2,50 | 2,46 | (400,0 MHz, d₆-DMSO): δ= 10,360(1,9);10,326(1,8);8,332(2,1);8,330(2,0);8,317(4, 9);8,021(1,5);8,018(1,6);8,016(1,6);8,000(2,3);7,997(2,3);7,995(2,2);7,901(0,9);7,89 6(1,5);7,892(1,0);7,882(7,4);7,862(0,6);7,857(1,1);7,852(0,6);7,428(5,2);7,193(1,0); 7,191(1,1);7,186(1,0);7,174(1,8);7,162(1,0);7,160(1,1);4,634(1,6);4,627(1,6);4,591( 2,5);4,584(2,6);4,492(2,6);4,479(2,5);4,448(1,5);4,436(1,5);4,317(0,6);4,307(0,3);4, 301(0,6);4,290(0,8);4,281(0,7);4,273(0,7);4,264(0,8);4,253(0,7);4,247(0,4);4,236(0, 6);3,850(0,6);3,834(0,7);3,823(0,9);3,813(0,7);3,807(0,8);3,797(0,8);3,786(0,6);3,77 0(0,6);3,328(328,0);2,680(2,2);2,676(4,1);2,671(5,8);2,667(4,3);2,604(0,4);2,573(0, 6);2,525(14,3);2,520(21,6);2,511(315,2);2,507(659,0);2,502(876,5);2,498(638,1);2,4 93(311,7);2,394(16,0);2,338(2,0);2,333(4,1);2,329(5,7);2,324(4,2);2,320(2,1);2,288( 0,6);2,255(14,5);2,075(0,8);1,148(0,4);0,146(1,4);0,008(10,5);0,000(355,8);-0,008(13,2);-0,150(1,4) |
| 119 | 2,65 | 2,65 | (400,0 MHz, d₆-DMSO): δ= 10,276(2,4);10,240(2,3);8,336(2,0);8,334(2,0);8,324(2, 0);8,313(0,6);8,092(4,9);8,090(4,9);8,011(1,4);8,008(1,4);8,005(1,4);7,990(2,0);7,98 7(2,0);7,984(2,0);7,904(0,8);7,900(1,4);7,895(0,8);7,881(1,8);7,865(0,6);7,860(1,0); 7,856(0,6);7,811(5,7);7,196(1,1);7,182(1,7);7,166(1,0);5,753(1,7);4,768(1,6);4,759( 1,6);4,723(2,2);4,715(2,2);4,550(2,3);4,538(2,2);4,506(1,5);4,494(1,5);4,403(0,5);4, 385(0,5);4,376(0,8);4,366(0,7);4,359(0,7);4,349(0,8);4,339(0,7);4,321(0,6);3,944(0, 7);3,939(0,7);3,917(0,8);3,912(0,9);3,907(0,8);3,902(0,7);3,890(0,3);3,880(0,7);3,87 5(0,6);3,317(123,4);2,675(0,8);2,670(1,1);2,666(0,8);2,540(0,5);2,523(3,1);2,506(12 9,8);2,501(168,4);2,497(125,9);2,436(16,0);2,416(0,5);2,332(0,8);2,328(1,1);2,324( 0,8);1,235(0,5);0,007(2,8);0,000(67,1);-0,008(3,2) |
| 120 | 4,02 | 3,92 | (400,0 MHz, d₆-DMSO): δ= 10,021(2,1);10,017(2,3);8,313(0,5);8,246(5,1);8,165(7, 1);8,061(1,5);7,822(1,1);7,802(1,3);7,625(0,5);7,617(0,5);7,605(0,9);7,597(1,0);7,58 5(0,5);7,577(0,5);7,495(1,1);7,475(0,8);4,764(1,3);4,759(1,2);4,719(1,8);4,715(1,7); 4,557(1,7);4,553(1,9);4,513(1,6);4,508(1,4);4,487(0,7);4,477(0,6);4,461(0,6);4,450( 0,9);4,424(0,6);4,102(0,5);4,075(0,5);4,064(0,5);4,038(0,7);4,010(0,6);3,999(0,4);3, 972(0,4);3,317(86,6);2,675(0,7);2,671(0,9);2,666(0,7);2,661(0,3);2,524(2,1);2,510(5 0,2);2,506(103,2);2,501(137,0);2,497(99,8);2,492(48,4);2,333(0,6);2,328(0,9);2,324 (0,7);1,398(16,0);0,146(0,5);0,008(3,9);0,000(116,5);-0,008(4,1);-0,150(0,5) |
| 121 | 3,03 | 2,99 | (400,0 MHz, d₆-DMSO): δ= 7,716(2,3);7,697(2,3);7,436(2,0);7,409(2,0);5,753(3,2); 4,554(8,3);3,925(0,9);3,899(2,9);3,874(3,0);3,848(1,0);3,318(27,5);2,945(16,0);2,89 8(0,7);2,885(1,0);2,871(0,8);2,524(0,8);2,510(17,0);2,506(34,5);2,501(45,9);2,497(3 4,3);2,446(12,5);2,073(4,3);0,720(2,6);0,703(2,1);0,000(5,6) |
| 122 | 2,62 | 2,58 | (400,0 MHz, d₆-DMSO): δ= 7,734(2,9);7,715(3,0);7,437(2,6);7,410(2,6);4,529(10,2) ;3,911(1,2);3,885(3,8);3,859(4,0);3,833(1,4);3,641(3,8);3,630(6,3);3,618(5,1);3,484( 4,5);3,473(5,7);3,463(3,5);3,316(9,9);2,506(34,5);2,501(46,3);2,497(35,3);2,472(0,4 );2,467(0,4);2,449(16,0);2,073(0,4);0,000(5,8) |
| 123 | 1,51 | 2,5 | (400,0 MHz, d₆-DMSO): δ= 7,734(2,9);7,716(3,0);7,436(2,5);7,409(2,5);5,753(0,4); 4,517(9,4);3,913(1,2);3,888(3,7);3,862(3,9);3,836(1,3);3,462(4,8);3,317(23,0);2,670 (0,4);2,524(1,1);2,510(22,0);2,506(44,8);2,502(59,5);2,497(44,7);2,449(16,0);2,361( 3,7);2,349(5,4);2,337(3,7);2,191(14,2);2,073(0,4);1,235(0,5);0,000(3,2) |
| 124 | 3,59 | 3,53 | (400,0 MHz, d₆-DMSO): δ= 7,740(2,9);7,722(2,9);7,438(2,6);7,411(2,6);4,496(10,4) ;3,919(1,2);3,893(3,8);3,868(3,9);3,842(1,4);3,317(21,4);3,208(1,0);2,993(9,2);2,67 1(0,3);2,505(38,3);2,502(50,4);2,498(42,0);2,450(16,0);2,328(0,3);2,073(0,8);1,984( 0,5);1,967(0,9);1,950(1,2);1,933(1,0);1,916(0,5);0,869(14,8);0,852(14,7);0,000(1,7) |
| 125 | 1,79 | 1,75 | (400,0 MHz, d₆-DMSO): δ= 8,052(3,0);8,034(3,0);7,545(2,6);7,519(2,5);5,753(15,6) ;4,528(8,2);4,327(0,9);4,318(0,4);4,300(1,1);4,291(1,2);4,273(0,4);4,263(1,1);4,236( 0,4);3,884(1,1);3,874(0,4);3,857(1,2);3,847(1,0);3,831(0,5);3,820(1,0);3,794(0,3);3, 642(4,0);3,631(6,5);3,619(5,3);3,492(5,1);3,481(6,4);3,470(3,8);3,317(46,0);2,670(0 ,5);2,506(60,3);2,501(78,5);2,497(58,6);2,427(16,0);2,328(0,5);0,008(2,4);0,000(62, 8) |
| 126 | 2,66 | 2,6 | (400,0 MHz, d₆-DMSO): δ= 8,059(2,9);8,041(2,9);7,548(2,6);7,521(2,6);5,753(12,4) ;4,494(9,3);4,324(0,9);4,315(0,4);4,297(1,0);4,288(1,1);4,270(0,4);4,260(1,1);4,233( 0,3);3,904(0,9);3,894(0,4);3,877(1,0);3,867(0,9);3,850(0,4);3,840(0,9);3,317(39,3);3,216(1,4);3,000(7,8);2,670(0,4);2,505(49,1);2,501(63,0);2,430(16,0);2,328(0,4);1,98 4(0,5);1,967(0,9);1,950(1,2);1,934(1,0);1,916(0,5);1,235(0,5);0,872(15,4);0,855(15, 0);0,008(1,9);0,000(46,9) |
| 127 | 1,61 | 1,72 | (400,0 MHz, d₆-DMSO): δ= 13,321(0,7);8,314(0,6);7,902(16,0);7,898(13,8);7,893(9 ,5);7,886(9,4);7,740(1,3);7,721(1,5);7,716(4,8);7,713(5,3);7,711(4,8);7,708(4,2);7,6 96(5,9);7,693(6,3);7,691(6,3);7,565(8,5);7,558(1,4);7,551(1,5);7,544(14,1);7,535(1, 3);7,524(6,1);7,453(1,1);7,440(0,4);7,426(1,0);7,413(0,4);4,535(3,4);4,519(1,2);4,08 7(0,8);4,051(1,0);3,897(0,6);3,885(0,6);3,871(1,5);3,859(0,7);3,846(1,5);3,820(0,6); 3,738(2,1);3,648(0,5);3,639(0,6);3,614(0,7);3,602(0,6);3,586(0,5);3,578(0,5);3,538( 4,7);3,520(1,1);3,507(1,7);3,320(19,9);2,676(1,1);2,671(1,6);2,667(1,2);2,524(4,1);2 ,507(186,3);2,502(245,9);2,498(184,1);2,461(6,7);2,451(2,6);2,434(1,0);2,333(1,7); 2,329(2,0);2,325(1,4);1,337(0,5);1,298(1,2);1,259(2,0);1,235(5,5);1,203(0,7);1,187( 1,0);1,166(1,0);1,149(0,5);0,870(0,5);0,862(0,5);0,854(1,0);0,845(0,7);0,837(0,5);0, 826(0,6);0,146(1,0);0,008(7,5);0,000(207,3);-0,150(1,0) |
| 128 | 2,44 | 2,4 | (400,0 MHz, d₆-DMSO): δ= 7,742(3,0);7,724(3,0);7,447(2,6);7,420(2,6);4,543(5,8); 3,901(1,6);3,875(8,5);3,850(5,3);3,824(1,5);3,316(28,2);3,280(5,3);2,671(0,5);2,505 (56,3);2,501(72,1);2,497(54,9);2,455(16,0);2,328(0,5);2,073(8,8);1,235(0,7);0,007(2 ,4);0,000(52,2) |
| 129 | 3,29 | 3,27 | (400,0 MHz, d₆-DMSO): δ= 7,743(2,9);7,725(2,9);7,442(2,5);7,415(2,5);5,753(3,9); 4,534(8,0);3,909(1,2);3,884(3,8);3,858(3,9);3,832(1,4);3,584(3,2);3,570(4,8);3,556( 3,4);3,319(23,4);2,524(0,8);2,510(18,1);2,506(37,1);2,501(49,4);2,497(36,7);2,493( 18,7);2,452(15,7);2,328(0,3);2,105(1,2);2,084(2,0);2,073(16,0);2,055(1,9);2,034(1,3 );2,022(0,8);0,008(2,4);0,000(64,0);-0,009(3,0) |
| 130 | 2,60 | 2,58 | (400,0 MHz, d₆-DMSO): δ= 7,734(1,9);7,716(1,9);7,438(1,6);7,411(1,6);5,753(0,9); 4,498(7,6);3,918(0,8);3,892(2,5);3,867(2,6);3,841(0,9);3,317(25,5);2,973(16,0);2,50 6(36,5);2,501(47,2);2,497(35,0);2,447(10,4);0,008(0,4);0,000(9,1);-0,008(0,5) |
| 131 | 2,16 | 2,08 | (400,0 MHz, d₆-DMSO): δ= 8,033(2,6);8,015(2,6);7,547(2,2);7,521(2,1);5,753(0,7); 4,558(8,4);4,326(0,8);4,317(0,4);4,299(0,9);4,289(1,0);4,272(0,4);4,262(1,0);3,909( 0,9);3,899(0,4);3,882(1,0);3,872(0,9);3,855(0,4);3,845(0,8);3,317(17,0);2,950(16,0); 2,912(0,3);2,899(0,8);2,886(1,1);2,871(0,9);2,505(29,9);2,501(38,5);2,497(29,5);2,4 28(13,4);2,085(7,2);2,073(2,8);1,235(0,5);0,725(3,7);0,709(2,9);0,000(4,5) |
| 132 | 2,46 | 2,41 | (400,0 MHz, d₆-DMSO): δ= 8,062(3,1);8,044(3,1);7,550(2,6);7,523(2,5);5,753(13,0) ;4,533(6,9);4,332(1,0);4,322(0,4);4,305(1,1);4,295(1,2);4,277(0,5);4,268(1,2);4,240( 0,4);3,881(1,1);3,871(0,4);3,854(1,3);3,844(1,1);3,827(0,5);3,817(1,0);3,791(0,3);3, 590(3,6);3,577(5,4);3,563(3,7);3,316(34,0);2,675(0,3);2,670(0,4);2,666(0,3);2,506(5 3,2);2,501(69,4);2,497(52,2);2,428(16,0);2,332(0,4);2,328(0,5);2,323(0,4);2,104(1,4 );2,086(4,0);2,073(8,0);2,056(2,1);2,036(1,5);1,234(0,5);0,008(0,4);0,000(9,6) |
| 133 | 1,78 | 1,75 | (400,0 MHz, d₆-DMSO): δ= 8,052(2,1);8,033(2,1);7,548(1,7);7,522(1,7);5,753(0,7); 4,498(7,0);4,330(0,7);4,303(0,8);4,294(0,8);4,276(0,3);4,266(0,8);3,890(0,8);3,863( 0,9);3,853(0,7);3,836(0,3);3,826(0,7);3,317(22,7);2,978(16,0);2,505(35,6);2,501(45, 7);2,497(34,4);2,428(10,9);2,086(1,6);2,073(2,7);0,000(3,4) |
| 134 | 1,69 | 1,66 | (400,0 MHz, d₆-DMSO): δ= 8,063(3,0);8,045(2,9);7,553(2,7);7,526(2,6);5,753(3,8); 4,544(5,5);4,363(0,3);4,336(1,0);4,327(0,6);4,309(1,2);4,299(1,3);4,281(0,6);4,272( 1,2);4,245(0,4);3,872(7,0);3,845(1,8);3,835(1,3);3,817(0,7);3,808(1,1);3,781(0,4);3, 506(0,4);3,316(38,3);3,282(6,9);2,670(0,8);2,501(112,4);2,426(16,0);2,328(0,8);2,0 73(2,0);1,235(0,9);0,000(4,4) |
| 135 | 2,44 | 2,4 | (400,0 MHz, d₆-DMSO): δ= 8,326(0,9);8,310(1,9);8,294(0,9);8,037(2,7);8,019(2,7); 7,582(2,1);7,555(2,1);4,494(0,7);4,355(0,8);4,345(0,4);4,328(0,9);4,318(1,0);4,300( 0,4);4,291(1,0);4,264(0,3);4,129(0,4);4,106(1,1);4,089(1,3);4,083(1,3);4,066(1,1);4, 043(0,4);3,880(0,7);3,853(0,8);3,843(0,7);3,827(0,3);3,817(0,6);3,317(81,6);2,674(0 ,4);2,670(0,5);2,666(0,4);2,523(1,3);2,510(34,3);2,506(69,0);2,501(90,5);2,497(66,0 );2,435(13,5);2,332(0,4);2,328(0,6);2,323(0,4);2,073(16,0);0,008(1,2);0,000(32,8);-0,008(1,2) |
| 136 | 2,43 | 2,4 | (400,0 MHz,d₆-DMSO): δ= 8,327(0,8);8,311(1,7);8,294(0,8);8,037(2,5);8,019(2,5); 7,582(2,0);7,555(1,9);4,495(0,6);4,493(0,6);4,355(0,8);4,345(0,3);4,328(0,9);4,318(0,9);4,301(0,4);4,291(0,9);4,129(0,3);4,106(1,0);4,089(1,1);4,083(1,2);4,065(1,0);4, 043(0,3);3,880(0,6);3,853(0,7);3,843(0,6);3,816(0,6);3,318(40,5);2,670(0,4);2,523(1 ,0);2,510(25,0);2,506(50,7);2,501(66,5);2,496(47,9);2,492(22,9);2,435(12,5);2,328( 0,4);2,073(16,0);0,008(1,0);0,000(26,4);-0,009(0,9) |

Ferner wurden gemäß den zuvor beschriebenen Verfahren die folgenden Verbindungen der Formel (II) hergestellt (vgl. Tabelle 3).

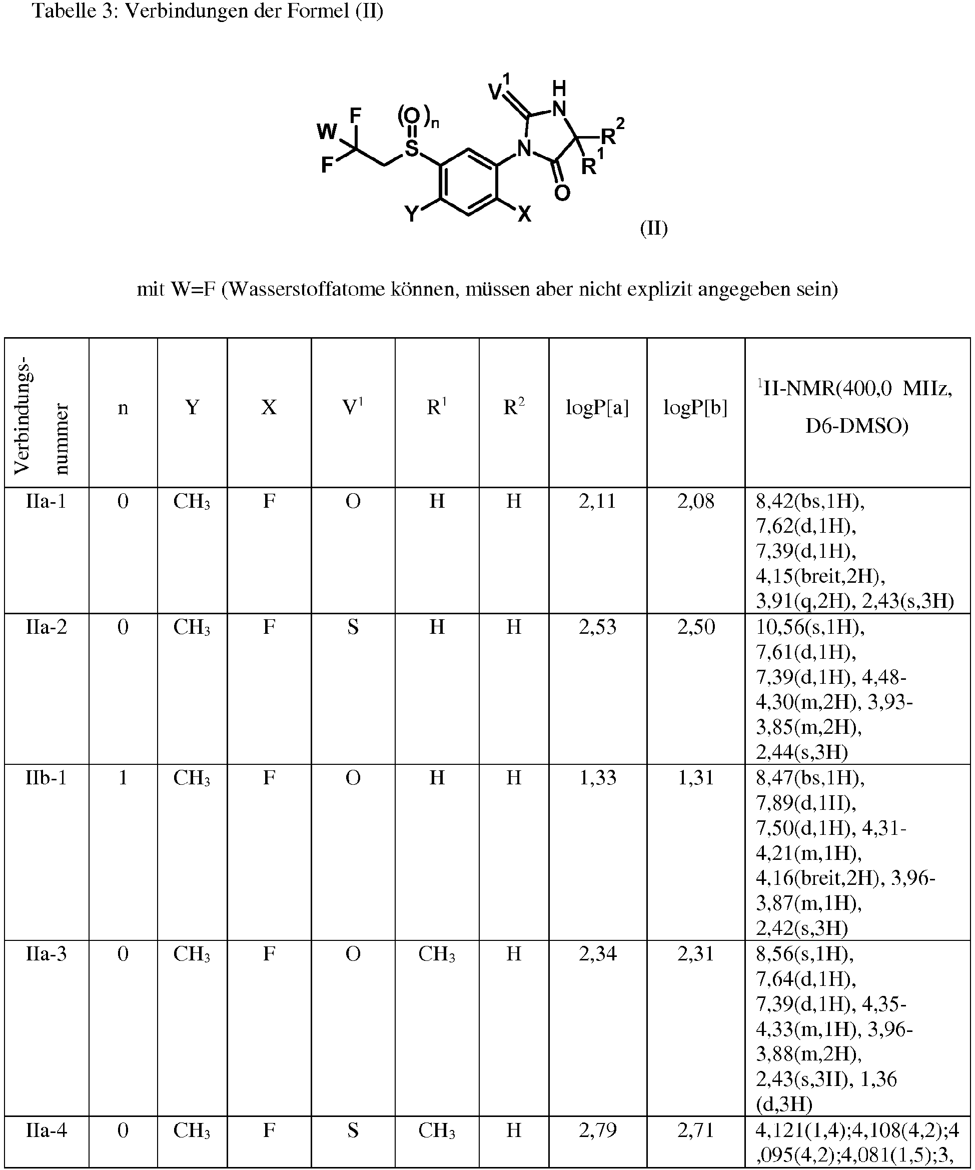

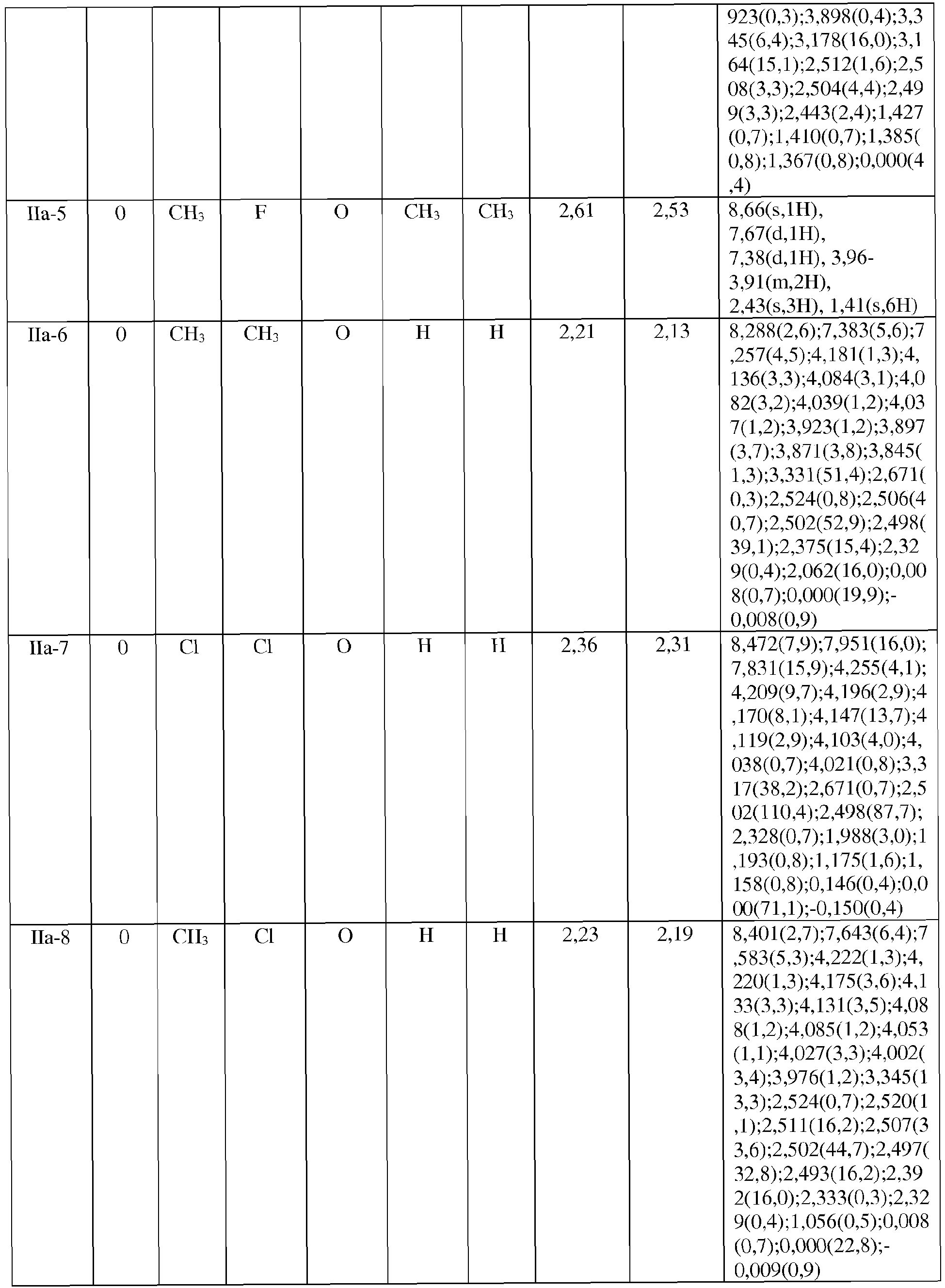

### Anwendungsbeispiele:

### 1. Boophilus microplus -Iniektionstest

| | |
|---|---|
| Lösungsmittel: | Dimethylsulfoxid |

Zur Herstellung einer zweckmäßigen Wirkstoffzubereitung vermischt man 10 mg Wirkstoff mit 0,5 ml Lösungsmittel und verdünnt das Konzentrat mit Lösungsmittel auf die gewünschte Konzentration.

1µl der Wirkstofflösung wird in das Abdomen von 5 vollgesogenen, adulten, weiblichen Rinderzecken *(Boophilus microplus)* injiziert. Die Tiere werden in Schalen überführt und in einem klimatisierten Raum aufbewahrt.

Die Wirkungskontrolle erfolgt nach 7 Tagen auf Ablage fertiler Eier. Eier, deren Fertilität nicht äußerlich sichtbar ist, werden bis zum Larvenschlupf nach etwa 42 Tagen im Klimaschrank aufbewahrt. Eine Wirkung von 100 % bedeutet, dass keine der Zecken fertile Eier gelegt hat, 0% bedeutet, dass alle Eier fertil sind.

Bei diesem Test zeigten z.B. die folgenden Verbindungen der Herstellungsbeispiele eine Wirkung von 100% bei einer Aufwandmenge von 20µg/Tier: 1, 6, 7, 9, 10, 15, 21, 23, 27, 28, 29, 36, 41, 42, 43, 46, 47, 48, 54, 62, 63, 68, 72, 79, 88, 89, 91, 99

### 2. Meloidogyne incognita- Test

| | |
|---|---|
| Lösungsmittel: | 125,0 Gewichtsteile Aceton |

Zur Herstellung einer zweckmäßigen Wirkstoffzubereitung vermischt man 1 Gewichtsteil Wirkstoff mit der angegebenen Menge Lösungsmittel und verdünnt das Konzentrat mit Wasser auf die gewünschte Konzentration.

Gefäße werden mit Sand, Wirkstofflösung, einer Ei-Larven-Suspension des südlichen Wurzelgallenälchens (*Meloidogyne incognita*) und Salatsamen gefüllt. Die Salatsamen keimen und die Pflänzchen entwickeln sich. An den Wurzeln entwickeln sich die Gallen.

Nach 14 Tagen wird die nematizide Wirkung anhand der Gallenbildung in % bestimmt. Dabei bedeutet 100 %, dass keine Gallen gefunden wurden; 0 % bedeutet, dass die Zahl der Gallen an den behandelten Pflanzen der unbehandelten Kontrolle entspricht.

Bei diesem Test zeigten z. B. die folgenden Verbindungen der Herstellungsbeispiele Wirkung von 100% bei einer Aufwandmenge von 20ppm: 9, 10, 20, 23, 43, 47, 54, 78, 125, 126, 128, 129, 130, 131, 132

Bei diesem Test zeigten z. B. die folgenden Verbindungen der Herstellungsbeispiele Wirkung von 90% bei einer Aufwandmenge von 20ppm: 1, 6, 7, 21, 36, 42, 48, 50, 68, 71, 72, 99, 101, 103, 108, 121, 122

### 3. Mvzus persicae - Sprühtest

| | |
|---|---|
| Lösungsmittel: | 78 Gewichtsteile Aceton |
| | 1,5 Gewichtsteile Dimethylformamid |
| Emulgator: | Alkylarylpolyglykolether |

Zur Herstellung einer zweckmäßigen Wirkstoffzubereitung löst man 1 Gewichtsteil Wirkstoff mit den angegebenen Gewichtsteilen Lösungsmittel und füllt mit Wasser, welches eine Emulgatorkonzentration von 1000 ppm enthält, bis zum Erreichen der gewünschten Konzentration auf. Zur Herstellung weiterer Testkonzentrationen wird mit emulgatorhaltigem Wasser verdünnt.

Chinakohlblattscheiben (*Brassica pekinensis*), die von allen Stadien der Grünen Pfirsichblattlaus (*Myzus persicae*) befallen sind, werden mit einer Wirkstoffzubereitung der gewünschten Konzentration gespritzt.

Nach 6 Tagen wird die Wirkung in % bestimmt. Dabei bedeutet 100 %, dass alle Blattläuse abgetötet wurden; 0 % bedeutet, dass keine Blattläuse abgetötet wurden.

Bei diesem Test zeigten z. B. die folgenden Verbindungen der Herstellungsbeispiele Wirkung von 90% bei einer Aufwandmenge von 500g/ha: 46

### 4. Phaedon cochleariae - Sprühtest

| | |
|---|---|
| Lösungsmittel: | 78,0 Gewichtsteile Aceton |
| | 1,5 Gewichtsteile Dimethylformamid |
| Emulgator: | Alkylarylpolyglykolether |

Zur Herstellung einer zweckmäßigen Wirkstoffzubereitung löst man 1 Gewichtsteil Wirkstoff mit den angegebenen Gewichtsteilen Lösungsmittel und füllt mit Wasser, welches eine Emulgatorkonzentration von 1000 ppm enthält, bis zum Erreichen der gewünschten Konzentration auf. Zur Herstellung weiterer Testkonzentrationen wird mit emulgatorhaltigem Wasser verdünnt.

Chinakohlblattscheiben *(Brassica pekinensis)* werden mit einer Wirkstoffzubereitung der gewünschten Konzentration gespritzt und nach dem Abtrocknen mit Larven des Meerrettichblattkäfers *(Phaedon cochleariae)* besetzt.

Nach 7 Tagen wird die Wirkung in % bestimmt. Dabei bedeutet 100 %, dass alle Käferlarven abgetötet wurden; 0 % bedeutet, dass keine Käferlarven abgetötet wurden.

Bei diesem Test zeigten z. B. die folgenden Verbindungen der Herstellungsbeispiele Wirkung von 100% bei einer Aufwandmenge von 500g/ha: 38, 39, 44, 54, 71, 72, 79, 118

Bei diesem Test zeigten z. B. die folgenden Verbindungen der Herstellungsbeispiele Wirkung von 83% bei einer Aufwandmenge von 500g/ha: 30, 37, 43, 48, 53, 101, 106

### 5. Tetranychus urticae - Sprühtest, OP-resistent

| | |
|---|---|
| Lösungsmittel: | 78,0 GewichtsteileAceton |
| | 1,5 Gewichtsteile Dimethylformamid |
| Emulgator: | Alkylarylpolyglykolether |

Zur Herstellung einer zweckmäßigen Wirkstoffzubereitung löst man 1 Gewichtsteil Wirkstoff mit den angegebenen Gewichtsteilen Lösungsmittel und füllt mit Wasser, welches eine Emulgatorkonzentration von 1000 ppm enthält, bis zum Erreichen der gewünschten Konzentration auf. Zur Herstellung weiterer Testkonzentrationen wird mit emulgatorhaltigem Wasser verdünnt.

Bohnenblattscheiben *(Phaseolus vulgaris),* die von allen Stadien der Gemeinen Spinnmilbe *(Tetranychus urticae)* befallen sind, werden mit einer Wirkstoffzubereitung der gewünschten Konzentration gespritzt.

Nach 6 Tagen wird die Wirkung in % bestimmt. Dabei bedeutet 100 %, dass alle Spinnmilben abgetötet wurden; 0 % bedeutet, dass keine Spinnmilben abgetötet wurden.

Bei diesem Test zeigten z. B. die folgenden Verbindungen der Herstellungsbeispiele eine Wirkung von 100% bei einer Aufwandmenge von 500g/ha: 1, 3, 4, 6, 7, 9, 10, 12, 14, 15, 16, 20, 21, 22, 24, 25, 26, 27, 28, 29, 32, 33, 34, 36, 37, 38, 39, 40, 41, 42, 43, 44, 45, 46, 47, 48, 49, 50, 51, 52, 53, 54, 57, 58, 59, 60, 61, 62, 63, 64, 66, 67, 68, 70, 71, 72, 74, 76, 77, 78, 79, 80, 81, 82, 84, 85, 86, 87, 88, 89, 90, 91, 94, 100, 101, 102, 104, 105, 106, 108, 110, 111, 112, 113, 114, 115, 116, 117, 120, 121, 122, 123, 125, 126, 127, 129, 130, 131, 133, IIa-2, IIb-1

Bei diesem Test zeigten z. B. die folgenden Verbindungen der Herstellungsbeispiele eine Wirkung von 90% bei einer Aufwandmenge von 500g/ha: 13, 18, 19, 30, 31, 55, 59, 76, 83, 92, 97, 98, 103, 109, 130, 132, 134, IIa-1

Bei diesem Test zeigten z. B. die folgenden Verbindungen der Herstellungsbeispiele eine Wirkung von 90% bei einer Aufwandmenge von 100g/ha: 65, 69, 99

### 6. Tetranychus urticae- Sprühtest; OP-resistent

| | |
|---|---|
| Lösungsmittel: | 7 Gewichtsteile Dimethylformamid |
| Emulgator: | Alkylarylpolyglykolether |

Zur Herstellung einer zweckmäßigen Wirkstoffzubereitung löst man 1 Gewichtsteil Wirkstoff mit den angegebenen Gewichtsteilen Lösungsmittel und füllt mit Wasser, welches eine Emulgatorkonzentration von 1000 ppm enthält, bis zum Erreichen der gewünschten Konzentration auf. Zur Herstellung weiterer Testkonzentrationen wird mit emulgatorhaltigem Wasser verdünnt. Bei erforderlicher Zugabe von Ammoniumsalzen oder/und Penetrationsförderern werden diese jeweils in einer Konzentration von 1000 ppm der Präparatelösung zugefügt.

Bohnenpflanzen (*Phaseolus vulgaris*), die stark von allen Stadien der Gemeinen Spinnmilbe (*Tetranychus urticae*) befallen sind, werden durch Spritzen mit der Wirkstoffzubereitung in der gewünschten Konzentration behandelt.

Nach 7 Tagen wird die Wirkung in % bestimmt. Dabei bedeutet 100 %, dass alle Spinnmilben abgetötet wurden; 0 % bedeutet, dass keine Spinnmilben abgetötet wurden.

Bei diesem Test zeigten z. B. die folgenden Verbindungen der Herstellungsbeispiele Wirkung von 100% bei einer Aufwandmenge von 20ppm: 136

## Patentansprüche

1. Verbindung der Formel (I) 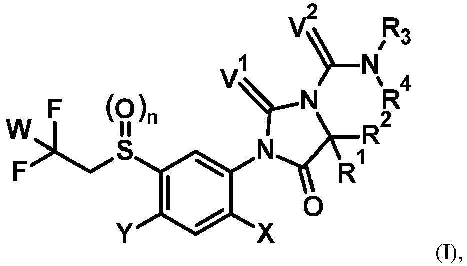 worin
W für Wasserstoff oder Halogen steht;
n für die Zahl 0, 1 oder 2 steht;
Y für Wasserstoff, Halogen, (C₁-C₆)Alkyl, Halogen(C₁-C₆)alkyl, (C₁-C₆)Alkoxy, Halogen(C₁-C₆)alkoxy oder Amino steht; oder
für NR"'R'''' steht,
wobei R'" und R'''' unabhängig voneinander für Wasserstoff, (C₁-C₆)Alkyl, oder Halogen(C₂-C₆)alkyl stehen;
X für Wasserstoff, Halogen, Cyano, (C₁-C₆)Alkyl, Halogen(C₁-C₆)alkyl oder (C₁-C₆)Alkoxy steht;
V¹ und V² unabhängig voneinander für Sauerstoff oder Schwefel stehen;
R¹ und R² unabhängig voneinander
für Wasserstoff, Halogen, Hydroxy, Cyano oder Nitro stehen; oder
für Alkyl, Alkenyl, Alkinyl, Alkoxy, Alkylsulfanyl, Alkylsulfinyl, Alkylsulfonyl, Halogenalkylcarbonyl, Alkylcarbonyl oder Alkoxycarbonyl stehen, wobei die vorgenannten Reste gegebenenfalls durch Halogen, Alkyl, Cycloalkyl, Cyano, Nitro, Alkoxy, Halogenalkyl oder Halogenalkoxy, insbesondere Fluor, Chlor, (C₁-C₃)Alkyl, (C₃-C₆)Cycloalkyl, Cyclopropyl, Cyano, (C₁-C₃)Alkoxy, (C₁-C₃)Halogenalkyl oder (C₁-C₃)Halogenalkoxy, substituiert sein können;
oder R¹ und R² bilden einen gegebenenfalls durch Halogen, Alkyl, Cycloalkyl, Cyano, Nitro, Alkoxy, Halogenalkyl oder Halogenalkoxy, insbesondere Fluor, Chlor, (C₁-C₃)Alkyl, (C₃-C₆)Cycloalkyl, Cyclopropyl, Cyano, (C₁-C₃)Alkoxy, (C₁-C₃)Halogenalkyl oder (C₁-C₃)Halogenalkoxy, substituierten und gegebenenfalls durch ein oder mehrere Heteroatome, welche unabhängig ausgewählt werden aus der Gruppe bestehend aus O, S und N mit der Maßgabe, dass zwei Sauerstoffatome nicht unmittelbar benachbart zueinander stehen, unterbrochenen gesättigten oder ungesättigten drei- bis sechs-gliedrigen Ring;
R³ für Alkyl, Halogenalkyl, Alkoxyalkyl, Halogenalkoxyalkyl, Alkenyl, Halogenalkenyl, Alkinyl, Halogenalkinyl, Alkyl-S(O)ₘ-alkyl, Halogenalkyl-S(O)ₘ-alkyl, *N*-Alkylaminocarbonylalkyl oder *N*,*N*-Dialkylaminocarbonylalkyl steht, oder
für gegebenenfalls einfach oder zweifach, gleich oder verschieden durch Halogen, Cyano, Nitro, Hydroxy, Amino, (C₁-C₄)Alkyl, Halogen(C₁-C₃)Alkyl, (C₁-C₃)Alkoxy, Halogen(C₁-C₃)Alkoxy oder Cyclopropyl substituiertes Cycloalkyl, Cycloalkylalkyl, Cycloalkenyl oder Cycloalkenylalkyl steht, oder
für gegebenenfalls einfach oder zweifach, gleich oder verschieden durch Halogen, Cyano, Nitro, Hydroxy, Amino, (C₁-C₄)Alkyl, Halogen(C₁-C₃)Alkyl, (C₁-C₃)Alkoxy, Halogen(C₁-C₃)Alkoxy oder Cyclopropyl substituiertes Heterocyclyl, Heterocyclylalkyl, Aryl, Arylalkyl, Hetaryl oder Hetarylalkyl steht;
R⁴ für Wasserstoff, Alkyl, Halogenalkyl, Alkoxyalkyl, Halogenalkoxyalkyl, Alkenyl, Halogenalkenyl, Alkinyl, Halogenalkinyl, Alkyl-S(O)ₘ-alkyl, Halogenalkyl-S(O)ₘ-alkyl, *N*-Alkylaminocarbonylalkyl oder *N*,*N-*Dialkylaminocarbonylalkyl steht, oder
für gegebenenfalls einfach oder zweifach, gleich oder verschieden durch Halogen, Cyano, Nitro, Hydroxy, Amino, (C₁-C₄)Alkyl, Halogen(C₁-C₃)Alkyl, (C₁-C₃)Alkoxy, Halogen(C₁-C₃)Alkoxy oder Cyclopropyl substituiertes Cycloalkyl, Cycloalkylalkyl, Cycloalkenyl oder Cycloalkenylalkyl steht, oder
für gegebenenfalls einfach oder zweifach, gleich oder verschieden durch Halogen, Cyano, Nitro, Hydroxy, Amino, (C₁-C₄)Alkyl, Halogen(C₁-C₃)Alkyl, (C₁-C₃)Alkoxy, Halogen(C₁-C₃)Alkoxy oder Cyclopropyl substituiertes Heterocyclyl, Heterocyclylalkyl, Aryl, Arylalkyl, Hetaryl oder Hetarylalkyl steht;
oder R³ und R⁴ bilden gemeinsam mit dem Stickstoffatom, an das sie gebunden sind, einen gegebenenfalls durch Halogen, Cyano, (C₁-C₄)Alkyl, Halogen(C₁-C₄)alkyl, (C₁-C₄)Alkoxy, Halogen(C₁-C₄)alkoxy oder (C₃-C₆)Cycloalkyl substituierten gesättigten bis dreifach ungesättigten 3- bis 6-gliedrigen Ring;
m für die Zahl 0, 1 oder 2 steht.

2. Verbindung nach Anspruch 1, worin die Strukturelemente wie folgt definiert sind:
W steht für Wasserstoff oder Halogen;
n steht für die Zahl 0 oder 1;
Y steht für Wasserstoff, Halogen, (C₁-C₃)Alkyl, (C₁-C₃)Halogenalkyl, (C₁-C₃)Alkoxy, (C₁-C₃)Halogenalkoxy oder Amino; oder
für NR"'R'''',
wobei R'" und R'''' unabhängig voneinander für Wasserstoff, (C₁-C₄)Alkyl, oder (C₂-C₄)Halogenalkyl stehen;
X steht für Wasserstoff, Halogen, Cyano, (C₁-C₃)Alkyl, (C₁-C₃)Halogenalkyl oder (C₁-C₃)Alkoxy;
V¹ und V² stehen unabhängig voneinander für Sauerstoff oder Schwefel;
R¹ und R² stehen unabhängig voneinander für Wasserstoff oder (C₁-C₃)Alkyl;
oder R¹ und R² bilden gemeinsam mit dem Kohlenstoffatom, an das sie gebunden sind, einen (C₃-C₆)Cycloalkyl-Ring;
R³ steht für (C₁-C₆)Alkyl, Halogen(C₁-C₃)alkyl, (C₁-C₃)Alkoxy(C₁-C₃)alkyl, Halogen(C₁-C₃)alkoxy(C₁-C₃)alkyl, (C₁-C₆)Alkenyl, Halogen(C₁-C₃)alkenyl, (C₁-C₆)Alkinyl, Halogen(C₁-C₃)alkinyl, (C₁-C₃)Alkyl-S(O)ₘ-(C₁-C₃)alkyl, Halogen(C₁-C₃)Alkyl-S(O)ₘ-(C₁-C₃)alkyl, *N*-(C₁-C₃)Alkylaminocarbonyl(C₁-C₃)alkyl oder *N*,*N*-Di(C₁-C₃)Alkylaminocarbonyl(C₁-C₃)alkyl, oder
für gegebenenfalls einfach oder zweifach, gleich oder verschieden durch Halogen, Cyano, Nitro, Hydroxy, Amino, (C₁-C₄)Alkyl, Halogen(C₁-C₃)Alkyl, (C₁-C₃)Alkoxy, Halogen(C₁-C₃)Alkoxy oder Cyclopropyl substituiertes (C₃-C₆)Cycloalkyl, (C₃-C₆)Cycloalkyl(C₁-C₃)alkyl, (C₃-C₆)Cycloalkenyl oder (C₃-C₆)Cycloalkenyl(C₁-C₃)alkyl, oder
für gegebenenfalls einfach oder zweifach, gleich oder verschieden durch Halogen, Cyano, Nitro, Hydroxy, Amino, (C₁-C₄)Alkyl, Halogen(C₁-C₃)Alkyl, (C₁-C₃)Alkoxy, Halogen(C₁-C₃)Alkoxy oder Cyclopropyl substituiertes Heterocyclyl, Heterocyclyl(C₁-C₃)alkyl, Aryl, Aryl(C₁-C₃)alkyl, Hetaryl oder Hetaryl(C₁-C₃)alkyl;
R⁴ steht für Wasserstoff, (C₁-C₆)Alkyl, Halogen(C₁-C₃)alkyl oder (C₁-C₃)Alkoxy(C₁-C₃)alkyl, oder
für gegebenenfalls einfach oder zweifach, gleich oder verschieden durch Halogen, Cyano, Nitro, Hydroxy, Amino, (C₁-C₄)Alkyl, Halogen(C₁-C₃)Alkyl, (C₁-C₃)Alkoxy, Halogen(C₁-C₃)Alkoxy oder Cyclopropyl substituiertes (C₃-C₆)Cycloalkyl, (C₃-C₆)Cycloalkyl(C₁-C₃)alkyl, Phenyl, Phenyl(C₁-C₃)alkyl, Pyridyl oder Pyridyl(C₁-C₃)alkyl;
oder R³ und R⁴ bilden gemeinsam mit dem Stickstoffatom, an das sie gebunden sind, einen gegebenenfalls durch Halogen, Cyano, (C₁-C₄)Alkyl, Halogen(C₁-C₄)alkyl, (C₁-C₄)Alkoxy, Halogen(C₁-C₄)alkoxy, (C₃-C₆)Cycloalkyl substituierten gesättigten bis dreifach ungesättigten 3- bis 6-gliedrigen Ring, der ausgewählt ist aus Aziridinyl, Azirenyl, Diaziridinyl, Diazirenyl, Azetidinyl, Dihydroazetyl, Diazetidinyl, Dihydrodiazetyl, Oxazetidinyl, Oxazetyl, Thiazetidinyl, Thiazetyl, Pyrrolidinyl, Dihydropyrrolyl, Pyrazolidinyl, Dihydropyrazolyl, Imidazolidinyl, Dihydroimidazolyl, Oxazolidinyl, Dihydrooxazolyl, Thiazolidinyl, Dihydrothyazolyl, Piperidinyl, Piperazinyl, Hexahydropyridazinyl, Hexahydropyrimidinyl, Morpholin, Dioxazinanyl, Thiomorpholin, Dithiazinan, Dioxothiazinan, Pyrrolyl, Pyrazolyl, Imidazolyl, Triazolyl und Tetrazolyl.
m steht für die Zahl 0, 1 oder 2.

3. Verbindung nach Anspruch 1 oder 2, worin die Strukturelemente wie folgt definiert sind:
W steht für Wasserstoff oder Fluor;
n steht für die Zahl 0 oder 1;
Y steht für Fluor, Chlor, Brom, Methyl, Trifluoromethyl oder Methoxy;
X steht für Wasserstoff, Chlor, Fluor oder Methyl;
insbesondere wobei X und Y für folgende Kombinationen (Y,X) stehen: (Me, F), (Me,H), (Me,Cl), (Me,Me), (Cl,Cl), (Cl,F), (MeO,F), (MeO,H), (Cl,H), (Br,H), (Br,F), (F,F), (CF₃,H), (CF₃,F), besonders bevorzugt für (Me, F), (Me,Cl), (Me,Me), (Cl,Cl);
V¹ und V² stehen unabhängig voneinander für Sauerstoff oder Schwefel;
R¹ und R² stehen unabhängig voneinander für Wasserstoff, Methyl oder Ethyl;
oder R¹ und R² bilden einen Cyclopropyl- oder Cyclobutyl-Ring;
R³ steht für Methyl, Ethyl, n-Propyl, Isopropyl, n-Butyl, Isobutyl, sec-Butyl, tert-Butyl, 1,1-Dimethylpropyl, 2,2,2-Trifluorethyl, 2,2-Difluorethyl, 2,2-Difluor-n-propyl, 2-Methoxyethyl, 2-Ethoxyethyl, 3-Methoxypropyl, 3-Ethoxypropyl, Dimethylaminocarbonylmethyl, Diethylaminocarbonylmethyl, *N*-Ethyl-*N-*methylaminocarbonylmethyl, *N*-Isopropyl-*N*-methylaminocarbonylmethyl, Dimethylaminocarbonylethyl, Diethylaminocarbonylethyl, *N*-Ethyl-*N-*methylaminocarbonylethyl, *N*-Isopropyl-*N*-methylaminocarbonylethyl, *N*-Cyclopropyl-*N*-methylaminocarbonylmethyl oder *N*-Cyclopropyl-*N*-methylaminocarbonylethyl, oder
für Cyclopropyl, Cyclobutyl, Cyclopentyl, Cyclohexyl, 2-Tetrahydrofurylmethyl, 3-Tetrahydrofurylmethyl, 2-Tetrahydrofurylethyl oder 3-Tetrahydrofurylethyl, oder
für gegebenenfalls einfach oder zweifach, gleich oder verschieden durch Fluor, Chlor, Brom, Cyano, Nitro, Hydroxy, Amino, Methyl, Ethyl, Isopropyl, tert-Butyl, Cyclopropyl, Trifluormethyl, Difluormethyl, Methoxy, Trifluormethoxy oder Difluormethoxy substituiertes Phenyl, oder
für gegebenenfalls einfach oder zweifach, gleich oder verschieden durch Fluor, Chlor, Brom, Cyano, Nitro, Hydroxy, Amino, Methyl, Ethyl, Isopropyl, tert-Butyl, Cyclopropyl, Trifluormethyl, Difluormethyl, Methoxy, Trifluormethoxy oder Difluormethoxy substituiertes Pyridyl, oder
für gegebenenfalls einfach oder zweifach, gleich oder verschieden durch Fluor, Chlor, Brom, Cyano, Nitro, Hydroxy, Amino, Methyl, Ethyl, Isopropyl, tert-Butyl, Trifluormethyl, Difluormethyl, Methoxy, Trifluormethoxy oder Difluormethoxy substituiertes Benzyl oder Pyridylmethyl;
R⁴ steht für Wasserstoff, Methyl, Ethyl, n-Propyl, Isopropyl, n-Butyl, Isobutyl, tert-Butyl, sec-Butyl, 2,2,2-Trifluorethyl, 2,2-Difluorethyl, 2-Methoxyethyl, 2-Ethoxyethyl, Cyclopropyl, Cyclobutyl, Cyclopropylmethyl, Phenyl oder Benzyl;
oder R³ und R⁴ bilden bilden gemeinsam mit dem Stickstoffatom, an das sie gebunden sind, einen Azetidin-, Oxetan-, Thiethan-, Morpholin-, Thiomorpholin-, Dioxothiazinan-, Piperidin- oder einen *N*-Methyl substituierten Piperazinring.

4. Verbindung nach einem der Ansprüche 1 bis 3, worin die Strukturelemente wie folgt definiert sind:
W steht für Fluor;
n steht für die Zahl 0 oder 1;
Y steht für Chlor oder Methyl;
X steht für Wasserstoff, Fluor, Chlor oder Methyl;
insbesondere wobei X und Y für folgende Kombinationen (Y,X) stehen: (Me,Cl), (Me, F), (Me,Me), (Cl,Cl);
V¹ und V² stehen unabhängig voneinander für Sauerstoff oder Schwefel;
R¹ und R² stehen unabhängig voneinander für Wasserstoff oder Methyl;
R³ steht für Methyl, Ethyl, Isopropyl, Isobutyl, sec-Butyl, tert-Butyl, 1,1-Dimethylpropyl, 2,2,2-Trifluorethyl, 2-Methoxyethyl, oder
für Cyclopropyl oder 2-Tetrahydrofurylmethyl, oder
für Phenyl, 3-Fluorphenyl, 4-Fluorphenyl, 3-Chlorphenyl, 3-Trifluormethylphenyl, 2-Pyridyl, 3-Pyridyl oder Benzyl;
R⁴ steht für Wasserstoff oder Methyl;
oder R³ und R⁴ bilden bilden gemeinsam einen der folgenden Ringe: 1-Morpholin, 1-(4-Methylpiperazin), 1-(1,1-Dioxo-1,4-thiazinan) oder 1-(4,4-Difluorpiperidin).

5. Verbindung nach einem der Ansprüche 1 bis 4, **dadurch gekennzeichnet, dass** sie eine Struktur gemäß Formel (I-A) aufweist 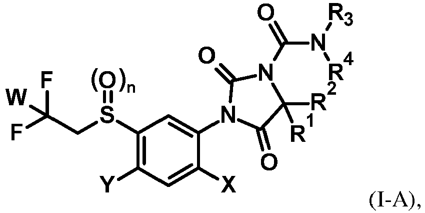 in der W, n, m, Y, X, R¹, R², R³ und R⁴ die in Anspruch 1 definierten Bedeutungen oder die in Anspruch 2 definierten Bedeutungen oder die in Anspruch 3 definierten Bedeutungen oder die in Anspruch 4 definierten Bedeutungen haben.

6. Verbindung der Formel (II) 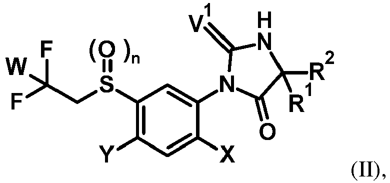 worin n, W, Y, X, V¹, R¹ und R² die in Anspruch 1 definierten Bedeutungen oder die in Anspruch 2 definierten Bedeutungen oder die in Anspruch 3 definierten Bedeutungen oder die in Anspruch 4 definierten Bedeutungen haben.

7. Verbindung der Formel (III) 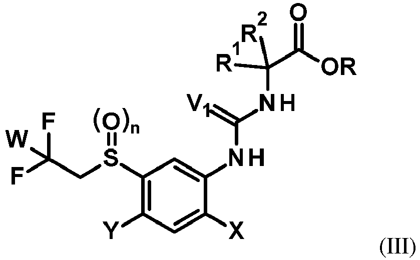 worin n, W, Y, X, V¹, R¹ und R² die in Anspruch 1 definierten Bedeutungen oder die in Anspruch 2 definierten Bedeutungen oder die in Anspruch 3 definierten Bedeutungen oder die in Anspruch 4 definierten Bedeutungen haben, und R für Wasserstoff oder eine Alkylgruppe, bevorzugt Methyl oder Ethyl, steht.

8. Verbindung der Formel (VI) 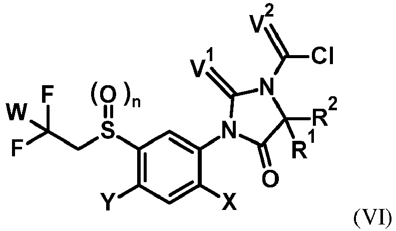 worin n, W, Y, X, V¹, V², R¹ und R² die in Anspruch 1 definierten Bedeutungen oder die in Anspruch 2 definierten Bedeutungen oder die in Anspruch 3 definierten Bedeutungen oder die in Anspruch 4 definierten Bedeutungen haben.

9. Formulierung, insbesondere agrochemische Formulierung, umfassend mindestens eine Verbindung der Formel (I) gemäß einem der Ansprüche 1 bis 5.

10. Formulierung nach Anspruch 9 ferner umfassend mindestens ein Streckmittel und/oder mindestens eine oberflächenaktive Substanz.

11. Formulierung nach Anspruch 9 oder 10, **dadurch gekennzeichnet, dass** die Verbindung der Formel (I) in Mischung mit mindestens einem weiteren Wirkstoff vorliegt.

12. Verfahren zur Bekämpfung von Schädlingen, insbesondere tierischen Schädlingen, **dadurch gekennzeichnet, dass** man eine Verbindung der Formel (I) gemäß einem der Ansprüche 1 bis 5 oder eine Formulierung gemäß einem der Ansprüche 9 bis 11 auf die Schädlinge und/oder ihren Lebensraum einwirken lässt.

13. Verfahren nach Anspruch 12, **dadurch gekennzeichnet, dass** der Schädling ein tierischer Schädling ist und ein Insekt, einen Akariden oder einen Nematoden umfasst, oder dass der Schädling ein Insekt, ein Akaride oder ein Nematode ist.

14. Verbindung der Formel (I) gemäß einem der Ansprüche 1 bis 5 oder einer Formulierung gemäß einem der Ansprüche 9 bis 11 zur Herstellung eines Wirkstoffs oder Arzneimittels zur Bekämpfung von tierischen Schädlingen.

15. Verbindung zur Herstellung eines Wirkstoffs oder Arzneimittels nach Anspruch 14, **dadurch gekennzeichnet, dass** der tierische Schädling ein Insekt, einen Akariden oder einen Nematoden umfasst, oder dass der tierische Schädling ein Insekt, ein Akaride oder ein Nematode ist.

16. Verbindung nach Anspruch 14 oder 15 zur Verwendung im Pflanzenschutz.

17. Verbindung der Formel (I) gemäß einem der Ansprüche 1-5 zur Herstellung eines Arzneimittels zur Verwendung auf dem Gebiet der Tiergesundheit.

18. Verfahren zum Schutz eines Saatgutes oder einer keimenden Pflanze vor Schädlingen, insbesondere tierischen Schädlingen, umfassend einen Verfahrensschritt, in welchem das Saatgut in Kontakt mit einer Verbindung der Formel (I) gemäß einem der Ansprüche 1 bis 5 oder mit einer Formulierung gemäß einem der Ansprüche 9 bis 11 gebracht wird.

## Claims

1. Compound of formula (I) 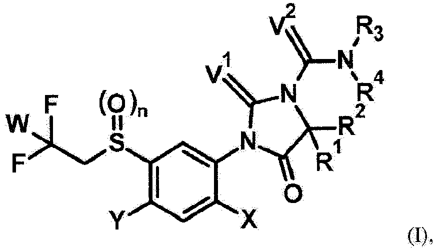 wherein
W represents hydrogen or halogen;
n represents the number 0, 1 or 2;
Y represents hydrogen, halogen, (C₁-C₆)-alkyl, halo(C₁-C₆)alkyl, (C₁-C₆)alkoxy, halo(C₁-C₆)alkoxy or amino; or
represents NR'''R'''',
wherein R''' and R'''' independently of one another represent hydrogen, (C₁-C₆)alkyl or halo (C₂-C₆)alkyl;
X represents hydrogen, halogen, cyano, (C₁-C₆)alkyl, halo (C₁-C₆)alkyl or (C₁-C₆) alkoxy;
V¹ and V² independently of one another represent oxygen or sulfur;
R¹ and R² independently of one another
represent hydrogen, halogen, hydroxy, cyano or nitro; or
represent alkyl, alkenyl, alkynyl, alkoxy, alkylsulfanyl, alkylsulfinyl, alkylsulfonyl, haloalkylcarbonyl, alkylcarbonyl or alkoxycarbonyl, wherein the above-mentioned radicals can be optionally substituted by halogen, alkyl, cycloalkyl, cyano, nitro, alkoxy, haloalkyl or haloalkoxy, in particular by fluorine, chlorine, (C₁-C₃)alkyl, (C₃-C₆)cycloalkyl, cyclopropyl, cyano, (C₁-C₃)alkoxy, (C₁-C₃)haloalkyl or (C₁-C₃)haloalkoxy;
or R¹ and R² form a saturated or unsaturated three-to six-membered ring optionally substituted by halogen, alkyl, cycloalkyl, cyano, nitro, alkoxy, haloalkyl or haloalkoxy, in particular by fluorine, chlorine, (C₁-C₃)alkyl, (C₃-C₆)-cycloalkyl, cyclopropyl, cyano, (C₁-C₃)-alkoxy, (C₁-C₃)haloalkyl or (C₁-C₃)haloalkoxy, and optionally interrupted by one or more heteroatoms selected independently from the group consisting of O, S and N, with the proviso that two oxygen atoms are not immediately adjacent to one another;
R³ represents alkyl, haloalkyl, alkoxyalkyl, haloalkoxyalkyl, alkenyl, haloalkenyl, alkynyl, haloalkynyl, alkyl-S(O)ₘ-alkyl, haloalkyl-S(O)ₘ-alkyl, *N-*alkylaminocarbonylalkyl or *N,N*-dialkylaminocarbonylalkyl, or
represents cycloalkyl, cycloalkylalkyl, cycloalkenyl or cycloalkenylalkyl optionally substituted by one or two identical or different substituents halogen, cyano, nitro, hydroxy, amino, (C₁-C₄)alkyl, halo (C₁-C₃) alkyl, (C₁-C₃)-alkoxy, halo(C₁-C₃)alkoxy or cyclopropyl, or
represents heterocyclyl, heterocyclylalkyl, aryl, arylalkyl, hetaryl or hetarylalkyl optionally substituted by one or two identical or different substituents halogen, cyano, nitro, hydroxy, amino, (C₁-C₄)alkyl, halo(C₁-C₃)-alkyl, (C₁-C₃)alkoxy, halo(C₁-C₃)alkoxy or cyclopropyl;
R⁴ represents hydrogen, alkyl, haloalkyl, alkoxyalkyl, haloalkoxyalkyl, alkenyl, haloalkenyl, alkynyl, haloalkynyl, alkyl-S(O)ₘ-alkyl, haloalkyl-S(O)ₘ-alkyl, *N*-alkylaminocarbonylalkyl or *N,N-*di-alkylaminocarbonylalkyl, or
represents cycloalkyl, cycloalkylalkyl, cycloalkenyl or cycloalkenylalkyl optionally substituted by one or two identical or different substituents halogen, cyano, nitro, hydroxy, amino, (C₁-C₄)alkyl, halo(C₁-C₃)alkyl, (C₁-C₃)-alkoxy, halo(C₁-C₃)alkoxy or cyclopropyl, or
represents heterocyclyl, heterocyclylalkyl, aryl, arylalkyl, hetaryl or hetarylalkyl optionally substituted by one or two identical or different substituents halogen, cyano, nitro, hydroxy, amino, (C₁-C₄)alkyl, halo(C₁-C₃)-alkyl, (C₁-C₃)alkoxy, halo(C₁-C₃)alkoxy or cyclopropyl;
or R³ and R⁴ form together with the nitrogen atom to which they are bonded a saturated to triunsaturated 3- to 6-membered ring optionally substituted by halogen, cyano, (C₁-C₄)alkyl, halo (C₁-C₄)alkyl, (C₁-C₄)alkoxy, halo(C₁-C₄)alkoxy or (C₃-C₆)cycloalkyl;
m represents the number 0, 1 or 2.

2. Compound according to Claim 1, wherein the structural elements are defined as follows:
W represents hydrogen or halogen;
n represents the number 0 or 1;
Y represents hydrogen, halogen, (C₁-C₃)alkyl, (C₁-C₃)haloalkyl, (C₁-C₃)alkoxy, (C₁-C₃)haloalkoxy or amino; or
represents NR'"R"",
wherein R'3 and R"" independently of one another represent hydrogen, (C₁-C₄)alkyl or (C₂-C₄)haloalkyl;
X represents hydrogen, halogen, cyano, (C₁-C₃)alkyl, (C₁-C₃)haloalkyl or (C₁-C₃)-alkoxy;
V¹ and V² independently of one another represent oxygen or sulfur;
R¹ and R² independently of one another represent hydrogen or (C₁-C₃)alkyl;
or R¹ and R² form together with the carbon atom to which they are bonded a (C₃-C₆)cycloalkyl ring;
R³ represents (C₁-C₆)alkyl, halo(C₁-C₃)alkyl, (C₁-C₃)alkoxy (C₁-C₃)alkyl, halo(C₁-C₃)alkoxy-(C₁-C₃)alkyl, (C₁-C₆)alkenyl, halo(C₁-C₃)-alkenyl, (C₁-C₆)alkynyl, halo(C₁-C₃)alkynyl, (C₁-C₃)alkyl-S(O)ₘ-(C₁-C₃)alkyl, halo(C₁-C₃)-alkyl-S(O)ₘ-(C₁-C₃)alkyl, *N*-(C₁-C₃)alkylaminocarbonyl(C₁-C₃)alkyl or *N,N*-di(C₁-C₃)alkylaminocarbonyl(C₁-C₃)alkyl, or
represents (C₃-C₆)cycloalkyl, (C₃-C₆)cycloalkyl(C₁-C₃)alkyl, (C₃-C₆)cycloalkenyl or (C₃-C₆)cycloalkenyl(C₁-C₃)alkyl optionally substituted by one or two identical or different substituents halogen, cyano, nitro, hydroxy, amino, (C₁-C₄)alkyl, halo(C₁-C₃)alkyl, (C₁-C₃)alkoxy, halo(C₁-C₃)alkoxy or cyclopropyl, or
represents heterocyclyl, heterocyclyl-(C₁-C₃)alkyl, aryl, aryl(C₁-C₃)alkyl, hetaryl or hetaryl(C₁-C₃)alkyl optionally substituted by one or two identical or different substituents halogen, cyano, nitro, hydroxy, amino, (C₁-C₄)alkyl, halo(C₁-C₃)alkyl, (C₁-C₃)-alkoxy, halo(C₁-C₃)alkoxy or cyclopropyl;
R⁴ represents hydrogen, (C₁-C₆)alkyl, halo(C₁-C₃)-alkyl or (C₁-C₃)alkoxy(C₁-C₃)alkyl, or
represents (C₃-C₆)cycloalkyl, (C₃-C₆)-cycloalkyl(C₁-C₃)alkyl, phenyl, phenyl(C₁-C₃)-alkyl, pyridyl or pyridyl(C₁-C₃)alkyl optionally substituted by one or two identical or different substituents halogen, cyano, nitro, hydroxy, amino, (C₁-C₄)alkyl, halo(C₁-C₃)alkyl, (C₁-C₃)alkoxy, halo(C₁-C₃)-alkoxy or cyclopropyl;
or R³ and R⁴ form together with the nitrogen atom to which they are bonded a saturated to triunsaturated 3- to 6-membered ring selected from aziridinyl, azirenyl, diaziridinyl, diazirenyl, azetidinyl, dihydroazetyl, diazetidinyl, dihydrodiazetyl, oxazetidinyl, oxazetyl, thiazetidinyl, thiazetyl, pyrrolidinyl, dihydropyrrolyl, pyrazolidinyl, dihydropyrazolyl, imidazolidinyl, dihydroimidazolyl, oxazolidinyl, dihydrooxazolyl, thiazolidinyl, dihydrothyazolyl, piperidinyl, piperazinyl, hexahydropyridazinyl, hexahydropyrimidinyl, morpholine, dioxazinanyl, thiomorpholine, dithiazinane, dioxothiazinane, pyrrolyl, pyrazolyl, imidazolyl, triazolyl and tetrazolyl which is optionally substituted by halogen, cyano, (C₁-C₄)alkyl, halo(C₁-C₄)alkyl, (C₁-C₄)alkoxy, halo(C₁-C₄)-alkoxy, (C₃-C₆)cycloalkyl;
m represents the number 0, 1 or 2.

3. Compound according to Claim 1 or 2, wherein the structural elements are defined as follows:
W represents hydrogen or fluorine;
n represents the number 0 or 1;
Y represents fluorine, chlorine, bromine, methyl, trifluoromethyl or methoxy;
X represents hydrogen, chlorine, fluorine or methyl;
in particular wherein X and Y represent the following combinations (Y,X): (Me,F), (Me,H), (Me,Cl), (Me,Me), (Cl,Cl), (Cl,F), (MeO,F), (MeO,H), (Cl,H), (Br,H), (Br,F), (F,F), (CF₃,H), (CF₃,F), particularly preferably (Me,F), (Me,Cl), (Me,Me), (Cl,Cl);
V¹ and V² independently of one another represent oxygen or sulfur;
R¹ and R² independently of one another represent hydrogen, methyl or ethyl; or R¹ and R² form a cyclopropyl or cyclobutyl ring;
R³ represents methyl, ethyl, n-propyl, isopropyl, n-butyl, isobutyl, sec-butyl, tert-butyl, 1,1-dimethylpropyl, 2,2,2-trifluoroethyl, 2,2-difluoroethyl, 2,2-difluoro-n-propyl, 2-methoxyethyl, 2-ethoxyethyl, 3-methoxypropyl, 3-ethoxypropyl, dimethylaminocarbonylmethyl, diethylaminocarbonylmethyl, *N*-ethyl-*N*-methylaminocarbonylmethyl, *N*-isopropyl-*N*-methylaminocarbonylmethyl, dimethylaminocarbonylethyl, diethylaminocarbonylethyl, *N*-ethyl-*N*-methylaminocarbonylethyl, *N-*isopropyl-*N*-methylaminocarbonylethyl, *N-*cyclopropyl-*N*-methylaminocarbonylmethyl or *N-*cyclopropyl-*N*-methylaminocarbonylethyl, or
represents cyclopropyl, cyclobutyl, cyclopentyl, cyclohexyl, 2-tetrahydrofurylmethyl, 3-tetrahydrofurylmethyl, 2-tetrahydrofurylethyl or 3-tetrahydrofurylethyl, or
represents phenyl optionally substituted by one or two identical or different substituents fluorine, chlorine, bromine, cyano, nitro, hydroxy, amino, methyl, ethyl, isopropyl, tert-butyl, cyclopropyl, trifluoromethyl, difluoromethyl, methoxy, trifluoromethoxy or difluoromethoxy, or
represents pyridyl optionally substituted by one or two identical or different substituents fluorine, chlorine, bromine, cyano, nitro, hydroxy, amino, methyl, ethyl, isopropyl, tert-butyl, cyclopropyl, trifluoromethyl, difluoromethyl, methoxy, trifluoromethoxy or difluoromethoxy, or
represents benzyl or pyridylmethyl optionally substituted by one or two identical or different substituents fluorine, chlorine, bromine, cyano, nitro, hydroxy, amino, methyl, ethyl, isopropyl, tert-butyl, trifluoromethyl, difluoromethyl, methoxy, trifluoromethoxy or difluoromethoxy;
R⁴ represents hydrogen, methyl, ethyl, n-propyl, isopropyl, n-butyl, isobutyl, tert-butyl, sec-butyl, 2,2,2-trifluoroethyl, 2,2-difluoroethyl, 2-methoxyethyl, 2-ethoxyethyl, cyclopropyl, cyclobutyl, cyclopropylmethyl, phenyl or benzyl;
or R³ and R⁴ form form together with the nitrogen atom to which they are bonded an azetidine, oxetane, thiethane, morpholine, thiomorpholine, dioxothiazinane, piperidine or an *N*-methyl-substituted piperazine ring.

4. Compound according to one of Claims 1 to 3, wherein the structural elements are defined as follows:
W represents fluorine;
n represents the number 0 or 1;
Y represents chlorine or methyl;
X represents hydrogen, fluorine, chlorine or methyl;
in particular wherein X and Y represent the following combinations (Y,X): (Me,Cl), (Me,F), (Me,Me), (Cl,Cl);
V¹ and V² independently of one another represent oxygen or sulfur;
R¹ and R² independently of one another represent hydrogen or methyl;
R³ represents methyl, ethyl, isopropyl, isobutyl, sec-butyl, tert-butyl, 1,1-dimethylpropyl, 2,2,2-trifluoroethyl, 2-methoxyethyl, or
represents cyclopropyl or 2-tetrahydrofurylmethyl, or
represents phenyl, 3-fluorophenyl, 4-fluorophenyl, 3-chlorophenyl, 3-trifluoromethylphenyl, 2-pyridyl, 3-pyridyl or benzyl;
R⁴ represents hydrogen or methyl;
or R³ and R⁴ form form together one of the following rings: 1-morpholine, 1-(4-methylpiperazine), 1-(1,1-dioxo-1,4-thiazinane) or 1-(4,4-difluoropiperidine).

5. Compound according to one of Claims 1 to 4, **characterized in that** it comprises a structure according to formula (I-A) 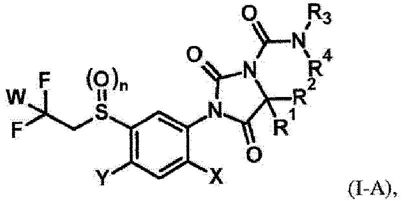 in which W, n, m, Y, X, R¹, R², R³ and R⁴ have the meanings defined in Claim 1 or the meanings defined in Claim 2 or the meanings defined in Claim 3 or the meanings defined in Claim 4.

6. Compound of formula (II) 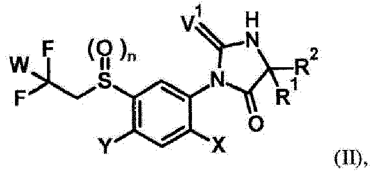 wherein n, W, Y, X, V¹, R¹ and R² have the meanings defined in Claim 1 or the meanings defined in Claim 2 or the meanings defined in Claim 3 or the meanings defined in Claim 4.

7. Compound of formula (III) 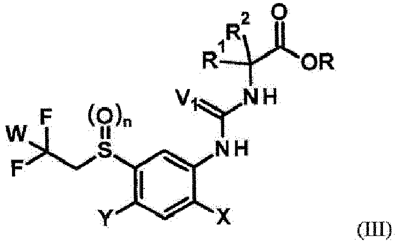 wherein n, W, Y, X, V¹, R¹ and R² have the meanings defined in Claim 1 or the meanings defined in Claim 2 or the meanings defined in Claim 3 or the meanings defined in Claim 4, and R represents hydrogen or an alkyl group, preferably methyl or ethyl.

8. Compound of formula (VI) 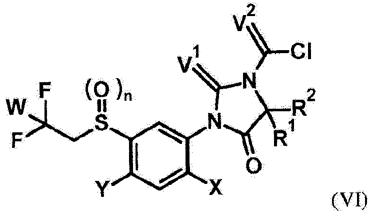 wherein n, W, Y, X, V¹, V², R¹ and R² have the meanings defined in Claim 1 or the meanings defined in Claim 2 or the meanings defined in Claim 3 or the meanings defined in Claim 4.

9. Formulation, in particular agrochemical formulation, comprising at least one compound of formula (I) according to one of Claims 1 to 5.

10. Formulation according to Claim 9, further comprising at least one extender and/or at least one surface-active substance.

11. Formulation according to Claim 9 or 10, **characterized in that** the compound of formula (I) is present in admixture with at least one further active ingredient.

12. Method for controlling pests, in particular animal pests, **characterized in that** a compound of formula (I) according to one of Claims 1 to 5 or a formulation according to one of Claims 9 to 11 is allowed to act on the pests and/or their habitat.

13. Method according to Claim 12, **characterized in that** the pest is an animal pest and comprises an insect, an acarid or a nematode, or **in that** the pest is an insect, an acarid or a nematode.

14. Compound of formula (I) according to one of Claims 1 to 5 or of a formulation according to one of Claims 9 to 11 for the preparation of an active ingredient or medicament for controlling animal pests.

15. Compound for the preparation of an active ingredient or medicament according to Claim 14, **characterized in that** the animal pest comprises an insect, an acarid or a nematode, or **in that** the animal pest is an insect, an acarid or a nematode.

16. Compound according to Claim 14 or 15 for use in crop protection.

17. Compound of formula (I) according to one of Claims 1 to 5 for the preparation of a medicament for use in the field of animal health.

18. Method for protecting seed or a germinating plant from pests, in particular animal pests, comprising a method step in which the seed is brought into contact with a compound of formula (I) according to one of Claims 1 to 5 or with a formulation according to one of Claims 9 to 11.

## Revendications

1. Composé de formule (I) 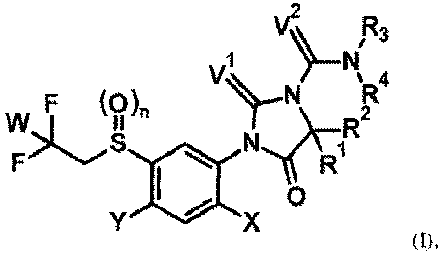 dans laquelle
W représente hydrogène ou halogène ;
n représente le nombre 0, 1 ou 2 ;
Y représente hydrogène, halogène, alkyle en (C₁-C₆), halogéno-alkyle en (C₁-C₆), alcoxy en (C₁-C₆), halogéno-alcoxy en (C₁-C₆) ou amino ; ou
représente NR'''R'''',
R''' et R'''' représentant indépendamment l'un de l'autre hydrogène, alkyle en (C₁-C₆) ou halogéno-alkyle en (C₂-C₆) ;
X représente hydrogène, halogène, cyano, alkyle en (C₁-C₆), halogéno-alkyle en (C₁-C₆) ou alcoxy en (C₁-C₆) ;
V¹ et V² représentent indépendamment l'un de l'autre oxygène ou soufre ;
R¹ et R² indépendamment l'un de l'autre
représentent hydrogène, halogène, hydroxy, cyano ou nitro ; ou
représentent alkyle, alcényle, alcynyle, alcoxy, alkylsulfanyle, alkylsulfinyle, alkylsulfonyle, halogénoalkylcarbonyle, alkylcarbonyle ou alcoxycarbonyle, les radicaux susmentionnés pouvant éventuellement être substitués par halogène, alkyle, cycloalkyle, cyano, nitro, alcoxy, halogénoalkyle ou halogénoalcoxy, notamment fluor, chlore, alkyle en (C₁-C₃), cycloalkyle en (C₃-C₆), cyclopropyle, cyano, alcoxy en (C₁-C₃), halogénoalkyle en (C₁-C₃) ou halogénoalcoxy en (C₁-C₃) ;
ou R¹ et R² forment un cycle saturé ou insaturé de trois à six chaînons éventuellement substitué par halogène, alkyle, cycloalkyle, cyano, nitro, alcoxy, halogénoalkyle ou halogénoalcoxy, notamment fluor, chlore, alkyle en (C₁-C₃), cycloalkyle en (C₃-C₆), cyclopropyle, cyano, alcoxy en (C₁-C₃), halogénoalkyle en (C₁-C₃) ou halogénoalcoxy en (C₁-C₃), et éventuellement interrompu par un ou plusieurs hétéroatomes, qui sont choisis indépendamment dans le groupe constitué par O, S et N, à condition que deux atomes d'oxygène ne soient pas directement voisins ;
R³ représente alkyle, halogénoalkyle, alcoxyalkyle, halogénoalcoxyalkyle, alcényle, halogénoalcényle, alcynyle, halogénoalcynyle, alkyl-S(O)ₘ-alkyle, halogénoalkyl-S(O)ₘ-alkyle, *N*-alkylaminocarbonylalkyle ou *N,N*-dialkylaminocarbonylalkyle, ou
représente cycloalkyle, cycloalkylalkyle, cycloalcényle ou cycloalcénylalkyle éventuellement substitué une fois ou deux fois, de manière identique ou différente, par halogène, cyano, nitro, hydroxy, amino, alkyle en (C₁-C₄), halogéno-alkyle en (C₁-C₃), alcoxy en (C₁-C₃), halogéno-alcoxy en (C₁-C₃) ou cyclopropyle, ou
représente hétérocyclyle, hétérocyclylalkyle, aryle, arylalkyle, hétaryle ou hétarylalkyle éventuellement substitué une fois ou deux fois, de manière identique ou différente, par halogène, cyano, nitro, hydroxy, amino, alkyle en (C₁-C₄), halogéno-alkyle en (C₁-C₃), alcoxy en (C₁-C₃), halogéno-alcoxy en (C₁-C₃) ou cyclopropyle ;
R⁴ représente hydrogène, alkyle, halogénoalkyle, alcoxyalkyle, halogénoalcoxyalkyle, alcényle, halogénoalcényle, alcynyle, halogénoalcynyle, alkyle-S(O)ₘ-alkyle, halogénoalkyl-S(O)ₘ-alkyle, *N-*alkylaminocarbonylalkyle ou *N,N-*dialkylaminocarbonylalkyle, ou
représente cycloalkyle, cycloalkylalkyle, cycloalcényle ou cycloalcénylalkyle éventuellement substitué une fois ou deux fois, de manière identique ou différente, par halogène, cyano, nitro, hydroxy, amino, alkyle en (C₁-C₄), halogéno-alkyle en (C₁-C₃), alcoxy en (C₁-C₃), halogéno-alcoxy en (C₁-C₃) ou cyclopropyle, ou
représente hétérocyclyle, hétérocyclylalkyle, aryle, arylalkyle, hétaryle ou hétarylalkyle éventuellement substitué une fois ou deux fois, de manière identique ou différente, par halogène, cyano, nitro, hydroxy, amino, alkyle en (C₁-C₄), halogéno-alkyle en (C₁-C₃), alcoxy en (C₁-C₃), halogéno-alcoxy en (C₁-C₃) ou cyclopropyle ;
ou R³ et R⁴ forment ensemble avec l'atome d'azote auquel ils sont reliés un cycle de 3 à 6 chaînons saturé à trois fois insaturé, éventuellement substitué par halogène, cyano, alkyle en (C₁-C₄), halogéno-alkyle en (C₁-C₄), alcoxy en (C₁-C₄), halogéno-alcoxy en (C₁-C₄) ou cycloalkyle en (C₃-C₆) ;
m représente le nombre 0, 1 ou 2.

2. Composé selon la revendication 1, dans lequel les éléments structuraux sont définis de la manière suivante :
W représente hydrogène ou halogène ;
n représente le nombre 0 ou 1 ;
Y représente hydrogène, halogène, alkyle en (C₁-C₃), halogéno-alkyle en (C₁-C₃), alcoxy en (C₁-C₃), halogéno-alcoxy en (C₁-C₃) ou amino ; ou
représente NR'''R'''',
R''' et R'''' représentant indépendamment l'un de l'autre hydrogène, alkyle en (C₁-C₄) ou halogéno-alkyle en (C₂-C₄) ;
X représente hydrogène, halogène, cyano, alkyle en (C₁-C₃), halogéno-alkyle en (C₁-C₃) ou alcoxy en (C₁-C3) ;
V¹ et V² représentent indépendamment l'un de l'autre oxygène ou soufre ;
R¹ et R² représentent indépendamment l'un de l'autre hydrogène ou alkyle en (C₁-C₃) ;
ou R¹ et R² forment avec l'atome de carbone auquel ils sont reliés un cycle cycloalkyle en (C₃-C₆) ;
R³ représente alkyle en (C₁-C₆), halogéno-alkyle en (C₁-C₃), alcoxy en (C₁-C₃)-alkyle en (C₁-C₃), halogéno-alcoxy en (C₁-C₃)-alkyle en (C₁-C₃), alcényle en (C₁-C₆), halogéno-alcényle en (C₁-C₃), alcynyle en (C₁-C₆), halogéno-alcynyle en (C₁-C₃), alkyle en (C₁-C₃)-S(O)ₘ-alkyle en (C₁-C₃), halogéno-alkyle en (C₁-C₃)-S(O)ₘ-alkyle en (C₁-C₃), *N*-alkyle en (C₁-C₃)-aminocarbonylalkyle en (C₁-C₃) ou *N,N*-dialkyle en (C₁-C₃)-aminocarbonylalkyle en (C₁-C₃), ou
représente cycloalkyle en (C₃-C₆), cycloalkyle en (C₃-C₆)-alkyle en (C₁-C₃), cycloalcényle en (C₃-C₆) ou cycloalcényle en (C₃-C₆)-alkyle en (C₁-C₃) éventuellement substitué une fois ou deux fois, de manière identique ou différente, par halogène, cyano, nitro, hydroxy, amino, alkyle en (C₁-C₄), halogéno-alkyle en (C₁-C₃), alcoxy en (C₁-C₃), halogéno-alcoxy en (C₁-C₃) ou cyclopropyle, ou
représente hétérocyclyle, hétérocyclyl-alkyle en (C₁-C₃), aryle, aryl-alkyle en (C₁-C₃), hétaryle ou hétaryl-alkyle en (C₁-C₃) éventuellement substitué une fois ou deux fois, de manière identique ou différente, par halogène, cyano, nitro, hydroxy, amino, alkyle en (C₁-C₄), halogéno-alkyle en (C₁-C₃), alcoxy en (C₁-C₃), halogéno-alcoxy en (C₁-C₃) ou cyclopropyle ;
R⁴ représente hydrogène, alkyle en (C₁-C₆), halogéno-alkyle en (C₁-C₃) ou alcoxy en (C₁-C₃)-alkyle en (C₁-C₃), ou
représente cycloalkyle en (C₃-C₆), cycloalkyle en (C₃-C₆)-alkyle en (C₁-C₃), phényle, phényl-alkyle en (C₁-C₃), pyridyle ou pyridyl-alkyle en (C₁-C₃) éventuellement substitué une fois ou deux fois, de manière identique ou différente, par halogène, cyano, nitro, hydroxy, amino, alkyle en (C₁-C₄), halogéno-alkyle en (C₁-C₃), alcoxy en (C₁-C₃), halogéno-alcoxy en (C₁-C₃) ou cyclopropyle ;
ou R³ et R⁴ forment ensemble avec l'atome d'azote auquel ils sont reliés un cycle de 3 à 6 chaînons saturé à trois fois insaturé, éventuellement substitué par halogène, cyano, alkyle en (C₁-C₄), halogéno-alkyle en (C₁-C₄), alcoxy en (C₁-C₄), halogéno-alcoxy en (C₁-C₄), cycloalkyle en (C₃-C₆) ; qui est choisi parmi aziridinyle, azirényle, diaziridinyle, diazirényle, azétidinyle, dihydroazétyle, diazétidinyle, dihydrodiazétyle, oxazétidinyle, oxazétyle, thiazétidinyle, thiazétyle, pyrrolidinyle, dihydropyrrolyle, pyrazolidinyle, dihydropyrazolyle, imidazolidinyle, dihydroimidazolyle, oxazolidinyle, dihydrooxazolyle, thiazolidinyle, dihydrothyazolyle, pipéridinyle, pipérazinyle, hexahydropyridazinyle, hexahydropyrimidinyle, morpholine, dioxazinanyle, thiomorpholine, dithiazinane, dioxothiazinane, pyrrolyle, pyrazolyle, imidazolyle, triazolyle et tétrazolyle,
m représente le nombre 0, 1 ou 2.

3. Composé selon la revendication 1 ou 2, dans lequel les éléments structuraux sont définis de la manière suivante :
W représente hydrogène ou fluor ;
n représente le nombre 0 ou 1 ;
Y représente fluor, chlore, brome, méthyle, trifluorométhyle ou méthoxy ;
X représente hydrogène, chlore, fluor ou méthyle ;
X et Y représentant notamment les combinaisons (Y, X) suivantes : (Me, F), (Me, H), (Me, Cl), (Me, Me), (Cl, Cl), (Cl, F), (MeO, F), (MeO, H), (Cl, H), (Br, H), (Br, F), (F, F), (CF₃, H), (CF₃, F), de manière particulièrement préférée (Me, F), (Me, Cl), (Me, Me), (Cl, Cl) ;
V¹ et V² représentent indépendamment l'un de l'autre oxygène ou soufre ;
R¹ et R² représentent indépendamment l'un de l'autre hydrogène, méthyle ou éthyle ;
ou R¹ et R² forment un cycle cyclopropyle ou cyclobutyle ;
R³ représente méthyle, éthyle, n-propyle, isopropyle, n-butyle, isobutyle, sec-butyle, tert-butyle, 1,1-diméthylpropyle, 2,2,2-trifluoroéthyle, 2,2-difluoroéthyle, 2,2-difluoro-n-propyle, 2-méthoxyéthyle, 2-éthoxyéthyle, 3-méthoxypropyle, 3-éthoxypropyle, diméthylaminocarbonylméthyle, diéthylaminocarbonylméthyle, *N*-éthyl-*N-*méthylaminocarbonylméthyle, *N*-isopropyl-*N-*méthylaminocarbonylméthyle, diméthylaminocarbonyléthyle, diéthylaminocarbonyléthyle, *N*-éthyl-*N-*méthylaminocarbonyléthyle, *N*-isopropyl-*N-*méthylaminocarbonyléthyle, *N*-cyclopropyl-*N-*méthylaminocarbonylméthyle ou *N*-cyclopropyl-*N-*méthylaminocarbonyléthyle, ou
représente cyclopropyle, cyclobutyle, cyclopentyle, cyclohexyle, 2-tétrahydrofurylméthyle, 3-tétrahydrofurylméthyle, 2-tétrahydrofuryléthyle ou 3-tétrahydrofuryléthyle, ou
représente phényle éventuellement substitué une fois ou deux fois, de manière identique ou différente, par fluor, chlore, brome, cyano, nitro, hydroxy, amino, méthyle, éthyle, isopropyle, tert-butyle, cyclopropyle, trifluorométhyle, difluorométhyle, méthoxy, trifluorométhoxy ou difluorométhoxy, ou
représente pyridyle éventuellement substitué une fois ou deux fois, de manière identique ou différente, par fluor, chlore, brome, cyano, nitro, hydroxy, amino, méthyle, éthyle, isopropyle, tert-butyle, cyclopropyle, trifluorométhyle, difluorométhyle, méthoxy, trifluorométhoxy ou difluorométhoxy, ou
représente benzyle ou pyridylméthyle éventuellement substitué une fois ou deux fois, de manière identique ou différente, par fluor, chlore, brome, cyano, nitro, hydroxy, amino, méthyle, éthyle, isopropyle, tert-butyle, trifluorométhyle, difluorométhyle, méthoxy, trifluorométhoxy ou difluorométhoxy ;
R⁴ représente hydrogène, méthyle, éthyle, n-propyle, isopropyle, n-butyle, isobutyle, tert-butyle, sec-butyle, 2,2,2-trifluoroéthyle, 2,2-difluoroéthyle, 2-méthoxyéthyle, 2-éthoxyéthyle, cyclopropyle, cyclobutyle, cyclopropylméthyle, phényle ou benzyle ;
ou R³ et R⁴ forment forment ensemble avec l'atome d'azote auquel ils sont reliés un cycle azétidine, oxétane, thiéthane, morpholine, thiomorpholine, dioxothiazinane, pipéridine ou un cycle pipérazine à substitution *N*-méthyle.

4. Composé selon l'une quelconque des revendications 1 à 3, dans lequel les éléments structuraux sont définis de la manière suivante :
W représente fluor ;
n représente le nombre 0 ou 1 ;
Y représente chlore ou méthyle ;
X représente hydrogène, fluor, chlore ou méthyle ;
X et Y représentant notamment les combinaisons (Y, X) suivantes : (Me, Cl), (Me, F), (Me, Me), (Cl, Cl) ;
V¹ et V² représentent indépendamment l'un de l'autre oxygène ou soufre ;
R¹ et R² représentent indépendamment l'un de l'autre hydrogène ou méthyle ;
R³ représente méthyle, éthyle, isopropyle, isobutyle, sec-butyle, tert-butyle, 1,1-diméthylpropyle, 2,2,2-trifluoroéthyle, 2-méthoxyéthyle, ou
représente cyclopropyle ou 2-tétrahydrofurylméthyle, ou
représente phényle, 3-fluorophényle, 4-fluorophényle, 3-chlorophényle, 3-trifluorométhylphényle, 2-pyridyle, 3-pyridyle ou benzyle ;
R⁴ représente hydrogène ou méthyle ;
ou R³ et R⁴ forment forment ensemble un des cycles suivants : 1-morpholine, 1-(4-méthylpipérazine), 1-(1,1-dioxo-1,4-thiazinane) ou 1-(4,4-difluoropipéridine).

5. Composé selon l'une quelconque des revendications 1 à 4, **caractérisé en ce qu'**il présente une structure selon la formule (I-A) 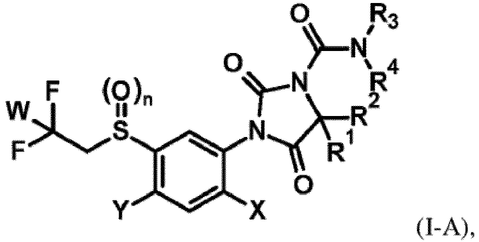 dans laquelle W, n, m, Y, X, R¹, R², R³ et R⁴ ont les significations indiquées dans la revendication 1 ou les significations indiquées dans la revendication 2 ou les significations indiquées dans la revendication 3 ou les significations indiquées dans la revendication 4.

6. Composé de formule (II) 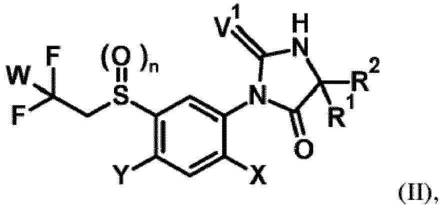 dans laquelle n, W, Y, X, V¹, R¹ et R² ont les significations indiquées dans la revendication 1 ou les significations indiquées dans la revendication 2 ou les significations indiquées dans la revendication 3 ou les significations indiquées dans la revendication 4.

7. Composé de formule (III) 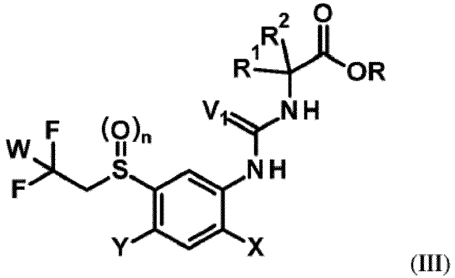 dans laquelle n, W, Y, X, V¹, R¹ et R² ont les significations indiquées dans la revendication 1 ou les significations indiquées dans la revendication 2 ou les significations indiquées dans la revendication 3 ou les significations indiquées dans la revendication 4, et R représente l'hydrogène ou un groupe alkyle, de préférence méthyle ou éthyle.

8. Composé de formule (VI) 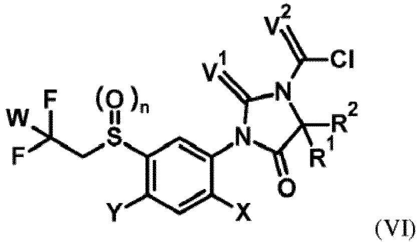 dans laquelle n, W, Y, X, V¹, V², R¹ et R² ont les significations indiquées dans la revendication 1 ou les significations indiquées dans la revendication 2 ou les significations indiquées dans la revendication 3 ou les significations indiquées dans la revendication 4.

9. Formulation, notamment formulation agrochimique, comprenant au moins un composé de formule (I) selon l'une quelconque des revendications 1 à 5.

10. Formulation selon la revendication 9, comprenant en outre au moins un extendeur et/ou au moins une substance tensioactive.

11. Formulation selon la revendication 9 ou 10, **caractérisée en ce que** le composé de formule (I) se présente en mélange avec au moins un autre agent actif.

12. Procédé de lutte contre des nuisibles, notamment des animaux nuisibles, **caractérisé en ce qu'**un composé de formule (I) selon l'une quelconque des revendications 1 à 5 ou une formulation selon l'une quelconque des revendications 9 à 11 est laissé agir sur les nuisibles et/ou leur habitat.

13. Procédé selon la revendication 12, **caractérisé en ce que** le nuisible est un animal nuisible et comprend un insecte, un acarien ou un nématode, ou **en ce que** le nuisible est un insecte, un acarien ou un nématode.

14. Composé de formule (I) selon l'une quelconque des revendications 1 à 5 ou d'une formulation selon l'une quelconque des revendications 9 à 11 pour la fabrication d'un agent actif ou d'un médicament pour lutter contre des animaux nuisibles.

15. Composé pour la fabrication d'un agent actif ou d'un médicament selon la revendication 14, **caractérisé en ce que** l'animal nuisible comprend un insecte, un acarien ou un nématode, ou **en ce que** l'animal nuisible est un insecte, un acarien ou un nématode.

16. Composé selon la revendication 14 ou 15 destiné à une utilisation dans la protection des plantes.

17. Composé de formule (I) selon l'une quelconque des revendications 1 à 5 pour la fabrication d'un médicament destiné à une utilisation dans le domaine de la santé animale.

18. Procédé de protection d'une graine ou d'une plante germée contre des nuisibles, notamment des animaux nuisibles, comprenant une étape de procédé lors de laquelle la graine est mise en contact avec un composé de formule (I) selon l'une quelconque des revendications 1 à 5 ou une formulation selon l'une quelconque des revendications 9 à 11.
